(19) Europäisches Patentamt
European Patent Office
Office européen des brevets

(11) EP 3 243 837 A1

(12) **EUROPEAN PATENT APPLICATION**
published in accordance with Art. 153(4) EPC

(43) Date of publication:
**15.11.2017 Bulletin 2017/46**

(21) Application number: **16735083.4**

(22) Date of filing: **08.01.2016**

(51) Int Cl.:
*C07K 16/28* (2006.01) *A61K 39/395* (2006.01)
*A61P 35/00* (2006.01) *C12N 5/10* (2006.01)
*C12N 15/09* (2006.01) *C12P 21/08* (2006.01)

(86) International application number:
**PCT/JP2016/050434**

(87) International publication number:
**WO 2016/111344 (14.07.2016 Gazette 2016/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.01.2015 US 201562101042 P**

(71) Applicant: **Kyowa Hakko Kirin Co., Ltd.**
**Tokyo 100-8185 (JP)**

(72) Inventors:
- **TAKAHASHI Nobuaki**
  **Tokyo 100-8185 (JP)**
- **NAKAYAMA Makoto**
  **Tokyo 100-8185 (JP)**
- **TAKAGI Sayaka**
  **Tokyo 100-8185 (JP)**

(74) Representative: **Hoffmann Eitle**
**Patent- und Rechtsanwälte PartmbB**
**Arabellastraße 30**
**81925 München (DE)**

(54) **BISPECIFIC ANTIBODY BINDING TO TRAILR2 AND PSMA**

(57) The present invention relates to: a bispecific antibody including an antigen-binding domain that binds to TRAILR2 and an antigen-binding domain that binds to PSMA, or a fragment of said antibody; a nucleic acid including a base sequence that encodes said antibody or said antibody fragment; a recombinant vector including said nucleic acid; a transformant strain including said recombinant vector; a method for producing said bispecific antibody or said antibody fragment by using said transformant strain; and a detection/measurement reagent, a diagnostic drug, and a therapeutic drug including said bispecific antibody or said antibody fragment.

[Fig. 1]

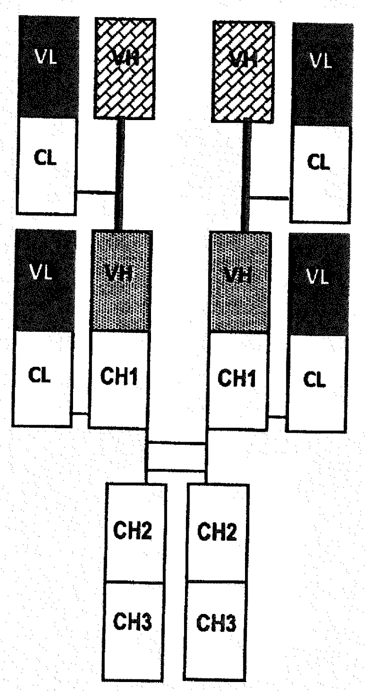


EP 3 243 837 A1

SEQUENCE LISTING

<110> Kyowa Hakko Kirin Co., Ltd.

<120> Bispecific antibody binding to TRAILR2 and PSMA

<130> W519750

<150> US62/101,042
<151> 2015-01-08

<160> 158

<170> PatentIn version 3.3

<210> 1
<211> 435
<212> DNA
<213> Artificial

<220>
<223> Description of the artificial sequence; nucleotide sequence of KMDA2_VH

<400> 1
atggactgga cctggaggat cctcttcttg gtggcagcag ctacaagtgc ccactcccag 60

gtgcagctgg tgcagtctgg ggctgaggtg aagaagcctg gggcctcagt gaaggtctcc 120

tgcaaggctt ctggatacac cttcaccagt tataaaatta actgggtgcg acaggccact 180

ggacaagggc ttgagtggat gggatggatg aaccctaaca gtggtaacac aggctatgca 240

cagaagttcc agggcagagt caccatgacc aggaacacct ccataagcac agcctacatg 300

gagctgagca gcctgagatc tgaggacacg gccgtgtatt actgtgcgag atcctatggt 360

tcggggagtt attatagaga ctattactac ggtatggacg tctggggcca agggaccacg 420

gtcaccgtct cctca 435

<210> 2
<211> 381
<212> DNA
<213> Artificial

<220>
<223> Description of the artificial sequence; nucleotide sequence of KMDA2_VL

<400> 2
atggaagccc cagctcagct tctcttcctc ctgctactct ggctcccaga taccaccgga 60

gaaattgtgt tgacacagtc tccagccacc ctgtctttgt ctccagggga aagagccacc 120

ctctcctgca gggccagtca gagtgttagc agctacttag cctggtacca acagaaacct 180

ggccaggctc ccaggctcct catctatgat gcatccaaca gggccactgg catcccagcc 240

aggttcagtg gcagtgggtc tgggacagac ttcactctca ccatcagcag cctagagcct 300

gaagattttg cagtttatta ctgtcagcag cgtagcaact ggccgctcac tttcggcgga 360

gggaccaagg tggagatcaa a 381

<210> 3
<211> 41
<212> DNA
<213> Artificial

<220>
<223> Description of the artificial sequence; primer1

<400> 3
atcacagatc tctcaccatg gaagccccag ctcagcttct c 41

<210> 4
<211> 33
<212> DNA
<213> Artificial

<220>
<223> Description of the artificial sequence; primer2

<400> 4
ggtgcagcca ccgtacgttt gatctccacc ttg 33

<210> 5
<211> 38
<212> DNA
<213> Artificial

<220>
<223> Description of the artificial sequence; primer3

<400> 5
gcgactaagt cgacaccatg gactggacct ggaggatc 38

<210> 6
<211> 32
<212> DNA
<213> Artificial

<220>
<223> Description of the artificial sequence; primer4

<400> 6
agagagagag gctagctgag gagacggtga cc 32

<210> 7
<211> 145
<212> PRT
<213> Artificial

<220>
<223> Description of the artificial sequence; amino acid sequence of KMDA2_VH

<400> 7

Met Asp Trp Thr Trp Arg Ile Leu Phe Leu Val Ala Ala Ala Thr Ser
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Lys Ile Asn Trp Val Arg Gln Ala Thr Gly Gln Gly Leu
    50                  55                  60

Glu Trp Met Gly Trp Met Asn Pro Asn Ser Gly Asn Thr Gly Tyr Ala
65                  70                  75                  80

Gln Lys Phe Gln Gly Arg Val Thr Met Thr Arg Asn Thr Ser Ile Ser
                85                  90                  95

Thr Ala Tyr Met Glu Leu Ser Ser Leu Arg Ser Glu Asp Thr Ala Val
            100                 105                 110

Tyr Tyr Cys Ala Arg Ser Tyr Gly Ser Gly Ser Tyr Tyr Arg Asp Tyr
        115                 120                 125

Tyr Tyr Gly Met Asp Val Trp Gly Gln Gly Thr Thr Val Thr Val Ser
    130                 135                 140

Ser
145

<210> 8
<211> 127
<212> PRT
<213> Artificial

<220>
<223> Description of the artificial sequence; amino acid sequence of KMDA2_VL

<400> 8

Met Glu Ala Pro Ala Gln Leu Leu Phe Leu Leu Leu Leu Trp Leu Pro
1               5                   10                  15

Asp Thr Thr Gly Glu Ile Val Leu Thr Gln Ser Pro Ala Thr Leu Ser
            20                  25                  30

Leu Ser Pro Gly Glu Arg Ala Thr Leu Ser Cys Arg Ala Ser Gln Ser
        35                  40                  45

```
Val Ser Ser Tyr Leu Ala Trp Tyr Gln Gln Lys Pro Gly Gln Ala Pro
    50                  55                  60


Arg Leu Leu Ile Tyr Asp Ala Ser Asn Arg Ala Thr Gly Ile Pro Ala
65                  70                  75                  80


Arg Phe Ser Gly Ser Gly Ser Gly Thr Asp Phe Thr Leu Thr Ile Ser
                85                  90                  95


Ser Leu Glu Pro Glu Asp Phe Ala Val Tyr Tyr Cys Gln Gln Arg Ser
            100                 105                 110


Asn Trp Pro Leu Thr Phe Gly Gly Gly Thr Lys Val Glu Ile Lys
        115                 120                 125


<210>  9
<211>  432
<212>  DNA
<213>  Artificial

<220>
<223>  Description of the artificial sequence; nucleotide sequence of
       E11_VH

<400>  9
atggatctca tgtgcaagaa aatgaagcac ctgtggttct tcctcctgct ggtggcggct     60

cccagatggg tcctgtccca gctgcagctg caggagtcgg gcccaggact ggtgaagcct    120

tcggagaccc tgtccctcac ctgcactgtc tctggtggct ccatcatcag taaaagttcc    180

tactggggct ggatccgcca gcccccaggg aaggggctgg agtggattgg gagtatctat    240

tatagtggga gtaccttcta caacccgtcc ctcaagagtc gagtcaccat atccgtagac    300

acgtccaaga accagttctc cctgaagctg agctctgtga ccgccgcaga cacggctgtg    360

tattactgtg cgagactgac agtggctgag tttgactact ggggccaggg aaccctggtc    420

accgtctcct ca                                                        432


<210>  10
<211>  384
<212>  DNA
<213>  Artificial

<220>
<223>  Description of the artificial sequence; nucleotide sequence of
       E11_VL

<400>  10
atggaagccc cagctcagct tctcttcctc ctgctactct ggctcccaga taccaccgga     60

gaaattgtgt tgacacagtc tccagccacc ctgtctttgt ctccagggga aagagccacc    120

ctctcctgca gggccagtca gagtgttagc agcttcttag cctggtacca acagaaacct    180
```

ggccaggctc ccaggctcct catctatgat gcatccaaca gggccactgg catcccagcc 240

aggttcagtg gcagtgggtc tgggacagac ttcactctca ccatcagcag cctagagcct 300

gaagattttg cagtttatta ctgtcagcag cgtagcaact ggcctctcac tttcggccct 360

gggaccaaag tggatatcaa acgt 384

<210> 11
<211> 14
<212> PRT
<213> Artificial

<220>
<223> Description of the artificial sequence; amino acid sequence of GL linker

<400> 11

Ala Ser Thr Lys Gly Pro Ser Val Phe Pro Leu Ala Pro Cys
1 5 10

<210> 12
<211> 14
<212> PRT
<213> Artificial

<220>
<223> Description of the artificial sequence; amino acid sequence of ML linker

<400> 12

Gly Ser Ala Ser Ala Pro Thr Leu Phe Pro Leu Val Ser Cys
1 5 10

<210> 13
<211> 414
<212> DNA
<213> Artificial

<220>
<223> Description of the artificial sequence; nucleotide sequence of PI134_VH

<400> 13
atggactgga cctggaggat cctcttcttg gtggcagcag ccacaggtgc ccactcccag 60

gtgcagctgg tgcagtctgg ggctgaggtg aagaagcctg gggcctcagt gaaggtctcc 120

tgcaaggctt ctggatacac cttcaccact tatgatatca actgggtgcg acaggccact 180

ggacaagggc ttgagtggat gggatggatg aaccctaaca atggttacac aggctatgca 240

cagaagttcc agggcagagt caccatgacc aggaacacct ccataagcac agcctacatg 300

gagctgagca gcctgagatc tgaggacacg gccgtgtatt actgtgcgag aactaactgg 360

gtctactggt acttcgatct ctggggccgt ggcaccctgg tcaccgtctc ctca 414

<210> 14
<211> 414
<212> DNA
<213> Artificial

<220>
<223> Description of the artificial sequence; nucleotide sequence of PI101_VH

<400> 14
atggggtcaa ccgccatcct cgccctcctc ctggctgttc tccaaggagt ctgttccgag 60

gtgcagctgg tgcagtctgg ggctgaggtg aagaagcctg gggcctcagt gaaggtctcc 120

tgcaaggctt ctggatataa tctcgccact tatgatatca actgggtgcg acaggccact 180

ggacaagggc ttgagtggat gggatggatg aaccctaaca gtggttacac aggctatgca 240

cagaagttcc agggcagagt caccatgacc agggacacct ccataaacac agcctacatg 300

gagctgagca gcctgagatc tgaggacacg gccgtgtatt actgtgcgag aactaactgg 360

gtctactggt acttcgatct ctggggccgt ggcaccctgg tcaccgtctc ctca 414

<210> 15
<211> 414
<212> DNA
<213> Artificial

<220>
<223> Description of the artificial sequence; nucleotide sequence of PN7_VH

<400> 15
atggagtttg ggctgagctg ggttttcctt gttgctattt taaaaggtgt ccagtgtgaa 60

gtgcagctgg tgcagtccgg agctgaggtg aagaagcctg gggcctcagt gaaggtctcc 120

tgcaaggctt ctggttacac ctttaccact tatgatatca actgggtgcg acaggccact 180

ggacaagggc ttgagtggat gggatggatg aaccctaaca gtggttacac aggctatgca 240

cagaagttcc agggcagagt caccatgacc agggacacct ccataagcac agcctacatg 300

gagctgagca gcctgagatc tgaggacacg gccgtgtatt actgtgcgcg gacgaactgg 360

gaatcctggt acttcgatct ctggggccgt ggaaccctgg tcaccgtctc ctca 414

<210> 16
<211> 414
<212> DNA
<213> Artificial

<220>
<223> Description of the artificial sequence; nucleotide sequence of PI105_VH

<400> 16
atggactgga cctggaggat cctcttcttg gtggcagcag ccacaggtgc ccactccgag 60

gtccagctgg tacagtctgg ggctgaggtg aagaagcctg gggcctcagt gaaggtctcc 120

tgcaaggctt ctggatacac cttcaccact tatgatatca actgggtgcg acaggccacg 180

ggacaagggc ttgagtggct gggatggatg aaccctaaca atggttacac aggctatgca 240

cagaagttcc agggcagagt caccatgacc aggaacacct ccataggcac agcctacatg 300

gagctgaaca gcctgagatc tgaggacacg gccgtgtatt actgtgcgag aactaactgg 360

gtctactggt acttcgatct ctggggccgt ggcaccctgg tcactgtctc ctca 414

<210> 17
<211> 414
<212> DNA
<213> Artificial

<220>
<223> Description of the artificial sequence; nucleotide sequence of PI108_VH

<400> 17
atggactgga cctggaggat cctcttcttg gtggcagcag ccacaggtgc ccactcccag 60

gtgcagctgg tgcagtctgg ggctgaggtg aagaagcctg gggcctcagt gaaggtctcc 120

tgcaaggctt ctggatacac cttcaccact tatgatatca actgggtgcg acaggccact 180

ggacaagggc ttgagtggat gggatggatg aaccctaaca atggttacac aggctatgca 240

cagaagttcc agggcagagt caccatgacc aggaacacct ccataagcac agcctacatg 300

gagctgacca gcctgagatc tgaggacacg gccgtgtatt actgtgcgag aactaactgg 360

gtctactggt acttcgatct ctggggccgt ggcaccctgg tcactgtctc ctca 414

<210> 18
<211> 414
<212> DNA
<213> Artificial

<220>
<223> Description of the artificial sequence; nucleotide sequence of PI115_VH

<400> 18
atggactgga cctggaggat cctcttcttg gtggcagcag ccacaggtgc ccactccgag 60

gtgcagctgg tgcagtccgg ggctgaggtg aagaagcctg gggcctcagt gaaggtctcc 120

tgcaaggctt ctggatacac cttcaccact tatgatatca actgggtgcg acaggccact 180

ggacaagggc ttgagtggat gggatggatg aaccctaaca atggttacac aggctatgca 240

cagaagttcc agggcagagt caccatgacc aggaacacct ccataagcac agcctacatg 300

gagctgagca gcctgagatc tgaggacacg gccgtgtatt actgtgcgag aactaactgg 360

gtctactggt acttcgatct ctggggccgt ggcaccctgg tcaccgtctc ctca 414

<210> 19
<211> 414
<212> DNA
<213> Artificial

<220>
<223> Description of the artificial sequence; nucleotide sequence of PI118_VH

<400> 19
atggactgga cctggaggat cctcttcttg gtggcagcag ccacaggtgc ccactcccag 60

gtgcagctgg tgcaatctgg ggctgaggtg aagaagcctg gggcctcagt gaaggtctcc 120

tgcaaggctt ctggatataa tctcaccact tatgatatca actgggtgcg acaggccact 180

ggacaagggc ttgagtggat gggatggatg aaccctaaca gtggttacac aggctatgca 240

cagaagttcc agggcagagt caccatgacc aggaacacct ccatagacac agcctacatg 300

gagctgagca acctgagatc tgaggacacg gccgtgtatt actgtgcgag aactaactgg 360

gtctactggt acttcgatct ctggggccgt ggaaccctgg tcaccgtctc ctca 414

<210> 20
<211> 414
<212> DNA
<213> Artificial

<220>
<223> Description of the artificial sequence; nucleotide sequence of PI127_VH

<400> 20
atggactgga cctggaggat cctcttcttg gtggcagcag ccacaggtgc ccactcccaa 60

gtccagctgg tacagtctgg ggctgaggtg aagaagcctg gggcctcagt gaaggtctcc 120

tgcaaggctt ctggatacac cttcaccact tatgatatca actgggtgcg acaggccacg 180

ggacaagggc ttgagtggct gggatggatg aaccctaaca atggttacac aggctatgca 240

cagaagttcc agggcagagt caccatgacc aggaacacct ccataggcac agcctacatg 300

gagctgaaca gcctgagatc tgaggacacg gccgtgtatt actgtgcgag aactaactgg 360

gtctactggt acttcgatct ctggggccgt ggcaccctgg tcactgtctc ctca 414

<210> 21
<211> 414
<212> DNA
<213> Artificial

<220>
<223> Description of the artificial sequence; nucleotide sequence of PI143_VH

<400> 21
atggactgga cctggaggat cctcttcttg gtggcagcag ccacaggtgc ccactcccag 60

gtccagctgg tgcagtctgg ggctgaggtg aagaagcctg gggcctcagt gaaggtctcc 120

tgcaaggctt ctggatacac cttcaccact tatgatatca actgggtgcg acaggccact 180

ggacaagggc ttgagtggat gggatggatg aaccctaaca atggttacac aggctatgca 240

cagaagttcc agggcagagt caccatgacc aggaacacct ccataagcac agcctacatg 300

gagctgagca gcctgagatc tgaggacacg gccgtgtatt actgtgcgag aactaactgg 360

gtctactggt acttcgatct ctggggccgt ggcaccacgg tcaccgtctc ctca 414

<210> 22
<211> 414
<212> DNA
<213> Artificial

<220>
<223> Description of the artificial sequence; nucleotide sequence of PL223_VH

<400> 22
atggactgga cctggaggat cctcttcttg gtggcagcag ccacaggtgc ccactccgaa 60

gtgcagctgg tgcagtctgg ggctgaggtg aagaagcctg gggcctcagt gaaggtctcc 120

tgcaaggctt ctggatacac cttcaccact tatgatatca actgggtgcg acaggccact 180

ggacaagggc ttgagtggat gggatggatg aaccctaaca atggttacac aggctatgca 240

cagaagttcc agggcagagt caccatgacc aggaacacct ccataagcac agcctacatg 300

gagctgagca gcctgagatc tgaggacacg gccgtgtatt actgtgcgag aactaactgg 360

gtctactggt acttcgatct ctggggccgt ggcaccatgg tcaccgtctc ttca 414

<210> 23
<211> 435
<212> DNA
<213> Artificial

<220>
<223> Description of the artificial sequence; nucleotide sequence of 2A10_VH

<400> 23
atggatctca tgtgcaagaa aatgaagcac ctgtggttct tcctcctgct ggtggcggct 60

cccagatggg tcctgtcgga ggtgcagctg gtgcagtctg gagcagaggt gaaaaagccc 120

ggggagtctc tgaagatctc ctgtaagggt tctggataca gctttaccag taactggatc 180

ggctgggtgc gccagatgcc cgggaaaggc ctggagtgga tggggatcat ctatcctggt 240

gactctgata ccagatacag cccgtccttc caaggccagg tcaccatctc agccgacaag 300

tccatcagca ccgcctacct gcagtggagc agcctgaagg cctcggacac cgccatgtat 360

tactgtgcga ggcaaactgg tttcctctgg tcctccgatc tctggggccg tggcaccctg 420

gtcactgtct cctca 435

<210> 24
<211> 145
<212> PRT
<213> Artificial

<220>
<223> Description of the artificial sequence; amino acid sequence of 2A10_VH

<400> 24

```
Met Asp Leu Met Cys Lys Lys Met Lys His Leu Trp Phe Phe Leu Leu
1               5                   10                  15

Leu Val Ala Ala Pro Arg Trp Val Leu Ser Glu Val Gln Leu Val Gln
            20                  25                  30

Ser Gly Ala Glu Val Lys Lys Pro Gly Glu Ser Leu Lys Ile Ser Cys
        35                  40                  45

Lys Gly Ser Gly Tyr Ser Phe Thr Ser Asn Trp Ile Gly Trp Val Arg
    50                  55                  60

Gln Met Pro Gly Lys Gly Leu Glu Trp Met Gly Ile Ile Tyr Pro Gly
65                  70                  75                  80

Asp Ser Asp Thr Arg Tyr Ser Pro Ser Phe Gln Gly Gln Val Thr Ile
                85                  90                  95

Ser Ala Asp Lys Ser Ile Ser Thr Ala Tyr Leu Gln Trp Ser Ser Leu
            100                 105                 110

Lys Ala Ser Asp Thr Ala Met Tyr Tyr Cys Ala Arg Gln Thr Gly Phe
        115                 120                 125

Leu Trp Ser Ser Asp Leu Trp Gly Arg Gly Thr Leu Val Thr Val Ser
    130                 135                 140

Ser
145
```

<210> 25
<211> 387
<212> DNA
<213> Artificial

<220>
<223> Description of the artificial sequence; nucleotide sequence of 2A10_VL

<400> 25

```
atggacatga gggtccccgc tcagctcctg gggcttctgc tgctctggct cccaggtgcc      60
```

agatgtgcca tccagttgac ccagtctcca tcctccctgt ctgcatctgt aggagacaga 120

gtcaccatca cttgccgggc aagtcaggac attagcagtg ctttagcctg gtatcaacag 180

aaaccaggga aagctcctaa gctcctgatc tatgatgcct ccagtttgga aagtggggtc 240

ccatcaaggt tcagcggcta tggatctggg acagatttca ctctcaccat caacagcctg 300

cagcctgaag attttgcaac ttattactgt caacagttta atagttaccc gctcactttc 360

ggcggaggga ccaaggtgga gatcaaa 387

<210> 26
<211> 129
<212> PRT
<213> Artificial

<220>
<223> Description of the artificial sequence; amino acid sequence of 2A10_VL

<400> 26

Met Asp Met Arg Val Pro Ala Gln Leu Leu Gly Leu Leu Leu Leu Trp
1 5 10 15

Leu Pro Gly Ala Arg Cys Ala Ile Gln Leu Thr Gln Ser Pro Ser Ser
20 25 30

Leu Ser Ala Ser Val Gly Asp Arg Val Thr Ile Thr Cys Arg Ala Ser
35 40 45

Gln Asp Ile Ser Ser Ala Leu Ala Trp Tyr Gln Gln Lys Pro Gly Lys
50 55 60

Ala Pro Lys Leu Leu Ile Tyr Asp Ala Ser Ser Leu Glu Ser Gly Val
65 70 75 80

Pro Ser Arg Phe Ser Gly Tyr Gly Ser Gly Thr Asp Phe Thr Leu Thr
85 90 95

Ile Asn Ser Leu Gln Pro Glu Asp Phe Ala Thr Tyr Tyr Cys Gln Gln
100 105 110

Phe Asn Ser Tyr Pro Leu Thr Phe Gly Gly Gly Thr Lys Val Glu Ile
115 120 125

Lys

<210> 27
<211> 68

<212> DNA
<213> Artificial

<220>
<223> Description of the artificial sequence; primer5

<400> 27
ctgtggttct tcctcctgct ggtggcggct cccagatggg tcctgtcgga ggtgcagctg 60

gtgcagtc 68

<210> 28
<211> 33
<212> DNA
<213> Artificial

<220>
<223> Description of the artificial sequence; primer6

<400> 28
ggtgctagct gaggagacag tgaccagggt gcc 33

<210> 29
<211> 65
<212> DNA
<213> Artificial

<220>
<223> Description of the artificial sequence; primer7

<400> 29
gacccgtcga cgctatggat ctcatgtgca agaaaatgaa gcacctgtgg ttcttcctcc 60

tgctg 65

<210> 30
<211> 33
<212> DNA
<213> Artificial

<220>
<223> Description of the artificial sequence; primer8

<400> 30
gtcaccgtct cctcagctag caccaagggg cca 33

<210> 31
<211> 20
<212> DNA
<213> Artificial

<220>
<223> Description of the artificial sequence; primer9

<400> 31
aactctcttg tccaccttgg 20

<210> 32

<211> 36
<212> DNA
<213> Artificial

<220>
<223> Description of the artificial sequence; primer10

<400> 32
aaggtggaca agagagttca ggtgcagctg gtgcag 36

<210> 33
<211> 33
<212> DNA
<213> Artificial

<220>
<223> Description of the artificial sequence; primer11

<400> 33
gcccttggtg ctagctgagg agacggtgac cag 33

<210> 34
<211> 1083
<212> DNA
<213> Artificial

<220>
<223> Description of the artificial sequence; nucleotide sequence of E11_VH-CH1-PI134_VH

<400> 34
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttcagg tgcagctggt gcagtctggg gctgaggtga agaagcctgg ggcctcagtg 780

aaggtctcct gcaaggcttc tggatacacc ttcaccactt atgatatcaa ctgggtgcga 840

caggccactg gacaagggct tgagtggatg ggatggatga accctaacaa tggttacaca 900

ggctatgcac agaagttcca gggcagagtc accatgacca ggaacacctc cataagcaca 960

gcctacatgg agctgagcag cctgagatct gaggacacgg ccgtgtatta ctgtgcgaga 1020

actaactggg tctactggta cttcgatctc tggggccgtg gcaccctggt caccgtctcc 1080

tca 1083

<210> 35
<211> 36
<212> DNA
<213> Artificial

<220>
<223> Description of the artificial sequence; primer12

<400> 35
aaggtggaca agagagttga ggtgcagctg gtgcag 36

<210> 36
<211> 1083
<212> DNA
<213> Artificial

<220>
<223> Description of the artificial sequence; nucleotide sequence of E11_VH-CH1-PI101_VH

<400> 36
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttgagg tgcagctggt gcagtctggg gctgaggtga agaagcctgg ggcctcagtg 780

aaggtctcct gcaaggcttc tggatataat ctcgccactt atgatatcaa ctgggtgcga 840

caggccactg gacaagggct tgagtggatg ggatggatga accctaacag tggttacaca 900

ggctatgcac agaagttcca gggcagagtc accatgacca gggacacctc cataaacaca 960

gcctacatgg agctgagcag cctgagatct gaggacacgg ccgtgtatta ctgtgcgaga 1020

actaactggg tctactggta cttcgatctc tggggccgtg gcaccctggt caccgtctcc 1080

tca 1083

<210> 37
<211> 36
<212> DNA
<213> Artificial

<220>
<223> Description of the artificial sequence; primer13

<400> 37
aaggtggaca agagagttga agtgcagctg gtgcag 36

<210> 38
<211> 33
<212> DNA
<213> Artificial

<220>
<223> Description of the artificial sequence; primer14

<400> 38
gcccttggtg ctagctgagg agacagtgac cag 33

<210> 39
<211> 1083
<212> DNA
<213> Artificial

<220>
<223> Description of the artificial sequence; nucleotide sequence of E11_VH-CH1-PI105_VH

<400> 39
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttgagg tccagctggt acagtctggg gctgaggtga agaagcctgg ggcctcagtg 780

aaggtctcct gcaaggcttc tggatacacc ttcaccactt atgatatcaa ctgggtgcga 840

caggccacgg gacaagggct tgagtggctg ggatggatga accctaacaa tggttacaca 900

ggctatgcac agaagttcca gggcagagtc accatgacca ggaacacctc cataggcaca 960

gcctacatgg agctgaacag cctgagatct gaggacacgg ccgtgtatta ctgtgcgaga 1020

actaactggg tctactggta cttcgatctc tggggccgtg gcaccctggt cactgtctcc 1080

tca 1083

<210> 40
<211> 1083
<212> DNA
<213> Artificial

<220>
<223> Description of the artificial sequence; nucleotide sequence of E11_VH-CH1-PI108_VH

<400> 40
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttcagg tgcagctggt gcagtctggg gctgaggtga agaagcctgg ggcctcagtg 780

aaggtctcct gcaaggcttc tggatacacc ttcaccactt atgatatcaa ctgggtgcga 840

caggccactg gacaagggct tgagtggatg ggatggatga accctaacaa tggttacaca 900

ggctatgcac agaagttcca gggcagagtc accatgacca ggaacacctc cataagcaca 960

gcctacatgg agctgaccag cctgagatct gaggacacgg ccgtgtatta ctgtgcgaga 1020

actaactggg tctactggta cttcgatctc tggggccgtg gcaccctggt cactgtctcc 1080

tca 1083

<210> 41
<211> 1083
<212> DNA
<213> Artificial

<220>
<223> Description of the artificial sequence; nucleotide sequence of E11_VH-CH1-PI115_VH

<400> 41
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttgagg tgcagctggt gcagtccggg gctgaggtga agaagcctgg ggcctcagtg 780

aaggtctcct gcaaggcttc tggatacacc ttcaccactt atgatatcaa ctgggtgcga 840

caggccactg gacaagggct tgagtggatg ggatggatga accctaacaa tggttacaca 900

ggctatgcac agaagttcca gggcagagtc accatgacca ggaacacctc cataagcaca 960

gcctacatgg agctgagcag cctgagatct gaggacacgg ccgtgtatta ctgtgcgaga 1020

actaactggg tctactggta cttcgatctc tggggccgtg gcaccctggt caccgtctcc 1080

tca 1083

<210> 42
<211> 36
<212> DNA
<213> Artificial

<220>
<223> Description of the artificial sequence; primer15

<400> 42
aaggtggaca agagagttca ggtgcagctg gtgcaa 36

<210> 43
<211> 1083
<212> DNA
<213> Artificial

<220>
<223> Description of the artificial sequence; nucleotide sequence of E11_VH-CH1-PI118_VH

<400> 43
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttcagg tgcagctggt gcaatctggg gctgaggtga agaagcctgg ggcctcagtg 780

aaggtctcct gcaaggcttc tggatataat ctcaccactt atgatatcaa ctgggtgcga 840

caggccactg gacaagggct tgagtggatg ggatggatga accctaacag tggttacaca 900

ggctatgcac agaagttcca gggcagagtc accatgacca ggaacacctc catagacaca 960

gcctacatgg agctgagcaa cctgagatct gaggacacgg ccgtgtatta ctgtgcgaga 1020

actaactggg tctactggta cttcgatctc tggggccgtg gaaccctggt caccgtctcc 1080

tca 1083

<210> 44
<211> 36
<212> DNA
<213> Artificial

<220>
<223> Description of the artificial sequence; primer16

<400> 44
aaggtggaca agagagttca agtccagctg gtacag 36

<210> 45

<211> 1083
<212> DNA
<213> Artificial

<220>
<223> Description of the artificial sequence; nucleotide sequence of E11_VH-CH1-PI127_VH

<400> 45
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttcaag tccagctggt acagtctggg gctgaggtga agaagcctgg ggcctcagtg 780

aaggtctcct gcaaggcttc tggatacacc ttcaccactt atgatatcaa ctgggtgcga 840

caggccacgg gacaagggct tgagtggctg ggatggatga accctaacaa tggttacaca 900

ggctatgcac agaagttcca gggcagagtc accatgacca ggaacacctc cataggcaca 960

gcctacatgg agctgaacag cctgagatct gaggacacgg ccgtgtatta ctgtgcgaga 1020

actaactggg tctactggta cttcgatctc tggggccgtg gcaccctggt cactgtctcc 1080

tca 1083

<210> 46
<211> 36
<212> DNA
<213> Artificial

<220>
<223> Description of the artificial sequence; primer17

<400> 46
aaggtggaca agagagttca ggtccagctg gtgcag 36

<210> 47
<211> 33
<212> DNA

<213> Artificial

<220>
<223> Description of the artificial sequence; primer18

<400> 47
gcccttggtg ctagctgagg agacggtgac cgt 33

<210> 48
<211> 1083
<212> DNA
<213> Artificial

<220>
<223> Description of the artificial sequence; nucleotide sequence of E11_VH-CH1-PI143_VH

<400> 48
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttcagg tccagctggt gcagtctggg gctgaggtga agaagcctgg ggcctcagtg 780

aaggtctcct gcaaggcttc tggatacacc ttcaccactt atgatatcaa ctgggtgcga 840

caggccactg gacaagggct tgagtggatg ggatggatga accctaacaa tggttacaca 900

ggctatgcac agaagttcca gggcagagtc accatgacca ggaacacctc cataagcaca 960

gcctacatgg agctgagcag cctgagatct gaggacacgg ccgtgtatta ctgtgcgaga 1020

actaactggg tctactggta cttcgatctc tggggccgtg gcaccacggt caccgtctcc 1080

tca 1083

<210> 49
<211> 36
<212> DNA
<213> Artificial

<220>
<223> Description of the artificial sequence; primer19

<400> 49
aaggtggaca agagagttga agtgcagctg gtgcag 36

<210> 50
<211> 33
<212> DNA
<213> Artificial

<220>
<223> Description of the artificial sequence; primer20

<400> 50
gcccttggtg ctagctgaag agacggtgac cat 33

<210> 51
<211> 1083
<212> DNA
<213> Artificial

<220>
<223> Description of the artificial sequence; nucleotide sequence of E11_VH-CH1_PL223_VH

<400> 51
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caaggggcca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttgaag tgcagctggt gcagtctggg gctgaggtga agaagcctgg ggcctcagtg 780

aaggtctcct gcaaggcttc tggatacacc ttcaccactt atgatatcaa ctgggtgcga 840

caggccactg gacaagggct tgagtggatg ggatggatga accctaacaa tggttacaca 900

ggctatgcac agaagttcca gggcagagtc accatgacca ggaacacctc cataagcaca 960

gcctacatgg agctgagcag cctgagatct gaggacacgg ccgtgtatta ctgtgcgaga 1020

actaactggg tctactggta cttcgatctc tggggccgtg gcaccatggt caccgtctct 1080

tca 1083

<210> 52
<211> 36
<212> DNA
<213> Artificial

<220>
<223> Description of the artificial sequence; primer21

<400> 52
aaggtggaca agagagttga agtgcagctg gtgcag 36

<210> 53
<211> 1083
<212> DNA
<213> Artificial

<220>
<223> Description of the artificial sequence; nucleotide sequence of E11_VH-CH1-PN7_VH

<400> 53
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caaggggcca tccgtcttcc ccctggcgcc ctgctccagg 480

agcacctccg agagcacagc cgccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacgaaga cctacacctg caacgtagat cacaagccca gcaacaccaa ggtggacaag 720

agagttgaag tgcagctggt gcagtccgga gctgaggtga agaagcctgg ggcctcagtg 780

aaggtctcct gcaaggcttc tggttacacc tttaccactt atgatatcaa ctgggtgcga 840

caggccactg gacaagggct tgagtggatg ggatggatga accctaacag tggttacaca 900

ggctatgcac agaagttcca gggcagagtc accatgacca gggacacctc cataagcaca 960

gcctacatgg agctgagcag cctgagatct gaggacacgg ccgtgtatta ctgtgcgcgg 1020

acgaactggg aatcctggta cttcgatctc tggggccgtg gaaccctggt caccgtctcc 1080

tca 1083

<210> 54
<211> 36
<212> DNA
<213> Artificial

<220>
<223> Description of the artificial sequence; primer22

<400> 54
aaggtggaca agagagttga ggtccagctg gtacag 36

<210> 55
<211> 33
<212> DNA
<213> Artificial

<220>
<223> Description of the artificial sequence; primer23

<400> 55
gcccttggtg ctagctgagg agacagtgac cag 33

<210> 56
<211> 1089
<212> DNA
<213> Artificial

<220>
<223> Description of the artificial sequence; nucleotide sequence of E11_VH-CH1-PN170_VH

<400> 56
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caaaggacct tctgtatttc ctcttgcgcc atgctctcgc 480

tctacgtcag aatcaactgc cgctctgggg tgcctggtta aagactactt cccggagcct 540

gtgacagtga gttggaactc cggcgccctg acatcaggag tgcatacatt tcccgccgtg 600

cttcagagca gcggacttta tagcctcagc agtgtggtga ccgtgccatc ttccagcctg 660

gggaccaaga cctacacctg taacgtggac cacaaaccca gcaacaccaa ggttgataag 720

agggtcgagg tccagctggt acagtctggg gctgaggtga agaagcctgg ggcctcagtg 780

aaggtctcct gcaaggcttc tggatacacc ctcaccagtt atgatatcaa ttgggtgcga 840

caggccactg gacaagggct tgagtggatg ggatggatga accctaacag tggttacaca 900

ggctatgcac agaagttcca ggacagagtc accatgacca ggaacaccgc cataaacaca 960

gcctacatgg agctgagcag cctgagatct gaggacacgg ccgtgtatta ctgtgcgaga 1020

gaggcgggtt acgatggtat ctggtacttc gatctctggg gccgtggcac cctggtcact 1080

gtctcctca 1089

<210> 57
<211> 75
<212> DNA
<213> Artificial

<220>
<223> Description of the artificial sequence; primer24

<400> 57
gtcaccgtct cctcagctag caccaagggg ccatccgtct tcccccctggc gccctgccag 60

gtgcagctgg tgcag 75

<210> 58
<211> 831
<212> DNA
<213> Artificial

<220>
<223> Description of the artificial sequence; nucleotide sequence of E11_VH-GL-PI134_VH

<400> 58
atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagctagcac caagggccca tccgtcttcc ccctggcgcc ctgccaggtg 480

cagctggtgc agtctggggc tgaggtgaag aagcctgggg cctcagtgaa ggtctcctgc 540

aaggcttctg gatacacctt caccacttat gatatcaact gggtgcgaca ggccactgga 600

caagggcttg agtggatggg atggatgaac cctaacaatg gttacacagg ctatgcacag 660

aagttccagg gcagagtcac catgaccagg aacacctcca taagcacagc ctacatggag 720

ctgagcagcc tgagatctga ggacacggcc gtgtattact gtgcgagaac taactgggtc 780

tactggtact tcgatctctg gggccgtggc accctggtca ccgtctcctc a 831

<210> 59
<211> 33
<212> DNA
<213> Artificial

<220>
<223> Description of the artificial sequence; primer25

<400> 59
agacccgtcg acgtcatgga tctcatgtgc aag 33

<210> 60
<211> 35
<212> DNA
<213> Artificial

<220>
<223> Description of the artificial sequence; primer26

<400> 60
ggggcggatg cgctccctga ggagacggtg accag 35

<210> 61
<211> 78
<212> DNA
<213> Artificial

<220>
<223> Description of the artificial sequence; primer27

<400> 61
ctggtcaccg tctcctcagg gagcgcatcc gccccaaccc ttttcccct cgtctcctgt 60

gaggtgcagc tggtgcag 78

<210> 62
<211> 39
<212> DNA
<213> Artificial

<220>
<223> Description of the artificial sequence; primer28

<400> 62
cccttggtgc tagctgagga gacggtgacc agggtgcca 39

<210> 63
<211> 831
<212> DNA
<213> Artificial

<220>
<223> Description of the artificial sequence; nucleotide sequence of E11_VH-ML-PI101_VH

<400> 63

atggatctca tgtgcaagaa aatgaagcac ctgtggttct tcctcctgct ggtggcggct 60

cccagatggg tcctgtccca gctgcagctg caggagtcgg gcccaggact ggtgaagcct 120

tcggagaccc tgtccctcac ctgcactgtc tctggtggct ccatcatcag taaaagttcc 180

tactggggct ggatccgcca gcccccaggg aaggggctgg agtggattgg gagtatctat 240

tatagtggga gtaccttcta caacccgtcc ctcaagagtc gagtcaccat atccgtagac 300

acgtccaaga accagttctc cctgaagctg agctctgtga ccgccgcaga cacggctgtg 360

tattactgtg cgagactgac agtggctgag tttgactact ggggccaggg aaccctggtc 420

accgtctcct cagggagcgc atccgcccca acccttttcc ccctcgtctc ctgtgaggtg 480

cagctggtgc agtctggggc tgaggtgaag aagcctgggg cctcagtgaa ggtctcctgc 540

aaggcttctg gatataatct cgccacttat gatatcaact gggtgcgaca ggccactgga 600

caagggcttg agtggatggg atggatgaac cctaacagtg gttacacagg ctatgcacag 660

aagttccagg gcagagtcac catgaccagg gacacctcca taaacacagc ctacatggag 720

ctgagcagcc tgagatctga ggacacggcc gtgtattact gtgcgagaac taactgggtc 780

tactggtact tcgatctctg gggccgtggc accctggtca ccgtctcctc a 831

<210> 64
<211> 2253
<212> DNA
<213> Homo sapiens

<400> 64
atgtggaatc tccttcacga aaccgactcg gctgtggcca ccgcgcgccg cccgcgctgg 60

ctgtgcgctg gggcgctggt gctggcgggt ggcttctttc tcctcggctt cctcttcggg 120

tggtttataa aatcctccaa tgaagctact aacattactc caaagcataa tatgaaagca 180

tttttggatg aattgaaagc tgagaacatc aagaagttct tatataattt tacacagata 240

ccacatttag caggaacaga acaaaacttt cagcttgcaa agcaaattca atcccagtgg 300

aaagaatttg gcctggattc tgttgagcta gcacattatg atgtcctgtt gtcctaccca 360

aataagactc atcccaacta catctcaata attaatgaag atggaaatga gattttcaac 420

acatcattat ttgaaccacc tcctccagga tatgaaaatg tttcggatat tgtaccacct 480

ttcagtgctt tctctcctca aggaatgcca gagggcgatc tagtgtatgt taactatgca 540

cgaactgaag acttctttaa attggaacgg gacatgaaaa tcaattgctc tgggaaaatt 600

gtaattgcca gatatgggaa agttttcaga ggaaataagg ttaaaaatgc ccagctggca 660

ggggccaaag gagtcattct ctactccgac cctgctgact actttgctcc tggggtgaag 720

tcctatccag atggttggaa tcttcctgga ggtggtgtcc agcgtggaaa tatcctaaat 780

ctgaatggtg caggagaccc tctcacacca ggttacccag caaatgaata tgcttatagg 840

cgtggaattg cagaggctgt tggtcttcca agtattcctg ttcatccaat tggatactat 900

gatgcacaga agctcctaga aaaaatgggt ggctcagcac caccagatag cagctggaga 960

ggaagtctca aagtgcccta caatgttgga cctggcttta ctggaaactt ttctacacaa 1020

aaagtcaaga tgcacatcca ctctaccaat gaagtgacaa gaatttacaa tgtgataggt 1080

actctcagag gagcagtgga accagacaga tatgtcattc tgggaggtca ccgggactca 1140

tgggtgtttg gtggtattga ccctcagagt ggagcagctg ttgttcatga aattgtgagg 1200

agctttggaa cactgaaaaa ggaagggtgg agacctagaa gaacaatttt gtttgcaagc 1260

tgggatgcag aagaatttgg tcttcttggt tctactgagt gggcagagga gaattcaaga 1320

ctccttcaag agcgtggcgt ggcttatatt aatgctgact catctataga aggaaactac 1380

actctgagag ttgattgtac accgctgatg tacagcttgg tacacaacct aacaaaagag 1440

ctgaaaagcc ctgatgaagg ctttgaaggc aaatctcttt atgaaagttg gactaaaaaa 1500

agtccttccc cagagttcag tggcatgccc aggataagca aattgggatc tggaaatgat 1560

tttgaggtgt tcttccaacg acttggaatt gcttcaggca gagcacggta tactaaaaat 1620

tgggaaacaa acaaattcag cggctatcca ctgtatcaca gtgtctatga aacatatgag 1680

ttggtggaaa agttttatga tccaatgttt aaatatcacc tcactgtggc ccaggttcga 1740

ggagggatgg tgtttgagct agccaattcc atagtgctcc cttttgattg tcgagattat 1800

gctgtagttt taagaaagta tgctgacaaa atctacagta tttctatgaa acatccacag 1860

gaaatgaaga catacagtgt atcatttgat tcactttttt ctgcagtaaa gaattttaca 1920

gaaattgctt ccaagttcag tgagagactc caggactttg acaaaagcaa cccaatagta 1980

ttaagaatga tgaatgatca actcatgttt ctggaaagag catttattga tccattaggg 2040

ttaccagaca ggccttttta taggcatgtc atctatgctc caagcagcca caacaagtat 2100

gcaggggagt cattcccagg aatttatgat gctctgtttg atattgaaag caaagtggac 2160

ccttccaagg cctggggaga agtgaagaga cagatttatg ttgcagcctt cacagtgcag 2220

gcagctgcag agactttgag tgaagtagcc taa 2253

<210> 65
<211> 80
<212> DNA
<213> Artificial

<220>
<223> Description of the artificial sequence; primer29

<400> 65
ggggcgctgg tgctggcggg tggcttcttt ctcctcggct tcctcttcgg gtggtttata 60

aaatcctcca atgaagctac 80

<210> 66
<211> 28
<212> DNA
<213> Artificial

<220>
<223> Description of the artificial sequence; primer30

<400> 66
gcggccgctt aggctacttc actcaaag 28

<210> 67
<211> 60
<212> DNA
<213> Artificial

<220>
<223> Description of the artificial sequence; primer31

<400> 67
ggctgtggcc accgcgcgcc gcccgcgctg gctgtgcgct ggggcgctgg tgctggcggg 60

<210> 68
<211> 59
<212> DNA
<213> Artificial

<220>
<223> Description of the artificial sequence; primer32

<400> 68
actagtcgtc atgtggaatc tccttcacga aaccgactcg gctgtggcca ccgcgcgcc 59

<210> 69
<211> 144
<212> PRT
<213> Artificial

<220>
<223> Description of the artificial sequence; amino acid sequence of E11_VH

<400> 69

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65                  70                  75                  80

Tyr Ser Gly Ser Thr Phe Tyr Asn Pro Ser Leu Lys Ser Arg Val Thr
                85                  90                  95

Ile Ser Val Asp Thr Ser Lys Asn Gln Phe Ser Leu Lys Leu Ser Ser
            100                 105                 110

Val Thr Ala Ala Asp Thr Ala Val Tyr Tyr Cys Ala Arg Leu Thr Val
        115                 120                 125

Ala Glu Phe Asp Tyr Trp Gly Gln Gly Thr Leu Val Thr Val Ser Ser
    130                 135                 140

<210> 70
<211> 7
<212> PRT
<213> Artificial

<220>
<223> Description of the artificial sequence; amino acid sequence of E11_HCDR1

<400> 70

Ser Lys Ser Ser Tyr Trp Gly
1               5

<210> 71
<211> 16
<212> PRT
<213> Artificial

<220>
<223> Description of the artificial sequence; amino acid sequence of E11_HCDR2

<400> 71

Ser Ile Tyr Tyr Ser Gly Ser Thr Phe Tyr Asn Pro Ser Leu Lys Ser
1               5                   10                  15

<210> 72
<211> 8
<212> PRT
<213> Artificial

<220>
<223> Description of the artificial sequence; amino acid sequence of E11_HCDR3

<400> 72

Leu Thr Val Ala Glu Phe Asp Tyr
1               5

<210> 73
<211> 128
<212> PRT
<213> Artificial

<220>
<223> Description of the artificial sequence; amino acid sequence of E11_VL

<400> 73

Met Glu Ala Pro Ala Gln Leu Leu Phe Leu Leu Leu Leu Trp Leu Pro
1 5 10 15

Asp Thr Thr Gly Glu Ile Val Leu Thr Gln Ser Pro Ala Thr Leu Ser
20 25 30

Leu Ser Pro Gly Glu Arg Ala Thr Leu Ser Cys Arg Ala Ser Gln Ser
35 40 45

Val Ser Ser Phe Leu Ala Trp Tyr Gln Gln Lys Pro Gly Gln Ala Pro
50 55 60

Arg Leu Leu Ile Tyr Asp Ala Ser Asn Arg Ala Thr Gly Ile Pro Ala
65 70 75 80

Arg Phe Ser Gly Ser Gly Ser Gly Thr Asp Phe Thr Leu Thr Ile Ser
85 90 95

Ser Leu Glu Pro Glu Asp Phe Ala Val Tyr Tyr Cys Gln Gln Arg Ser
100 105 110

Asn Trp Pro Leu Thr Phe Gly Pro Gly Thr Lys Val Asp Ile Lys Arg
115 120 125

<210> 74
<211> 11
<212> PRT
<213> Artificial

<220>
<223> Description of the artificial sequence; amino acid sequence of E11_LCDR1

<400> 74

Arg Ala Ser Gln Ser Val Ser Ser Phe Leu Ala
1 5 10

<210> 75
<211> 7
<212> PRT

<213> Artificial

<220>
<223> Description of the artificial sequence; amino acid sequence of E11_LCDR2

<400> 75

Asp Ala Ser Asn Arg Ala Thr
1 5

<210> 76
<211> 9
<212> PRT
<213> Artificial

<220>
<223> Description of the artificial sequence; amino acid sequence of E11_LCDR3

<400> 76

Gln Gln Arg Ser Asn Trp Pro Leu Thr
1 5

<210> 77
<211> 138
<212> PRT
<213> Artificial

<220>
<223> Description of the artificial sequence; amino acid sequence of PI134_VH

<400> 77

Met Asp Trp Thr Trp Arg Ile Leu Phe Leu Val Ala Ala Ala Thr Gly
1 5 10 15

Ala His Ser Gln Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Thr Tyr Asp Ile Asn Trp Val Arg Gln Ala Thr Gly Gln Gly Leu
50 55 60

Glu Trp Met Gly Trp Met Asn Pro Asn Asn Gly Tyr Thr Gly Tyr Ala
65 70 75 80

Gln Lys Phe Gln Gly Arg Val Thr Met Thr Arg Asn Thr Ser Ile Ser
85 90 95

Thr Ala Tyr Met Glu Leu Ser Ser Leu Arg Ser Glu Asp Thr Ala Val

100 105 110

Tyr Tyr Cys Ala Arg Thr Asn Trp Val Tyr Trp Tyr Phe Asp Leu Trp
115 120 125

Gly Arg Gly Thr Leu Val Thr Val Ser Ser
130 135

<210> 78
<211> 5
<212> PRT
<213> Artificial

<220>
<223> Description of the artificial sequence; amino acid sequence of PI134 PI143 PI105 PI127 PI108 PI115 PL223 PN7 PI101 PI118_HCDR1

<400> 78

Thr Tyr Asp Ile Asn
1 5

<210> 79
<211> 17
<212> PRT
<213> Artificial

<220>
<223> Description of the artificial sequence; amino acid sequence of PI134 PI143 PI105 PI127 PI108 PI115 PL223_HCDR2

<400> 79

Trp Met Asn Pro Asn Asn Gly Tyr Thr Gly Tyr Ala Gln Lys Phe Gln
1 5 10 15

Gly

<210> 80
<211> 10
<212> PRT
<213> Artificial

<220>
<223> Description of the artificial sequence; amino acid sequence of PI134 PI143 PI105 PI127 PI108 PI115 PL223 PI101 PI118_HCDR3

<400> 80

Thr Asn Trp Val Tyr Trp Tyr Phe Asp Leu
1 5 10

<210> 81
<211> 138
<212> PRT

<213> Artificial

<220>
<223> Description of the artificial sequence; amino acid sequence of PI101_VH

<400> 81

```
Met Gly Ser Thr Ala Ile Leu Ala Leu Leu Leu Ala Val Leu Gln Gly
1               5                   10                  15

Val Cys Ser Glu Val Gln Leu Val Gln Ser Gly Ala Glu Val Lys Lys
            20                  25                  30

Pro Gly Ala Ser Val Lys Val Ser Cys Lys Ala Ser Gly Tyr Asn Leu
        35                  40                  45

Ala Thr Tyr Asp Ile Asn Trp Val Arg Gln Ala Thr Gly Gln Gly Leu
    50                  55                  60

Glu Trp Met Gly Trp Met Asn Pro Asn Ser Gly Tyr Thr Gly Tyr Ala
65                  70                  75                  80

Gln Lys Phe Gln Gly Arg Val Thr Met Thr Arg Asp Thr Ser Ile Asn
                85                  90                  95

Thr Ala Tyr Met Glu Leu Ser Ser Leu Arg Ser Glu Asp Thr Ala Val
            100                 105                 110

Tyr Tyr Cys Ala Arg Thr Asn Trp Val Tyr Trp Tyr Phe Asp Leu Trp
        115                 120                 125

Gly Arg Gly Thr Leu Val Thr Val Ser Ser
    130                 135
```

<210> 82
<211> 17
<212> PRT
<213> Artificial

<220>
<223> Description of the artificial sequence; amino acid sequence of PI101 PN7 PI118_HCDR2

<400> 82

```
Trp Met Asn Pro Asn Ser Gly Tyr Thr Gly Tyr Ala Gln Lys Phe Gln
1               5                   10                  15

Gly
```

<210> 83
<211> 138
<212> PRT
<213> Artificial

<220>
<223> Description of the artificial sequence; amino acid sequence of PN7_VH

<400> 83

Met Glu Phe Gly Leu Ser Trp Val Phe Leu Val Ala Ile Leu Lys Gly
1               5                   10                  15

Val Gln Cys Glu Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Thr Tyr Asp Ile Asn Trp Val Arg Gln Ala Thr Gly Gln Gly Leu
    50                  55                  60

Glu Trp Met Gly Trp Met Asn Pro Asn Ser Gly Tyr Thr Gly Tyr Ala
65                  70                  75                  80

Gln Lys Phe Gln Gly Arg Val Thr Met Thr Arg Asp Thr Ser Ile Ser
                85                  90                  95

Thr Ala Tyr Met Glu Leu Ser Ser Leu Arg Ser Glu Asp Thr Ala Val
            100                 105                 110

Tyr Tyr Cys Ala Arg Thr Asn Trp Glu Ser Trp Tyr Phe Asp Leu Trp
        115                 120                 125

Gly Arg Gly Thr Leu Val Thr Val Ser Ser
    130                 135

<210> 84
<211> 10
<212> PRT
<213> Artificial

<220>
<223> Description of the artificial sequence; amino acid sequence of PN7_HCDR3

<400> 84

Thr Asn Trp Glu Ser Trp Tyr Phe Asp Leu
1               5                   10

<210> 85

<211> 138
<212> PRT
<213> Artificial

<220>
<223> Description of the artificial sequence; amino acid sequence of PI105_VH

<400> 85

```
Met Gly Ser Thr Ala Ile Leu Ala Leu Leu Leu Ala Val Leu Gln Gly
1               5                   10                  15

Val Cys Ser Glu Val Gln Leu Val Gln Ser Gly Ala Glu Val Lys Lys
            20                  25                  30

Pro Gly Ala Ser Val Lys Val Ser Cys Lys Ala Ser Gly Tyr Asn Leu
        35                  40                  45

Ala Thr Tyr Asp Ile Asn Trp Val Arg Gln Ala Thr Gly Gln Gly Leu
    50                  55                  60

Glu Trp Met Gly Trp Met Asn Pro Asn Ser Gly Tyr Thr Gly Tyr Ala
65                  70                  75                  80

Gln Lys Phe Gln Gly Arg Val Thr Met Thr Arg Asp Thr Ser Ile Asn
                85                  90                  95

Thr Ala Tyr Met Glu Leu Ser Ser Leu Arg Ser Glu Asp Thr Ala Val
            100                 105                 110

Tyr Tyr Cys Ala Arg Thr Asn Trp Val Tyr Trp Tyr Phe Asp Leu Trp
        115                 120                 125

Gly Arg Gly Thr Leu Val Thr Val Ser Ser
    130                 135
```

<210> 86
<211> 138
<212> PRT
<213> Artificial

<220>
<223> Description of the artificial sequence; amino acid sequence of PI108_VH

<400> 86

```
Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30
```

Pro Gly Ala Ser Val Lys Val Ser Cys Lys Ala Ser Gly Tyr Thr Phe
35 40 45

Thr Thr Tyr Asp Ile Asn Trp Val Arg Gln Ala Thr Gly Gln Gly Leu
50 55 60

Glu Trp Met Gly Trp Met Asn Pro Asn Asn Gly Tyr Thr Gly Tyr Ala
65 70 75 80

Gln Lys Phe Gln Gly Arg Val Thr Met Thr Arg Asn Thr Ser Ile Ser
85 90 95

Thr Ala Tyr Met Glu Leu Thr Ser Leu Arg Ser Glu Asp Thr Ala Val
100 105 110

Tyr Tyr Cys Ala Arg Thr Asn Trp Val Tyr Trp Tyr Phe Asp Leu Trp
115 120 125

Gly Arg Gly Thr Leu Val Thr Val Ser Ser
130 135

<210> 87
<211> 138
<212> PRT
<213> Artificial

<220>
<223> Description of the artificial sequence; amino acid sequence of PI115_VH

<400> 87

Met Asp Trp Thr Trp Arg Ile Leu Phe Leu Val Ala Ala Ala Thr Gly
1 5 10 15

Ala His Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Thr Tyr Asp Ile Asn Trp Val Arg Gln Ala Thr Gly Gln Gly Leu
50 55 60

Glu Trp Met Gly Trp Met Asn Pro Asn Asn Gly Tyr Thr Gly Tyr Ala
65 70 75 80

Gln Lys Phe Gln Gly Arg Val Thr Met Thr Arg Asn Thr Ser Ile Ser
85 90 95

Thr Ala Tyr Met Glu Leu Ser Ser Leu Arg Ser Glu Asp Thr Ala Val
            100                 105                 110

Tyr Tyr Cys Ala Arg Thr Asn Trp Val Tyr Trp Tyr Phe Asp Leu Trp
        115                 120                 125

Gly Arg Gly Thr Leu Val Thr Val Ser Ser
    130                 135

<210> 88
<211> 138
<212> PRT
<213> Artificial

<220>
<223> Description of the artificial sequence; amino acid sequence of PI118_VH

<400> 88

Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Asn Leu
        35                  40                  45

Thr Thr Tyr Asp Ile Asn Trp Val Arg Gln Ala Thr Gly Gln Gly Leu
    50                  55                  60

Glu Trp Met Gly Trp Met Asn Pro Asn Ser Gly Tyr Thr Gly Tyr Ala
65                  70                  75                  80

Gln Lys Phe Gln Gly Arg Val Thr Met Thr Arg Asn Thr Ser Ile Asp
                85                  90                  95

Thr Ala Tyr Met Glu Leu Ser Asn Leu Arg Ser Glu Asp Thr Ala Val
            100                 105                 110

Tyr Tyr Cys Ala Arg Thr Asn Trp Val Tyr Trp Tyr Phe Asp Leu Trp
        115                 120                 125

Gly Arg Gly Thr Leu Val Thr Val Ser Ser
    130                 135

<210> 89
<211> 138

<212> PRT
<213> Artificial

<220>
<223> Description of the artificial sequence; amino acid sequence of PI127_VH

<400> 89

```
Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Thr Tyr Asp Ile Asn Trp Val Arg Gln Ala Thr Gly Gln Gly Leu
    50                  55                  60

Glu Trp Leu Gly Trp Met Asn Pro Asn Asn Gly Tyr Thr Gly Tyr Ala
65                  70                  75                  80

Gln Lys Phe Gln Gly Arg Val Thr Met Thr Arg Asn Thr Ser Ile Gly
                85                  90                  95

Thr Ala Tyr Met Glu Leu Asn Ser Leu Arg Ser Glu Asp Thr Ala Val
            100                 105                 110

Tyr Tyr Cys Ala Arg Thr Asn Trp Val Tyr Trp Tyr Phe Asp Leu Trp
        115                 120                 125

Gly Arg Gly Thr Leu Val Thr Val Ser Ser
    130                 135
```

<210> 90
<211> 138
<212> PRT
<213> Artificial

<220>
<223> Description of the artificial sequence; amino acid sequence of PI143_VH

<400> 90

```
Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30
```

Pro Gly Ala Ser Val Lys Val Ser Cys Lys Ala Ser Gly Tyr Thr Phe
35 40 45

Thr Thr Tyr Asp Ile Asn Trp Val Arg Gln Ala Thr Gly Gln Gly Leu
50 55 60

Glu Trp Met Gly Trp Met Asn Pro Asn Asn Gly Tyr Thr Gly Tyr Ala
65 70 75 80

Gln Lys Phe Gln Gly Arg Val Thr Met Thr Arg Asn Thr Ser Ile Ser
85 90 95

Thr Ala Tyr Met Glu Leu Ser Ser Leu Arg Ser Glu Asp Thr Ala Val
100 105 110

Tyr Tyr Cys Ala Arg Thr Asn Trp Val Tyr Trp Tyr Phe Asp Leu Trp
115 120 125

Gly Arg Gly Thr Thr Val Thr Val Ser Ser
130 135

<210> 91
<211> 138
<212> PRT
<213> Artificial

<220>
<223> Description of the artificial sequence; amino acid sequence of PL223_VH

<400> 91

Met Asp Trp Thr Trp Arg Ile Leu Phe Leu Val Ala Ala Ala Thr Gly
1 5 10 15

Ala His Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Thr Tyr Asp Ile Asn Trp Val Arg Gln Ala Thr Gly Gln Gly Leu
50 55 60

Glu Trp Met Gly Trp Met Asn Pro Asn Asn Gly Tyr Thr Gly Tyr Ala
65 70 75 80

Gln Lys Phe Gln Gly Arg Val Thr Met Thr Arg Asn Thr Ser Ile Ser
85 90 95

Thr Ala Tyr Met Glu Leu Ser Ser Leu Arg Ser Glu Asp Thr Ala Val
100 105 110

Tyr Tyr Cys Ala Arg Thr Asn Trp Val Tyr Trp Tyr Phe Asp Leu Trp
115 120 125

Gly Arg Gly Thr Met Val Thr Val Ser Ser
130 135

<210> 92
<211> 2214
<212> DNA
<213> Artificial

<220>
<223> Description of the artificial sequence; nucleotide sequence of N_FLAG-PSMA_ECD

<400> 92
atgagggtcc ccgctcagct cctggggctc ctgctgctct ggctcccagg tgcacgatgt 60

gactacaagg acgacgatga caagactagt aaatcctcca atgaagctac taacattact 120

ccaaagcata atatgaaagc atttttggat gaattgaaag ctgagaacat caagaagttc 180

ttatataatt ttacacagat accacattta gcaggaacag aacaaaactt tcagcttgca 240

aagcaaattc aatcccagtg gaaagaattt ggcctggatt ctgttgagct agcacattat 300

gatgtcctgt tgtcctaccc aaataagact catcccaact acatctcaat aattaatgaa 360

gatggaaatg agattttcaa cacatcatta tttgaaccac ctcctccagg atatgaaaat 420

gtttcggata ttgtaccacc tttcagtgct ttctctcctc aaggaatgcc agagggcgat 480

ctagtgtatg ttaactatgc acgaactgaa gacttcttta aattggaacg ggacatgaaa 540

atcaattgct ctgggaaaat tgtaattgcc agatatggga aagttttcag aggaaataag 600

gttaaaaatg cccagctggc aggggccaaa ggagtcattc tctactccga ccctgctgac 660

tactttgctc ctggggtgaa gtcctatcca gatggttgga atcttcctgg aggtggtgtc 720

cagcgtggaa atatcctaaa tctgaatggt gcaggagacc ctctcacacc aggttaccca 780

gcaaatgaat atgcttatag gcgtggaatt gcagaggctg ttggtcttcc aagtattcct 840

gttcatccaa ttggatacta tgatgcacag aagctcctag aaaaaatggg tggctcagca 900

ccaccagata gcagctggag aggaagtctc aaagtgccct acaatgttgg acctggcttt 960

actggaaact tttctacaca aaaagtcaag atgcacatcc actctaccaa tgaagtgaca 1020

agaatttaca atgtgatagg tactctcaga ggagcagtgg aaccagacag atatgtcatt 1080

ctgggaggtc accgggactc atgggtgttt ggtggtattg accctcagag tggagcagct 1140

gttgttcatg aaattgtgag gagctttgga acactgaaaa aggaagggtg gagacctaga 1200

agaacaattt tgtttgcaag ctgggatgca gaagaatttg gtcttcttgg ttctactgag 1260

tgggcagagg agaattcaag actccttcaa gagcgtggcg tggcttatat taatgctgac 1320

tcatctatag aaggaaacta cactctgaga gttgattgta caccgctgat gtacagcttg 1380

gtacacaacc taacaaaaga gctgaaaagc cctgatgaag gctttgaagg caaatctctt 1440

tatgaaagtt ggactaaaaa aagtccttcc ccagagttca gtggcatgcc caggataagc 1500

aaattgggat ctggaaatga ttttgaggtg ttcttccaac gacttggaat tgcttcaggc 1560

agagcacggt atactaaaaa ttgggaaaca aacaaattca gcggctatcc actgtatcac 1620

agtgtctatg aaacatatga gttggtggaa aagttttatg atccaatgtt taaatatcac 1680

ctcactgtgg cccaggttcg aggagggatg gtgtttgagc tagccaattc catagtgctc 1740

ccttttgatt gtcgagatta tgctgtagtt ttaagaaagt atgctgacaa aatctacagt 1800

atttctatga aacatccaca ggaaatgaag acatacagtg tatcatttga ttcacttttt 1860

tctgcagtaa agaattttac agaaattgct tccaagttca gtgagagact ccaggacttt 1920

gacaaaagca acccaatagt attaagaatg atgaatgatc aactcatgtt tctggaaaga 1980

gcatttattg atccattagg gttaccagac aggccttttt ataggcatgt catctatgct 2040

ccaagcagcc acaacaagta tgcaggggag tcattcccag gaatttatga tgctctgttt 2100

gatattgaaa gcaaagtgga cccttccaag gcctggggag aagtgaagag acagatttat 2160

gttgcagcct tcacagtgca ggcagctgca gagactttga gtgaagtagc ctaa 2214

<210> 93
<211> 98
<212> PRT
<213> Artificial

<220>
<223> Description of the artificial sequence; amino acid sequence of IgG4_CH1_linker

<400> 93

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Lys Thr

65 70 75 80

Tyr Thr Cys Asn Val Asp His Lys Pro Ser Asn Thr Lys Val Asp Lys
85 90 95

Arg Val

<210> 94
<211> 420
<212> DNA
<213> Artificial

<220>
<223> Description of the artificial sequence; nucleotide sequence of PN170_VH

<400> 94
atggactgga cctggaggat cctcttcttg gtggcagcag ccacaggtgc ccactccgag 60

gtccagctgg tacagtctgg ggctgaggtg aagaagcctg gggcctcagt gaaggtctcc 120

tgcaaggctt ctggatacac cctcaccagt tatgatatca attgggtgcg acaggccact 180

ggacaagggc ttgagtggat gggatggatg aaccctaaca gtggttacac aggctatgca 240

cagaagttcc aggacagagt caccatgacc aggaacaccg ccataaacac agcctacatg 300

gagctgagca gcctgagatc tgaggacacg gccgtgtatt actgtgcgag agaggcgggt 360

tacgatggta tctggtactt cgatctctgg ggccgtggca ccctggtcac tgtctcctca 420

<210> 95
<211> 140
<212> PRT
<213> Artificial

<220>
<223> Description of the artificial sequence; amino acid sequence of PN170_VH

<400> 95

Met Asp Trp Thr Trp Arg Ile Leu Phe Leu Val Ala Ala Ala Thr Gly
1 5 10 15

Ala His Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Leu
35 40 45

Thr Ser Tyr Asp Ile Asn Trp Val Arg Gln Ala Thr Gly Gln Gly Leu
50 55 60

Glu Trp Met Gly Trp Met Asn Pro Asn Ser Gly Tyr Thr Gly Tyr Ala
65                  70                  75                  80

Gln Lys Phe Gln Asp Arg Val Thr Met Thr Arg Asn Thr Ala Ile Asn
                85                  90                  95

Thr Ala Tyr Met Glu Leu Ser Ser Leu Arg Ser Glu Asp Thr Ala Val
            100                 105                 110

Tyr Tyr Cys Ala Arg Glu Ala Gly Tyr Asp Gly Ile Trp Tyr Phe Asp
        115                 120                 125

Leu Trp Gly Arg Gly Thr Leu Val Thr Val Ser Ser
    130                 135                 140

<210> 96
<211> 5
<212> PRT
<213> Artificial

<220>
<223> Description of the artificial sequence; amino acid sequence of PN170_HCDR1

<400> 96

Ser Tyr Asp Ile Asn
1               5

<210> 97
<211> 17
<212> PRT
<213> Artificial

<220>
<223> Description of the artificial sequence; amino acid sequence of PN170_HCDR2

<400> 97

Trp Met Asn Pro Asn Ser Gly Tyr Thr Gly Tyr Ala Gln Lys Phe Gln
1               5                   10                  15

Asp

<210> 98
<211> 12
<212> PRT
<213> Artificial

<220>
<223> Description of the artificial sequence; amino acid sequence of PN170_HCDR3

<400> 98

Glu Ala Gly Tyr Asp Gly Ile Trp Tyr Phe Asp Leu
1 5 10

<210> 99
<211> 361
<212> PRT
<213> Artificial

<220>
<223> Description of the artificial sequence; amino acid sequence of E11_VH-CH1-PI134_VH

<400> 99

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val
115 120 125

Ala Glu Phe Asp Tyr Trp Gly Gln Gly Thr Leu Val Thr Val Ser Ser
130 135 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Lys Thr
210 215 220

Tyr Thr Cys Asn Val Asp His Lys Pro Ser Asn Thr Lys Val Asp Lys
225 230 235 240

Arg Val Gln Val Gln Leu Val Gln Ser Gly Ala Glu Val Lys Lys Pro
245 250 255

Gly Ala Ser Val Lys Val Ser Cys Lys Ala Ser Gly Tyr Thr Phe Thr
260 265 270

Thr Tyr Asp Ile Asn Trp Val Arg Gln Ala Thr Gly Gln Gly Leu Glu
275 280 285

Trp Met Gly Trp Met Asn Pro Asn Asn Gly Tyr Thr Gly Tyr Ala Gln
290 295 300

Lys Phe Gln Gly Arg Val Thr Met Thr Arg Asn Thr Ser Ile Ser Thr
305 310 315 320

Ala Tyr Met Glu Leu Ser Ser Leu Arg Ser Glu Asp Thr Ala Val Tyr
325 330 335

Tyr Cys Ala Arg Thr Asn Trp Val Tyr Trp Tyr Phe Asp Leu Trp Gly
340 345 350

Arg Gly Thr Leu Val Thr Val Ser Ser
355 360

<210> 100
<211> 361
<212> PRT
<213> Artificial

<220>
<223> Description of the artificial sequence; amino acid sequence of E11_VH-CH1-PI101_VH

<400> 100

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

```
Ser Gly Pro Gly Leu Val Lys Pro Ser Glu Thr Leu Ser Leu Thr Cys
        35                  40                  45

Thr Val Ser Gly Gly Ser Ile Ile Ser Lys Ser Ser Tyr Trp Gly Trp
    50                  55                  60

Ile Arg Gln Pro Pro Gly Lys Gly Leu Glu Trp Ile Gly Ser Ile Tyr
65                  70                  75                  80

Tyr Ser Gly Ser Thr Phe Tyr Asn Pro Ser Leu Lys Ser Arg Val Thr
                85                  90                  95

Ile Ser Val Asp Thr Ser Lys Asn Gln Phe Ser Leu Lys Leu Ser Ser
            100                 105                 110

Val Thr Ala Ala Asp Thr Ala Val Tyr Tyr Cys Ala Arg Leu Thr Val
        115                 120                 125

Ala Glu Phe Asp Tyr Trp Gly Gln Gly Thr Leu Val Thr Val Ser Ser
    130                 135                 140

Ala Ser Thr Lys Gly Pro Ser Val Phe Pro Leu Ala Pro Cys Ser Arg
145                 150                 155                 160

Ser Thr Ser Glu Ser Thr Ala Ala Leu Gly Cys Leu Val Lys Asp Tyr
                165                 170                 175

Phe Pro Glu Pro Val Thr Val Ser Trp Asn Ser Gly Ala Leu Thr Ser
            180                 185                 190

Gly Val His Thr Phe Pro Ala Val Leu Gln Ser Ser Gly Leu Tyr Ser
        195                 200                 205

Leu Ser Ser Val Val Thr Val Pro Ser Ser Ser Leu Gly Thr Lys Thr
    210                 215                 220

Tyr Thr Cys Asn Val Asp His Lys Pro Ser Asn Thr Lys Val Asp Lys
225                 230                 235                 240

Arg Val Glu Val Gln Leu Val Gln Ser Gly Ala Glu Val Lys Lys Pro
                245                 250                 255

Gly Ala Ser Val Lys Val Ser Cys Lys Ala Ser Gly Tyr Asn Leu Ala
            260                 265                 270

Thr Tyr Asp Ile Asn Trp Val Arg Gln Ala Thr Gly Gln Gly Leu Glu
```

275 280 285

Trp Met Gly Trp Met Asn Pro Asn Ser Gly Tyr Thr Gly Tyr Ala Gln
290 295 300

Lys Phe Gln Gly Arg Val Thr Met Thr Arg Asp Thr Ser Ile Asn Thr
305 310 315 320

Ala Tyr Met Glu Leu Ser Ser Leu Arg Ser Glu Asp Thr Ala Val Tyr
325 330 335

Tyr Cys Ala Arg Thr Asn Trp Val Tyr Trp Tyr Phe Asp Leu Trp Gly
340 345 350

Arg Gly Thr Leu Val Thr Val Ser Ser
355 360

<210> 101
<211> 361
<212> PRT
<213> Artificial

<220>
<223> Description of the artificial sequence; amino acid sequence of E11_VH-CH1-PI105_VH

<400> 101

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val

115 120 125

Ala Glu Phe Asp Tyr Trp Gly Gln Gly Thr Leu Val Thr Val Ser Ser
130 135 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Lys Thr
210 215 220

Tyr Thr Cys Asn Val Asp His Lys Pro Ser Asn Thr Lys Val Asp Lys
225 230 235 240

Arg Val Glu Val Gln Leu Val Gln Ser Gly Ala Glu Val Lys Lys Pro
245 250 255

Gly Ala Ser Val Lys Val Ser Cys Lys Ala Ser Gly Tyr Thr Phe Thr
260 265 270

Thr Tyr Asp Ile Asn Trp Val Arg Gln Ala Thr Gly Gln Gly Leu Glu
275 280 285

Trp Leu Gly Trp Met Asn Pro Asn Asn Gly Tyr Thr Gly Tyr Ala Gln
290 295 300

Lys Phe Gln Gly Arg Val Thr Met Thr Arg Asn Thr Ser Ile Gly Thr
305 310 315 320

Ala Tyr Met Glu Leu Asn Ser Leu Arg Ser Glu Asp Thr Ala Val Tyr
325 330 335

Tyr Cys Ala Arg Thr Asn Trp Val Tyr Trp Tyr Phe Asp Leu Trp Gly
340 345 350

Arg Gly Thr Leu Val Thr Val Ser Ser
355 360

<210> 102
<211> 361
<212> PRT
<213> Artificial

<220>
<223> Description of the artificial sequence; amino acid sequence of E11_VH-CH1-PI108_VH

<400> 102

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val
115 120 125

Ala Glu Phe Asp Tyr Trp Gly Gln Gly Thr Leu Val Thr Val Ser Ser
130 135 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

```
Leu Ser Ser Val Val Thr Val Pro Ser Ser Ser Leu Gly Thr Lys Thr
    210                 215                 220

Tyr Thr Cys Asn Val Asp His Lys Pro Ser Asn Thr Lys Val Asp Lys
225                 230                 235                 240

Arg Val Gln Val Gln Leu Val Gln Ser Gly Ala Glu Val Lys Lys Pro
                245                 250                 255

Gly Ala Ser Val Lys Val Ser Cys Lys Ala Ser Gly Tyr Thr Phe Thr
            260                 265                 270

Thr Tyr Asp Ile Asn Trp Val Arg Gln Ala Thr Gly Gln Gly Leu Glu
        275                 280                 285

Trp Met Gly Trp Met Asn Pro Asn Asn Gly Tyr Thr Gly Tyr Ala Gln
    290                 295                 300

Lys Phe Gln Gly Arg Val Thr Met Thr Arg Asn Thr Ser Ile Ser Thr
305                 310                 315                 320

Ala Tyr Met Glu Leu Thr Ser Leu Arg Ser Glu Asp Thr Ala Val Tyr
                325                 330                 335

Tyr Cys Ala Arg Thr Asn Trp Val Tyr Trp Tyr Phe Asp Leu Trp Gly
            340                 345                 350

Arg Gly Thr Leu Val Thr Val Ser Ser
        355                 360


<210>  103
<211>  361
<212>  PRT
<213>  Artificial

<220>
<223>  Description of the artificial sequence; amino acid sequence of
       E11_VH-CH1-PI115_VH

<400>  103

Met Asp Leu Met Cys Lys Lys Met Lys His Leu Trp Phe Phe Leu Leu
1               5                   10                  15

Leu Val Ala Ala Pro Arg Trp Val Leu Ser Gln Leu Gln Leu Gln Glu
            20                  25                  30

Ser Gly Pro Gly Leu Val Lys Pro Ser Glu Thr Leu Ser Leu Thr Cys
        35                  40                  45
```

```
Thr Val Ser Gly Gly Ser Ile Ile Ser Lys Ser Ser Tyr Trp Gly Trp
    50                  55                  60

Ile Arg Gln Pro Pro Gly Lys Gly Leu Glu Trp Ile Gly Ser Ile Tyr
65                  70                  75                  80

Tyr Ser Gly Ser Thr Phe Tyr Asn Pro Ser Leu Lys Ser Arg Val Thr
                85                  90                  95

Ile Ser Val Asp Thr Ser Lys Asn Gln Phe Ser Leu Lys Leu Ser Ser
            100                 105                 110

Val Thr Ala Ala Asp Thr Ala Val Tyr Tyr Cys Ala Arg Leu Thr Val
        115                 120                 125

Ala Glu Phe Asp Tyr Trp Gly Gln Gly Thr Leu Val Thr Val Ser Ser
    130                 135                 140

Ala Ser Thr Lys Gly Pro Ser Val Phe Pro Leu Ala Pro Cys Ser Arg
145                 150                 155                 160

Ser Thr Ser Glu Ser Thr Ala Ala Leu Gly Cys Leu Val Lys Asp Tyr
                165                 170                 175

Phe Pro Glu Pro Val Thr Val Ser Trp Asn Ser Gly Ala Leu Thr Ser
            180                 185                 190

Gly Val His Thr Phe Pro Ala Val Leu Gln Ser Ser Gly Leu Tyr Ser
        195                 200                 205

Leu Ser Ser Val Val Thr Val Pro Ser Ser Ser Leu Gly Thr Lys Thr
    210                 215                 220

Tyr Thr Cys Asn Val Asp His Lys Pro Ser Asn Thr Lys Val Asp Lys
225                 230                 235                 240

Arg Val Glu Val Gln Leu Val Gln Ser Gly Ala Glu Val Lys Lys Pro
                245                 250                 255

Gly Ala Ser Val Lys Val Ser Cys Lys Ala Ser Gly Tyr Thr Phe Thr
            260                 265                 270

Thr Tyr Asp Ile Asn Trp Val Arg Gln Ala Thr Gly Gln Gly Leu Glu
        275                 280                 285

Trp Met Gly Trp Met Asn Pro Asn Asn Gly Tyr Thr Gly Tyr Ala Gln
    290                 295                 300
```

Lys Phe Gln Gly Arg Val Thr Met Thr Arg Asn Thr Ser Ile Ser Thr
305                 310                 315                 320

Ala Tyr Met Glu Leu Ser Ser Leu Arg Ser Glu Asp Thr Ala Val Tyr
                325                 330                 335

Tyr Cys Ala Arg Thr Asn Trp Val Tyr Trp Tyr Phe Asp Leu Trp Gly
            340                 345                 350

Arg Gly Thr Leu Val Thr Val Ser Ser
        355                 360

<210>  104
<211>  361
<212>  PRT
<213>  Artificial

<220>
<223>  Description of the artificial sequence; amino acid sequence of E11_VH-CH1-PI118_VH

<400>  104

Met Asp Leu Met Cys Lys Lys Met Lys His Leu Trp Phe Phe Leu Leu
1               5                   10                  15

Leu Val Ala Ala Pro Arg Trp Val Leu Ser Gln Leu Gln Leu Gln Glu
            20                  25                  30

Ser Gly Pro Gly Leu Val Lys Pro Ser Glu Thr Leu Ser Leu Thr Cys
        35                  40                  45

Thr Val Ser Gly Gly Ser Ile Ile Ser Lys Ser Ser Tyr Trp Gly Trp
    50                  55                  60

Ile Arg Gln Pro Pro Gly Lys Gly Leu Glu Trp Ile Gly Ser Ile Tyr
65                  70                  75                  80

Tyr Ser Gly Ser Thr Phe Tyr Asn Pro Ser Leu Lys Ser Arg Val Thr
                85                  90                  95

Ile Ser Val Asp Thr Ser Lys Asn Gln Phe Ser Leu Lys Leu Ser Ser
            100                 105                 110

Val Thr Ala Ala Asp Thr Ala Val Tyr Tyr Cys Ala Arg Leu Thr Val
        115                 120                 125

Ala Glu Phe Asp Tyr Trp Gly Gln Gly Thr Leu Val Thr Val Ser Ser
    130                 135                 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Lys Thr
210 215 220

Tyr Thr Cys Asn Val Asp His Lys Pro Ser Asn Thr Lys Val Asp Lys
225 230 235 240

Arg Val Gln Val Gln Leu Val Gln Ser Gly Ala Glu Val Lys Lys Pro
245 250 255

Gly Ala Ser Val Lys Val Ser Cys Lys Ala Ser Gly Tyr Asn Leu Thr
260 265 270

Thr Tyr Asp Ile Asn Trp Val Arg Gln Ala Thr Gly Gln Gly Leu Glu
275 280 285

Trp Met Gly Trp Met Asn Pro Asn Ser Gly Tyr Thr Gly Tyr Ala Gln
290 295 300

Lys Phe Gln Gly Arg Val Thr Met Thr Arg Asn Thr Ser Ile Asp Thr
305 310 315 320

Ala Tyr Met Glu Leu Ser Asn Leu Arg Ser Glu Asp Thr Ala Val Tyr
325 330 335

Tyr Cys Ala Arg Thr Asn Trp Val Tyr Trp Tyr Phe Asp Leu Trp Gly
340 345 350

Arg Gly Thr Leu Val Thr Val Ser Ser
355 360

<210> 105
<211> 361
<212> PRT
<213> Artificial

<220>

<223> Description of the artificial sequence; amino acid sequence of E11_VH-CH1-PI127_VH

<400> 105

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val
115 120 125

Ala Glu Phe Asp Tyr Trp Gly Gln Gly Thr Leu Val Thr Val Ser Ser
130 135 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Lys Thr
210 215 220

Tyr Thr Cys Asn Val Asp His Lys Pro Ser Asn Thr Lys Val Asp Lys
225 230 235 240

Arg Val Gln Val Gln Leu Val Gln Ser Gly Ala Glu Val Lys Lys Pro
245 250 255

Gly Ala Ser Val Lys Val Ser Cys Lys Ala Ser Gly Tyr Thr Phe Thr
260 265 270

Thr Tyr Asp Ile Asn Trp Val Arg Gln Ala Thr Gly Gln Gly Leu Glu
275 280 285

Trp Leu Gly Trp Met Asn Pro Asn Asn Gly Tyr Thr Gly Tyr Ala Gln
290 295 300

Lys Phe Gln Gly Arg Val Thr Met Thr Arg Asn Thr Ser Ile Gly Thr
305 310 315 320

Ala Tyr Met Glu Leu Asn Ser Leu Arg Ser Glu Asp Thr Ala Val Tyr
325 330 335

Tyr Cys Ala Arg Thr Asn Trp Val Tyr Trp Tyr Phe Asp Leu Trp Gly
340 345 350

Arg Gly Thr Leu Val Thr Val Ser Ser
355 360

<210> 106
<211> 361
<212> PRT
<213> Artificial

<220>
<223> Description of the artificial sequence; amino acid sequence of E11_VH-CH1-PI143_VH

<400> 106

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

```
Tyr Ser Gly Ser Thr Phe Tyr Asn Pro Ser Leu Lys Ser Arg Val Thr
                85                  90                  95

Ile Ser Val Asp Thr Ser Lys Asn Gln Phe Ser Leu Lys Leu Ser Ser
            100                 105                 110

Val Thr Ala Ala Asp Thr Ala Val Tyr Tyr Cys Ala Arg Leu Thr Val
        115                 120                 125

Ala Glu Phe Asp Tyr Trp Gly Gln Gly Thr Leu Val Thr Val Ser Ser
    130                 135                 140

Ala Ser Thr Lys Gly Pro Ser Val Phe Pro Leu Ala Pro Cys Ser Arg
145                 150                 155                 160

Ser Thr Ser Glu Ser Thr Ala Ala Leu Gly Cys Leu Val Lys Asp Tyr
                165                 170                 175

Phe Pro Glu Pro Val Thr Val Ser Trp Asn Ser Gly Ala Leu Thr Ser
            180                 185                 190

Gly Val His Thr Phe Pro Ala Val Leu Gln Ser Ser Gly Leu Tyr Ser
        195                 200                 205

Leu Ser Ser Val Val Thr Val Pro Ser Ser Ser Leu Gly Thr Lys Thr
    210                 215                 220

Tyr Thr Cys Asn Val Asp His Lys Pro Ser Asn Thr Lys Val Asp Lys
225                 230                 235                 240

Arg Val Gln Val Gln Leu Val Gln Ser Gly Ala Glu Val Lys Lys Pro
                245                 250                 255

Gly Ala Ser Val Lys Val Ser Cys Lys Ala Ser Gly Tyr Thr Phe Thr
            260                 265                 270

Thr Tyr Asp Ile Asn Trp Val Arg Gln Ala Thr Gly Gln Gly Leu Glu
        275                 280                 285

Trp Met Gly Trp Met Asn Pro Asn Asn Gly Tyr Thr Gly Tyr Ala Gln
    290                 295                 300

Lys Phe Gln Gly Arg Val Thr Met Thr Arg Asn Thr Ser Ile Ser Thr
305                 310                 315                 320

Ala Tyr Met Glu Leu Ser Ser Leu Arg Ser Glu Asp Thr Ala Val Tyr
```

325 330 335

Tyr Cys Ala Arg Thr Asn Trp Val Tyr Trp Tyr Phe Asp Leu Trp Gly
340 345 350

Arg Gly Thr Thr Val Thr Val Ser Ser
355 360

<210> 107
<211> 361
<212> PRT
<213> Artificial

<220>
<223> Description of the artificial sequence; amino acid sequence of E11_VH-CH1-PL223_VH

<400> 107

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val
115 120 125

Ala Glu Phe Asp Tyr Trp Gly Gln Gly Thr Leu Val Thr Val Ser Ser
130 135 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr

165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Lys Thr
210 215 220

Tyr Thr Cys Asn Val Asp His Lys Pro Ser Asn Thr Lys Val Asp Lys
225 230 235 240

Arg Val Glu Val Gln Leu Val Gln Ser Gly Ala Glu Val Lys Lys Pro
245 250 255

Gly Ala Ser Val Lys Val Ser Cys Lys Ala Ser Gly Tyr Thr Phe Thr
260 265 270

Thr Tyr Asp Ile Asn Trp Val Arg Gln Ala Thr Gly Gln Gly Leu Glu
275 280 285

Trp Met Gly Trp Met Asn Pro Asn Asn Gly Tyr Thr Gly Tyr Ala Gln
290 295 300

Lys Phe Gln Gly Arg Val Thr Met Thr Arg Asn Thr Ser Ile Ser Thr
305 310 315 320

Ala Tyr Met Glu Leu Ser Ser Leu Arg Ser Glu Asp Thr Ala Val Tyr
325 330 335

Tyr Cys Ala Arg Thr Asn Trp Val Tyr Trp Tyr Phe Asp Leu Trp Gly
340 345 350

Arg Gly Thr Met Val Thr Val Ser Ser
355 360

<210> 108
<211> 361
<212> PRT
<213> Artificial

<220>
<223> Description of the artificial sequence; amino acid sequence of E11_VH-CH1-PN7_VH

<400> 108

Met Asp Leu Met Cys Lys Lys Met Lys His Leu Trp Phe Phe Leu Leu

1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val
115 120 125

Ala Glu Phe Asp Tyr Trp Gly Gln Gly Thr Leu Val Thr Val Ser Ser
130 135 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Lys Thr
210 215 220

Tyr Thr Cys Asn Val Asp His Lys Pro Ser Asn Thr Lys Val Asp Lys
225 230 235 240

Arg Val Glu Val Gln Leu Val Gln Ser Gly Ala Glu Val Lys Lys Pro
245 250 255

Gly Ala Ser Val Lys Val Ser Cys Lys Ala Ser Gly Tyr Thr Phe Thr
260 265 270

Thr Tyr Asp Ile Asn Trp Val Arg Gln Ala Thr Gly Gln Gly Leu Glu
275 280 285

Trp Met Gly Trp Met Asn Pro Asn Ser Gly Tyr Thr Gly Tyr Ala Gln
290 295 300

Lys Phe Gln Gly Arg Val Thr Met Thr Arg Asp Thr Ser Ile Ser Thr
305 310 315 320

Ala Tyr Met Glu Leu Ser Ser Leu Arg Ser Glu Asp Thr Ala Val Tyr
325 330 335

Tyr Cys Ala Arg Thr Asn Trp Glu Ser Trp Tyr Phe Asp Leu Trp Gly
340 345 350

Arg Gly Thr Leu Val Thr Val Ser Ser
355 360

<210> 109
<211> 363
<212> PRT
<213> Artificial

<220>
<223> Description of the artificial sequence; amino acid sequence of E11_VH-CH1-PN170_VH

<400> 109

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val
115 120 125

Ala Glu Phe Asp Tyr Trp Gly Gln Gly Thr Leu Val Thr Val Ser Ser
130 135 140

Ala Ser Thr Lys Gly Pro Ser Val Phe Pro Leu Ala Pro Cys Ser Arg
145 150 155 160

Ser Thr Ser Glu Ser Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Lys Thr
210 215 220

Tyr Thr Cys Asn Val Asp His Lys Pro Ser Asn Thr Lys Val Asp Lys
225 230 235 240

Arg Val Glu Val Gln Leu Val Gln Ser Gly Ala Glu Val Lys Lys Pro
245 250 255

Gly Ala Ser Val Lys Val Ser Cys Lys Ala Ser Gly Tyr Thr Leu Thr
260 265 270

Ser Tyr Asp Ile Asn Trp Val Arg Gln Ala Thr Gly Gln Gly Leu Glu
275 280 285

Trp Met Gly Trp Met Asn Pro Asn Ser Gly Tyr Thr Gly Tyr Ala Gln
290 295 300

Lys Phe Gln Asp Arg Val Thr Met Thr Arg Asn Thr Ala Ile Asn Thr
305 310 315 320

Ala Tyr Met Glu Leu Ser Ser Leu Arg Ser Glu Asp Thr Ala Val Tyr
325 330 335

Tyr Cys Ala Arg Glu Ala Gly Tyr Asp Gly Ile Trp Tyr Phe Asp Leu
340 345 350

```
Trp Gly Arg Gly Thr Leu Val Thr Val Ser Ser
        355                 360


<210>  110
<211>  277
<212>  PRT
<213>  Artificial

<220>
<223>  Description of the artificial sequence; amino acid sequence of
       E11_VH-GL-PI134_VH

<400>  110

Met Asp Leu Met Cys Lys Lys Met Lys His Leu Trp Phe Phe Leu Leu
1               5                   10                  15


Leu Val Ala Ala Pro Arg Trp Val Leu Ser Gln Leu Gln Leu Gln Glu
            20                  25                  30


Ser Gly Pro Gly Leu Val Lys Pro Ser Glu Thr Leu Ser Leu Thr Cys
        35                  40                  45


Thr Val Ser Gly Gly Ser Ile Ile Ser Lys Ser Ser Tyr Trp Gly Trp
    50                  55                  60


Ile Arg Gln Pro Pro Gly Lys Gly Leu Glu Trp Ile Gly Ser Ile Tyr
65                  70                  75                  80


Tyr Ser Gly Ser Thr Phe Tyr Asn Pro Ser Leu Lys Ser Arg Val Thr
                85                  90                  95


Ile Ser Val Asp Thr Ser Lys Asn Gln Phe Ser Leu Lys Leu Ser Ser
            100                 105                 110


Val Thr Ala Ala Asp Thr Ala Val Tyr Tyr Cys Ala Arg Leu Thr Val
        115                 120                 125


Ala Glu Phe Asp Tyr Trp Gly Gln Gly Thr Leu Val Thr Val Ser Ser
    130                 135                 140


Ala Ser Thr Lys Gly Pro Ser Val Phe Pro Leu Ala Pro Cys Gln Val
145                 150                 155                 160


Gln Leu Val Gln Ser Gly Ala Glu Val Lys Lys Pro Gly Ala Ser Val
                165                 170                 175


Lys Val Ser Cys Lys Ala Ser Gly Tyr Thr Phe Thr Thr Tyr Asp Ile
            180                 185                 190
```

Asn Trp Val Arg Gln Ala Thr Gly Gln Gly Leu Glu Trp Met Gly Trp
195 200 205

Met Asn Pro Asn Asn Gly Tyr Thr Gly Tyr Ala Gln Lys Phe Gln Gly
210 215 220

Arg Val Thr Met Thr Arg Asn Thr Ser Ile Ser Thr Ala Tyr Met Glu
225 230 235 240

Leu Ser Ser Leu Arg Ser Glu Asp Thr Ala Val Tyr Tyr Cys Ala Arg
245 250 255

Thr Asn Trp Val Tyr Trp Tyr Phe Asp Leu Trp Gly Arg Gly Thr Leu
260 265 270

Val Thr Val Ser Ser
275

<210> 111
<211> 277
<212> PRT
<213> Artificial

<220>
<223> Description of the artificial sequence; amino acid sequence of E11_VH-ML-PI101_VH

<400> 111

Met Asp Leu Met Cys Lys Lys Met Lys His Leu Trp Phe Phe Leu Leu
1 5 10 15

Leu Val Ala Ala Pro Arg Trp Val Leu Ser Gln Leu Gln Leu Gln Glu
20 25 30

Ser Gly Pro Gly Leu Val Lys Pro Ser Glu Thr Leu Ser Leu Thr Cys
35 40 45

Thr Val Ser Gly Gly Ser Ile Ile Ser Lys Ser Ser Tyr Trp Gly Trp
50 55 60

Ile Arg Gln Pro Pro Gly Lys Gly Leu Glu Trp Ile Gly Ser Ile Tyr
65 70 75 80

Tyr Ser Gly Ser Thr Phe Tyr Asn Pro Ser Leu Lys Ser Arg Val Thr
85 90 95

Ile Ser Val Asp Thr Ser Lys Asn Gln Phe Ser Leu Lys Leu Ser Ser
100 105 110

Val Thr Ala Ala Asp Thr Ala Val Tyr Tyr Cys Ala Arg Leu Thr Val
115 120 125

Ala Glu Phe Asp Tyr Trp Gly Gln Gly Thr Leu Val Thr Val Ser Ser
130 135 140

Gly Ser Ala Ser Ala Pro Thr Leu Phe Pro Leu Val Ser Cys Glu Val
145 150 155 160

Gln Leu Val Gln Ser Gly Ala Glu Val Lys Lys Pro Gly Ala Ser Val
165 170 175

Lys Val Ser Cys Lys Ala Ser Gly Tyr Asn Leu Ala Thr Tyr Asp Ile
180 185 190

Asn Trp Val Arg Gln Ala Thr Gly Gln Gly Leu Glu Trp Met Gly Trp
195 200 205

Met Asn Pro Asn Ser Gly Tyr Thr Gly Tyr Ala Gln Lys Phe Gln Gly
210 215 220

Arg Val Thr Met Thr Arg Asp Thr Ser Ile Asn Thr Ala Tyr Met Glu
225 230 235 240

Leu Ser Ser Leu Arg Ser Glu Asp Thr Ala Val Tyr Tyr Cys Ala Arg
245 250 255

Thr Asn Trp Val Tyr Trp Tyr Phe Asp Leu Trp Gly Arg Gly Thr Leu
260 265 270

Val Thr Val Ser Ser
275

<210> 112
<211> 750
<212> PRT
<213> Homo sapiens

<400> 112

Met Trp Asn Leu Leu His Glu Thr Asp Ser Ala Val Ala Thr Ala Arg
1 5 10 15

Arg Pro Arg Trp Leu Cys Ala Gly Ala Leu Val Leu Ala Gly Gly Phe
20 25 30

Phe Leu Leu Gly Phe Leu Phe Gly Trp Phe Ile Lys Ser Ser Asn Glu
35 40 45

```
Ala Thr Asn Ile Thr Pro Lys His Asn Met Lys Ala Phe Leu Asp Glu
    50                  55                  60

Leu Lys Ala Glu Asn Ile Lys Lys Phe Leu Tyr Asn Phe Thr Gln Ile
65                  70                  75                  80

Pro His Leu Ala Gly Thr Glu Gln Asn Phe Gln Leu Ala Lys Gln Ile
                85                  90                  95

Gln Ser Gln Trp Lys Glu Phe Gly Leu Asp Ser Val Glu Leu Ala His
            100                 105                 110

Tyr Asp Val Leu Leu Ser Tyr Pro Asn Lys Thr His Pro Asn Tyr Ile
        115                 120                 125

Ser Ile Ile Asn Glu Asp Gly Asn Glu Ile Phe Asn Thr Ser Leu Phe
    130                 135                 140

Glu Pro Pro Pro Pro Gly Tyr Glu Asn Val Ser Asp Ile Val Pro Pro
145                 150                 155                 160

Phe Ser Ala Phe Ser Pro Gln Gly Met Pro Glu Gly Asp Leu Val Tyr
                165                 170                 175

Val Asn Tyr Ala Arg Thr Glu Asp Phe Phe Lys Leu Glu Arg Asp Met
            180                 185                 190

Lys Ile Asn Cys Ser Gly Lys Ile Val Ile Ala Arg Tyr Gly Lys Val
        195                 200                 205

Phe Arg Gly Asn Lys Val Lys Asn Ala Gln Leu Ala Gly Ala Lys Gly
    210                 215                 220

Val Ile Leu Tyr Ser Asp Pro Ala Asp Tyr Phe Ala Pro Gly Val Lys
225                 230                 235                 240

Ser Tyr Pro Asp Gly Trp Asn Leu Pro Gly Gly Gly Val Gln Arg Gly
                245                 250                 255

Asn Ile Leu Asn Leu Asn Gly Ala Gly Asp Pro Leu Thr Pro Gly Tyr
            260                 265                 270

Pro Ala Asn Glu Tyr Ala Tyr Arg Arg Gly Ile Ala Glu Ala Val Gly
        275                 280                 285

Leu Pro Ser Ile Pro Val His Pro Ile Gly Tyr Tyr Asp Ala Gln Lys
    290                 295                 300
```

Leu Leu Glu Lys Met Gly Gly Ser Ala Pro Pro Asp Ser Ser Trp Arg
305                 310                 315                 320

Gly Ser Leu Lys Val Pro Tyr Asn Val Gly Pro Gly Phe Thr Gly Asn
                325                 330                 335

Phe Ser Thr Gln Lys Val Lys Met His Ile His Ser Thr Asn Glu Val
            340                 345                 350

Thr Arg Ile Tyr Asn Val Ile Gly Thr Leu Arg Gly Ala Val Glu Pro
        355                 360                 365

Asp Arg Tyr Val Ile Leu Gly Gly His Arg Asp Ser Trp Val Phe Gly
    370                 375                 380

Gly Ile Asp Pro Gln Ser Gly Ala Ala Val Val His Glu Ile Val Arg
385                 390                 395                 400

Ser Phe Gly Thr Leu Lys Lys Glu Gly Trp Arg Pro Arg Arg Thr Ile
                405                 410                 415

Leu Phe Ala Ser Trp Asp Ala Glu Glu Phe Gly Leu Leu Gly Ser Thr
            420                 425                 430

Glu Trp Ala Glu Glu Asn Ser Arg Leu Leu Gln Glu Arg Gly Val Ala
        435                 440                 445

Tyr Ile Asn Ala Asp Ser Ser Ile Glu Gly Asn Tyr Thr Leu Arg Val
    450                 455                 460

Asp Cys Thr Pro Leu Met Tyr Ser Leu Val His Asn Leu Thr Lys Glu
465                 470                 475                 480

Leu Lys Ser Pro Asp Glu Gly Phe Glu Gly Lys Ser Leu Tyr Glu Ser
                485                 490                 495

Trp Thr Lys Lys Ser Pro Ser Pro Glu Phe Ser Gly Met Pro Arg Ile
            500                 505                 510

Ser Lys Leu Gly Ser Gly Asn Asp Phe Glu Val Phe Phe Gln Arg Leu
        515                 520                 525

Gly Ile Ala Ser Gly Arg Ala Arg Tyr Thr Lys Asn Trp Glu Thr Asn
    530                 535                 540

Lys Phe Ser Gly Tyr Pro Leu Tyr His Ser Val Tyr Glu Thr Tyr Glu

545 550 555 560

Leu Val Glu Lys Phe Tyr Asp Pro Met Phe Lys Tyr His Leu Thr Val
565 570 575

Ala Gln Val Arg Gly Gly Met Val Phe Glu Leu Ala Asn Ser Ile Val
580 585 590

Leu Pro Phe Asp Cys Arg Asp Tyr Ala Val Val Leu Arg Lys Tyr Ala
595 600 605

Asp Lys Ile Tyr Ser Ile Ser Met Lys His Pro Gln Glu Met Lys Thr
610 615 620

Tyr Ser Val Ser Phe Asp Ser Leu Phe Ser Ala Val Lys Asn Phe Thr
625 630 635 640

Glu Ile Ala Ser Lys Phe Ser Glu Arg Leu Gln Asp Phe Asp Lys Ser
645 650 655

Asn Pro Ile Val Leu Arg Met Met Asn Asp Gln Leu Met Phe Leu Glu
660 665 670

Arg Ala Phe Ile Asp Pro Leu Gly Leu Pro Asp Arg Pro Phe Tyr Arg
675 680 685

His Val Ile Tyr Ala Pro Ser Ser His Asn Lys Tyr Ala Gly Glu Ser
690 695 700

Phe Pro Gly Ile Tyr Asp Ala Leu Phe Asp Ile Glu Ser Lys Val Asp
705 710 715 720

Pro Ser Lys Ala Trp Gly Glu Val Lys Arg Gln Ile Tyr Val Ala Ala
725 730 735

Phe Thr Val Gln Ala Ala Ala Glu Thr Leu Ser Glu Val Ala
740 745 750

<210> 113
<211> 737
<212> PRT
<213> Artificial

<220>
<223> Description of the artificial sequence; amino acid sequence of N_FLAG-hPSMA_ECD

<400> 113

Met Arg Val Pro Ala Gln Leu Leu Gly Leu Leu Leu Leu Trp Leu Pro

```
1               5                   10                  15

Gly Ala Arg Cys Asp Tyr Lys Asp Asp Asp Asp Lys Thr Ser Lys Ser
            20                  25                  30

Ser Asn Glu Ala Thr Asn Ile Thr Pro Lys His Asn Met Lys Ala Phe
        35                  40                  45

Leu Asp Glu Leu Lys Ala Glu Asn Ile Lys Lys Phe Leu Tyr Asn Phe
    50                  55                  60

Thr Gln Ile Pro His Leu Ala Gly Thr Glu Gln Asn Phe Gln Leu Ala
65                  70                  75                  80

Lys Gln Ile Gln Ser Gln Trp Lys Glu Phe Gly Leu Asp Ser Val Glu
                85                  90                  95

Leu Ala His Tyr Asp Val Leu Leu Ser Tyr Pro Asn Lys Thr His Pro
            100                 105                 110

Asn Tyr Ile Ser Ile Ile Asn Glu Asp Gly Asn Glu Ile Phe Asn Thr
        115                 120                 125

Ser Leu Phe Glu Pro Pro Pro Pro Gly Tyr Glu Asn Val Ser Asp Ile
    130                 135                 140

Val Pro Pro Phe Ser Ala Phe Ser Pro Gln Gly Met Pro Glu Gly Asp
145                 150                 155                 160

Leu Val Tyr Val Asn Tyr Ala Arg Thr Glu Asp Phe Phe Lys Leu Glu
                165                 170                 175

Arg Asp Met Lys Ile Asn Cys Ser Gly Lys Ile Val Ile Ala Arg Tyr
            180                 185                 190

Gly Lys Val Phe Arg Gly Asn Lys Val Lys Asn Ala Gln Leu Ala Gly
        195                 200                 205

Ala Lys Gly Val Ile Leu Tyr Ser Asp Pro Ala Asp Tyr Phe Ala Pro
    210                 215                 220

Gly Val Lys Ser Tyr Pro Asp Gly Trp Asn Leu Pro Gly Gly Gly Val
225                 230                 235                 240

Gln Arg Gly Asn Ile Leu Asn Leu Asn Gly Ala Gly Asp Pro Leu Thr
                245                 250                 255
```

```
Pro Gly Tyr Pro Ala Asn Glu Tyr Ala Tyr Arg Arg Gly Ile Ala Glu
            260                 265                 270

Ala Val Gly Leu Pro Ser Ile Pro Val His Pro Ile Gly Tyr Tyr Asp
        275                 280                 285

Ala Gln Lys Leu Leu Glu Lys Met Gly Gly Ser Ala Pro Pro Asp Ser
    290                 295                 300

Ser Trp Arg Gly Ser Leu Lys Val Pro Tyr Asn Val Gly Pro Gly Phe
305                 310                 315                 320

Thr Gly Asn Phe Ser Thr Gln Lys Val Lys Met His Ile His Ser Thr
                325                 330                 335

Asn Glu Val Thr Arg Ile Tyr Asn Val Ile Gly Thr Leu Arg Gly Ala
            340                 345                 350

Val Glu Pro Asp Arg Tyr Val Ile Leu Gly Gly His Arg Asp Ser Trp
        355                 360                 365

Val Phe Gly Gly Ile Asp Pro Gln Ser Gly Ala Ala Val Val His Glu
    370                 375                 380

Ile Val Arg Ser Phe Gly Thr Leu Lys Lys Glu Gly Trp Arg Pro Arg
385                 390                 395                 400

Arg Thr Ile Leu Phe Ala Ser Trp Asp Ala Glu Glu Phe Gly Leu Leu
                405                 410                 415

Gly Ser Thr Glu Trp Ala Glu Glu Asn Ser Arg Leu Leu Gln Glu Arg
            420                 425                 430

Gly Val Ala Tyr Ile Asn Ala Asp Ser Ser Ile Glu Gly Asn Tyr Thr
        435                 440                 445

Leu Arg Val Asp Cys Thr Pro Leu Met Tyr Ser Leu Val His Asn Leu
    450                 455                 460

Thr Lys Glu Leu Lys Ser Pro Asp Glu Gly Phe Glu Gly Lys Ser Leu
465                 470                 475                 480

Tyr Glu Ser Trp Thr Lys Lys Ser Pro Ser Pro Glu Phe Ser Gly Met
                485                 490                 495

Pro Arg Ile Ser Lys Leu Gly Ser Gly Asn Asp Phe Glu Val Phe Phe
            500                 505                 510
```

```
Gln Arg Leu Gly Ile Ala Ser Gly Arg Ala Arg Tyr Thr Lys Asn Trp
        515                 520                 525

Glu Thr Asn Lys Phe Ser Gly Tyr Pro Leu Tyr His Ser Val Tyr Glu
    530                 535                 540

Thr Tyr Glu Leu Val Glu Lys Phe Tyr Asp Pro Met Phe Lys Tyr His
545                 550                 555                 560

Leu Thr Val Ala Gln Val Arg Gly Gly Met Val Phe Glu Leu Ala Asn
                565                 570                 575

Ser Ile Val Leu Pro Phe Asp Cys Arg Asp Tyr Ala Val Val Leu Arg
            580                 585                 590

Lys Tyr Ala Asp Lys Ile Tyr Ser Ile Ser Met Lys His Pro Gln Glu
        595                 600                 605

Met Lys Thr Tyr Ser Val Ser Phe Asp Ser Leu Phe Ser Ala Val Lys
    610                 615                 620

Asn Phe Thr Glu Ile Ala Ser Lys Phe Ser Glu Arg Leu Gln Asp Phe
625                 630                 635                 640

Asp Lys Ser Asn Pro Ile Val Leu Arg Met Met Asn Asp Gln Leu Met
                645                 650                 655

Phe Leu Glu Arg Ala Phe Ile Asp Pro Leu Gly Leu Pro Asp Arg Pro
            660                 665                 670

Phe Tyr Arg His Val Ile Tyr Ala Pro Ser Ser His Asn Lys Tyr Ala
        675                 680                 685

Gly Glu Ser Phe Pro Gly Ile Tyr Asp Ala Leu Phe Asp Ile Glu Ser
    690                 695                 700

Lys Val Asp Pro Ser Lys Ala Trp Gly Glu Val Lys Arg Gln Ile Tyr
705                 710                 715                 720

Val Ala Ala Phe Thr Val Gln Ala Ala Ala Glu Thr Leu Ser Glu Val
                725                 730                 735

Ala


<210>  114
<211>  126
```

<212> PRT
<213> Artificial

<220>
<223> Description of the artificial sequence; amino acid sequence of KMDA2_VH excluding signal sequence

<400> 114

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Lys Ile Asn Trp Val Arg Gln Ala Thr Gly Gln Gly Leu Glu Trp Met
        35                  40                  45

Gly Trp Met Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Tyr Gly Ser Gly Ser Tyr Tyr Arg Asp Tyr Tyr Tyr Gly
            100                 105                 110

Met Asp Val Trp Gly Gln Gly Thr Thr Val Thr Val Ser Ser
        115                 120                 125

<210> 115
<211> 107
<212> PRT
<213> Artificial

<220>
<223> Description of the artificial sequence; amino acid sequence of KMDA2_VL excluding signal sequence

<400> 115

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Leu
                85                  90                  95

Thr Phe Gly Gly Gly Thr Lys Val Glu Ile Lys
            100                 105

<210> 116
<211> 119
<212> PRT
<213> Artificial

<220>
<223> Description of the artificial sequence; amino acid sequence of 2A10_VH excluding signal sequence

<400> 116

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Asn
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gln Thr Gly Phe Leu Trp Ser Ser Asp Leu Trp Gly Arg Gly
            100                 105                 110

Thr Leu Val Thr Val Ser Ser
        115

<210> 117
<211> 106
<212> PRT

<213> Artificial

<220>
<223> Description of the artificial sequence; amino acid sequence of 2A10_VL excluding signal sequence

<400> 117

```
Ile Gln Leu Thr Gln Ser Pro Ser Ser Leu Ser Ala Ser Val Gly Asp
1               5                   10                  15

Arg Val Thr Ile Thr Cys Arg Ala Ser Gln Asp Ile Ser Ser Ala Leu
            20                  25                  30

Ala Trp Tyr Gln Gln Lys Pro Gly Lys Ala Pro Lys Leu Leu Ile Tyr
        35                  40                  45

Asp Ala Ser Ser Leu Glu Ser Gly Val Pro Ser Arg Phe Ser Gly Tyr
    50                  55                  60

Gly Ser Gly Thr Asp Phe Thr Leu Thr Ile Asn Ser Leu Gln Pro Glu
65                  70                  75                  80

Asp Phe Ala Thr Tyr Tyr Cys Gln Gln Phe Asn Ser Tyr Pro Leu Thr
                85                  90                  95

Phe Gly Gly Gly Thr Lys Val Glu Ile Lys
            100                 105
```

<210> 118
<211> 118
<212> PRT
<213> Artificial

<220>
<223> Description of the artificial sequence; amino acid sequence of E11_VH excluding signal sequence

<400> 118

```
Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ile Ser Lys
            20                  25                  30

Ser Ser Tyr Trp Gly Trp Ile Arg Gln Pro Pro Gly Lys Gly Leu Glu
        35                  40                  45

Trp Ile Gly Ser Ile Tyr Tyr Ser Gly Ser Thr Phe Tyr Asn Pro Ser
    50                  55                  60
```

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser
115

<210> 119
<211> 108
<212> PRT
<213> Artificial

<220>
<223> Description of the artificial sequence; amino acid sequence of E11_VL excluding signal sequence

<400> 119

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Phe
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Leu
85 90 95

Thr Phe Gly Pro Gly Thr Lys Val Asp Ile Lys Arg
100 105

<210> 120
<211> 119
<212> PRT
<213> Artificial

<220>
<223> Description of the artificial sequence; amino acid sequence of PI134_VH excluding signal sequence

<400>  120

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Tyr
            20                  25                  30

Asp Ile Asn Trp Val Arg Gln Ala Thr Gly Gln Gly Leu Glu Trp Met
        35                  40                  45

Gly Trp Met Asn Pro Asn Asn Gly Tyr Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Thr Asn Trp Val Tyr Trp Tyr Phe Asp Leu Trp Gly Arg Gly
            100                 105                 110

Thr Leu Val Thr Val Ser Ser
        115

<210>  121
<211>  119
<212>  PRT
<213>  Artificial

<220>
<223>  Description of the artificial sequence; amino acid sequence of PI101_VH excluding signal sequence

<400>  121

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Asn Leu Ala Thr Tyr
            20                  25                  30

Asp Ile Asn Trp Val Arg Gln Ala Thr Gly Gln Gly Leu Glu Trp Met
        35                  40                  45

Gly Trp Met Asn Pro Asn Ser Gly Tyr Thr Gly Tyr Ala Gln Lys Phe
    50                  55                  60

Gln Gly Arg Val Thr Met Thr Arg Asp Thr Ser Ile Asn Thr Ala Tyr

65 70 75 80

Met Glu Leu Ser Ser Leu Arg Ser Glu Asp Thr Ala Val Tyr Tyr Cys
85 90 95

Ala Arg Thr Asn Trp Val Tyr Trp Tyr Phe Asp Leu Trp Gly Arg Gly
100 105 110

Thr Leu Val Thr Val Ser Ser
115

<210> 122
<211> 119
<212> PRT
<213> Artificial

<220>
<223> Description of the artificial sequence; amino acid sequence of PN7_VH excluding signal sequence

<400> 122

Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Thr Tyr
20 25 30

Asp Ile Asn Trp Val Arg Gln Ala Thr Gly Gln Gly Leu Glu Trp Met
35 40 45

Gly Trp Met Asn Pro Asn Ser Gly Tyr Thr Gly Tyr Ala Gln Lys Phe
50 55 60

Gln Gly Arg Val Thr Met Thr Arg Asp Thr Ser Ile Ser Thr Ala Tyr
65 70 75 80

Met Glu Leu Ser Ser Leu Arg Ser Glu Asp Thr Ala Val Tyr Tyr Cys
85 90 95

Ala Arg Thr Asn Trp Glu Ser Trp Tyr Phe Asp Leu Trp Gly Arg Gly
100 105 110

Thr Leu Val Thr Val Ser Ser
115

<210> 123
<211> 119
<212> PRT
<213> Artificial

<220>
<223> Description of the artificial sequence; amino acid sequence of PI105_VH excluding signal sequence

<400> 123

Glu Val Gln Leu Val Gln Ser Gly Ala Glu Val Lys Lys Pro Gly Ala
1 5 10 15

Ser Val Lys Val Ser Cys Lys Ala Ser Gly Tyr Asn Leu Ala Thr Tyr
20 25 30

Asp Ile Asn Trp Val Arg Gln Ala Thr Gly Gln Gly Leu Glu Trp Met
35 40 45

Gly Trp Met Asn Pro Asn Ser Gly Tyr Thr Gly Tyr Ala Gln Lys Phe
50 55 60

Gln Gly Arg Val Thr Met Thr Arg Asp Thr Ser Ile Asn Thr Ala Tyr
65 70 75 80

Met Glu Leu Ser Ser Leu Arg Ser Glu Asp Thr Ala Val Tyr Tyr Cys
85 90 95

Ala Arg Thr Asn Trp Val Tyr Trp Tyr Phe Asp Leu Trp Gly Arg Gly
100 105 110

Thr Leu Val Thr Val Ser Ser
115

<210> 124
<211> 119
<212> PRT
<213> Artificial

<220>
<223> Description of the artificial sequence; amino acid sequence of PI108_VH excluding signal sequence

<400> 124

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Thr Tyr
20 25 30

Asp Ile Asn Trp Val Arg Gln Ala Thr Gly Gln Gly Leu Glu Trp Met
35 40 45

Gly Trp Met Asn Pro Asn Asn Gly Tyr Thr Gly Tyr Ala Gln Lys Phe
50 55 60

```
Gln Gly Arg Val Thr Met Thr Arg Asn Thr Ser Ile Ser Thr Ala Tyr
65                  70                  75                  80

Met Glu Leu Thr Ser Leu Arg Ser Glu Asp Thr Ala Val Tyr Tyr Cys
                85                  90                  95

Ala Arg Thr Asn Trp Val Tyr Trp Tyr Phe Asp Leu Trp Gly Arg Gly
            100                 105                 110

Thr Leu Val Thr Val Ser Ser
        115
```

<210> 125
<211> 119
<212> PRT
<213> Artificial

<220>
<223> Description of the artificial sequence; amino acid sequence of PI115_VH excluding signal sequence

<400> 125

```
Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Tyr
            20                  25                  30

Asp Ile Asn Trp Val Arg Gln Ala Thr Gly Gln Gly Leu Glu Trp Met
        35                  40                  45

Gly Trp Met Asn Pro Asn Asn Gly Tyr Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Thr Asn Trp Val Tyr Trp Tyr Phe Asp Leu Trp Gly Arg Gly
            100                 105                 110

Thr Leu Val Thr Val Ser Ser
        115
```

<210> 126
<211> 119

<212> PRT
<213> Artificial

<220>
<223> Description of the artificial sequence; amino acid sequence of PI118_VH excluding signal sequence

<400> 126

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Asn Leu Thr Thr Tyr
            20                  25                  30

Asp Ile Asn Trp Val Arg Gln Ala Thr Gly Gln Gly Leu Glu Trp Met
        35                  40                  45

Gly Trp Met Asn Pro Asn Ser Gly Tyr Thr Gly Tyr Ala Gln Lys Phe
    50                  55                  60

Gln Gly Arg Val Thr Met Thr Arg Asn Thr Ser Ile Asp Thr Ala Tyr
65                  70                  75                  80

Met Glu Leu Ser Asn Leu Arg Ser Glu Asp Thr Ala Val Tyr Tyr Cys
                85                  90                  95

Ala Arg Thr Asn Trp Val Tyr Trp Tyr Phe Asp Leu Trp Gly Arg Gly
            100                 105                 110

Thr Leu Val Thr Val Ser Ser
        115

<210> 127
<211> 119
<212> PRT
<213> Artificial

<220>
<223> Description of the artificial sequence; amino acid sequence of PI127_VH excluding signal sequence

<400> 127

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Tyr
            20                  25                  30

Asp Ile Asn Trp Val Arg Gln Ala Thr Gly Gln Gly Leu Glu Trp Leu
        35                  40                  45

Gly Trp Met Asn Pro Asn Asn Gly Tyr Thr Gly Tyr Ala Gln Lys Phe
50 55 60

Gln Gly Arg Val Thr Met Thr Arg Asn Thr Ser Ile Gly Thr Ala Tyr
65 70 75 80

Met Glu Leu Asn Ser Leu Arg Ser Glu Asp Thr Ala Val Tyr Tyr Cys
85 90 95

Ala Arg Thr Asn Trp Val Tyr Trp Tyr Phe Asp Leu Trp Gly Arg Gly
100 105 110

Thr Leu Val Thr Val Ser Ser
115

<210> 128
<211> 119
<212> PRT
<213> Artificial

<220>
<223> Description of the artificial sequence; amino acid sequence of PI143_VH excluding signal sequence

<400> 128

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Thr Tyr
20 25 30

Asp Ile Asn Trp Val Arg Gln Ala Thr Gly Gln Gly Leu Glu Trp Met
35 40 45

Gly Trp Met Asn Pro Asn Asn Gly Tyr Thr Gly Tyr Ala Gln Lys Phe
50 55 60

Gln Gly Arg Val Thr Met Thr Arg Asn Thr Ser Ile Ser Thr Ala Tyr
65 70 75 80

Met Glu Leu Ser Ser Leu Arg Ser Glu Asp Thr Ala Val Tyr Tyr Cys
85 90 95

Ala Arg Thr Asn Trp Val Tyr Trp Tyr Phe Asp Leu Trp Gly Arg Gly
100 105 110

Thr Thr Val Thr Val Ser Ser
115

<210> 129
<211> 119
<212> PRT
<213> Artificial

<220>
<223> Description of the artificial sequence; amino acid sequence of PL223_VH excluding signal sequence

<400> 129

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Tyr
            20                  25                  30

Asp Ile Asn Trp Val Arg Gln Ala Thr Gly Gln Gly Leu Glu Trp Met
        35                  40                  45

Gly Trp Met Asn Pro Asn Asn Gly Tyr Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Thr Asn Trp Val Tyr Trp Tyr Phe Asp Leu Trp Gly Arg Gly
            100                 105                 110

Thr Met Val Thr Val Ser Ser
        115

<210> 130
<211> 121
<212> PRT
<213> Artificial

<220>
<223> Description of the artificial sequence; amino acid sequence of PN170_VH excluding signal sequence

<400> 130

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Leu Thr Ser Tyr
            20                  25                  30

Asp Ile Asn Trp Val Arg Gln Ala Thr Gly Gln Gly Leu Glu Trp Met
35 40 45

Gly Trp Met Asn Pro Asn Ser Gly Tyr Thr Gly Tyr Ala Gln Lys Phe
50 55 60

Gln Asp Arg Val Thr Met Thr Arg Asn Thr Ala Ile Asn Thr Ala Tyr
65 70 75 80

Met Glu Leu Ser Ser Leu Arg Ser Glu Asp Thr Ala Val Tyr Tyr Cys
85 90 95

Ala Arg Glu Ala Gly Tyr Asp Gly Ile Trp Tyr Phe Asp Leu Trp Gly
100 105 110

Arg Gly Thr Leu Val Thr Val Ser Ser
115 120

<210> 131
<211> 335
<212> PRT
<213> Artificial

<220>
<223> Description of the artificial sequence; amino acid sequence of E11_VH-CH1-PI134_VH excluding signal sequence

<400> 131

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

```
Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Gln Val Gln Leu Val Gln Ser Gly
    210                 215                 220

Ala Glu Val Lys Lys Pro Gly Ala Ser Val Lys Val Ser Cys Lys Ala
225                 230                 235                 240

Ser Gly Tyr Thr Phe Thr Thr Tyr Asp Ile Asn Trp Val Arg Gln Ala
                245                 250                 255

Thr Gly Gln Gly Leu Glu Trp Met Gly Trp Met Asn Pro Asn Asn Gly
            260                 265                 270

Tyr Thr Gly Tyr Ala Gln Lys Phe Gln Gly Arg Val Thr Met Thr Arg
        275                 280                 285

Asn Thr Ser Ile Ser Thr Ala Tyr Met Glu Leu Ser Ser Leu Arg Ser
    290                 295                 300

Glu Asp Thr Ala Val Tyr Tyr Cys Ala Arg Thr Asn Trp Val Tyr Trp
305                 310                 315                 320

Tyr Phe Asp Leu Trp Gly Arg Gly Thr Leu Val Thr Val Ser Ser
                325                 330                 335
```

<210> 132
<211> 335
<212> PRT
<213> Artificial

<220>
<223> Description of the artificial sequence; amino acid sequence of

E11_VH-CH1-PI101_VH excluding signal sequence

<400> 132

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser Ala Ser Thr Lys Gly Pro Ser Val Phe Pro
115 120 125

Leu Ala Pro Cys Ser Arg Ser Thr Ser Glu Ser Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Lys Thr Tyr Thr Cys Asn Val Asp His Lys Pro Ser
195 200 205

Asn Thr Lys Val Asp Lys Arg Val Glu Val Gln Leu Val Gln Ser Gly
210 215 220

Ala Glu Val Lys Lys Pro Gly Ala Ser Val Lys Val Ser Cys Lys Ala
225 230 235 240

Ser Gly Tyr Asn Leu Ala Thr Tyr Asp Ile Asn Trp Val Arg Gln Ala
245 250 255

Thr Gly Gln Gly Leu Glu Trp Met Gly Trp Met Asn Pro Asn Ser Gly
260 265 270

Tyr Thr Gly Tyr Ala Gln Lys Phe Gln Gly Arg Val Thr Met Thr Arg
275 280 285

Asp Thr Ser Ile Asn Thr Ala Tyr Met Glu Leu Ser Ser Leu Arg Ser
290 295 300

Glu Asp Thr Ala Val Tyr Tyr Cys Ala Arg Thr Asn Trp Val Tyr Trp
305 310 315 320

Tyr Phe Asp Leu Trp Gly Arg Gly Thr Leu Val Thr Val Ser Ser
325 330 335

<210> 133
<211> 335
<212> PRT
<213> Artificial

<220>
<223> Description of the artificial sequence; amino acid sequence of E11_VH-CH1-PI105_VH excluding signal sequence

<400> 133

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Val Gln Leu Val Gln Ser Gly
    210                 215                 220

Ala Glu Val Lys Lys Pro Gly Ala Ser Val Lys Val Ser Cys Lys Ala
225                 230                 235                 240

Ser Gly Tyr Thr Phe Thr Thr Tyr Asp Ile Asn Trp Val Arg Gln Ala
                245                 250                 255

Thr Gly Gln Gly Leu Glu Trp Leu Gly Trp Met Asn Pro Asn Asn Gly
            260                 265                 270

Tyr Thr Gly Tyr Ala Gln Lys Phe Gln Gly Arg Val Thr Met Thr Arg
        275                 280                 285

Asn Thr Ser Ile Gly Thr Ala Tyr Met Glu Leu Asn Ser Leu Arg Ser
    290                 295                 300

Glu Asp Thr Ala Val Tyr Tyr Cys Ala Arg Thr Asn Trp Val Tyr Trp
305                 310                 315                 320

Tyr Phe Asp Leu Trp Gly Arg Gly Thr Leu Val Thr Val Ser Ser
                325                 330                 335

<210>  134
<211>  335
<212>  PRT
<213>  Artificial

<220>

<223> Description of the artificial sequence; amino acid sequence of E11_VH-CH1-PI108_VH excluding signal sequence

<400> 134

```
Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ile Ser Lys
            20                  25                  30

Ser Ser Tyr Trp Gly Trp Ile Arg Gln Pro Pro Gly Lys Gly Leu Glu
        35                  40                  45

Trp Ile Gly Ser Ile Tyr Tyr Ser Gly Ser Thr Phe Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Leu Thr Val Ala Glu Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Gln Val Gln Leu Val Gln Ser Gly
    210                 215                 220

Ala Glu Val Lys Lys Pro Gly Ala Ser Val Lys Val Ser Cys Lys Ala
225                 230                 235                 240
```

Ser Gly Tyr Thr Phe Thr Thr Tyr Asp Ile Asn Trp Val Arg Gln Ala
245 250 255

Thr Gly Gln Gly Leu Glu Trp Met Gly Trp Met Asn Pro Asn Asn Gly
260 265 270

Tyr Thr Gly Tyr Ala Gln Lys Phe Gln Gly Arg Val Thr Met Thr Arg
275 280 285

Asn Thr Ser Ile Ser Thr Ala Tyr Met Glu Leu Thr Ser Leu Arg Ser
290 295 300

Glu Asp Thr Ala Val Tyr Tyr Cys Ala Arg Thr Asn Trp Val Tyr Trp
305 310 315 320

Tyr Phe Asp Leu Trp Gly Arg Gly Thr Leu Val Thr Val Ser Ser
325 330 335

<210> 135
<211> 335
<212> PRT
<213> Artificial

<220>
<223> Description of the artificial sequence; amino acid sequence of E11_VH-CH1-PI115_VH excluding signal sequence

<400> 135

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

```
Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Val Gln Leu Val Gln Ser Gly
    210                 215                 220

Ala Glu Val Lys Lys Pro Gly Ala Ser Val Lys Val Ser Cys Lys Ala
225                 230                 235                 240

Ser Gly Tyr Thr Phe Thr Thr Tyr Asp Ile Asn Trp Val Arg Gln Ala
                245                 250                 255

Thr Gly Gln Gly Leu Glu Trp Met Gly Trp Met Asn Pro Asn Asn Gly
            260                 265                 270

Tyr Thr Gly Tyr Ala Gln Lys Phe Gln Gly Arg Val Thr Met Thr Arg
        275                 280                 285

Asn Thr Ser Ile Ser Thr Ala Tyr Met Glu Leu Ser Ser Leu Arg Ser
    290                 295                 300

Glu Asp Thr Ala Val Tyr Tyr Cys Ala Arg Thr Asn Trp Val Tyr Trp
305                 310                 315                 320

Tyr Phe Asp Leu Trp Gly Arg Gly Thr Leu Val Thr Val Ser Ser
                325                 330                 335

<210>  136
<211>  335
<212>  PRT
<213>  Artificial
```

<220>
<223> Description of the artificial sequence; amino acid sequence of E11_VH-CH1-PI118_VH excluding signal sequence

<400> 136

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser Ala Ser Thr Lys Gly Pro Ser Val Phe Pro
115 120 125

Leu Ala Pro Cys Ser Arg Ser Thr Ser Glu Ser Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Lys Thr Tyr Thr Cys Asn Val Asp His Lys Pro Ser
195 200 205

Asn Thr Lys Val Asp Lys Arg Val Gln Val Gln Leu Val Gln Ser Gly
210 215 220

Ala Glu Val Lys Lys Pro Gly Ala Ser Val Lys Val Ser Cys Lys Ala

225 230 235 240

Ser Gly Tyr Asn Leu Thr Thr Tyr Asp Ile Asn Trp Val Arg Gln Ala
245 250 255

Thr Gly Gln Gly Leu Glu Trp Met Gly Trp Met Asn Pro Asn Ser Gly
260 265 270

Tyr Thr Gly Tyr Ala Gln Lys Phe Gln Gly Arg Val Thr Met Thr Arg
275 280 285

Asn Thr Ser Ile Asp Thr Ala Tyr Met Glu Leu Ser Asn Leu Arg Ser
290 295 300

Glu Asp Thr Ala Val Tyr Tyr Cys Ala Arg Thr Asn Trp Val Tyr Trp
305 310 315 320

Tyr Phe Asp Leu Trp Gly Arg Gly Thr Leu Val Thr Val Ser Ser
325 330 335

<210> 137
<211> 335
<212> PRT
<213> Artificial

<220>
<223> Description of the artificial sequence; amino acid sequence of E11_VH-CH1-PI127_VH excluding signal sequence

<400> 137

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr

```
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Gln Val Gln Leu Val Gln Ser Gly
    210                 215                 220

Ala Glu Val Lys Lys Pro Gly Ala Ser Val Lys Val Ser Cys Lys Ala
225                 230                 235                 240

Ser Gly Tyr Thr Phe Thr Thr Tyr Asp Ile Asn Trp Val Arg Gln Ala
                245                 250                 255

Thr Gly Gln Gly Leu Glu Trp Leu Gly Trp Met Asn Pro Asn Asn Gly
            260                 265                 270

Tyr Thr Gly Tyr Ala Gln Lys Phe Gln Gly Arg Val Thr Met Thr Arg
        275                 280                 285

Asn Thr Ser Ile Gly Thr Ala Tyr Met Glu Leu Asn Ser Leu Arg Ser
    290                 295                 300

Glu Asp Thr Ala Val Tyr Tyr Cys Ala Arg Thr Asn Trp Val Tyr Trp
305                 310                 315                 320

Tyr Phe Asp Leu Trp Gly Arg Gly Thr Leu Val Thr Val Ser Ser
                325                 330                 335
```

<210>  138
<211>  335
<212>  PRT
<213>  Artificial

<220>
<223> Description of the artificial sequence; amino acid sequence of E11_VH-CH1-PI143_VH excluding signal sequence

<400> 138

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser Ala Ser Thr Lys Gly Pro Ser Val Phe Pro
115 120 125

Leu Ala Pro Cys Ser Arg Ser Thr Ser Glu Ser Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Lys Thr Tyr Thr Cys Asn Val Asp His Lys Pro Ser
195 200 205

Asn Thr Lys Val Asp Lys Arg Val Gln Val Gln Leu Val Gln Ser Gly
210 215 220

Ala Glu Val Lys Lys Pro Gly Ala Ser Val Lys Val Ser Cys Lys Ala
225 230 235 240

Ser Gly Tyr Thr Phe Thr Thr Tyr Asp Ile Asn Trp Val Arg Gln Ala
245 250 255

Thr Gly Gln Gly Leu Glu Trp Met Gly Trp Met Asn Pro Asn Asn Gly
260 265 270

Tyr Thr Gly Tyr Ala Gln Lys Phe Gln Gly Arg Val Thr Met Thr Arg
275 280 285

Asn Thr Ser Ile Ser Thr Ala Tyr Met Glu Leu Ser Ser Leu Arg Ser
290 295 300

Glu Asp Thr Ala Val Tyr Tyr Cys Ala Arg Thr Asn Trp Val Tyr Trp
305 310 315 320

Tyr Phe Asp Leu Trp Gly Arg Gly Thr Thr Val Thr Val Ser Ser
325 330 335

<210> 139
<211> 335
<212> PRT
<213> Artificial

<220>
<223> Description of the artificial sequence; amino acid sequence of E11_VH-CH1-PL223_VH excluding signal sequence

<400> 139

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

```
Cys Ala Arg Leu Thr Val Ala Glu Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Val Gln Leu Val Gln Ser Gly
    210                 215                 220

Ala Glu Val Lys Lys Pro Gly Ala Ser Val Lys Val Ser Cys Lys Ala
225                 230                 235                 240

Ser Gly Tyr Thr Phe Thr Thr Tyr Asp Ile Asn Trp Val Arg Gln Ala
                245                 250                 255

Thr Gly Gln Gly Leu Glu Trp Met Gly Trp Met Asn Pro Asn Asn Gly
            260                 265                 270

Tyr Thr Gly Tyr Ala Gln Lys Phe Gln Gly Arg Val Thr Met Thr Arg
        275                 280                 285

Asn Thr Ser Ile Ser Thr Ala Tyr Met Glu Leu Ser Ser Leu Arg Ser
    290                 295                 300

Glu Asp Thr Ala Val Tyr Tyr Cys Ala Arg Thr Asn Trp Val Tyr Trp
305                 310                 315                 320

Tyr Phe Asp Leu Trp Gly Arg Gly Thr Met Val Thr Val Ser Ser
                325                 330                 335

<210>  140
<211>  335
<212>  PRT
```

<213> Artificial

<220>
<223> Description of the artificial sequence; amino acid sequence of E11_VH-CH1-PN7_VH excluding signal sequence

<400> 140

```
Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ile Ser Lys
            20                  25                  30

Ser Ser Tyr Trp Gly Trp Ile Arg Gln Pro Pro Gly Lys Gly Leu Glu
        35                  40                  45

Trp Ile Gly Ser Ile Tyr Tyr Ser Gly Ser Thr Phe Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Leu Thr Val Ala Glu Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Val Gln Leu Val Gln Ser Gly
    210                 215                 220
```

Ala Glu Val Lys Lys Pro Gly Ala Ser Val Lys Val Ser Cys Lys Ala
225 230 235 240

Ser Gly Tyr Thr Phe Thr Thr Tyr Asp Ile Asn Trp Val Arg Gln Ala
245 250 255

Thr Gly Gln Gly Leu Glu Trp Met Gly Trp Met Asn Pro Asn Ser Gly
260 265 270

Tyr Thr Gly Tyr Ala Gln Lys Phe Gln Gly Arg Val Thr Met Thr Arg
275 280 285

Asp Thr Ser Ile Ser Thr Ala Tyr Met Glu Leu Ser Ser Leu Arg Ser
290 295 300

Glu Asp Thr Ala Val Tyr Tyr Cys Ala Arg Thr Asn Trp Glu Ser Trp
305 310 315 320

Tyr Phe Asp Leu Trp Gly Arg Gly Thr Leu Val Thr Val Ser Ser
325 330 335

<210> 141
<211> 337
<212> PRT
<213> Artificial

<220>
<223> Description of the artificial sequence; amino acid sequence of E11_VH-CH1-PN170_VH excluding signal sequence

<400> 141

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser Ala Ser Thr Lys Gly Pro Ser Val Phe Pro
115 120 125

Leu Ala Pro Cys Ser Arg Ser Thr Ser Glu Ser Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Lys Thr Tyr Thr Cys Asn Val Asp His Lys Pro Ser
195 200 205

Asn Thr Lys Val Asp Lys Arg Val Glu Val Gln Leu Val Gln Ser Gly
210 215 220

Ala Glu Val Lys Lys Pro Gly Ala Ser Val Lys Val Ser Cys Lys Ala
225 230 235 240

Ser Gly Tyr Thr Leu Thr Ser Tyr Asp Ile Asn Trp Val Arg Gln Ala
245 250 255

Thr Gly Gln Gly Leu Glu Trp Met Gly Trp Met Asn Pro Asn Ser Gly
260 265 270

Tyr Thr Gly Tyr Ala Gln Lys Phe Gln Asp Arg Val Thr Met Thr Arg
275 280 285

Asn Thr Ala Ile Asn Thr Ala Tyr Met Glu Leu Ser Ser Leu Arg Ser
290 295 300

Glu Asp Thr Ala Val Tyr Tyr Cys Ala Arg Glu Ala Gly Tyr Asp Gly
305 310 315 320

Ile Trp Tyr Phe Asp Leu Trp Gly Arg Gly Thr Leu Val Thr Val Ser
325 330 335

Ser

<210> 142
<211> 251
<212> PRT
<213> Artificial

<220>
<223> Description of the artificial sequence; amino acid sequence of E11_VH-GL-PI134_VH excluding signal sequence

<400> 142

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser Ala Ser Thr Lys Gly Pro Ser Val Phe Pro
115 120 125

Leu Ala Pro Cys Gln Val Gln Leu Val Gln Ser Gly Ala Glu Val Lys
130 135 140

Lys Pro Gly Ala Ser Val Lys Val Ser Cys Lys Ala Ser Gly Tyr Thr
145 150 155 160

Phe Thr Thr Tyr Asp Ile Asn Trp Val Arg Gln Ala Thr Gly Gln Gly
165 170 175

Leu Glu Trp Met Gly Trp Met Asn Pro Asn Asn Gly Tyr Thr Gly Tyr
180 185 190

Ala Gln Lys Phe Gln Gly Arg Val Thr Met Thr Arg Asn Thr Ser Ile
195 200 205

Ser Thr Ala Tyr Met Glu Leu Ser Ser Leu Arg Ser Glu Asp Thr Ala
    210                 215                 220

Val Tyr Tyr Cys Ala Arg Thr Asn Trp Val Tyr Trp Tyr Phe Asp Leu
225                 230                 235                 240

Trp Gly Arg Gly Thr Leu Val Thr Val Ser Ser
                245                 250

<210> 143
<211> 251
<212> PRT
<213> Artificial

<220>
<223> Description of the artificial sequence; amino acid sequence of E11_VH-ML-PI101_VH excluding signal sequence

<400> 143

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ile Ser Lys
            20                  25                  30

Ser Ser Tyr Trp Gly Trp Ile Arg Gln Pro Pro Gly Lys Gly Leu Glu
        35                  40                  45

Trp Ile Gly Ser Ile Tyr Tyr Ser Gly Ser Thr Phe Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Leu Thr Val Ala Glu Phe Asp Tyr Trp Gly Gln Gly Thr
            100                 105                 110

Leu Val Thr Val Ser Ser Gly Ser Ala Ser Ala Pro Thr Leu Phe Pro
        115                 120                 125

Leu Val Ser Cys Glu Val Gln Leu Val Gln Ser Gly Ala Glu Val Lys
    130                 135                 140

Lys Pro Gly Ala Ser Val Lys Val Ser Cys Lys Ala Ser Gly Tyr Asn
145                 150                 155                 160

```
Leu Ala Thr Tyr Asp Ile Asn Trp Val Arg Gln Ala Thr Gly Gln Gly
                165                 170                 175

Leu Glu Trp Met Gly Trp Met Asn Pro Asn Ser Gly Tyr Thr Gly Tyr
            180                 185                 190

Ala Gln Lys Phe Gln Gly Arg Val Thr Met Thr Arg Asp Thr Ser Ile
        195                 200                 205

Asn Thr Ala Tyr Met Glu Leu Ser Ser Leu Arg Ser Glu Asp Thr Ala
    210                 215                 220

Val Tyr Tyr Cys Ala Arg Thr Asn Trp Val Tyr Trp Tyr Phe Asp Leu
225                 230                 235                 240

Trp Gly Arg Gly Thr Leu Val Thr Val Ser Ser
                245                 250


<210>  144
<211>  37
<212>  DNA
<213>  Artificial

<220>
<223>   Description of the artificial sequence; primer33

<400>  144
tcaccgtctc ctcagctagc accaaaggac cttctgt                             37


<210>  145
<211>  24
<212>  DNA
<213>  Artificial

<220>
<223>  Description of the artificial sequence; primer34

<400>  145
gaccctctta tcaaccttgg tgtt                                          24


<210>  146
<211>  36
<212>  DNA
<213>  Artificial

<220>
<223>  Description of the artificial sequence; primer35

<400>  146
aaggttgata agagggtcga agtgcagctg gtgcag                              36


<210>  147
```

<211>  34
<212>  DNA
<213>  Artificial

<220>
<223>  Description of the artificial sequence; primer36

<400>  147
gccccttggt gctagctgag gagacggtga ccag    34

<210>  148
<211>  33
<212>  DNA
<213>  Artificial

<220>
<223>  Description of the artificial sequence; primer37

<400>  148
cgtctcctca gctagcacca aggggccatc cgt    33

<210>  149
<211>  79
<212>  DNA
<213>  Artificial

<220>
<223>  Description of the artificial sequence; primer38

<400>  149
aaccgttaac ggatcctcag tggtggtggt ggtgatgctt atcatcgtcg tctttgtaat    60

ccggacatgg tggacaagg    79

<210>  150
<211>  1380
<212>  DNA
<213>  Artificial

<220>
<223>  Description of the artificial sequence; nucleotide sequence of E11-CH1-PN7VH F(ab')2

<220>
<221>  CDS
<222>  (1)..(1380)

<400>  150
cag ctg cag ctg cag gag tcg ggc cca gga ctg gtg aag cct tcg gag    48
Gln Leu Gln Leu Gln Glu Ser Gly Pro Gly Leu Val Lys Pro Ser Glu
1               5                   10                  15

acc ctg tcc ctc acc tgc act gtc tct ggt ggc tcc atc atc agt aaa    96
Thr Leu Ser Leu Thr Cys Thr Val Ser Gly Gly Ser Ile Ile Ser Lys
            20                  25                  30

agt tcc tac tgg ggc tgg att cgc cag ccc cca ggg aag ggg ctg gag    144
Ser Ser Tyr Trp Gly Trp Ile Arg Gln Pro Pro Gly Lys Gly Leu Glu
        35                  40                  45

```
tgg att ggg agt atc tat tat agt ggg agt acc ttc tac aac ccg tcc      192
Trp Ile Gly Ser Ile Tyr Tyr Ser Gly Ser Thr Phe Tyr Asn Pro Ser
    50                  55                  60

ctc aag agt cga gtc acc ata tcc gta gac acg tcc aag aac cag ttc      240
Leu Lys Ser Arg Val Thr Ile Ser Val Asp Thr Ser Lys Asn Gln Phe
65                  70                  75                  80

tcc ctg aag ctg agc tct gtg acc gcc gca gac acg gct gtg tat tac      288
Ser Leu Lys Leu Ser Ser Val Thr Ala Ala Asp Thr Ala Val Tyr Tyr
                85                  90                  95

tgt gcg aga ctg aca gtg gct gag ttt gac tac tgg ggc cag gga acc      336
Cys Ala Arg Leu Thr Val Ala Glu Phe Asp Tyr Trp Gly Gln Gly Thr
            100                 105                 110

ctg gtc acc gtc tcc tca gct agc acc aaa gga ccg tct gta ttt cct      384
Leu Val Thr Val Ser Ser Ala Ser Thr Lys Gly Pro Ser Val Phe Pro
        115                 120                 125

ctt gcg cca tgc tct cgc tct acg tca gaa tca act gcc gct ctg ggg      432
Leu Ala Pro Cys Ser Arg Ser Thr Ser Glu Ser Thr Ala Ala Leu Gly
    130                 135                 140

tgc ctg gtt aaa gac tac ttc ccg gag cct gtg aca gtg agt tgg aac      480
Cys Leu Val Lys Asp Tyr Phe Pro Glu Pro Val Thr Val Ser Trp Asn
145                 150                 155                 160

tcc ggc gcc ctg aca tca gga gtg cat aca ttt ccc gcc gtg ctt cag      528
Ser Gly Ala Leu Thr Ser Gly Val His Thr Phe Pro Ala Val Leu Gln
                165                 170                 175

agc agc gga ctt tat agc ctc agc agt gtg gtg acc gtg cca tct tcc      576
Ser Ser Gly Leu Tyr Ser Leu Ser Ser Val Val Thr Val Pro Ser Ser
            180                 185                 190

agc ctg ggg acc aag acc tac acc tgt aac gtg gac cac aaa ccc agc      624
Ser Leu Gly Thr Lys Thr Tyr Thr Cys Asn Val Asp His Lys Pro Ser
        195                 200                 205

aac acc aag gtt gat aag agg gtc gaa gtg cag ctg gtg cag tcc gga      672
Asn Thr Lys Val Asp Lys Arg Val Glu Val Gln Leu Val Gln Ser Gly
    210                 215                 220

gct gag gtg aag aag cct ggg gcc tca gtg aag gtc tcc tgc aag gct      720
Ala Glu Val Lys Lys Pro Gly Ala Ser Val Lys Val Ser Cys Lys Ala
225                 230                 235                 240

tct ggt tac acc ttt acc act tat gat atc aac tgg gtg cga cag gcc      768
Ser Gly Tyr Thr Phe Thr Thr Tyr Asp Ile Asn Trp Val Arg Gln Ala
                245                 250                 255

act gga caa ggg ctt gag tgg atg gga tgg atg aac cct aac agt ggt      816
Thr Gly Gln Gly Leu Glu Trp Met Gly Trp Met Asn Pro Asn Ser Gly
            260                 265                 270

tac aca ggc tat gca cag aag ttc cag ggc aga gtc acc atg acc agg      864
Tyr Thr Gly Tyr Ala Gln Lys Phe Gln Gly Arg Val Thr Met Thr Arg
        275                 280                 285

gac acc tcc ata agc aca gcc tac atg gag ctg agc agc ctg aga tct      912
Asp Thr Ser Ile Ser Thr Ala Tyr Met Glu Leu Ser Ser Leu Arg Ser
```

```
    290                 295                 300

gag gac acg gcc gtg tat tac tgt gcg cgg acg aac tgg gaa tcc tgg       960
Glu Asp Thr Ala Val Tyr Tyr Cys Ala Arg Thr Asn Trp Glu Ser Trp
305                 310                 315                 320

tac ttc gat ctc tgg ggc cgt gga acc ctg gtc acc gtc tcc tca gct      1008
Tyr Phe Asp Leu Trp Gly Arg Gly Thr Leu Val Thr Val Ser Ser Ala
                325                 330                 335

agc acc aag ggg cca tcc gtg ttc cct ctt gcc cca tgc tct agg tct      1056
Ser Thr Lys Gly Pro Ser Val Phe Pro Leu Ala Pro Cys Ser Arg Ser
            340                 345                 350

acc agc gaa tct act gcc gct ctt ggt tgt ctt gtc aaa gac tac ttc      1104
Thr Ser Glu Ser Thr Ala Ala Leu Gly Cys Leu Val Lys Asp Tyr Phe
        355                 360                 365

ccg gaa cct gtc aca gtc agc tgg aat agt ggc gct ttg acc tct ggt      1152
Pro Glu Pro Val Thr Val Ser Trp Asn Ser Gly Ala Leu Thr Ser Gly
    370                 375                 380

gtc cac act ttt ccc gcg gtt ctg cag tcc tca ggc ctg tat agc ctg      1200
Val His Thr Phe Pro Ala Val Leu Gln Ser Ser Gly Leu Tyr Ser Leu
385                 390                 395                 400

agt tcc gtg gtg acc gtt cct tct tct agc ctg ggg acg aaa acg tac      1248
Ser Ser Val Val Thr Val Pro Ser Ser Ser Leu Gly Thr Lys Thr Tyr
                405                 410                 415

acc tgt aat gtg gat cac aaa ccc agc aat aca aag gtt gac aag cgc      1296
Thr Cys Asn Val Asp His Lys Pro Ser Asn Thr Lys Val Asp Lys Arg
            420                 425                 430

gta gag agt aag tat gga cct cct tgt cca cca tgt ccg gat tac aaa      1344
Val Glu Ser Lys Tyr Gly Pro Pro Cys Pro Pro Cys Pro Asp Tyr Lys
        435                 440                 445

gac gac gat gat aag cat cac cac cac cac cac tga                      1380
Asp Asp Asp Asp Lys His His His His His His
    450                 455


<210>  151
<211>  459
<212>  PRT
<213>  Artificial

<220>
<223>  Synthetic Construct

<400>  151

Gln Leu Gln Leu Gln Glu Ser Gly Pro Gly Leu Val Lys Pro Ser Glu
1               5                   10                  15


Thr Leu Ser Leu Thr Cys Thr Val Ser Gly Gly Ser Ile Ile Ser Lys
            20                  25                  30


Ser Ser Tyr Trp Gly Trp Ile Arg Gln Pro Pro Gly Lys Gly Leu Glu
        35                  40                  45
```

```
Trp Ile Gly Ser Ile Tyr Tyr Ser Gly Ser Thr Phe Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Leu Thr Val Ala Glu Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Val Gln Leu Val Gln Ser Gly
    210                 215                 220

Ala Glu Val Lys Lys Pro Gly Ala Ser Val Lys Val Ser Cys Lys Ala
225                 230                 235                 240

Ser Gly Tyr Thr Phe Thr Thr Tyr Asp Ile Asn Trp Val Arg Gln Ala
                245                 250                 255

Thr Gly Gln Gly Leu Glu Trp Met Gly Trp Met Asn Pro Asn Ser Gly
            260                 265                 270

Tyr Thr Gly Tyr Ala Gln Lys Phe Gln Gly Arg Val Thr Met Thr Arg
        275                 280                 285

Asp Thr Ser Ile Ser Thr Ala Tyr Met Glu Leu Ser Ser Leu Arg Ser
```

290 295 300

Glu Asp Thr Ala Val Tyr Tyr Cys Ala Arg Thr Asn Trp Glu Ser Trp
305 310 315 320

Tyr Phe Asp Leu Trp Gly Arg Gly Thr Leu Val Thr Val Ser Ser Ala
325 330 335

Ser Thr Lys Gly Pro Ser Val Phe Pro Leu Ala Pro Cys Ser Arg Ser
340 345 350

Thr Ser Glu Ser Thr Ala Ala Leu Gly Cys Leu Val Lys Asp Tyr Phe
355 360 365

Pro Glu Pro Val Thr Val Ser Trp Asn Ser Gly Ala Leu Thr Ser Gly
370 375 380

Val His Thr Phe Pro Ala Val Leu Gln Ser Ser Gly Leu Tyr Ser Leu
385 390 395 400

Ser Ser Val Val Thr Val Pro Ser Ser Ser Leu Gly Thr Lys Thr Tyr
405 410 415

Thr Cys Asn Val Asp His Lys Pro Ser Asn Thr Lys Val Asp Lys Arg
420 425 430

Val Glu Ser Lys Tyr Gly Pro Pro Cys Pro Pro Cys Pro Asp Tyr Lys
435 440 445

Asp Asp Asp Asp Lys His His His His His His
450 455

<210> 152
<211> 79
<212> DNA
<213> Artificial

<220>
<223> Description of the artificial sequence; primer39

<400> 152
aaccgttaac ggatcctcag tggtggtggt ggtggtgttt gtcgtcgtcg tctttgtagt 60

caactctctt gtccacctt 79

<210> 153
<211> 1344
<212> DNA
<213> Artificial

<220>

<223> Description of the artificial sequence; nucleotide sequence of E11-CH1-PN7VH Fab

<220>
<221> CDS
<222> (1)..(1344)

<400> 153
```
cag ctg cag ctg cag gag tcg ggc cca gga ctg gtg aag cct tcg gag      48
Gln Leu Gln Leu Gln Glu Ser Gly Pro Gly Leu Val Lys Pro Ser Glu
1               5                   10                  15

acc ctg tcc ctc acc tgc act gtc tct ggt ggc tcc atc atc agt aaa      96
Thr Leu Ser Leu Thr Cys Thr Val Ser Gly Gly Ser Ile Ile Ser Lys
            20                  25                  30

agt tcc tac tgg ggc tgg att cgc cag ccc cca ggg aag ggg ctg gag      144
Ser Ser Tyr Trp Gly Trp Ile Arg Gln Pro Pro Gly Lys Gly Leu Glu
        35                  40                  45

tgg att ggg agt atc tat tat agt ggg agt acc ttc tac aac ccg tcc      192
Trp Ile Gly Ser Ile Tyr Tyr Ser Gly Ser Thr Phe Tyr Asn Pro Ser
    50                  55                  60

ctc aag agt cga gtc acc ata tcc gta gac acg tcc aag aac cag ttc      240
Leu Lys Ser Arg Val Thr Ile Ser Val Asp Thr Ser Lys Asn Gln Phe
65                  70                  75                  80

tcc ctg aag ctg agc tct gtg acc gcc gca gac acg gct gtg tat tac      288
Ser Leu Lys Leu Ser Ser Val Thr Ala Ala Asp Thr Ala Val Tyr Tyr
                85                  90                  95

tgt gcg aga ctg aca gtg gct gag ttt gac tac tgg ggc cag gga acc      336
Cys Ala Arg Leu Thr Val Ala Glu Phe Asp Tyr Trp Gly Gln Gly Thr
            100                 105                 110

ctg gtc acc gtc tcc tca gct agc acc aaa gga cct tct gta ttt cct      384
Leu Val Thr Val Ser Ser Ala Ser Thr Lys Gly Pro Ser Val Phe Pro
        115                 120                 125

ctt gcg cca tgc tct cgc tct acg tca gaa tca act gcc gct ctg ggg      432
Leu Ala Pro Cys Ser Arg Ser Thr Ser Glu Ser Thr Ala Ala Leu Gly
    130                 135                 140

tgc ctg gtt aaa gac tac ttc ccg gag cct gtg aca gtg agt tgg aac      480
Cys Leu Val Lys Asp Tyr Phe Pro Glu Pro Val Thr Val Ser Trp Asn
145                 150                 155                 160

tcc ggc gcc ctg aca tca gga gtg cat aca ttt ccc gcc gtg ctt cag      528
Ser Gly Ala Leu Thr Ser Gly Val His Thr Phe Pro Ala Val Leu Gln
                165                 170                 175

agc agc gga ctt tat agc ctc agc agt gtg gtg acc gtg cca tct tcc      576
Ser Ser Gly Leu Tyr Ser Leu Ser Ser Val Val Thr Val Pro Ser Ser
            180                 185                 190

agc ctg ggg acc aag acc tac acc tgt aac gtg gac cac aaa ccc agc      624
Ser Leu Gly Thr Lys Thr Tyr Thr Cys Asn Val Asp His Lys Pro Ser
        195                 200                 205

aac acc aag gtt gat aag agg gtc gaa gtg cag ctg gtg cag tcc gga      672
Asn Thr Lys Val Asp Lys Arg Val Glu Val Gln Leu Val Gln Ser Gly
```

```
    210                 215                 220

gct gag gtg aag aag cct ggg gcc tca gtg aag gtc tcc tgc aag gct      720
Ala Glu Val Lys Lys Pro Gly Ala Ser Val Lys Val Ser Cys Lys Ala
225                 230                 235                 240

tct ggt tac acc ttt acc act tat gat atc aac tgg gtg cga cag gcc      768
Ser Gly Tyr Thr Phe Thr Thr Tyr Asp Ile Asn Trp Val Arg Gln Ala
                245                 250                 255

act gga caa ggg ctt gag tgg atg gga tgg atg aac cct aac agt ggt      816
Thr Gly Gln Gly Leu Glu Trp Met Gly Trp Met Asn Pro Asn Ser Gly
            260                 265                 270

tac aca ggc tat gca cag aag ttc cag ggc aga gtc acc atg acc agg      864
Tyr Thr Gly Tyr Ala Gln Lys Phe Gln Gly Arg Val Thr Met Thr Arg
        275                 280                 285

gac acc tcc ata agc aca gcc tac atg gag ctg agc agc ctg aga tct      912
Asp Thr Ser Ile Ser Thr Ala Tyr Met Glu Leu Ser Ser Leu Arg Ser
    290                 295                 300

gag gac acg gcc gtg tat tac tgt gcg cgg acg aac tgg gaa tcc tgg      960
Glu Asp Thr Ala Val Tyr Tyr Cys Ala Arg Thr Asn Trp Glu Ser Trp
305                 310                 315                 320

tac ttc gat ctc tgg ggc cgt gga acc ctg gtc acc gtc tcc tca gct     1008
Tyr Phe Asp Leu Trp Gly Arg Gly Thr Leu Val Thr Val Ser Ser Ala
                325                 330                 335

agc acc aag ggg cca tcc gtc ttc ccc ctg gcg ccc tgc tcc agg agc     1056
Ser Thr Lys Gly Pro Ser Val Phe Pro Leu Ala Pro Cys Ser Arg Ser
            340                 345                 350

acc tcc gag agc aca gcc gcc ctg ggc tgc ctg gtc aag gac tac ttc     1104
Thr Ser Glu Ser Thr Ala Ala Leu Gly Cys Leu Val Lys Asp Tyr Phe
        355                 360                 365

ccc gaa ccg gtg acg gtg tcg tgg aac tca ggc gcc ctg acc agc ggc     1152
Pro Glu Pro Val Thr Val Ser Trp Asn Ser Gly Ala Leu Thr Ser Gly
    370                 375                 380

gtg cac acc ttc ccg gct gtc cta cag tcc tca gga ctc tac tcc ctc     1200
Val His Thr Phe Pro Ala Val Leu Gln Ser Ser Gly Leu Tyr Ser Leu
385                 390                 395                 400

agc agc gtg gtg acc gtg ccc tcc agc agc ttg ggc acg aag acc tac     1248
Ser Ser Val Val Thr Val Pro Ser Ser Ser Leu Gly Thr Lys Thr Tyr
                405                 410                 415

acc tgc aac gta gat cac aag ccc agc aac acc aag gtg gac aag aga     1296
Thr Cys Asn Val Asp His Lys Pro Ser Asn Thr Lys Val Asp Lys Arg
            420                 425                 430

gtt gac tac aaa gac gac gac gac aaa cac cac cac cac cac cac tga     1344
Val Asp Tyr Lys Asp Asp Asp Asp Lys His His His His His His
        435                 440                 445
```

<210> 154
<211> 447
<212> PRT
<213> Artificial

<220>
<223> Synthetic Construct

<400> 154

Gln Leu Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ile Ser Lys
20 25 30

Ser Ser Tyr Trp Gly Trp Ile Arg Gln Pro Pro Gly Lys Gly Leu Glu
35 40 45

Trp Ile Gly Ser Ile Tyr Tyr Ser Gly Ser Thr Phe Tyr Asn Pro Ser
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Leu Thr Val Ala Glu Phe Asp Tyr Trp Gly Gln Gly Thr
100 105 110

Leu Val Thr Val Ser Ser Ala Ser Thr Lys Gly Pro Ser Val Phe Pro
115 120 125

Leu Ala Pro Cys Ser Arg Ser Thr Ser Glu Ser Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Lys Thr Tyr Thr Cys Asn Val Asp His Lys Pro Ser
195 200 205

Asn Thr Lys Val Asp Lys Arg Val Glu Val Gln Leu Val Gln Ser Gly
210 215 220

Ala Glu Val Lys Lys Pro Gly Ala Ser Val Lys Val Ser Cys Lys Ala

```
225                 230                 235                 240

Ser Gly Tyr Thr Phe Thr Thr Tyr Asp Ile Asn Trp Val Arg Gln Ala
                245                 250                 255

Thr Gly Gln Gly Leu Glu Trp Met Gly Trp Met Asn Pro Asn Ser Gly
            260                 265                 270

Tyr Thr Gly Tyr Ala Gln Lys Phe Gln Gly Arg Val Thr Met Thr Arg
        275                 280                 285

Asp Thr Ser Ile Ser Thr Ala Tyr Met Glu Leu Ser Ser Leu Arg Ser
    290                 295                 300

Glu Asp Thr Ala Val Tyr Tyr Cys Ala Arg Thr Asn Trp Glu Ser Trp
305                 310                 315                 320

Tyr Phe Asp Leu Trp Gly Arg Gly Thr Leu Val Thr Val Ser Ser Ala
                325                 330                 335

Ser Thr Lys Gly Pro Ser Val Phe Pro Leu Ala Pro Cys Ser Arg Ser
            340                 345                 350

Thr Ser Glu Ser Thr Ala Ala Leu Gly Cys Leu Val Lys Asp Tyr Phe
        355                 360                 365

Pro Glu Pro Val Thr Val Ser Trp Asn Ser Gly Ala Leu Thr Ser Gly
    370                 375                 380

Val His Thr Phe Pro Ala Val Leu Gln Ser Ser Gly Leu Tyr Ser Leu
385                 390                 395                 400

Ser Ser Val Val Thr Val Pro Ser Ser Ser Leu Gly Thr Lys Thr Tyr
                405                 410                 415

Thr Cys Asn Val Asp His Lys Pro Ser Asn Thr Lys Val Asp Lys Arg
            420                 425                 430

Val Asp Tyr Lys Asp Asp Asp Asp Lys His His His His His His
        435                 440                 445
```

<210>  155
<211>  1347
<212>  DNA
<213>  Artificial

<220>
<223>  Description of the artificial sequence; nucleotide sequence of E11 K409R

<220>
<221> CDS
<222> (1)..(1347)

<400> 155

```
cag ctg cag ctg cag gag tcg ggc cca gga ctg gtg aag cct tcg gag       48
Gln Leu Gln Leu Gln Glu Ser Gly Pro Gly Leu Val Lys Pro Ser Glu
1               5                   10                  15

acc ctg tcc ctc acc tgc act gtc tct ggt ggc tcc atc atc agt aaa       96
Thr Leu Ser Leu Thr Cys Thr Val Ser Gly Gly Ser Ile Ile Ser Lys
            20                  25                  30

agt tcc tac tgg ggc tgg att cgc cag ccc cca ggg aag ggg ctg gag      144
Ser Ser Tyr Trp Gly Trp Ile Arg Gln Pro Pro Gly Lys Gly Leu Glu
        35                  40                  45

tgg att ggg agt atc tat tat agt ggg agt acc ttc tac aac ccg tcc      192
Trp Ile Gly Ser Ile Tyr Tyr Ser Gly Ser Thr Phe Tyr Asn Pro Ser
    50                  55                  60

ctc aag agt cga gtc acc ata tcc gta gac acg tcc aag aac cag ttc      240
Leu Lys Ser Arg Val Thr Ile Ser Val Asp Thr Ser Lys Asn Gln Phe
65                  70                  75                  80

tcc ctg aag ctg agc tct gtg acc gcc gca gac acg gct gtg tat tac      288
Ser Leu Lys Leu Ser Ser Val Thr Ala Ala Asp Thr Ala Val Tyr Tyr
                85                  90                  95

tgt gcg aga ctg aca gtg gct gag ttt gac tac tgg ggc cag gga acc      336
Cys Ala Arg Leu Thr Val Ala Glu Phe Asp Tyr Trp Gly Gln Gly Thr
            100                 105                 110

ctg gtc acc gtc tcc tca gct agc acc aag ggc cca tcg gtc ttc ccc      384
Leu Val Thr Val Ser Ser Ala Ser Thr Lys Gly Pro Ser Val Phe Pro
        115                 120                 125

ctg gca ccc tcc tcc aag agc acc tct ggg ggc aca gcg gcc ctg ggc      432
Leu Ala Pro Ser Ser Lys Ser Thr Ser Gly Gly Thr Ala Ala Leu Gly
    130                 135                 140

tgc ctg gtc aag gac tac ttc ccc gaa ccg gtg acg gtg tcg tgg aac      480
Cys Leu Val Lys Asp Tyr Phe Pro Glu Pro Val Thr Val Ser Trp Asn
145                 150                 155                 160

tca ggc gcc ctg acc agc ggc gtg cac acc ttc ccg gct gtc cta cag      528
Ser Gly Ala Leu Thr Ser Gly Val His Thr Phe Pro Ala Val Leu Gln
                165                 170                 175

tcc tca gga ctc tac tcc ctc agc agc gtg gtg acc gtg ccc tcc agc      576
Ser Ser Gly Leu Tyr Ser Leu Ser Ser Val Val Thr Val Pro Ser Ser
            180                 185                 190

agc ttg ggc acc cag acc tac atc tgc aac gtg aat cac aag ccc agc      624
Ser Leu Gly Thr Gln Thr Tyr Ile Cys Asn Val Asn His Lys Pro Ser
        195                 200                 205

aac acc aag gtg gac aag aaa gtt gag ccc aaa tct tgt gac aaa act      672
Asn Thr Lys Val Asp Lys Lys Val Glu Pro Lys Ser Cys Asp Lys Thr
    210                 215                 220
```

```
cac aca tgc cca ccg tgc cca gca cct gaa ctc ctg ggg gga ccg tca       720
His Thr Cys Pro Pro Cys Pro Ala Pro Glu Leu Leu Gly Gly Pro Ser
225                 230                 235                 240

gtc ttc ctc ttc ccc cca aaa ccc aag gac acc ctc atg atc tcc cgg       768
Val Phe Leu Phe Pro Pro Lys Pro Lys Asp Thr Leu Met Ile Ser Arg
                245                 250                 255

acc cct gag gtc aca tgc gtg gtg gtg gac gtg agc cac gaa gac cct       816
Thr Pro Glu Val Thr Cys Val Val Val Asp Val Ser His Glu Asp Pro
            260                 265                 270

gag gtc aag ttc aac tgg tac gtg gac ggc gtg gag gtg cat aat gcc       864
Glu Val Lys Phe Asn Trp Tyr Val Asp Gly Val Glu Val His Asn Ala
        275                 280                 285

aag aca aag ccg cgg gag gag cag tac aac agc acg tac cgt gtg gtc       912
Lys Thr Lys Pro Arg Glu Glu Gln Tyr Asn Ser Thr Tyr Arg Val Val
    290                 295                 300

agc gtc ctc acc gtc ctg cac cag gac tgg ctg aat ggc aag gag tac       960
Ser Val Leu Thr Val Leu His Gln Asp Trp Leu Asn Gly Lys Glu Tyr
305                 310                 315                 320

aag tgc aag gtc tcc aac aaa gcc ctc cca gcc ccc atc gag aaa acc      1008
Lys Cys Lys Val Ser Asn Lys Ala Leu Pro Ala Pro Ile Glu Lys Thr
                325                 330                 335

atc tcc aaa gcc aaa ggg cag ccc cga gaa cca cag gtg tac acc ctg      1056
Ile Ser Lys Ala Lys Gly Gln Pro Arg Glu Pro Gln Val Tyr Thr Leu
            340                 345                 350

ccc cca tcc cgg gat gag ctg acc aag aac cag gtc agc ctg acc tgc      1104
Pro Pro Ser Arg Asp Glu Leu Thr Lys Asn Gln Val Ser Leu Thr Cys
        355                 360                 365

ctg gtc aaa ggc ttc tat ccc agc gac atc gcc gtg gag tgg gag agc      1152
Leu Val Lys Gly Phe Tyr Pro Ser Asp Ile Ala Val Glu Trp Glu Ser
    370                 375                 380

aat ggg cag ccg gag aac aac tac aag acc acg cct ccc gtg ctg gac      1200
Asn Gly Gln Pro Glu Asn Asn Tyr Lys Thr Thr Pro Pro Val Leu Asp
385                 390                 395                 400

tcc gac ggc tcc ttc ttc ctc tac agc agg ctc acc gtg gac aag agc      1248
Ser Asp Gly Ser Phe Phe Leu Tyr Ser Arg Leu Thr Val Asp Lys Ser
                405                 410                 415

agg tgg cag cag ggg aac gtc ttc tca tgc tcc gtg atg cat gag gct      1296
Arg Trp Gln Gln Gly Asn Val Phe Ser Cys Ser Val Met His Glu Ala
            420                 425                 430

ctg cac aac cac tac acg cag aag agc ctc tcc ctg tct ccg ggt aaa      1344
Leu His Asn His Tyr Thr Gln Lys Ser Leu Ser Leu Ser Pro Gly Lys
        435                 440                 445

tga                                                                  1347
```

<210> 156
<211> 448
<212> PRT
<213> Artificial

<220>
<223>  Synthetic Construct

<400>  156

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ile Ser Lys
            20                  25                  30

Ser Ser Tyr Trp Gly Trp Ile Arg Gln Pro Pro Gly Lys Gly Leu Glu
        35                  40                  45

Trp Ile Gly Ser Ile Tyr Tyr Ser Gly Ser Thr Phe Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Leu Thr Val Ala Glu Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser

225 230 235 240

Val Phe Leu Phe Pro Pro Lys Pro Lys Asp Thr Leu Met Ile Ser Arg
245 250 255

Thr Pro Glu Val Thr Cys Val Val Val Asp Val Ser His Glu Asp Pro
260 265 270

Glu Val Lys Phe Asn Trp Tyr Val Asp Gly Val Glu Val His Asn Ala
275 280 285

Lys Thr Lys Pro Arg Glu Glu Gln Tyr Asn Ser Thr Tyr Arg Val Val
290 295 300

Ser Val Leu Thr Val Leu His Gln Asp Trp Leu Asn Gly Lys Glu Tyr
305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Tyr Thr Leu
340 345 350

Pro Pro Ser Arg Asp Glu Leu Thr Lys Asn Gln Val Ser Leu Thr Cys
355 360 365

Leu Val Lys Gly Phe Tyr Pro Ser Asp Ile Ala Val Glu Trp Glu Ser
370 375 380

Asn Gly Gln Pro Glu Asn Asn Tyr Lys Thr Thr Pro Pro Val Leu Asp
385 390 395 400

Ser Asp Gly Ser Phe Phe Leu Tyr Ser Arg Leu Thr Val Asp Lys Ser
405 410 415

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro Gly Lys
435 440 445

<210> 157
<211> 1350
<212> DNA
<213> Artificial

<220>
<223> Description of the artificial sequence; nucleotide sequence of PN7 F405L

<220>
<221> CDS
<222> (1)..(1350)

<400> 157

```
gaa gtg cag ctg gtg cag tcc gga gct gag gtg aag aag cct ggg gcc       48
Glu Val Gln Leu Val Gln Ser Gly Ala Glu Val Lys Lys Pro Gly Ala
1               5                   10                  15

tca gtg aag gtc tcc tgc aag gct tct ggt tac acc ttt acc act tat       96
Ser Val Lys Val Ser Cys Lys Ala Ser Gly Tyr Thr Phe Thr Thr Tyr
            20                  25                  30

gat atc aac tgg gtg cga cag gcc act gga caa ggg ctt gag tgg atg      144
Asp Ile Asn Trp Val Arg Gln Ala Thr Gly Gln Gly Leu Glu Trp Met
        35                  40                  45

gga tgg atg aac cct aac agt ggt tac aca ggc tat gca cag aag ttc      192
Gly Trp Met Asn Pro Asn Ser Gly Tyr Thr Gly Tyr Ala Gln Lys Phe
    50                  55                  60

cag ggc aga gtc acc atg acc agg gac acc tcc ata agc aca gcc tac      240
Gln Gly Arg Val Thr Met Thr Arg Asp Thr Ser Ile Ser Thr Ala Tyr
65                  70                  75                  80

atg gag ctg agc agc ctg aga tct gag gac acg gcc gtg tat tac tgt      288
Met Glu Leu Ser Ser Leu Arg Ser Glu Asp Thr Ala Val Tyr Tyr Cys
                85                  90                  95

gcg cgg acg aac tgg gaa tcc tgg tac ttc gat ctc tgg ggc cgt gga      336
Ala Arg Thr Asn Trp Glu Ser Trp Tyr Phe Asp Leu Trp Gly Arg Gly
            100                 105                 110

acc ctg gtc acc gtc tcc tca gct agc acc aag ggc cca tcg gtc ttc      384
Thr Leu Val Thr Val Ser Ser Ala Ser Thr Lys Gly Pro Ser Val Phe
        115                 120                 125

ccc ctg gca ccc tcc tcc aag agc acc tct ggg ggc aca gcg gcc ctg      432
Pro Leu Ala Pro Ser Ser Lys Ser Thr Ser Gly Gly Thr Ala Ala Leu
    130                 135                 140

ggc tgc ctg gtc aag gac tac ttc ccc gaa ccg gtg acg gtg tcg tgg      480
Gly Cys Leu Val Lys Asp Tyr Phe Pro Glu Pro Val Thr Val Ser Trp
145                 150                 155                 160

aac tca ggc gcc ctg acc agc ggc gtg cac acc ttc ccg gct gtc cta      528
Asn Ser Gly Ala Leu Thr Ser Gly Val His Thr Phe Pro Ala Val Leu
                165                 170                 175

cag tcc tca gga ctc tac tcc ctc agc agc gtg gtg acc gtg ccc tcc      576
Gln Ser Ser Gly Leu Tyr Ser Leu Ser Ser Val Val Thr Val Pro Ser
            180                 185                 190

agc agc ttg ggc acc cag acc tac atc tgc aac gtg aat cac aag ccc      624
Ser Ser Leu Gly Thr Gln Thr Tyr Ile Cys Asn Val Asn His Lys Pro
        195                 200                 205

agc aac acc aag gtg gac aag aaa gtt gag ccc aaa tct tgt gac aaa      672
Ser Asn Thr Lys Val Asp Lys Lys Val Glu Pro Lys Ser Cys Asp Lys
    210                 215                 220
```

```
act cac aca tgc cca ccg tgc cca gca cct gaa ctc ctg ggg gga ccg        720
Thr His Thr Cys Pro Pro Cys Pro Ala Pro Glu Leu Leu Gly Gly Pro
225                 230                 235                 240

tca gtc ttc ctc ttc ccc cca aaa ccc aag gac acc ctc atg atc tcc        768
Ser Val Phe Leu Phe Pro Pro Lys Pro Lys Asp Thr Leu Met Ile Ser
                245                 250                 255

cgg acc cct gag gtc aca tgc gtg gtg gtg gac gtg agc cac gaa gac        816
Arg Thr Pro Glu Val Thr Cys Val Val Val Asp Val Ser His Glu Asp
            260                 265                 270

cct gag gtc aag ttc aac tgg tac gtg gac ggc gtg gag gtg cat aat        864
Pro Glu Val Lys Phe Asn Trp Tyr Val Asp Gly Val Glu Val His Asn
        275                 280                 285

gcc aag aca aag ccg cgg gag gag cag tac aac agc acg tac cgt gtg        912
Ala Lys Thr Lys Pro Arg Glu Glu Gln Tyr Asn Ser Thr Tyr Arg Val
    290                 295                 300

gtc agc gtc ctc acc gtc ctg cac cag gac tgg ctg aat ggc aag gag        960
Val Ser Val Leu Thr Val Leu His Gln Asp Trp Leu Asn Gly Lys Glu
305                 310                 315                 320

tac aag tgc aag gtc tcc aac aaa gcc ctc cca gcc ccc atc gag aaa       1008
Tyr Lys Cys Lys Val Ser Asn Lys Ala Leu Pro Ala Pro Ile Glu Lys
                325                 330                 335

acc atc tcc aaa gcc aaa ggg cag ccc cga gaa cca cag gtg tac acc       1056
Thr Ile Ser Lys Ala Lys Gly Gln Pro Arg Glu Pro Gln Val Tyr Thr
            340                 345                 350

ctg ccc cca tcc cgg gat gag ctg acc aag aac cag gtc agc ctg acc       1104
Leu Pro Pro Ser Arg Asp Glu Leu Thr Lys Asn Gln Val Ser Leu Thr
        355                 360                 365

tgc ctg gtc aaa ggc ttc tat ccc agc gac atc gcc gtg gag tgg gag       1152
Cys Leu Val Lys Gly Phe Tyr Pro Ser Asp Ile Ala Val Glu Trp Glu
    370                 375                 380

agc aat ggg cag ccg gag aac aac tac aag acc acg cct ccc gtg ctg       1200
Ser Asn Gly Gln Pro Glu Asn Asn Tyr Lys Thr Thr Pro Pro Val Leu
385                 390                 395                 400

gac tcc gac ggc tcc ttc ttg ctc tac agc aag ctc acc gtg gac aag       1248
Asp Ser Asp Gly Ser Phe Leu Leu Tyr Ser Lys Leu Thr Val Asp Lys
                405                 410                 415

agc agg tgg cag cag ggg aac gtc ttc tca tgc tcc gtg atg cat gag       1296
Ser Arg Trp Gln Gln Gly Asn Val Phe Ser Cys Ser Val Met His Glu
            420                 425                 430

gct ctg cac aac cac tac acg cag aag agc ctc tcc ctg tct ccg ggt       1344
Ala Leu His Asn His Tyr Thr Gln Lys Ser Leu Ser Leu Ser Pro Gly
        435                 440                 445

aaa tga                                                                1350
Lys


<210>  158
<211>  449
```

<212> PRT
<213> Artificial

<220>
<223> Synthetic Construct

<400> 158

```
Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Tyr
            20                  25                  30

Asp Ile Asn Trp Val Arg Gln Ala Thr Gly Gln Gly Leu Glu Trp Met
        35                  40                  45

Gly Trp Met Asn Pro Asn Ser Gly Tyr Thr Gly Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Thr Asn Trp Glu Ser Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220
```

```
Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Leu Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys
```

## Description

### Technical Field

The present invention is related to a bispecific antibody or an antibody fragment thereof comprising an antigen binding domain that binds to a tumor necrosis factor-related apoptosis including ligand receptor 2 (TRAILR2), and an antigen binding domain that binds to a prostate-specific membrane antigen (PSMA); a nucleic acid comprising a nucleotide sequence that encodes the antibody or the antibody fragment thereof; a recombinant vector comprising the nucleic acid; a transformant comprising the recombinant vector; a method for producing the bispecific antibody or the antibody fragment thereof by using the transformant; and a reagent for detection or measurement, a diagnostic agent, and a therapeutic agent, each of which comprises the bispecific antibody or the antibody fragment thereof.

### Background Art

An antibody is a glycoprotein present in serum and tissue fluid of all mammals and recognizes foreign antigens *in vivo* (Non-Patent Document 1). The antibody is involved in the body's defense by activating the complement system, and activating effector functions of FcR-expressing cells such as phagocytic capacity, antibody-dependent cellular cytotoxicity capacity, mediator liberation capacity, and antigen presenting capability by binding to a receptor (FcR) present on the cell surface. One molecule of antibody consists of two homologous light chains (L chains) and two homologous heavy chains (H chains) and includes two antigen binding sites. The classes and subclasses of the antibody are determined by the H chain, and each class and subclass has unique functional differences. There are five different classes of human antibodies; IgG, IgA, IgM, IgD, and IgE. IgG is further broken down into the subclasses of IgG1, IgG2, IgG3 and IgG4, and IgA is broken down into the subclasses of IgA1 and IgA2.

A multivalent antibody is an antibody having multiple antigen binding sites in one molecule. As the example of the multivalent antibody, first, it has been reported that a multivalent antibody having the H chains and the L chains as a plurality of different polypeptide chains that bind to different antigens was produced using hybrid hybridomas (Non-Patent Document 3). In this method, since two different types of the H chains and the L chains are expressed on one cell, there occur about 10 combinations of the H chains and the L chains of the antibody. Therefore, the amount of the multivalent antibody produced with a desired combination of the H chains and the L chains is low, and because it is difficult to selectively isolate and purify such a multivalent antibody, the yield of a desired antibody decreases.

To overcome this problem, it has been reported that attempts have been made to produce an antibody with a desired combination by expressing a single polypeptide chain through linking a plurality of antigen binding sites and therefore reducing the variation of combinations between subunits. As an example, an antibody comprising a single chain Fv (scFv) in which antigen binding sites of an H chain and an L chain are linked as one polypeptide (Non-Patent Document 4) is known. Furthermore, an antibody in which two antigen binding sites are linked using a CH1 domain of an H chain constant region of IgG1 or a partial fragment of the domain, and an L chain constant region or a flexible linker (Gly-Gly-Gly-Gly-Ser), and the like have been reported (Non-Patent Document 5, Patent Document 1, and Patent Document 2). These traditional multivalent antibodies had drawbacks in terms of a propensity to aggregate, and low stability and productivity. On the other hand, it had been found that a multivalent antibody that comprises multiple antigen binding sites in a single H chain polypeptide and in which the antigen binding sites are not close to each other, has high stability and productivity (Patent Document 3).

The physiological cell death *in vivo* caused for generation change of normal cells is called apoptosis and is distinguished from necrosis which is pathological cell death (Non-Patent Document 6). Apoptosis is a phenomenon commonly found in processes such as embryogenesis and selection of lymphocytes (T cells and B cells) (Non-Patent Document 7).

As molecules involved in apoptosis, molecules such as tumor necrosis factor-$\alpha$ (TNF-$\alpha$), tumor necrosis factor-$\beta$ (TNF-$\beta$), Fas ligand, and CD40 ligand (CD 154) which belong to the tumor necrosis factor family (TNF family), have been identified. The TNF family molecule binds to a specific corresponding receptor (TNF receptor family molecule) on the cell surface, induces trimerization of the receptor so as to transmit signals intracellularly, and therefore causes apoptosis in various cells (Non-Patent Documents 8 to 11).

The TNF receptor family molecules are defined by the presence of cysteine-rich repeats in the extracellular domain. Among these, Fas and TNFR1, which are the receptor of the Fas ligand and the TNF-$\alpha$, respectively, have an intracellular region termed a death domain, which is required for apoptosis signal transduction. The activation of Fas promotes the association of adaptor molecules FADD/MORT1 having the death domain and causes the activation of caspase-8 binding to FADD/MORT1. The activated caspase-8 sequentially activates the downstream caspase molecule group and finally induces the cell apoptosis (Non-Patent Document 12). Signal is also transmitted intracellularly by cross-linking antibodies specific to the TNF receptor family molecules, and therefore the association of the TNF receptor family molecules is considered necessary for the signal transduction (Non-Patent Documents 13 and 14).

TRAIL is the TNF family molecule that induces apoptosis discovered by Wiley et al., and is the abbreviation for TNF-related apoptosis-inducing ligand (Non-Patent Document 15). This molecule is also called Apo-2 ligand or Apo-2L (Non-Patent Document 16). Unlike the Fas ligand, TRAIL is detected at significant levels in many human tissues such as spleen, lung, prostate, thymus, ovary, small intestine, large intestine, peripheral blood lymphocytes, placenta, and kidney, and is constantly expressed in some cell lines. TRAIL binds to a TRAIL receptor and induces apoptosis very rapidly than TNF in a time frame similar to the death signal transduction by the Fas (Non-Patent Document 17).

As the TRAIL receptor (TRAILR), five proteins have been identified so far. Among them, two receptors TRAILR1 (death receptor 4; also referred to as DR4) and TRAILR2 (death receptor 5; also referred to as DR5) have the death domain in the intracellular region. Transcripts of TRAILR1 are found in many human tissues such as spleen, peripheral blood leukocytes, small intestine, and thymus, and transcripts of TRAILR2 are found in many tissues such as spleen, peripheral blood lymphocytes, and ovary (Non-Patent Documents 18 to 20). It has been reported that TRAILR2 is a type I membrane protein having two forms by alternative splicing, and that the expression level of the form consisting of 440 amino acid residues is high in cancer cells (Non-Patent Documents 21 and 22). TRAILR2 also trimerizes by binding of TRAIL, induces the cell death signal through its own death domain, and therefore causes apoptosis in cancer cells (Non-Patent Document 23).

A recombinant human TRAIL comprising the extracellular region of TRAIL induces apoptosis in various cancer cells (Non-Patent Document 22), and exhibits remarkable anti-tumor effects in tumor-bearing mouse models obtained by using human colon cancer cells and breast cancer cells (Non-Patent Document 25). In addition, unlike the TNF-$\alpha$ and the Fas ligand which belong to the same TNF family as the TRAIL and have apoptosis-inducing activity, TRAIL does not damage normal tissues of a mouse and a cynomolgus monkey (Non-Patent Document 26). On the other hand, the recombinant human TRAIL is known to also induce apoptosis in human normal hepatic parenchymal cells and also in human brain cells (Non-Patent Documents 28 and 32).

It has been reported that monoclonal antibodies against TRAILR1 and TRAILR2 induce apoptosis in cancer cells similarly to the above described recombinant human TRAIL (Non-Patent Documents 24 and 27, and Patent Document 4). These antibodies exhibit anti-tumor effects also in tumor-bearing mouse models obtained by using human large intestine cancer cells and the like (Non-Patent Document 24 and Patent Document 4). Generally, it has been reported that in an experiment *in vitro,* traditional antibodies against TRAILR require cross-linking between antibodies when exhibiting the anti-tumor activity by inducing cell death (Non-Patent Documents 24 and 27).

As the main mechanisms of the anti-tumor activity of antibodies, there are two mechanisms. One of the mechanisms is the elimination by host's immune system such as complement-dependent cytotoxicity (CDC) and antibody-dependent cellular cytotoxicity (ADCC). The other mechanism is causing of direct inhibition of the cell proliferation or inducing the cell death via target molecules on cancer cells. For example, with respect to an anti-CD20 antibody and an anti-Her2/neu antibody, an experiment using mouse models indicates that ADCC via the Fc receptor expressed on immunocompetent cells, is responsible for the anti-tumor activity of the antibodies (Non-Patent Document 24). Furthermore, it has been reported that cross-linking of the anti-CD20 antibody enhances the anti-tumor activity of the antibody and causes the effect of inhibiting the cell proliferation in cancer cells (Non-Patent Document 24).

On the other hand, PSMA consists of a total of 750 amino acid residues from three domains of an intracellular domain consisting of 19 amino acid residues, a transmembrane domain consisting of 24 amino acid residues, and an extracellular domain consisting of 707 amino acid residues, and is a type II membrane glycoprotein of approximately 110 kDa. PSMA functions as glutamate carboxypeptidase, N-acetyl $\alpha$-linked acidic dipeptidase, and folate hydrolase (Non-Patent Document 29). PSMA is expressed on prostate epithelial cells *in vivo,* and its expression level increases in prostate cancer, and remarkably increases particularly in poorly differentiated, metastatic, and hormone refractory adenocarcinoma (Non-Patent Documents 30 and 31). In addition, PSMA is expressed in small intestine, salivary gland, duodenal mucosa, proximal tubule, and brain in small amounts (Non-Patent Document 31). Furthermore, PSMA is related to tumor angiogenesis (Non-Patent Document 31), and also expressed in the epithelium of new blood vessels related to tumors of large intestine cancer, breast cancer, bladder cancer, pancreatic cancer, kidney cancer, and melanoma (Non-Patent Document 32).

As an antibody against PSMA, J591 (ATCC Accession No. HB-12126) is known (Patent Document 5), and a radiolabeled substance such as $^{177}$Lu (ruthenium) or $^{89}$Zr (zirconium) has been developed as a therapeutic agent and a diagnostic agent for cancer.

Related Art Document

Patent Document

Patent Document 1: US Patent Application Publication No. 2007/0071675

Patent Document 2: International Publication No. 2001/077342
Patent Document 3: International Publication No. 2009/131239
Patent Document 4: International Publication No. 2002/094880
Patent Document 5: US Patent No. 5,773,292

Non-Patent Document

Non-Patent Document 1: Charles A. J. et. al., Immunobiology, 1997, Current Biology Ltd/Garland Publishing Inc.
Non-Patent Document 2: Emmanuelle Laffy et. al., Human Antibodies 14, 33-55, 2005
Non-Patent Document 3: Suresh et. al., Methods Enzymol. 121, 210-228, 1986
Non-Patent Document 4: Kranz et. al., J. Hematother Immunol. 5, 403-408, 1995
Non-Patent Document 5: Wu et. al., Nat. Biothech. 25, 1290-1297, 2007
Non-Patent Document 6: Kerr, et al., Br. J. Cancer 26,239,1972
Non-Patent Document 7: Itoh, S., et al., Cell 66, 233-243, 1991
Non-Patent Document 8: Schmid, et al., Proc.Natl.Acad.Sci., 83, 1881, 1986
Non-Patent Document 9: Dealtry et al., Eur.J.Immunol. 17, 689, 1987
Non-Patent Document 10: Krammer, et al., Curr.Op.Immunol. 6, 279-289, 1994
Non-Patent Document 11: Nagata, et al., Science 267, 1449-1456, 1995
Non-Patent Document 12: Nagata et al., Cell, 88, 355-365, 1997
Non-Patent Document 13: Chuntharapai A et. al., J. Immunol. 166(8), 4891, 2001
Non-Patent Document 14: Ashkenazi A et. al., Nat Rev Cancer 2,420,2002
Non-Patent Document 15: Wiley et al., Immunity, 3, 673-682, 1995
Non-Patent Document 16: Pitt, R. M., et al., J.Biol.Chem. 271, 12687-12690, 1996
Non-Patent Document 17: Marsters, S. A., et al., Curr. Biol. 6, 750-752, 1996
Non-Patent Document 18: Pan, Gel, et al., Science 276, 111-113, 1997
Non-Patent Document 19: Pan, G, et al., Science 277, 815-818, 1997
Non-Patent Document 20: Walczak, H., et al., EMBO J., 16, 5386-5397, 1997
Non-Patent Document 21: Screaton, G R., et al., Curr Biol., 7, 693-696, 1997
Non-Patent Document 22: Arai, T., et al., Cancer Letters, 133, 197-204, 1998
Non-Patent Document 23: Hymowitz S. G. et al., Mol Cell., 4, 563-71, 1999
Non-Patent Document 24: Griffith, T. S., et al., Curr.Opin.Immunol., 10, 559-563, 1998
Non-Patent Document 25: Walczak, H., et al., Nature Medicine 5, 2, 157-163, 1999
Non-Patent Document 26: Ashkenazi, A., et al., J.Clin.Invest. 104, 155-162, 1999
Non-Patent Document 27: Mori, E., et al, Cell Death and Differentiation, 11, 203-207, 2004
Non-Patent Document 28: Jo, M., et al., Nature Medicine, 6, 564-567, 2000
Non-Patent Document 29: Elsasser-Beile U. et al., Curr. Drug Targets, 10, 118-125, 2009
Non-Patent Document 30: Gregorakis, A.K. et al., Semin. Urol. Oncol., 16, 2-12, 1998
Non-Patent Document 31: Silver, D.A., Clin. Cancer Reseach, 3, 81-85,1997
Non-Patent Document 32: Chang, S.S., Curr. Opin. Invest. Drugs, 5,611-615,2004

Disclosure of Invention

Problems to Be Solved by the Invention

The problems to be solved by the present invention is to provide a bispecific antibody or an antibody fragment thereof comprising an antigen binding domain that binds to TRAILR2, and an antigen binding domain that binds to PSMA; a nucleic acid comprising a nucleotide sequence that encodes the antibody or the antibody fragment thereof; a recombinant vector comprising the nucleic acid; a transformant comprising the recombinant vector; a method for producing the bispecific antibody or the antibody fragment thereof by using the transformant; and a reagent for detection or measurement, a diagnostic agent, and a therapeutic agent, each of which comprises including the bispecific antibody or the antibody fragment thereof.

Means for Solving the Problems

As means for solving the problems above, the present invention provides a bispecific antibody or an antibody fragment thereof including an antigen binding domain that binds to TRAILR2, and an antigen binding domain that binds to PSMA.

That is, the present invention is related to (1) to (30) below.

(1) A bispecific antibody or an antibody fragment thereof, comprising:

an antigen binding domain that binds to TRAILR2; and
an antigen binding domain that binds to PSMA.

(2) The bispecific antibody or the antibody fragment thereof according to (1),
which activates TRAILR2, only when binding to TRAILR2 and PSMA.
(3) The bispecific antibody or the antibody fragment thereof according to (1) or (2), which divalently binds to each TRAILR2 and PSMA.
(4) The bispecific antibody or the antibody fragment thereof according to any one of (1) to (3),
wherein the antigen binding domain that binds to TRAILR2 is located closer to the N-terminus side than the antigen binding domain that binds to PSMA.
(5) The bispecific antibody or the antibody fragment thereof according to any one of (1) to (4),
wherein the antigen binding domain that binds to TRAILR2 is a variable region (V region) of an antibody that binds to TRAILR2, and the antigen binding domain that binds to PSMA is a V region of an antibody that binds to PSMA.
(6) The bispecific antibody or the antibody fragment thereof according to (5),
wherein amino acid sequences of complementarity determining regions (CDRs) 1 to 3 of a heavy chain variable region (hereinafter will be described as VH) of the monoclonal antibody that binds to TRAILR2 comprise the amino acid sequences represented by SEQ ID NOs: 70 to 72, respectively, and amino acid sequences of CDRs 1 to 3 of a light chain variable region (hereinafter will be described as VL) of the monoclonal antibody that binds to TRAILR2 comprise the amino acid sequences represented by SEQ ID NOs: 74 to 76, respectively.
(7) The bispecific antibody or the antibody fragment thereof according to (5) or (6),
wherein an amino acid sequence of the VH of the antibody that binds to TRAILR2 comprises the amino acid sequence represented by SEQ ID NO: 118, and an amino acid sequence of the VL of the antibody that binds to TRAILR2 comprises the amino acid sequence represented by SEQ ID NO: 119.
(8) The bispecific antibody or the antibody fragment thereof according to any one of (5) to (7),
wherein amino acid sequences of CDRs 1 to 3 of the VL of the antibody that binds to PSMA comprise the amino acid sequences represented by SEQ ID NOs: 74 to 76, respectively, and amino acid sequences of CDRs 1 to 3 of the VH of the antibody that binds to PSMA comprise any one of amino acid sequences selected from the group consisting of (a) to (d) below;

(a) the amino acid sequences represented by SEQ ID NOs: 78, 82, and 84, respectively,
(b) the amino acid sequences represented by SEQ ID NOs: 78, 79, and 80, respectively,
(c) the amino acid sequences represented by SEQ ID NOs: 78, 82, and 80, respectively, and
(d) the amino acid sequences represented by SEQ ID NOs: 96 to 98, respectively.

(9) The bispecific antibody or the antibody fragment thereof according to any one of (5) to (8),
wherein an amino acid sequence of the VL of the antibody that binds to PSMA comprises the amino acid sequence represented by SEQ ID NO: 119, and an amino acid sequence of the VH of the antibody that binds to PSMA comprises any one of amino acid sequences selected from the groups consisting of (e) to (o) below;

(e) the amino acid sequence represented by SEQ ID NO: 122,
(f) the amino acid sequence represented by SEQ ID NO: 120,
(g) the amino acid sequence represented by SEQ ID NO: 128,
(h) the amino acid sequence represented by SEQ ID NO: 123,
(i) the amino acid sequence represented by SEQ ID NO: 127,
(j) the amino acid sequence represented by SEQ ID NO: 124,
(k) the amino acid sequence represented by SEQ ID NO: 125,
(1) the amino acid sequence represented by SEQ ID NO: 129,
(m) the amino acid sequence represented by SEQ ID NO: 121,
(n) the amino acid sequence represented by SEQ ID NO: 126, and
(o) the amino acid sequence represented by SEQ ID NO: 130.

(10) The bispecific antibody or the antibody fragment thereof according to any one of (5) to (9),
which comprises a heavy chain comprising a polypeptide wherein the VH of the antibody that binds to TRAILR2 and the VH of the antibody that binds to PSMA are linked via a linker containing an immunoglobulin domain or a domain fragment thereof.

(11) The bispecific antibody or the antibody fragment thereof according to (10),
wherein the linker is a linker derived from the subclass of IgG4 or IgM.

(12) The bispecific antibody or the antibody fragment thereof according to (10) or (11),
wherein an amino acid sequence of a polypeptide consisting of the VH of the antibody that binds to TRAILR2, the linker and the VH of the antibody that binds to PSMA is any one of amino acid sequences selected from the group consisting of (A) to (M) below;

(A) the amino acid sequence represented by SEQ ID NO: 131,
(B) the amino acid sequence represented by SEQ ID NO: 132,
(C) the amino acid sequence represented by SEQ ID NO: 133,
(D) the amino acid sequence represented by SEQ ID NO: 134,
(E) the amino acid sequence represented by SEQ ID NO: 135,
(F) the amino acid sequence represented by SEQ ID NO: 136,
(G) the amino acid sequence represented by SEQ ID NO: 137,
(H) the amino acid sequence represented by SEQ ID NO: 138,
(I) the amino acid sequence represented by SEQ ID NO: 139,
(J) the amino acid sequence represented by SEQ ID NO: 140,
(K) the amino acid sequence represented by SEQ ID NO: 141,
(L) the amino acid sequence represented by SEQ ID NO: 142, and
(M) the amino acid sequence represented by SEQ ID NO: 143.

(13) A nucleic acid, comprising:

a nucleotide sequence that encodes the bispecific antibody or the antibody fragment thereof according to any one of (1) to (12).

(14) A recombinant vector, comprising:

the nucleic acid according to (13).

(15) A transformant, comprising:

the recombinant vector according to (14).

(16) A method for producing the bispecific antibody or the antibody fragment thereof according to any one of (1) to (12), comprising:

culturing the transformant according to (15) in a medium and collecting the bispecific antibody or the antibody fragment thereof from a culture supernatant.

(17) A reagent for detecting or measuring at least one of TRAILR2 and PSMA, comprising:

the bispecific antibody or the antibody fragment thereof according to any one of (1) to (12).

(18) A diagnostic agent for a disease related to a TRAILR2- and PSMA-expressing cell, comprising:

the bispecific antibody or the antibody fragment thereof according to any one of (1) to (12).

(19) The diagnostic agent according to (18),
wherein the disease related to a TRAILR2- and PSMA-expressing cell is a malignant tumor and a cancer.

(20) A therapeutic agent for a disease related to a TRAILR2- and PSMA-expressing cell, comprising:

the bispecific antibody or the antibody fragment thereof according to any one of (1) to (12) as an active ingredient.

(21) The therapeutic agent according to (20),
wherein the disease related to a TRAILR2- and PSMA-expressing cell is a malignant tumor and a cancer.

(22) A method for detecting or measuring at least one of TRAILR2 and PSMA,
by using the bispecific antibody or the antibody fragment thereof according to any one of (1) to (12).
(23) A diagnostic method for a disease related to a TRAILR2- and PSMA-expressing cell, comprising:

detecting or measuring at least one of TRAILR2 and PSMA by using the bispecific antibody or the antibody fragment thereof according to any one of (1) to (12).

(24) The diagnostic method according to (23),
wherein the disease related to a TRAILR2- and PSMA-expressing cell is a malignant tumor and a cancer.
(25) A therapeutic method for a disease related to a TRAILR2- and PSMA-expressing cell,
by using the bispecific antibody or the antibody fragment thereof according to any one of (1) to (12).
(26) The therapeutic method according to (25),
wherein the disease related to TRAILR2- and PSMA-expressing cell is a malignant tumor and a cancer.
(27) Use of the bispecific antibody or the antibody fragment thereof according to any one of (1) to (12), for the manufacture of a diagnostic agent for a disease related to a TRAILR2- and PSMA-expressing cell.
(28) The use according to (27),
wherein the disease related to a TRAILR2- and PSMA-expressing cell is a malignant tumor and a cancer.
(29) Use of the bispecific antibody or the antibody fragment thereof according to any one of (1) to (12), for the manufacture of a therapeutic agent for a disease related to a TRAILR2- and PSMA-expressing cell.
(30) The use according to (29),
wherein the disease related to a TRAILR2- and PSMA-expressing cell is a malignant tumor and a cancer.

Effects of the Invention

Only when binding to TRAILR2 and PSMA, a bispecific antibody or an antibody fragment thereof of the present invention induces various reactions in accordance with the activation of TRAILR2. Therefore, the bispecific antibody or the antibody fragment thereof of the present invention can be used as a therapeutic agent and a diagnostic agent which are for a disease related to TRAILR2- and PSMA-expressing cell.

Brief Description of Drawings

[Fig. 1] Fig. 1 is a diagram illustrating the structure of a bispecific antibody of the present invention. The thick line represents a linker that links two VHs.
[Fig. 2] Fig. 2 illustrates the results of analysis of the affinity of an anti-hPSMA monoclonal antibody or an hTRAILR2-hPSMA bispecific antibody to hPSMA/L929 cells by using a flow cytometer. The vertical axis represents the number of cells and the horizontal axis represents the fluorescence intensity. As the anti-hPSMA monoclonal antibody, 2A10 antibody or PI134 antibody was used. As the hTRAILR2-hPSMA bispecific antibody, E11-GL-PI134VH antibody, E11-CH1-PI134VH antibody, E11-CH1-PI101VH antibody, E11-CH1-PN7VH antibody, or E11-CH1-PN170VH antibody was used. The solid line represents the affinity of each antibody, and the dotted line represents the affinity of a known anti-DNP monoclonal antibody used as a negative control.
[Fig. 3] Fig. 3 illustrates the results of analysis of the affinity of an anti-hTRAILR2 monoclonal antibody, the anti-hPSMA monoclonal antibody, or the hTRAILR2-hPSMA bispecific antibody to hTRAILR2/L929 cells by using a flow cytometer. The vertical axis represents the number of cells and the horizontal axis represents the fluorescence intensity. As the anti-hTRAILR2 monoclonal antibody, E11 antibody was used. As the anti-hPSMA monoclonal antibody, the PI134 antibody was used. As the hTRAILR2-hPSMA bispecific antibody, E11-CH1-PI101VH, E11-CH1-PN7VH antibody, or E11-CH1-PN170VH antibody was used. The solid line represents the affinity of each antibody, and the dotted line represents the affinity of the anti-DNP monoclonal antibody used as a negative control.
[Fig. 4] Figs. 4(A) and 4(B) are diagrams illustrating the expression level of hTRAILR2 or hPSMA in PC3 cells [Fig. 4(A)] and hPSMA/PC3 cells [Fig. 4(B)]. The vertical axis represents the number of cells and the horizontal axis represents the fluorescence intensity. Each cell was stained with the anti-hTRAILR2 monoclonal antibody E11 antibody, the anti-hPSMA monoclonal antibody PI134 antibody, the TRAILR2-hPSMA bispecific antibody E11-CH1-PI101 VH antibody, or the anti-DNP monoclonal antibody used as a negative control.
[Fig. 5] Fig. 5 is a diagram illustrating the expression level of hTRAILR2 or hPSMA in LNCaP clone FGC cells. The vertical axis represents the number of cells and the horizontal axis represents the fluorescence intensity. Cells were stained with the anti-hTRAILR2 monoclonal antibody E11 antibody, the anti-hPSMA monoclonal antibody 2A10 antibody, or the anti-DNP monoclonal antibody used as a negative control.

[Fig. 6] Fig. 6 is a diagram illustrating the result of a cell proliferation test on PC3 cells using the anti-hTRAILR2 agonistic monoclonal antibody KMDA2 antibody or the hTRAILR2-hPSMA bispecific antibody E11-CH1-PI134VH antibody or the E11-CH1-PI101VH antibody. The vertical axis represents the cell survival rate (%) and the horizontal axis represents the antibody concentration (ng/mL). The anti-DNP monoclonal antibody was used as a negative control.

[Fig. 7] Fig. 7 is a diagram illustrating the result of a cell proliferation test on PC3 cells using the anti-hTRAILR2 agonistic monoclonal antibody KMDA2 antibody, the anti-hTRAILR2 monoclonal antibody E11 antibody or the hTRAILR2-hPSMAbispecific antibody E11-CH1-PI101VH antibody, the E11-CH1-PN7VH antibody, or the E11-CH1-PN170VH antibody. The vertical axis represents the cell survival rate (%) and the horizontal axis represents the antibody concentration (ng/mL). The anti-DNP monoclonal antibody was used as a negative control.

[Fig. 8] Fig. 8 is a diagram illustrating the result of a cell proliferation test on hPSMA/PC3 cells using the anti-hTRAILR2 agonistic monoclonal antibody KMDA2 antibody or the hTRAILR2-hPSMA bispecific antibody E11-GL-PI134VH antibody or the E11-CH1-PI134VH antibody. The vertical axis represents the cell survival rate (%) and the horizontal axis represents the antibody concentration (ng/mL). The anti-DNP monoclonal antibody was used as a negative control.

[Fig. 9] Fig. 9 is a diagram illustrating the result of a cell proliferation test on hPSMA/PC3 cells using the anti-hTRAILR2 agonistic monoclonal antibody KMDA2 antibody, the anti-hTRAILR2 monoclonal antibody E11 antibody or the hTRAILR2-hPSMA bispecific antibody E11-CH1-PI101VH antibody, E11-CH1-PN7VH antibody, or E11-CH1-PN170VH antibody. The vertical axis represents the cell survival rate (%) and the horizontal axis represents the antibody concentration (ng/mL). The anti-DNP monoclonal antibody was used as a negative control.

[Fig. 10] Fig. 10 is a diagram illustrating the result of a cell proliferation test on hPSMA/PC3 cells using the anti-hTRAILR2 agonistic monoclonal antibody KMDA2 antibody or the hTRAILR2-hPSMA bispecific antibody. The vertical axis represents the cell survival rate (%) and the horizontal axis represents the antibody concentration (ng/mL). As the hTRAILR2-hPSMA bispecific antibody, E11-CH1-PI101VH antibody, E11-CH1-PI105VH antibody, E11-CH1-PI108VH antibody, E11-CH1-PI115VH antibody, E11-CH1-PI118VH antibody, E11-CH1-PI127VH antibody, E11-CH1-PI143VH antibody, or E11-CH1-PL223VH antibody was used. The anti-DNP monoclonal antibody was used as a negative control.

[Fig. 11] Fig. 11 is a diagram illustrating the result of a cell proliferation test on LNCaP clone FGC cells using the anti-hTRAILR2 agonistic monoclonal antibody KMDA2 antibody, the anti-hTRAILR2 monoclonal antibody E11 antibody or the hTRAILR2-hPSMA bispecific antibody E11-CH1-PI101VH antibody, E11-CH1-PN7VH antibody, or E11-CH1-PN170VH antibody. The vertical axis represents the cell survival rate (%) and the horizontal axis represents the antibody concentration (ng/mL). The anti-DNP monoclonal antibody was used as a negative control.

[Fig. 12] Figs. 12(A) and 12(B) illustrates the result of detection of caspase activated when adding the hTRAILR2-hPSMA bispecific antibody E11-CH1-PI101VH antibody or the TRAILR2 ligand TRAIL/Apo2 to cells by using flow cytometry. The vertical axis represents the number of cells and the horizontal axis represents the fluorescence intensity. Fig. 12(A) illustrates the result when PC3 cells were used, and Fig. 12(B) illustrates the result when PSMA/PC3 cells were used. The anti-DNP monoclonal antibody was used as a negative control.

[Fig. 13] Fig. 13 is a diagram illustrating the result of a cell proliferation test on normal human hepatocytes using the anti-hTRAILR2 agonistic monoclonal antibody KMDA2 antibody, the anti-hTRAILR2 monoclonal antibody E11 antibody, the hTRAILR2-hPSMA bispecific antibody E11-CH1-PI101 VH antibody, or the TRAILR2 ligand TRAIL/Apo2. The vertical axis represents the cell survival rate (%) and the horizontal axis represents the protein concentration (ng/mL). The anti-DNP monoclonal antibody was used as a negative control.

[Fig. 14] Fig. 14 is a diagram illustrating the result of a cell proliferation test on normal human hepatocytes using the anti-hTRAILR2 agonistic monoclonal antibody KMDA2 antibody, the anti-hTRAILR2 monoclonal antibody E11 antibody, the hTRAILR2-hPSMA bispecific antibody E11-CH1-PN7VH antibody, or the TRAILR2 ligand TRAIL/Apo2. The vertical axis represents the cell survival rate (%) and the horizontal axis represents the protein concentration (ng/mL). The anti-DNP monoclonal antibody was used as a negative control.

[Fig. 15] Figs. 15(A) to 15(D) illustrate the structures of the hTRAILR2-hPSMA bispecific antibody E11-CH1-PN7VH antibody [Fig. 15(A)], the hTRAILR2-hPSMA bispecific antibody fragment E11-CH1-PN7VH $F(ab')_2$ [Fig. 15(B)], E11-CH1-PN7VH Fab [Fig. 15(C)], and the hTRAILR2-hPSMA heterobispecific antibody E11_PN7 Hetero antibody [Fig. 15(D)]. The thick line in the structures of Figs. 15(A) to 15(C) represents a linker.

[Fig. 16] Fig. 16 illustrates the result of a cell proliferation test on PC3 cells using each antibody or antibody fragment. The vertical axis represents the absorbance and the horizontal axis represents the antibody concentration (nM). As the antibody or the antibody fragment, the anti-hTRAILR2 agonistic monoclonal antibody KMDA2 antibody, the hTRAILR2-hPSMAheterobispecific antibody E11_PN7 Hetero antibody, the hTRAILR2-hPSMA bispecific antibody E11-CH1-PN7VH antibody, the hTRAILR2-hPSMA bispecific antibody fragment E11-CH1-PN7VH $F(ab')_2$ or E11-CH1-PN7VH Fab, or the anti-DNP monoclonal antibody as a negative control was used.

[Fig. 17] Fig. 17 illustrates the result of a cell proliferation test on hPSMA/PC3 cells using each antibody or antibody fragment. The vertical axis represents the absorbance and the horizontal axis represents the antibody concentration (nM). As the antibody or the antibody fragment, the anti-hTRAILR2 agonistic monoclonal antibody KMDA2 antibody, the hTRAILR2-hPSMAheterobispecific antibody E11_PN7 Hetero antibody, the hTRAILR2-hPSMA bispecific antibody E11-CH1-PN7VH antibody, the hTRAILR2-hPSMA bispecific antibody fragment E11-CH1-PN7VH F(ab')$_2$ or E11-CH1-PN7VH Fab, or the anti-DNP monoclonal antibody as a negative control was used.

Embodiments for Carrying Out the Invention

The present invention is related to a bispecific antibody or an antibody fragment thereof (hereinafter will be described as a bispecific antibody or an antibody fragment thereof of the present invention) comprising an antigen binding domain that binds to TRAILR2 and an antigen binding domain that binds to PSMA.

In the present invention, an antigen binding domain that binds to TRAILR2 or PSMA may be any domain as long as it specifically recognizes and binds to TRAILR2 or PSMA. For example, the domain may be in any form of a polypeptide such as an antibody, a ligand, a receptor, interacting molecules in nature, or the like which can be produced by genetic recombination technology, protein molecules and a fragment thereof, a conjugate of the low molecular weight proteins or natural products, and the like.

In addition, the antigen binding domain may be a binding protein recombined by using a binding domain of known binding molecules such as an antibody, a ligand, a receptor, and the like. Specific examples include a recombinant protein comprising a CDR of an antibody that binds to each antigen, an antibody variable region comprising the CDR, a recombinant protein comprising the antibody variable region and a binding domain of a ligand that binds to each antigen, and the like. Among these, it is preferable that the antigen binding domain is the antibody variable region in the present invention.

As the bispecific antibody or the antibody fragment thereof of the present invention, there is a bispecific antibody or an antibody fragment thereof which activates TRAILR2, only when binding to TRAILR2 and PSMA.

The bispecific antibody or the antibody fragment thereof of the present invention binds to TRAILR2 and PSMA on cells, and thereby trimerizes and activates the TRAILR2, and induces various cellular reactions such as association of the adaptor molecule FADD/MORT1 via the death domain in TRAILR2-expressed cell, caspase-8 activation, and the like. As a result, in each cell, the inhibition of cell proliferation, cell death by apoptosis and the like, and the like are induced.

That is, specific examples of the bispecific antibody or the antibody fragment thereof of the present invention include a bispecific antibody or an antibody fragment thereof, and the like which causes at least one of reactions selected from the group consisting of association of FADD/MORT1 in TRAILR2-expressed cell, caspase-8 activation, and induction of the inhibition of the proliferation of TRAILR2-expressed cell and the cell death when binds to TRAILR2 and PSMA.

The bispecific antibody or the antibody fragment thereof of the present invention can bind to TRAIL2 and PSMA which are expressed in the same cell, or can bind to TRAILR2 and PSMA which are expressed in different cells.

In addition, in the present invention, the cell death includes any one of apoptosis and necrosis, but preferably means apoptosis.

TRAILR2 of the present invention is used synonymously with death receptor 5 (DR5), killer/dr5, and TRICK2. Examples of TRAILR2 include human TRAILR2 comprising an amino acid sequence represented by NCBI accession No. NP_003833, mouse TRAILR2 comprising an amino acid sequence represented by NCBI accession No. NP_064671, and the like in NCBI (http://www.ncbi.nlm.nih.gov/). Furthermore, examples include a polypeptide comprising an amino acid sequence in which one or more of an amino acid is lost, substituted, or added in an amino acid sequence represented by NCBI accession No. NP_003833 or NCBI accession No. NP_064671, and that has a function of TRAILR2.

Examples of TRAILR2 of the present invention also include a polypeptide comprising an amino acid sequence having 70% or higher, preferably 80% or higher, and more preferably 90% or higher homology to an amino acid sequence represented by NCBI accession No. NP_003833 or NCBI accession No. NP_064671, and a polypeptide that is formed from an amino acid sequence having most preferably 95%, 96%, 97%, 98%, and 99% or higher homology, and that has a function of TRAILR2.

The polypeptide comprising an amino acid sequence in which one or more of an amino acid residue is lost, substituted, or added in an amino acid sequence represented by NCBI accession No. NP_003833 or NCBI accession No. NP_064671 can be obtained by, for example, introducing site-specific mutations to DNA that encodes an amino acid sequence represented by NCBI accession No. NP_003833 or NCBI accession No. NP_064671 by using the site-directed mutagenesis [Molecular Cloning, A Laboratory Manual, Second Edition, Cold Spring Harbor Laboratory Press (1989), Current Protocols in Molecular Biology, John Wiley & Sons (1987-1997), Nucleic Acids Research, 10, 6487 (1982), Proc. Natl. Acad. Sci. USA, 79, 6409 (1982), Gene, 34, 315 (1985), Nucleic Acids Research, 13,4431 (1985), Proceeding of the National Academy of Sciences in USA, 82, 488 (1985)], or the like. The number of an amino acids that are lost, substituted, or added is not particularly limited, and is preferably one to tens, for example, 1 to 20, and more preferably one to a few, for example, 1 to 5 amino acids.

Examples of a gene that encodes TRAILR2 include 294$^{th}$ to 1616$^{th}$ nucleotide sequences represented by NCBI accession No. NM_003842 of human TRAILR2, a nucleotide sequence represented by NCBI accession No. NM_020275 of mouse TRAILR2, and the like. Furthermore, examples of a gene that encodes TAILR2 of the present invention also include a gene comprising DNA that encodes a polypeptide that is consisting of a nucleotide sequence in which one or more of a nucleotide is deleted, substituted, or added in the $294^{th}$ to $1616^{th}$ nucleotide sequences represented by NCBI accession No. NM_003842 of human TRAILR2, and that has a function of TRAILR2, a gene comprising DNA that encodes a polypeptide that is consisting of a nucleotide sequence having preferably 60% or higher homology to the $294^{th}$ to $1616^{th}$ nucleotide sequences represented by NCBI accession No. NM_003842, more preferably a nucleotide sequence having 80% or higher homology, and further more preferably a nucleotide sequence having 95% or higher homology, and that has a function of TRAILR2, a gene comprising DNA that encodes a polypeptide that is consisting of DNA that hybridizes with the $294^{th}$ to $1616^{th}$ DNAs of NCBI accession No. NM 003842 under stringent conditions, and that has a function of TRAILR2, and the like.

DNA that hybridizes under stringent conditions means, for example, hybridizable DNA that can be obtained by using a colony hybridization method, a plaque hybridization method, a southern blot hybridization method, a DNA microarray method, and the like using DNA comprising the $294^{th}$ to $1616^{th}$ nucleotide sequences represented by NCBI accession No. NM_003842 as a probe. Specifically, it is possible to exemplify DNA that can be identified by washing a filter or a glass slide under the condition of 65°C using a SSC solution of the concentration of 0.1 to 2 times (a composition of the SSC solution with the concentration of 1 time is 150 mmol/L of sodium chloride and 15 mmol/L of sodium citrate), after performing hybridization [Molecular Cloning, A Laboratory Manual, Second Edition, Cold Spring Harbor Laboratory Press (1989), Current Protocols in Molecular Biology, John Wiley & Sons (1987-1997), DNA Cloning 1: Core Techniques, A Practical Approach, Second Edition, Oxford University (1995)] at 65°C under the presence of 0.7 to 1.0 mol/L of sodium chloride using a filter or a glass slide on which DNA derived from hybridized colonies or plaque, a PCR product or DNA oligo having the sequence are fixed. Examples of hybridizable DNA include DNA having preferably 60% or higher homology to the $294^{th}$ to $1616^{th}$ nucleotide sequences represented by NCBI accession NO: NM_003842, more preferably DNA having 80% or higher homology, and further more preferably DNA having 95% or higher homology.

Genetic polymorphism is often recognized in a nucleotide sequence of a gene that encodes proteins of a eukaryote. A gene that encodes TRAILR2 of the present invention also includes a gene in which small scale mutations arise in a nucleotide sequence by such polymorphism among genes used in the present invention.

A value of homology in the present invention may be a value calculated by using a homology detection program known to those skilled in the art unless particularly specified. Regarding a nucleotide sequence, there are a value calculated by using a default parameter of BLAST [J. Mol. Biol., 215, 403 (1990)], and the like. Regarding an amino acid sequence, there are a value calculated by using a default parameter of BLAST2 [Nucleic Acids Research, 25, 3389 (1997), Genome Research, 7, 649 (1997), http://www.ncbi.nlm.nih.gov/Education/BLASTinfo/information3.htm], and the like.

Regarding the default parameters, G (Cost to open gap) is 5 for a nucleotide sequence and 11 for an amino acid sequence, -E (Cost to extend gap) is 2 for a nucleotide sequence and 1 for an amino acid sequence, -q (Penalty for nucleotide mismatch) is -3, -r (reward for nucleotide match) is 1, -e (expect value) is 10, -W (wordsize) is 11 residue for a nucleotide sequence and 3 residue for an amino acid sequence, -y [Dropoff (X) for blast extensions in bits] is 20 for the blastn and 7 for programs other than the blastn, -X (X dropoff value for gapped alignment in bits) is 15, and -Z (final X dropoff value for gapped alignment in bits) is 50 for the blastn and 25 for programs other than the blastn (http://www.ncbi.nlm.nih.gov/blast/html/blastcgihelp.html).

A polypeptide that is consisting of partial amino acid sequences of TRAILR2 can be produced by a method known for those skilled in the art, and can be produced by, for example, deleting a part of DNA that encodes an amino acid sequence represented by NCBI accession No. NP_003833 and culturing a transfectant into which an expression vector including the deleted part is introduced. In addition, for example, a polypeptide comprising an amino acid sequence in which one or more of an amino acid is deleted, substituted, or added in partial amino acid sequences represented by NCBI accession No. NP_003833 can be obtained by using the same method as above based on the polypeptide or DNA produced by the above method. Furthermore, a polypeptide that is consisting of partial amino acid sequences of TRAILR2, or a polypeptide comprising an amino acid sequence in which one or more of an amino acid is deleted, substituted, or added in partial amino acid sequences of TRAILR2 can be produced by using a chemical synthesis method such as a fluorenylmethyloxycarbonyl (Fmoc) method, a t-butyloxycarbonyl (tBoc) method, and the like.

Examples of an extracellular region of TRAILR2 of the present invention include region in which an amino acid sequence represented by NCBI accession No. NP -003833 of human TRAILR2 is predicted by using a known transmembrane region prediction program SOSUI (http://sosui.proteome.bio.tuat.ac.jp/sosuiframe0.html), TMHMM ver.2 (http://www.cbs.dtu.dk/services/TMHMM-2.0/), ExPASy Proteomics Server (http://Ca.expasy.org/), or the like. Specifically, there are $54^{th}$ to $212^{th}$ amino acid sequences among an amino acid sequence represented by NCBI accession No. NP_003833.

Examples of a function of TRAILR2 include a function in which when TRAIL (Apo-2L) ligand binds to TRAILR2, a trimer of TRAILR2 is formed, which promotes the association of the adaptor molecule FADD/MORT1 via the death domain in the cells and causes the activation of caspase-8 that binds to FADD/MORT1, and as a result, the inhibition of cell proliferation and the apoptosis are induced.

PSMA of the present invention is used synonymously with folate hydrolase (FOLH), glutamate carboxypeptidase II (GCP2), prostate-specific membrane antigen (PSM or PSMA), N-acetylated alpha-linked acidic dipeptidase 1 (NAALAD1), and NAALADase I.

Examples of PSMA include human PSMA comprising an amino acid sequence represented by NCBI accession No. NP_004467 or SEQ ID NO: 112, mouse PSMA comprising an amino acid sequence represented by NCBI accession No. NP_058050, or the like. In addition, examples include a polypeptide that is formed from an amino acid sequence in which one or more of an amino acid is lost, substituted, or added in an amino acid sequence represented by NCBI accession No. NP_004467, SEQ ID NO: 112, or NCBI accession No. NP_058050, and that has a function of PSMA.

Examples of PSMA of the present invention also include a polypeptide comprising an amino acid sequence having preferably 70% or higher, more preferably 80% or higher, and further more preferably 90% or higher homology to an amino acid sequence represented by NCBI accession No. NP_004467, SEQ ID NO: 112, or NCBI accession No. NP_058050, and most preferably a polypeptide that is consisting of an amino acid sequence having 95%, 96%, 97%, 98%, and 99% or higher homology, and that has a function of PSMA.

The polypeptide comprising an amino acid sequence in which one or more of an amino acid residue is deleted, substituted, or added in an amino acid sequence represented by NCBI accession No. NP_004467, SEQ ID NO: 112, or NCBI accession No. NP_058050 can be obtained by, for example, introducing site-specific mutations to DNA that codes for an amino acid sequence represented by NCBI accession No. NP_004467, SEQ ID NO: 112, or NCBI accession No. NP_058050 by using the site-directed mutagenesis described above or the like. The number of an amino acids that are deleted, substituted, or added is not particularly limited, and is preferably one to tens, for example, 1 to 20, and more preferably one to a few, for example, 1 to 5 amino acids.

Examples of a gene that encodes PSMA of the present invention include a gene of human PSMA comprising a nucleotide sequence represented by NCBI accession No. NM_004476 or SEQ ID NO: 64, or a gene of mouse PSMA including a nucleotide sequence represented by NCBI accession No. NMO16770. Furthermore, examples of a gene that codes for PSMA of the present invention include a gene comprising DNA consisting of a nucleotide sequence in which one or more of a nucleotide is deleted, substituted, or added in a nucleotide sequence represented by NCBI accession No. NM_004476, SEQ ID NO: 64, or NCBI accession No. NM016770, and that encodes a polypeptide which has a function of PSMA, a gene comprising DNA consisting of a nucleotide sequence having 60% or higher homology to a nucleotide sequence represented by NCBI accession No. NM_004476, SEQ ID NO: 64, or NCBI accession No. NM016770, preferably a nucleotide sequence having 80% or higher homology, and more preferably a nucleotide sequence having 95% or higher homology, and that encodes a polypeptide which has a function of PSMA, a gene comprising DNA consisting of DNA that hybridizes with DNA represented by NCBI accession No. NM_004476, SEQ ID NO: 64, or NCBI accession No. NM016770 under stringent conditions, and that encodes a polypeptide which has a function of PSMA, and the like.

Examples of a function of PSMA include a function as glutamic acid carboxypeptidase, N-acetyl $\alpha$-linked acidic dipeptidase, and folate hydrolase.

In the present invention, binding to TRAILR2 means recognizing and binding to an extracellular region of TRAILR2. In addition, in the present invention, binding to PSMA means recognizing and binding to an extracellular region of PSMA.

In the present invention, binding of an antibody or an antibody fragment thereof to at least one of TRAILR2 and PSMA can be confirmed by a method by which the affinity of the antibody to a cell which expresses at least one of TRAILR2 and PSMA is confirmed by using, for example, a known immunological detection method, preferably a fluorescent cell straining method, and the like. Furthermore, it is possible to use known immunological detection methods [Monoclonal Antibodies - Principles and Practice, Third Edition, Academic Press (1996), Antibodies - A Laboratory Manual, Cold Spring Harbor Laboratory (1988), A manual for monoclonal antibody experiments, Kodansha scientific books (1987)], and the like in combination.

In the present invention, that an antibody has apoptotic activity means that the cell death is induced by binding of an antibody to TRAILR2 on the cells. That the bispecific antibody of the present invention has apoptotic activity can be confirmed by a method below. For example, TRAILR2- and PSMA-expressing cells were seeded in a 96-well plate, an antibody was added thereto and culturing was performed for a certain period of time, followed by reaction with WST-8 reagent (manufactured by Dojindo Molecular Technologies, Inc.). The absorbance was measured at 450 nm using a plate reader, and the cell survival rate was measured.

An antibody is a protein derived from a gene (called "antibody genes") that encodes all or a part of a heavy chain variable region, a heavy chain constant region, a light chain variable region and a light chain constant region constituting an immunoglobulin. The antibody of the present invention includes an antibody or an antibody fragment having any one of classes and subclasses of immunoglobulins.

The H chain refers to a polypeptide with heavier molecular weight among two types of polypeptides constituting immunoglobulin molecules. The heavy chain determines a class and a subclass of an antibody. IgA, IgD, IgE, IgG, and IgM include $\alpha$ chain, $\delta$ chain, $\varepsilon$ chain, $\gamma$ chain, and $\mu$ chain as the heavy chain, respectively, and the heavy chain constant region is characterized by different amino acid sequences. The light chain (L chain) refers to a polypeptide with lighter molecular weight among two types of polypeptides constituting immunoglobulin molecules. For a human antibody, two types of the light chain, $\kappa$ chain and $\lambda$ chain are present.

The V region normally refers to a region which is present in an amino acid sequence of the N-terminus of an immunoglobulin and rich in diversity. Because a part other than the variable region has less diverse structures, it is called a constant region (C region). An antigen binding site is formed by association of each variable region of the heavy chain and the light chain, and the affinity of the antibody to the antigen is determined.

In the heavy chain of a human antibody, a variable region corresponds to the 1$^{st}$ to 117$^{th}$ amino acid sequences in the EU index by Kabat et al. (Kabat et al., Sequences of proteins of immunological interest, 1991 Fifth edition), and a constant region corresponds to the 118$^{th}$ and succeeding amino acid sequences. In the light chain of a human antibody, a variable region corresponds to the 1$^{st}$ to 107$^{th}$ amino acid sequences in the Kabat numbering by Kabat et al., and a constant region corresponds to the 108$^{th}$ and succeeding amino acid sequences. Hereinafter, the heavy chain variable region or the light chain variable region will be abbreviated to VH or VL.

The antigen binding site is a site that recognizes and binds to an antigen of an antibody, and refers to a site that forms a complementary three-dimensional structure with respect to an antigenic determinant (epitope). The antigen binding site generates strong molecular interactions between the antigenic determinants. The antigen binding site is constituted of VH and VL including at least three CDRs. For a human antibody, VH and VL include three CDRs, respectively. These CDRs are called CDR 1, CDR 2, and CDR 3, respectively in order from the N-terminus side.

In the constant region, the heavy chain constant region or the light chain constant region is referred as CH or CL, respectively. CH is classified into $\alpha$ chain, $\delta$ chain, $\varepsilon$ chain, $\gamma$ chain, and $\mu$ chain which are subclasses of the heavy chain. CH is constituted of a CH1 domain, a hinge domain, a CH2 domain, and a CH3 domain arranged in order from the N-terminus side, and the CH2 domain and the CH3 domain together are called a Fc region. On the other hand, CL is classified into two subclasses called a C$\lambda$ chain and a C$\kappa$ chain.

In the present invention, the antibody that binds to TRAILR2 refers to a monoclonal antibody that recognizes and binds to the extracellular region of TRAILR2. In addition, in the present invention, the antibody that binds to PSMA refers to a monoclonal antibody that recognizes and binds to the extracellular region of PMSA. Furthermore, in the present invention, the antibody includes a polyclonal antibody and an oligoclonal antibody.

A monoclonal antibody is an antibody that is secreted by antibody-producing cells retaining monoclonality, and recognizes a single epitope (also called as the antigenic determinant). The monoclonal antibody molecules have the same amino acid sequence (primary structure) and have a unitary structure. The polyclonal antibody is a group of antibody molecules that are secreted by the antibody-producing cells of different clones. The oligoclonal antibody is a group of antibody molecules in which a plurality of different monoclonal antibodies are mixed.

The epitope is a structural site of an antigen to which an antibody binds by recognizing it. Examples of the epitope include a single amino acid sequence, a three-dimensional structure made of an amino acid sequence, and a three-dimensional structure made of an amino acid sequence to which a sugar chain binds and an amino acid sequence to which a sugar chain binds to which the monoclonal antibody binds by recognizing it, , and the like.

Examples of the monoclonal antibody of the present invention include an antibody that is produced from hybridoma, and a gene recombinant antibody that is produced by a transfectant that is transformed with an expression vector including antibody genes.

The hybridoma can be produced by, for example, producing an antigen, obtaining antibody-producing cells having antigenic specificity from an animal to which the antigen was immunized, and then fusing antibody-producing cells with myeloma cells. A desired monoclonal antibody can be obtained either by culturing the hybridoma or by administering the hybridoma to an animal, causing the hybridoma to become an ascites tumor, isolating the culture solution or ascites, and purifying. As the animal to which the antigen is immunized, any animals can be used as long as they enable the production of hybridoma, and mice, rats, hamsters, rabbits, and the like are favorably used. In addition, the hybridoma can be produced by obtaining cells having antibody producibility from such immune animals, subjecting the cells to an immune step in vitro, and then fusing the cells with myeloma cells.

Examples of the gene recombinant antibody of the present invention include a recombinant mouse antibody, a recombinant rat antibody, a recombinant hamster antibody, a recombinant rabbit antibody, a human chimeric antibody (also called a chimeric antibody), a humanized antibody (also called a CDR-implanted antibody), a human antibody, and the like, which are produced using gene recombinant technology. Regarding the gene recombinant antibody, in accordance with animal species as a target and the purpose, it is possible to determine which animal species-derived heavy chain and the light chain variable regions and the constant region, is to be applied. For example, if the animal species as a target is human, it is possible to set the variable region to be derived from humans, or mice and the like that are non-human animals, and the constant region and the linker to be derived from humans.

The chimeric antibody refers to an antibody that is formed from VH and VL of an antibody of animals other than humans (non-human animals) and from CH and CL of a human antibody. As the non-human animals, any one of mice, rats, hamsters, rabbits, and the like can be used as long as they enable the production of hybridoma. The chimeric antibody can be produced by obtaining cDNA that encodes VH and VL from hybridoma derived from the non-human animals producing the monoclonal antibody, inserting the cDNA into each an expression vector for animal cells having DNA that encodes CH and CL of a human antibody, and thereby constructing a chimeric antibody-expressing vector, and introducing it to animal cells to be expressed.

The humanized antibody refers to an antibody in which CDR of VH and VL from an antibody of the non-human animals is implanted to CDR corresponding to VH and VL from a human antibody. A region other than CDR of VH and VL is called a framework region (hereinafter will be marked as FR). The humanized antibody can be produced by constructing cDNA that encodes an amino acid sequence of VH formed from an amino acid sequence of FR of VH from an arbitrary human antibody and from an amino acid sequence of CDR of VH from an antibody of the non-human animals, and cDNA encodes for an amino acid sequence of VL formed from an amino acid sequence of FR of VL from an arbitrary human antibody and from an amino acid sequence of CDR of VL from an antibody of the non-human animals, inserting each of them to an expression vector for animal cells comprising DNA that encodes CH and CL of a human antibody respectively, and thereby constructing a humanized antibody-expressed vector, and introducing it to animal cells to be expressed.

The human antibody is originally an antibody that is naturally present in the human body, but also includes antibodies that are obtained from a human antibody phage library and human antibody-producing transgenic animals which are produced by technological advancement in genetic engineering, cellular engineering, and developmental engineering,.

The antibody that is naturally present in the human body can be obtained by, for example, infecting human peripheral blood lymphocytes with the EB virus or the like so to be immortalized, culturing lymphocytes that produce the antibody by cloning, and then purifying the antibody from the culture supernatant.

The human antibody phage library is a library in which an antibody fragment such as Fab and scFv is expressed on the surface of phages by inserting antibody genes produced from human B cells to phage genes. It is possible to collect the phages of which an antibody fragment having a desired antigen binding activity is expressed on the surface thereof by using binding activity with respect to a substrate to which an antigen is fixed as an index from the library. Furthermore, the antibody fragment can be converted to human antibody molecules formed from two whole H chains and two whole L chains by using the genetic engineering technique.

The human antibody-producing transgenic animals mean animals of which human antibody genes are incorporated into the cells. Specifically, for example, it is possible to produce a human antibody-producing transgenic mouse by introducing human antibody genes to mouse ES cells, implanting the ES cells to the mouse early embryo, and then generating its individuality. A human antibody derived from the human antibody-producing transgenic animals can be produced by obtaining hybridoma using a hybridoma production method performed for normal non-human animals, culturing it, and generating and accumulating an antibody in the culture supernatant.

CH of the gene recombinant antibody may be any one as long as it belongs to a human immunoglobulin, but CH of the human immunoglobulin G (hIgG) class is preferable. Furthermore, it is possible to use any one of subclasses of hIgG1, hIgG2, hIgG3, and hIgG4 which belong to the hIgG class. In addition, CL of the gene recombinant antibody may be any one as long as it belongs to a human immunoglobulin, and it can be CL of the κ class or the λ class.

In the present invention, the bispecific antibody is an antibody that comprises two types of antigen binding domain with different specificities. Each antigen binding domain of the bispecific antibody may bind to different epitopes of a single antigen or bind to different antigens.

A single molecule of bispecific antibody binds to different epitopes of a single antigen or different antigens, respectively via one or more of antigen binding domains bind to, that is, binding of the antibody is monovalent or higher. For example, in the present invention, in a case where a single molecule of bispecific antibody comprises two antigen binding domains that bind to TRAILR2 and two antigen binding domains that bind to PSMA, the bispecific antibody divalently binds to each TRAILR2 and PSMA.

In the present invention, one molecule of the bispecific antibody can binds to TRAILR2 or PSMA in any valences, but it is preferable that the antibody binds at least divalently to each TRAILR2 and PSMA.

In addition, the bispecific antibody of the present invention also includes an antibody comprising multiple antigen binding domains that are linked via an appropriate linker such as a linker comprising an immunoglobulin domain or a fragment thereof.

In the bispecific antibody of the present invention, a position of an antigen binding domain that binds to TRAILR2 and an antigen binding domain that binds to PSMA in the antibody can be appropriately selected.

The bispecific antibody of the present invention can be produced by using a known production technology ([Nature Protocols, 9, 2450-2463 (2014)], International Publication No. 1998/050431, International Publication No. 2001/7734, International Publication No. 2002/002773, and International Publication No. 2009/131239) or the like.

In the bispecific antibody of the present invention, the antigen binding domain that binds to TRAILR2 may be located closer to the N-terminus side or C-terminus side than the antigen binding domain that binds to PSMA, but it is preferable to be located closer to the N-terminus side.

As the bispecific antibody of the present invention, the V region of the antibody can be used as an antigen binding domain, and examples thereof include an antibody comprising a heavy chain comprising multiple VHs for one heavy chain and an antibody comprising two heavy chains in which one VH is respectively comprised.

Specific examples of the bispecific antibody comprising a heavy chain comprising multiple VHs for one heavy chain include an antibody comprising a heavy chain comprising two or more VHs that are linked via a linker comprising an immunoglobulin domain or a fragment thereof

In a case of binding three or more of VHs, different immunoglobulin domains or a fragment thereof may be used, and the same immunoglobulin domains or a fragment thereof may be used. In addition, in a case of linking two or more of VHs, it is possible to change the length or the type of an immunoglobulin domain or a fragment thereof so that each VH can bind specifically to an antigen.

Specifically, the bispecific antibody of the present invention has at least one of the features represented in (a) to (e) below.

(a) One heavy chain polypeptide comprises multiple different VHs (for example, 2 to 5) and the VHs are not close to each other and separated.

(b) VHs are tandemly linked via a polypeptide linker comprising 10 or more amino acids. Specifically, VHs are linked via, for example, a linker comprising all or a part of amino acid sequences of an immunoglobulin domain.

(c) A light chain is associated with a heavy chain, which forms an antigen binding site.

(d) As illustrated in Fig. 1, a structure is formed of two heavy chain polypeptides and at least four light chain polypeptides, the two heavy chain polypeptides are linked to each other via disulfide bonds in a hinge region, and the light chain polypeptides and the heavy chain polypeptides are linked to each other via disulfide bonds.

(e) A constant region of the heavy chain is formed of, for example, whole or a portion of a constant region of a natural antibody heavy chain (for example, CH1 fragment, CH1, CH2, CH3, CH1-hinge, CH1-hinge-CH2, CH1-hinge-CH2-CH3, and the like).

In the present invention, it is possible to appropriately select the position of VH of an antibody that binds to TRAILR2 and the position of VH of an antibody that binds to PSMA, which are comprised in the bispecific antibody. For example, regarding the bispecific antibody having the structure illustrated in Fig. 1, the VH of an antibody that binds to TRAILR2 may be located closer to the N-terminus side or C-terminus side than the VH of an antibody that binds to PSMA, but it is preferable to be located closer to the N-terminus side.

In the present invention, the VLs comprised in the bispecific antibody may be the same VLs or different VLs. The VH of the bispecific antibody that comprises the same VL and that can binds to two different epitopes on two different antigens or the same antigen, may be an optimized or altered VH so that each variable region can bind specifically to an antigen or an epitope. For example, it is possible to select an appropriate VH by using a method such as screening with amino-acid alteration, or phage display.

In the present invention, the linker refers to a chemical structure through which multiple antigen binding domains are linked, and is preferably polypeptides. Examples of the linker used for the bispecific antibody of the present invention include a linker comprising all or a part of amino acid sequences of an immunoglobulin domain, a linker comprising all or a part of amino acid sequences of a linker of multiple immunoglobulin domains, and the like.

Amino acid sequences selected from an immunoglobulin as a linker comprising a part of amino acid sequences of an immunoglobulin domain may be intermittent or consecutive, but consecutive amino acid sequences are preferable.

In the present invention, the immunoglobulin domain comprises a peptide that comprises amino acid sequences similar to the immunoglobulin and that is consisting of approximately 100 amino acid residues in which at least two cysteine residues are present as the smallest units. In the present invention, the immunoglobulin domain comprises polypeptides comprising plural of the above small units of immunoglobulin domains. Examples of the immunoglobulin domain include VH, CH1, CH2, and CH3 of an immunoglobulin heavy chain, VL and CL of an immunoglobulin light chain, and the like.

The types of animals of the immunoglobulin are not particularly limited, but humans are preferable. In addition, the subclasses of the constant region of the immunoglobulin heavy chain may be any one of IgD, IgM, IgG1, IgG2, IgG3, IgG4, IgA1, IgA2, and IgE, and IgG-derived and IgM-derived subclasses are preferable. In addition, the subclasses of the constant region of the immunoglobulin light chain may be any one of κ and λ

Furthermore, the immunoglobulin domain is also present in proteins other than the immunoglobulin. Examples thereof include an immunoglobulin domain comprised in proteins such as major histocompatibility antigen (MHC), CD1, B7, and T cell receptor (TCR), which belong to the immunoglobulin superfamily. As the immunoglobulin domain used for the bispecific antibody of the present invention, any one of the immunoglobulin domains can be applied.

In a case of the human antibody, CH1 refers to a region having the $118^{th}$ to $215^{th}$ amino acid sequences represented in the EU index. Similarly, the hinge region refers to a region comprising the 216th to 230th amino acid sequences represented in the EU index by Kabat et al., CH2 refers to a region comprising the 231th to 340th amino acid sequences represented in the EU index by Kabat et al., and CH3 refers to a region comprising the 341th to 446th amino acid sequences represented in the EU index by Kabat et al. Between CH1 and CH2, an amino acid region with great flexibility called a hinge region is present.

CL refers to a region comprising the 108th to 214th amino acid sequences represented in the Kabat numberings in a case of the κ chain of the human antibody, and refers to a region comprising the 108th to 215th amino acid sequences in a case of the λ chain.

Examples of the linker used for the bispecific antibody of the present invention include an immunoglobulin domain formed of CH1-hinge-CH2-CH3 arranged in order in the direction from the N-terminus to the C-terminus, an immunoglobulin domain formed of CH1-hinge-CH2, an immunoglobulin domain formed of CH1-hinge, an immunoglobulin domain formed of CH1,a fragment in the N-terminus side of CH1, a CH1 fragment formed of a 14-amino acid residue of which the 14th amino acid residue is Cys and a CH1 fragment formed of the 1st to 14th amino acid residues in the N-terminus of CH1, and the fragments in which one or more of the amino acid residues altered from the amino acid sequences of the fragments of the immunoglobulin domain, but examples are not limited thereto.

In addition, in the present invention, as an example of the linker, it is possible to appropriately combine and use all or some of the fragments having amino acid sequences formed of CH1, hinge, CH2, and CH3 of an antibody. Furthermore, it is possible to use the fragments by partially deleting the amino acid sequences or changing orders thereof. Furthermore, the subclasses of the antibody used for the linker are not particularly limited, but IgM or IgG4 is preferable, and IgG4 is more preferable.

In the present invention, specific examples of the linker include a linker formed of the 1st to 14th 14-amino acid residues in the N-terminus of CH1 of IgG4 represented by SEQ ID NO: 11, a linker formed of the 1st to 14th amino acid residues in the N-terminus of CH1 of IgM represented by SEQ ID NO: 12, a linker formed of CH1 of IgG4 represented by SEQ ID NO: 93, and the like, and the linker formed of CH1 of IgG4 represented by SEQ ID NO: 93 is preferable.

The bispecific antibody or the antibody fragment thereof of the present invention also includes an antibody and a fragment thereof in which one or more of amino acid residues are deleted, added, substituted, or inserted regarding the amino acid sequences constituting the bispecific antibody or the antibody fragment thereof of the present invention, and which includes the same activity as the above the antibody or the fragment thereof.

The number of amino acids which are deleted, substituted, inserted, and/or added is one or more, and is not particularly limited, and is the amount that can be deleted, substituted, inserted, or added by using a known method such as the site-directed mutagenesis described in Molecular Cloning, The Second Edition, Cold Spring Harbor Laboratory Press (1989), Current Protocols in Molecular Biology, John Willy & Sons (1987-1997), Nucleic Acids Research, 10, 6487 (1982), Proc. Natl. Acad. Sci., USA, 79,6409 (1982), Gene, 34, 315 (1985), Nucleic Acids Research, 13,4431 (1985), Proc. Natl. Acad. Sci USA, 82, 488 (1985), and the like. For example, it is one to tens, preferably 1 to 20, more preferably 1 to 10, and further more preferably 1 to 5.

That one or more of amino acid residues are deleted, substituted, inserted, or added in the amino acid sequences of the bispecific antibody of the present invention as above indicates the following matter. This means in one or plural arbitrary amino acid sequences represented by the same SEQ ID NO:, there is one or plural amino acid residues being deleted, substituted, inserted, or added. In addition, there is a case where deletion, substitution, insertion, or addition occurs at the same time, and either of natural or unnatural amino acid residues may be substituted, inserted, or added.

Examples of the natural amino acid residues include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine, and the like.

Preferable examples of amino acid residues capable of being substituted for each other are shown below. Amino acid residues included in the same group are capable of being substituted for each other.

A group: leucine, isoleucine, norleucine, valine, norvaline, alanine, 2-aminobutanoic acid, methionine, O-methylserine, t-butylglycine, t-butylalanine, cyclohexylalanine
B group: aspartic acid, glutamic acid, isoaspartic acid, isoglutamic acid, 2-aminoadipic acid, 2-aminosuberic acid
C group: asparagine, glutamine
D group: lysine, arginine, ornithine, 2,4-diaminobutanoic acid, 2,3-diaminopropionic acid
E group: proline, 3-hydroxyproline, 4-hydroxyproline
F group: serine, threonine, homoserine
G group: phenylalanine, tyrosine

The bispecific antibody or the antibody fragment thereof of the present invention includes an antibody having one of any amino acid residues in which post-translational modification occurred. Regarding the post-translational modification, there are, for example, deletion of a lysine residue in the C-terminus of the H chain (lysine clipping), conversion to pyroglutamic acid (puroGlu) of a glutamine residue in the N-terminus of polypeptide, and the like [Beck et al, Analytical Chemistry, 85, 715-736 (2013)].

Specific examples of the bispecific antibody of the present invention include any one of bispecific antibodies selected from the group consisting of (1) to (3) below, and the like.

(1) a bispecific antibody comprising V region of the antibody that binds to TRAILR2 and V region of the antibody that binds to PSMA
(2) a bispecific antibody comprising CDRs 1 to 3 of VH and CDRs 1 to 3 of VL of the antibody that binds to TRAILR2, and CDRs 1 to 3 of VH and CDRs 1 to 3 of VL of the antibody that binds to PSMA, and
(3) a bispecific antibody comprising VH and VL of the antibody that binds to TRAILR2, and VH and VL of the antibody that binds to PSMA

In the bispecific antibody described in (2) above, CDRs 1 to 3 of VL of the antibody that binds to TRAILR2 and CDRs 1 to 3 of VL of the antibody that binds to PSMA may be the same or different from each other, but it is preferable that they are the same.

In addition, in the bispecific antibody described in (3) above, VL of the antibody that binds to TRAILR2 and VL of the antibody that binds to PSMA may be the same or different from each other, but it is preferable that they are the same.

Further specific examples of the bispecific antibody of the present invention include a bispecific antibody in which the amino acid sequences of CDRs 1 to 3 of VH of the antibody that binds to TRAILR2 comprise the amino acid sequences represented by SEQ ID NOs: 70 to 72, respectively, and the amino acid sequences of CDRs 1 to 3 of VL comprise the amino acid sequences represented by SEQ ID NOs: 74 to 76, respectively.

As one aspect of the antibody that binds to TRAILR2 in which the amino acid sequences of CDRs 1 to 3 of VH comprise the amino acid sequences represented by SEQ ID NOs: 70 to 72, respectively, and the amino acid sequences of CDRs 1 to 3 of VL comprise the amino acid sequences represented by SEQ ID NOs: 74 to 76, respectively, there are the anti-human TRAILR2 monoclonal antibody E11 antibody, and the like.

The bispecific antibody of the present invention also includes a bispecific antibody in which the amino acid sequences of CDRs 1 to 3 of VH of the antibody that binds to TRAIL2 and the amino acid sequences of CDRs 1 to 3 of VL are at least 35%, at least 40%, at least 45%, at least 50%, at least 55%, at least 60%, at least 65%, at least 70%, at least 75%, at least 80%, at least 85%, at least 90%, at least 95%, and at least 99% homology to the amino acid sequences represented by SEQ ID NOs: 70 to 72, respectively, and the amino acid sequences represented by SEQ ID NOs: 74 to 76, respectively.

In addition, specific examples of the bispecific antibody of the present invention also includes a bispecific antibody in which the amino acid sequences of VH of the antibody that binds to TRAILR2 comprise the amino acid sequence represented by SEQ ID NO: 118, and the amino acid sequences of VL comprise an amino acid sequence represented by SEQ ID NO: 119.

As one aspect of the antibody that binds to TRAILR2 in which the amino acid sequences of VH comprise the amino acid sequence represented by SEQ ID NO: 118, and the amino acid sequences of VL comprise the amino acid sequence represented by SEQ ID NO: 119, there are the anti-human TRAILR2 monoclonal antibody E11 antibody, and the like.

The bispecific antibody of the present invention also includes a bispecific antibody in which the in which the amino acid sequences of VH and VL of the antibody that binds to TRAILR2 are at least 35%, at least 40%, at least 45%, at least 50%, at least 55%, at least 60%, at least 65%, at least 70%, at least 75%, at least 80%, at least 85%, at least 90%, at least 95%, and at least 99% homology to the amino acid sequence of VH represented by SEQ ID NO: 118 and the amino acid sequence of VL represented by SEQ ID NO: 119, respectively.

The antibody that binds to TRAILR2 in the present invention also includes any one of antibodies described in (1) to (3) below.

(1) A bispecific antibody of which TRAILR2 antibody itself doesn't exhibit at least one of the activities of the inhibition of cell proliferation and the induction of cell death but when the antibody is formed with a binding domains for other antigens, at least one of the activities of the inhibition of cell proliferation and the induction of cell death is exhibited.
(2) An antibody that binds to TRAILR2 by competing with the antibody comprising the amino acid sequences of CDRs 1 to 3 of VH represented by SEQ ID NOs: 70 to 72, respectively, and the amino acid sequences of CDRs 1 to 3 of VL represented by SEQ ID NOs: 74 to 76, respectively.
(3) An antibody that binds to the same epitope as the antibody comprising the amino acid sequences of CDRs 1 to 3 of VH represented by SEQ ID NOs: 70 to 72, respectively, and the amino acid sequences of CDRs 1 to 3 of VL represented by SEQ ID NOs: 74 to 76, respectively.

In addition, examples of the bispecific antibody of the present invention include a bispecific antibody in which the amino acid sequences of CDRs 1 to 3 of VH of the antibody that binds to PSMA comprise any one of amino acid sequences selected from the group consisting of (A) to (D) below, and the amino acid sequences of CDRs 1 to 3 of VL comprise the amino acid sequences represented by SEQ ID NOs: 74 to 76, respectively.

(A) the amino acid sequences of CDRs 1 to 3 of VH that are the amino acid sequences represented by SEQ ID NOs: 78, 82, and 84, respectively
(B) the amino acid sequences of CDRs 1 to 3 of VH that are the amino acid sequences represented by SEQ ID NOs: 78, 79, and 80, respectively
(C) the amino acid sequences of CDRs 1 to 3 of VH that are the amino acid sequences represented by SEQ ID NOs: 78, 82, and 80, respectively, and
(D) the amino acid sequences of CDRs 1 to 3 of VH that are the amino acid sequences represented by SEQ ID NOs: 96 to 98, respectively

Accordingly, as specific examples of the variable region of the antibody that binds to PSMA, which is comprised in the bispecific antibody of the present invention, there are any one of VHs selected from the group consisting of (a) to (d) below, and VL comprising CDRs 1 to 3 that are the amino acid sequences represented by SEQ ID NOs: 74 to 76, respectively.

(a) VH comprising the amino acid sequences represented by SEQ ID NOs: 78, 82, and 84, respectively, as CDRs 1 to 3
(b) VH comprising the amino acid sequences represented by SEQ ID NOs: 78, 79, and 80, respectively, as CDRs 1 to 3
(c) VH comprising the amino acid sequences represented by SEQ ID NOs: 78, 82, and 80, respectively, as CDRs 1 to 3, and
(d) VH comprising the amino acid sequences represented by SEQ ID NOs: 96 to 98, respectively, as CDRs 1 to 3

As one aspect of the antibody that binds to PSMA comprising VH comprising CDRs 1 to 3 that are the amino acid sequences represented by SEQ ID NOs: 78, 82, and 84, respectively, and VL comprising CDRs 1 to 3 that are the amino acid sequences represented by SEQ ID NOs: 74 to 76, respectively, there are anti-human PSMA monoclonal antibody PN7 antibody, and the like.

As one aspect of the antibody that binds to PSMA comprising VH comprising CDRs 1 to 3 that are the amino acid sequences represented by SEQ ID NOs: 78, 79, and 80, respectively, and VL comprising CDRs 1 to 3 that are the amino acid sequences represented by SEQ ID NOs: 74 to 76, respectively, there are anti-human PSMA monoclonal antibody PI134 antibody, PI143 antibody, PI105 antibody, PI127 antibody, PI108 antibody, PI115 antibody, PL223 antibody, and the like.

As one aspect of the antibody that binds to PSMA comprising VH comprising CDRs 1 to 3 that are the amino acid sequences represented by SEQ ID NOs: 78, 82, and 80, respectively, and VL comprising CDRs 1 to 3 that are the amino acid sequences represented by SEQ ID NOs: 74 to 76, respectively, there are anti-human PSMA monoclonal antibody PI101 antibody, PI118 antibody, and the like.

As one aspect of the antibody that binds to PSMA comprising VH comprising CDRs 1 to 3 that are the amino acid sequences represented by SEQ ID NOs: 96 to 98, respectively, and VL comprising CDRs 1 to 3 that are the amino acid sequences represented by SEQ ID NOs: 74 to 76, respectively, there are anti-human PSMA monoclonal antibody PN170 antibody, and the like.

The bispecific antibody of the present invention also includes a bispecific antibody comprising VH and VL comprise the amino acid sequences that are at least 35%, at least 40%, at least 45%, at least 50%, at least 55%, at least 60%, at least 65%, at least 70%, at least 75%, at least 80%, at least 85%, at least 90%, at least 95%, and at least 99% homology to the amino acid sequences of any one of VHs selected from the group consisting of (a) to (d) above which are VHs of the antibody that binds to PSMA, and VL comprising CDRs 1 to 3 that are the amino acid sequences represented by SEQ ID NOs: 74 to 76, respectively.

In addition, as specific examples of the variable region of the antibody that binds to PSMA, which is comprised in the bispecific antibody of the present invention, there are any one of VHs selected from the group consisting of (e) to (o) below, and VL comprising the amino acid sequence represented by SEQ ID NO: 119.

(e) VH comprising the amino acid sequence represented by SEQ ID NO: 122
(f) VH comprising the amino acid sequence represented by SEQ ID NO: 120
(g) VH comprising the amino acid sequence represented by SEQ ID NO: 128
(h) VH comprising the amino acid sequence represented by SEQ ID NO: 123
(i) VH comprising the amino acid sequence represented by SEQ ID NO: 127
(j) VH comprising the amino acid sequence represented by SEQ ID NO: 124

(k) VH comprising the amino acid sequence represented by SEQ ID NO: 125
(l) VH comprising the amino acid sequence represented by SEQ ID NO: 129
(m) VH comprising the amino acid sequence represented by SEQ ID NO: 121
(n) VH comprising the amino acid sequence represented by SEQ ID NO: 126, and
(o) VH comprising the amino acid sequence represented by SEQ ID NO: 130

As one aspect of the antibody that binds to human PSMA comprising VH comprising the amino acid sequence represented by SEQ ID NO: 122, and VL comprising the amino acid sequence represented by SEQ ID NO: 119, there are anti-human PSMA monoclonal antibody PN7 antibody, and the like.
As one aspect of the antibody that binds to human PSMA comprising VH comprising the amino acid sequence represented by SEQ ID NO: 120, and VL comprising the amino acid sequence represented by SEQ ID NO: 119, there are anti-human PSMA monoclonal antibody PI134 antibody, and the like.
As one aspect of the antibody that binds to human PSMA comprising VH comprising the amino acid sequence represented by SEQ ID NO: 128, and VL comprising the amino acid sequence represented by SEQ ID NO: 119, there are anti-human PSMA monoclonal antibody PI 143 antibody, and the like.
As one aspect of the antibody that binds to human PSMA comprising VH comprising the amino acid sequence represented by SEQ ID NO: 123, and VL comprising the amino acid sequence represented by SEQ ID NO: 119, there are anti-human PSMA monoclonal antibody PI105 antibody, and the like.
As one aspect of the antibody that binds to human PSMA comprising VH comprising the amino acid sequence represented by SEQ ID NO: 127, and VL comprising the amino acid sequence represented by SEQ ID NO: 119, there are anti-human PSMA monoclonal antibody PI127 antibody, and the like.
As one aspect of the antibody that binds to human PSMA comprising VH comprising the amino acid sequence represented by SEQ ID NO: 124, and VL comprising the amino acid sequence represented by SEQ ID NO: 119, there are anti-human PSMA monoclonal antibody PI108 antibody, and the like.
As one aspect of the antibody that binds to human PSMA comprising VH comprising the amino acid sequence represented by SEQ ID NO: 125, and VL comprising the amino acid sequence represented by SEQ ID NO: 119, there are anti-human PSMA monoclonal antibody PI115 antibody, and the like.
As one aspect of the antibody that binds to human PSMA comprising VH comprising the amino acid sequence represented by SEQ ID NO: 129, and VL comprising the amino acid sequence represented by SEQ ID NO: 119, there are anti-human PSMA monoclonal antibody PL223 antibody, and the like.
As one aspect of the antibody that binds to human PSMA comprising VH comprising the amino acid sequence represented by SEQ ID NO: 121, and VL comprising the amino acid sequence represented by SEQ ID NO: 119, there are anti-human PSMA monoclonal antibody PI101 antibody, and the like.
As one aspect of the antibody that binds to human PSMA comprising VH comprising the amino acid sequence represented by SEQ ID NO: 126, and VL comprising the amino acid sequence represented by SEQ ID NO: 119, there are anti-human PSMA monoclonal antibody PI118 antibody, and the like.
As one aspect of the antibody that binds to human PSMA comprising VH comprising the amino acid sequence represented by SEQ ID NO: 130, and VL comprising the amino acid sequence represented by SEQ ID NO: 119, there are anti-human PSMA monoclonal antibody PN170 antibody, and the like.
The bispecific antibody of the present invention also includes a bispecific antibody comprising VH and VL comprising the amino acid sequences that are at least 35%, at least 40%, at least 45%, at least 50%, at least 55%, at least 60%, at least 65%, at least 70%, at least 75%, at least 80%, at least 85%, at least 90%, at least 95%, and at least 99% homology to the amino acid sequences of any one of VHs selected from the group consisting of (e) to (o) above which are VHs of the antibody that binds to PSMA, and VL comprising the amino acid sequence represented by SEQ ID NO: 119, respectively.
In addition, the anti-PSMA antibody used for the bispecific antibody of the present invention includes antibodies described in (1) or (2) below, and the like.
(1) An antibody that binds to PSMA by competing with the antibody comprising the amino acid sequences of CDRs 1 to 3 of VH that are the amino acid sequences represented by SEQ ID NOs: 78, 82, and 84, respectively, and the amino acid sequences of CDRs 1 to 3 of VL that are the amino acid sequences represented by SEQ ID NOs: 74 to 76, respectively; the antibody comprising the amino acid sequences of CDRs 1 to 3 of VH that are the amino acid sequences represented by SEQ ID NOs: 78, 89, and 80, respectively, and the amino acid sequences of CDRs 1 to 3 of VL that are the amino acid sequences represented by SEQ ID NOs: 74 to 76, respectively; the antibody comprising the amino acid sequences of CDRs 1 to 3 of VH that are the amino acid sequences represented by SEQ ID NOs: 78, 82, and 80, respectively, and the amino acid sequences of CDRs 1 to 3 of VL that are the amino acid sequences represented by SEQ ID NOs: 74 to 76, respectively; or the antibody comprising the amino acid sequences of CDRs 1 to 3 of VH that are the amino acid sequences represented by SEQ ID NOs: 96 to 98, respectively, and the amino acid sequences of CDRs 1 to 3 of VL that are the amino acid sequences represented by SEQ ID NOs: 74 to 76, respectively.

(2) An antibody that binds to the same epitope as the antibody comprising the amino acid sequences of CDRs 1 to 3 of VH that are the amino acid sequences represented by SEQ ID NOs: 78, 82, and 84, respectively, and the amino acid sequences of CDRs 1 to 3 of VL that are the amino acid sequences represented by SEQ ID NOs: 74 to 76, respectively; the antibody comprising the amino acid sequences of CDRs 1 to 3 of VH that are the amino acid sequences represented by SEQ ID NOs: 78, 89, and 80, respectively, and the amino acid sequences of CDRs 1 to 3 of VL that are the amino acid sequences represented by SEQ ID NOs: 74 to 76, respectively; the antibody comprising the amino acid sequences of CDRs 1 to 3 of VH that are the amino acid sequences represented by SEQ ID NOs: 78, 82, and 80, respectively, and the amino acid sequences of CDRs 1 to 3 of VL that are the amino acid sequences represented by SEQ ID NOs: 74 to 76, respectively; or the antibody comprising the amino acid sequences of CDRs 1 to 3 of VH that are the amino acid sequences represented by SEQ ID NOs: 96 to 98, respectively, and the amino acid sequences of CDRs 1 to 3 of VL that are the amino acid sequences represented by SEQ ID NOs: 74 to 76, respectively.

In the bispecific antibody of the present invention, the variable region of the antibody that binds to TRAILR2 may be located closer to the N-terminus side than the variable region of the antibody that binds to PSMA via a linker, or may be located closer to the C-terminus side, but it is preferable to be located closer to the N-terminus side via a linker. As the bispecific antibody of which the variable region of the antibody that binds to TRAILR2 is located closer to the N-terminus side than the variable region of the antibody that binds to PSMA, there is a bispecific antibody of which the amino acid sequences of the polypeptide constituted of VH of the antibody that binds to TRAILR2, a linker and VH of the antibody that binds to PSMA, are any one of amino acid sequences selected from the group consisting of (A) to (M) below.

(A) the amino acid sequence represented by SEQ ID NO: 131
(B) the amino acid sequence represented by SEQ ID NO: 132
(C) the amino acid sequence represented by SEQ ID NO: 133
(D) the amino acid sequence represented by SEQ ID NO: 134
(E) the amino acid sequence represented by SEQ ID NO: 135
(F) the amino acid sequence represented by SEQ ID NO: 136
(G) the amino acid sequence represented by SEQ ID NO: 137
(H) the amino acid sequence represented by SEQ ID NO: 138
(I) the amino acid sequence represented by SEQ ID NO: 139
(J) the amino acid sequence represented by SEQ ID NO: 140
(K) the amino acid sequence represented by SEQ ID NO: 141
(L) the amino acid sequence represented by SEQ ID NO: 142, and
(M) the amino acid sequence represented by SEQ ID NO: 143

As one aspect of the bispecific antibody that binds to TRAILR2 and PSMA, of which the amino acid sequences of the polypeptide constituted of VH of the antibody that binds to TRAILR2, a linker and VH of the antibody that binds to PSMA, are the amino acid sequences described in (A) to (M) above, there are bispecific antibody E11-CH1-PI134 antibody, E11-CH1-PI101VH antibody, E11-CHI-PI105VH antibody, E11-CH1-PI108VH antibody, E11-CH1-PI115VH antibody, E11-CH1-PI118VH antibody, E11-CH1-PI127VH antibody, E11-CH1-PI143VH antibody, E11-CH1-PL223VH antibody, E11-CH1-PN7VH antibody, E11-CH1-PN170VH antibody, E11-GL-PI134VH antibody, E11-ML-PI101VH antibody, and the like, each of which binds to hTRAILR2 and hPSMA.

The bispecific antibody or the antibody fragment thereof of the present invention includes an antibody or an antibody fragment thereof having effector activity.

The effector activity refers to antibody-dependent cellular cytotoxicity activity that is caused via the Fc region of the antibody, and examples thereof include Antibody-Dependent Cellular Cytotoxicity activity (ADCC activity), Complement-Dependent Cytotoxicity activity (CDC activity), Antibody-dependent cellular phagocytosis activity (ADCP activity) that is caused by phagocytes such as macrophages and dendritic cells, opsonin effect, and the like.

The ADCC activity and the CDC activity in the present invention can be measured by using a known measuring method [Cancer Immunol. Immunother., 36, 373 (1993)].

The ADCC activity is activity in which an antibody that binds to an antigen on target cells, binds to an Fc receptor on immunocytes via the Fc region of the antibody, which activates the immunocytes (natural killer cells and the like), and therefore the target cells are damaged.

The Fc receptor (FcR) is a receptor that binds to the Fc region of the antibody, and binding to the antibody induces various effector activities. Each FcR corresponds to the subclasses of the antibody, and IgG, IgE, IgA, and IgM bind specifically to FcγR, FcεR, FcαR, and FcμR, respectively. Furthermore, as the subtypes of FcγR, there are FcγRI(CD64), FcγRII (CD32), and FcγRIII(CD16). Each of the subtypes have isoforms of FcγRIA, FcγRIB, FcγRIC, FcγRIIA, FcγRIIB, FcγRIIC, FcγRIIIA, and FcγRIIIB. The different types of FcγRs are present on different cells [Annu. Rev. Immunol. 9:457-492 (1991)]. In humans, FcγRIIIB is expressed specifically in neutrophils, and FcγRIIIA is expressed in monocytes, natural killer cells (NK cells), macrophages, and some of T cells. NK cells-dependent ADCC activity is induced by binding of the antibody to FcγRIIIA.

The CDC activity is an activity in which an antibody that binds to an antigen on target cells activates a series of a cascade (complement activation pathway) formed by complement related protein groups in blood, and therefore the target cells are damaged. In addition, protein fragments generated by the activation of the complement induce the migration and activation of the immunocytes. In the cascade in the CDC activity, C1q binds to the Fc region first and then binds to C1r and C1s which are two serine proteases, and therefore the C1 complex is formed to initiate the activity.

The CDC activity or the ADCC activity of the bispecific antibody or the antibody fragment thereof of the present invention against antigen-expressing cells can be evaluated by a known measuring method [Cancer Immunol. Immunother., 36, 373 (1993)].

As a method for controlling the effector activity of the bispecific antibody of the present invention, a method for controlling the amount of α1,6-fucose (also called as a core fucose) binding to N-acetylglucosamine (GlcNAc) present on the reducing terminal of N-linked complex sugar chains that binds to the 297$^{th}$ asparagine (Asn) in the Fc region (the constant region formed of the CH2 and CH3 domains) of the antibody (International Publication NO: 2005/035586, International Publication NO: 2002/31140, and International Publication NO: 00/61739), a method for controlling by altering an amino acid residue in the Fc region of the antibody (International Publication NO: 00/42072), and the like are known.

It is possible to promote or suppress the ADCC activity of the antibody by controlling the fucose amount added to the bispecific antibody. For example, using a method for decreasing the fucose amount binding to N-linked complex sugar chains that binds to the Fc of the antibody by expressing the bispecific antibody using host cells in which α1,6-fucosyltransferase genes are deleted, it is possible to obtain a bispecific antibody having a high level of ADCC. On the other hand, using a method for increasing the fucose amount binding to N-linked complex sugar chains that binds to the Fc of the bispecific antibody by expressing the antibody using host cells into which α1,6-fucosyltransferase genes are introduced, it is possible to obtain a bispecific antibody having a low level of the ADCC activity.

In addition, it is possible to promote or suppress the ADCC activity or the CDC activity by altering an amino acid residue in the Fc region of the bispecific antibody. For example, it is possible to promote the CDC activity of the bispecific antibody by using the amino acid sequences of the Fc region described in United States Patent Application, Publication No. 2007/0148165. In addition, it is possible to either promote or suppress the ADCC activity or the CDC activity by performing alteration of amino acids described in United States Patent No. 6,737,056, United States Patent No. 7,297,775, United States Patent No. 7,317,091, or the like.

Furthermore, a bispecific antibody in which the effector activity is controlled may be obtained by combining the above methods.

The stability of the bispecific antibody of the present invention can be evaluated by measuring an aggregate (oligomer) amount formed in a sample obtained during purification process, or preserved under the certain conditions. That is, a case where the aggregate amount is decreased under the same conditions is evaluated as an increase in the stability of the antibody. The aggregate amount can be measured by separating aggregated antibodies and non-aggregated antibodies using appropriate chromatography including gel-filtration chromatography.

The productivity of the bispecific antibody of the present invention can be evaluated by measuring an amount of antibodies produced in the culture solution from antibody-producing cells. More specifically, it is possible to evaluate the productivity by measuring the amount of antibodies included in the culture supernatant of which the producing cells are removed from the culture solution using an appropriate method such as HPLC or ELISA.

In the present invention, the antibody fragment is an antibody fragment that comprises an antigen binding domain that binds to TRAILR2 and an antigen binding domain that binds to PSMA, and that has the antigen binding activity. Examples of the antibody fragment of the present invention include Fab, Fab', $F(ab')_2$, scFv, diabody, dsFv, peptides comprising CDR, and the like.

Fab is an antibody fragment that has the antigen binding activity and approximately fifty thousand molecular weight, and in which about a half of the H chain in the N-terminus side and the entire L chain are linked to each other via a disulfide bond (S-S bond) (cleaved by the 224$^{th}$ amino acid residue in the H chain) among the fragments obtained by treating the IgG antibody with papain, that is, the protease.

$F(ab')_2$ is an antibody fragment that has the antigen binding activity and approximately a hundred thousand molecular weight, and that is slightly larger than the one of which Fab is bound via the S-S bond in the hinge region (cleaved by the 234$^{th}$ amino acid residue in the H chain) among the fragments obtained by treating the IgG with pepsin, that is, the protease.

Fab' is an antibody fragment that has the antigen binding activity and approximately fifty thousand molecular weight, and in which the S-S bond in the hinge region of the above $F(ab')_2$ is cleaved.

scFv is a VH-P-VL or VL-P-VH polypeptide in which one VH and one VL are linked using an appropriate peptide linker (P) of 12 or more residues, and is an antibody fragment having the antigen binding activity.

Diabody is an antibody fragment in which scFvs having the same or different antigen binding specificities form a dimer, and is an antibody fragment having a divalent antigen binding activity to the same antigen or the antigen binding activity specific for each different antigen.

dsFv refers to a polypeptide in which each one amino acid residue in VH and VL is substituted with a cysteine residue, and is bound through the S-S bond between the cysteine residues.

Peptide comprising CDR is formed comprising at least one region of CDR of VH or VL. Peptide comprising a plurality of CDRs can be produced by binding CDRs directly or via an appropriate peptide linker. The peptide comprising CDRs can be produced by constructing DNA that encodes CDRs of VH and VL of the bispecific antibody of the present invention, inserting the DNA into an expression vector for a prokaryote or an expression vector for a eukaryote, and then introducing the expression vector into a prokaryote or a eukaryote for expression. In addition, the peptide comprising CDRs can also be produced by a chemical synthesis method such as the Fmoc method or the tBoc method.

The present invention further includes fusion protein in which the Fc of the bispecific antibody and the antibody fragment of the present invention are bound to each other, Fc fusion protein in which the Fc and a natural ligand or a receptor bind are bound to each other (also referred to as an immunoadhesin), and Fc fusion protein in which a plurality of Fc regions are fused, and the like. In addition, an Fc region that comprises the modification of amino acid residues, and is for enhancing or eliminating the effector activity of the antibody, stabilizing the antibody, and controlling blood half-life can also be used for the bispecific antibody of the present invention.

Examples of the bispecific antibody or the antibody fragment of the present invention include a derivate of the antibody in which the radioisotope, a low molecular weight drug, a polymer drug, a protein, or an antibody drug is bound to the bispecific antibody or the antibody fragment of the present invention in a chemical or genetically engineered way.

The derivative of the antibody of the present invention can be produced by binding the radioisotope, a low molecular weight drug, a polymer drug, an immunostimulant, a protein, or an antibody drug to the N-terminus side or C-terminus side of the H chain or L chain of the bispecific antibody or the antibody fragment thereof of the present invention, an appropriate substituent or a side chain in the antibody or the antibody fragment thereof, a sugar chain in the antibody or the antibody fragment thereof, and the like, using a chemical method [Introduction to Antibody Engineering, Chijinshokan Co., Ltd. (1994)].

Further, the derivative of the antibody of the present invention can be produced by a genetic engineering technique in which DNA that encodes the bispecific antibody or the antibody fragment of the present invention is linked to DNA that encodes a desired protein or antibody drug, inserted into the expression vector, and the expression vector is introduced in an appropriate host cell for expression.

Examples of the radioisotope include $^{111}In$, $^{131}I$, $^{125}I$, $^{90}Y$, $^{64}Cu$, $^{99}Tc$, $^{77}Lu$, or $^{211}At$. The radioisotope can be directly bound to the antibody by the chloramine T method or the like. In addition, a substance that chelates the radioisotope may be bound to the antibody. Examples of the chelating agent include 1-isothiocyanatobenzyl-3-methyldiethylenetriaminepentaacetic acid (MX-DTPA) and the like.

Examples of the low molecular weight drug include anti-cancer drugs such as alkylating agents, nitrosoureas, antimetabolites, antibiotics, plant alkaloids, topoisomerase inhibitors, hormonal therapy agents, hormone antagonists, aromatase inhibitors, P-glycoprotein inhibitors, platinum complex derivatives, M cycle inhibitor, or kinase inhibitors [Clinical oncology, Cancer and chemotherapy (1996)], anti-inflammatory agents such as steroids such as hydrocortisone or prednisone, nonsteroidal drugs such as aspirin or indomethacin, immune modulating drugs such as gold thiomalate or penicillamine, immunosuppressive drugs such as cyclophosphamide or azathioprine, antihistamine drugs such as chlorpheniramine maleate or clemastine [Inflammation and anti-inflammatory therapy, Ishiyaku Pub, Inc. (1982)], and the like.

Examples of the anti-cancer drugs include amifostine (Ethyol), cisplatin, dacarbazine (DTIC), dactinomycin, mechlorethamine (nitrogen mustard), streptozocin, cyclophosphamide, ifosfamide, carmustine (BCNU), lomustine (CCNU), doxorubicin (Adriamycin), epirubicin, gemcitabine (Gemzar), daunorubicin, procarbazine, mitomycin, cytarabine, etoposide, 5-fluorouracil, fluorouracil, vinblastine, vincristine, bleomycin, daunomycin, peplomycin, estramustine, paclitaxel (Taxol), docetaxel (Taxotere), Aldesleukin, asparaginase, busulfan, carboplatin, oxaliplatin, nedaplatin, cladribine, camptothecin, 7-ethyl-10-hydroxycamptothecin (SN38), floxuridine, fludarabine, hydroxyurea, idarubicin, mesna, irinotecan (CPT-11), nogitecan, mitoxantrone, topotecan, leuprolide, megestrol, melphalan, mercaptopurine, hydroxycarbamide, plicamycin, mitotane, pegaspargase, pentostatin, pipobroman, streptozocin, tamoxifen, goserelin, leuprorelin, flutamide, teniposide, testolactone, thioguanine, thiotepa, uracil mustard, vinorelbine, chlorambucil, hydrocortisone, prednisolone, methylprednisolone, vindesine, nimustine, semustine, capecitabine, Tomudex, azacitidine, UFT, oxaloplatin, gefitinib (Iressa), imatinib (STI571), erlotinib, FMS-like tyrosine kinase 3 (Flt3) inhibitor, vascular endothelial growth facotr receptor (VEGFR) inhibitor, fibroblast growth factor receptor (FGFR) inhibitor, epidermal growth factor receptor (EGFR) inhibitor such as Tarceva, radicicol, 17-allylamino-17-demethoxygeldanamycin, rapamycin, amsacrine, all-trans retinoic acid, thalidomide, lenalidomide, anastrozole, fadrozole, letrozole, exemestane, gold thiomalate, D-penicillamine, bucillamine, azathioprine, mizoribine, cyclosporine, rapamycin, hydrocortisone, bexarotene (Targretin), tamoxifen, dexamethasone, progestins, estrogens, anastrozole (Arimidex), Leuplin, Aspirin, indomethacin, celecoxib, azathioprine, penicillamine, gold thiomalate, chlorpheniramine maleate, chloropheniramine, clemastine, tretinoin, bexarotene, arsenic, bortezomib, allopurinol, calicheamicin, ibritumomab tiuxetan, targretin, ozogamine, clarithromycin, leucovorin, ketoconazole, aminoglutethimide, suramin, methotrexate or maytansinoid or a derivative thereof, and the like.

Examples of a method for binding a low molecular weight drug and the bispecific antibody of the present invention include a method of binding the drug to an amino group of the antibody via glutaraldehyde, or a method of binding an amino group of the drug to a carboxyl group of the antibody via water-soluble carbodiimide, and the like.

Examples of a polymer drug include polyethylene glycol (PEG), albumin, dextran, polyoxyethylene, styrene-maleic acid copolymer, polyvinylpyrrolidone, pyran copolymer, or hydroxypropyl methacrylamide, and the like. By binding these polymer compounds to the bispecific antibody or the antibody fragment of the present invention, effect such as (1) improvement in stability against various chemical, physical or biological factors, (2) significant extension of blood half-life, or (3) disappearance of immunogenicity or inhibition of antibody production, is expected [Bioconjugate pharmaceutical product, Hirokawa-Shoten Ltd. (1993)].

Examples of a method for binding PEG to the bispecific antibody of the present invention include a method of reacting with a PEGylation reagent, and the like [Bioconjugate pharmaceutical product, Hirokawa-Shoten Ltd. (1993)]. Examples of the PEGylation reagent include a modifying agent to an ε-amino group of lysine (JP-A-S61-178926), a modifying agent to a carboxyl group of aspartic acid and glutamic acid (JP-A-S56-23587), or a modifying agent to a guanidino group of arginine (JP-A-H2-117920), and the like.

The immunostimulant may be a natural product known as an immunoadjuvant. Specific examples thereof include a drug that promotes immunity such as a $\beta(1\rightarrow3)$ glucan (for example, lentinan or schizophyllan), or α-galactosylceramide (KRN7000).

Examples of the protein include cytokines or growth factors which activate immunocompetent cells such as NK cells, macrophages or neutrophils, or toxic proteins, and the like.

Examples of cytokines or the growth factors include interferon (hereinafter referred to as IFN)-α, IFN-β, and IFN-γ, interleukin (hereinafter referred to as IL)-2, IL-12, IL-15, IL-18, IL-21, and IL-23, granulocyte colony stimulating factor (G-CSF), granulocyte-macrophage colony stimulating factor (GM-CSF) or macrophage colony stimulating factor (M-CSF) and the like.

Examples of the toxic proteins include ricin, diphtheria toxin or ONTAK and the like, and also include protein toxins in which a mutation has been introduced into the protein for regulating toxicity.

It is possible to produce an antibody fused with a protein or an antibody drug by linking cDNA that encodes a protein to cDNA that encodes the bispecific antibody or the antibody fragment of the present invention to construct DNA that encodes a fused antibody, inserting the DNA into an expression vector for a prokaryote or a eukaryote, and then introducing the expression vector into a prokaryote or a eukaryote for expression.

When the derivative of the antibody is used for a detection method or a quantification method, and as a reagent for detection, a reagent for quantification or a diagnostic agent, examples of the drug binding to the bispecific antibody or the antibody fragment thereof of the present invention include a labeling substance used for a general method for immunological detection or measurement. Examples of the labeling substance include enzymes such as alkaline phosphatase, peroxidase or luciferase, luminescent substances such as acridinium esters or lophines, or fluorescent substances such as fluorescein isothiocyanate (FITC) or tetramethylrhodamine isothiocyanate (RITC), Alexa (registered trademark) Fluor 488, R-phycoerythrin (R-PE), and the like.

The present invention includes the bispecific antibody and the antibody fragment thereof having the cytotoxic activity such as CDC activity or ADCC activity. It is possible to evaluate CDC activity or ADCC activity of the bispecific antibody or the antibody fragment thereof of the present invention against the antigen-expressing cells by a known measuring method [Cancer Immunol. Immunother., 36, 373 (1993)].

The present invention is further related to a composition comprising a bispecific antibody or an antibody fragment thereof which specifically recognizes and binds to TRAILR2 and PSMA or a therapeutic agent for a disease related to TRAILR2- and PSMA-expressing cell, comprising, the bispecific antibody or the antibody fragment thereof as an active ingredient.

The disease related to TRAILR2- and PSMA-expressing cell, may be, for example, any diseases related to TRAILR2- and PSMA-expressing cell, and examples thereof include a malignant tumor and a cancer, and the like.

Examples of the malignant tumor and the cancer in the present invention include, large intestine cancer, colorectal cancer, lung cancer, breast cancer, glioma, malignant melanoma (melanoma), thyroid cancer, renal cell carcinoma, leukemia, lymphoma, T cell lymphoma, stomach cancer, pancreatic cancer, cervical cancer, endometrial cancer, ovarian cancer, esophageal cancer, liver cancer, head and neck squamous cell carcinoma, skin cancer, urinary tract cancer, bladder cancer, prostate cancer, chorionic carcinoma, pharyngeal cancer, laryngeal cancer, mesothelioma, pleural tumor, arrhenoblastoma, endometrial hyperplasia, endometriosis, embryoma, fibrosarcoma, kaposi’s sarcoma, angioma, cavernous hemangioma, angioblastoma, retinoblastoma, astrocytoma, neurofibroma, oligodendroglioma, medulloblastoma, neuroblastoma, glioma, rhabdomyosarcoma, glioblastoma, osteogenic sarcoma, leiomyosarcoma, thyroid sarcoma, Wilm's tumor, and the like.

The therapeutic agent containing the bispecific antibody or an antibody fragment thereof of the present invention, or a derivative thereof may contain only the antibody or the antibody fragment, or a derivative thereof as an active ingredient, but it is generally preferable that the agent is mixed with one or more pharmacologically acceptable carriers to be provided as medicinal formulation that is produced by arbitrary methods known in the technical field of pharmaceutical science.

As the route of administration, it is preferable to use the most effective route for the treatment, and examples thereof include oral administration or parenteral administration such as intraoral, airway, intrarectal, subcutaneous, intramuscular or intravenous administration. Among these, the intravenous administration is preferable.

Examples of the form of administration include a spray, a capsule, a tablet, a powder, a granule, a syrup, an emulsion, a suppository, an injection, an ointment, or a tape, and the like.

The dose or the frequency of administration varies according to the desired therapeutic effect, administration method, treatment period, age, body weight and the like, but is usually 10 $\mu$g/kg to 10 mg/kg per day for adult.

The present invention is still further related to a reagent for immunological detection or measurement of at least one of TRAILR2 and PSMA, which includes the bispecific antibody or the antibody fragment thereof of the present invention, or a diagnostic agent for a disease related to TRAILR2- and PSMA-expressing cell. The present invention is still further related to a method for immunological detecting or measuring at least one of TRAILR2 and PSMA by using the bispecific antibody or the antibody fragment thereof of the present invention, or a diagnostic method for a disease related to TRAILR2- and PSMA-expressing cell.

Examples of a method for detecting or measuring the amount of at least one of TRAILR2 and PSMA in the present invention, include known arbitrary methods. For example, there is a method for immunological detection or measurement.

The method for immunological detection or measurement is a method of detecting or measuring the antibody amount or the antigen amount using a labeled antigen or antibody. Examples of the method for immunological detection or measurement include a radioimmunoassay method (RIA), an enzyme immunoassay method (EIA or ELISA), a fluorescence immunoassay method (FIA), a luminescent immunoassay method, a western blotting method, or a physicochemical method, and the like.

By detecting or measuring cell expressing at least one of TRAILR2 and PSMA by using the bispecific antibody or the antibody fragment of the present invention, it is possible to diagnose a disease related to TRAILR2 and PSMA-expressing cell.

It is possible to use a known immunological detection method for detecting cells expressing a polypeptide formed of at least one of TRAILR2 and PSMA, and examples thereof include an immunoprecipitation method, an immunocytochemical staining method, an immunohistochemical staining method, or a fluorescent antibody staining method, and the like. In addition, examples thereof include a fluorescent antibody staining method such as FMAT 8100 HTS system (manufactured by Applied Biosystems) and the like.

As a biological sample that is a target to be subjected to the detection or measurement of at least one of TRAILR2 and PSMA in the present invention, there are tissue cells, blood, plasma, serum, pancreatic juice, urine, feces, tissue fluid, culture solution, and the like. As long as there is a possibility that the sample contains the cell in which at least one of TRAILR2 and PSMA is expressed, it is not particularly limited.

The diagnostic agent containing the bispecific antibody or the antibody fragment thereof of the present invention, or a derivative thereof, may contain a reagent for performing antigen-antibody reaction and a reagent for detecting the reaction in accordance with to the desired diagnostic method. Examples of the reagent for performing the antigen-antibody reaction include a buffer, a salt and the like.

Examples of the reagent for detection include reagents such as the bispecific antibody or the antibody fragment thereof, or a labeled secondary antibody that binds to a derivative thereof, or a substrate corresponding to a label, which are used for a general method for immunological detection or measurement.

Hereinafter, a method for producing the bispecific antibody of the present invention, a method for measuring at least one of TRAILR2 and PSMA using the antibody, and a diagnostic method and a therapeutic method for a disease related to TRAILR2- and PSMA-expressing cell will be described in detail.

1. Method for Producing Monoclonal Antibody

A method for producing a monoclonal antibody of the present invention includes production steps as below. That is, (1) at least one of the purification of an antigen used as an immunogen, and the production of cells in which the antigen is overly expressed on the cell surface, (2) a step of preparing an antibody-producing cell by immunizing animals with the antigen, followed by collecting blood, examining the antibody valency thereof, and then determining when to resecting the spleen or the like, (3) preparing a myeloma cell, (4) fusing the antibody-producing cell with the myeloma, (5) sorting hybridoma groups that produce a target antibody, (6) separating a monoclonal cell from the hybridoma groups (cloning), (7) in some cases, culturing hybridomas to produce monoclonal antibodies in large amounts, or breeding of animals implanted with the hybridomas, (8) investigating the bioactivity of the monoclonal antibody produced in this manner, and the antigen binding specificity thereof, or examining the characteristics as a labeling reagent, and the like.

Hereinafter, the method for producing a monoclonal antibody that binds to TRAILR2 and a monoclonal antibody that binds to PSMA, which are used for producing the bispecific antibody that binds to TRAILR2 and PSMA of the present invention, will be described according to the above steps. The method for producing the antibody is not particularly limited, and for example, it is possible to use an antibody-producing cell other than a spleen cell, and a myeloma.

(1) Purification of Antigen

It is possible to obtain at least one of TRAILR2- and PSMA-expressing cell by introducing an expression vector comprising cDNA that encodes the full length or the partial length thereof of at least one of TRAILR2 and PSMA, to E. coli, yeast, insect cells or animal cells, and the like. In addition, it is possible to purify at least one of TRAILR2 and PSMA from various human tumor-cultured cells or human tissues in which at least one of TRAILR2 and PSMA are expressed in large amounts, and the like so as to use it as an antigen. In addition, the tumor-cultured cell or the tissue and the like can be used as it is as an antigen. Furthermore, it is possible to prepare synthetic peptides comprising a partial sequence of at least one of TRAILR2 and PSMA by using the method for chemical synthesis such as the Fmoc method or the tBoc method, and the like so as to use it as an antigen.

It is possible to produce at least one of TRAILR2 and PSMA used in the present invention by using a method such as the method described in Molecular Cloning, A Laboratory Manual, Second Edition, Cold Spring Harbor Laboratory Press (1989), or Current Protocols In Molecular Biology, John Wiley & Sons (1987-1997), and the like, for example, by using the following method of expressing DNA that codes for at least one of the TRAILR2 and the PSMA in a host cell.

A recombinant vector is produced by inserting full-length cDNA comprising a part that encodes at least one of TRAILR2 and PSMA, into downstream of a promoter in an appropriate expression vector. An appropriate length of a DNA fragment which comprises a part that encodes polypeptides, and which is prepared based on the full-length cDNA, may be used in place of the full-length cDNA. Next, it is possible to obtain a transfectant that produces at least one of TRAILR2 and PSMA by introducing the obtained recombinant vector in a host cell suitable for the expression vector.

As the expression vector, any vector can be used as long as it can be inserted into an autonomous replicating element or a chromosome in a host cell to be used, and comprises a suitable promoter in the position that enables the transcription of DNA that encodes at least one of TRAILR2 and PSMA.

As the host cell, any cell, for example, a microorganism belonging to the genus Escherichia such as E. coli, yeast, an insect cell or an animal cell, and the like, can be used as long as it enables the expression of a target gene.

In a case where a prokaryote such as E. coli is used as a host cell, it is preferable that a recombinant vector is a vector that can replicate autonomously in the prokaryote and that comprises a promoter, a ribosomal binding sequence, DNA comprising a part that encodes at least one of TRAILR2 and PSMA, and a transcription termination sequence. In addition, a transcription termination sequence is not essentially needed for the recombinant vector, it is preferable that a transcription termination sequence is arranged immediately below a structural gene. Furthermore, the recombinant vector may include a gene controlling a promoter.

As the recombinant vector, it is preferable to use a plasmid in which a distance between the Shine-Dalgarno sequence that is a ribosomal binding sequence, and a start codon is appropriately adjusted (to, for example, 6 to 18 nucleotides).

In addition, regarding a nucleotide sequence of DNA that encodes at least one of TRAILR2 and PSMA, it is possible to substitute a nucleotide so that a codon becomes optimum to be expressed in a host, which enables the enhancement in the production rate of at least one of the target TRAILR2 and PSMA.

As the expression vector, any vector can be used as long as it can exhibit its function in a host cell to be used. Examples thereof include pBTrp2, pBTac1, and pBTac2 (manufactured by Roche Diagnostics K.K.), pKK233-2 (manufactured by Pharmacia), pSE280 (manufactured by Invitrogen), pGEMEX-1 (manufactured by Promega Corporation), pQE-8 (manufactured by QIAGEN), pKYP10 (JP-A-S58-110600), pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82,4306 (1985)], pBluescript II SK(-) (manufactured by Stratagene Corporation), pTrs30 [prepared from Escherichia coli JM109/pTrS30 (FERM BP-5407)], pTrs32 [prepared from Escherichia coli JM109/pTrS32 (FERM BP-5408)], pGHA2 [prepared from Escherichia coli IGHA2(FERM BP-400), JP-A-S60-221091], pGKA2 [prepared from Escherichia coli IGKA2 (FERM BP-6798), JP-A-S60-221091], pTerm2 (United States Patent No. 4,686,191, United States Patent No. 4,939,094, United States Patent No. 5,160,735), pSupex, pUB110, pTP5, pC194, pEG400 [J. Bacteriol., 172,2392 (1990)], pGEX (manufactured by Pharmacia), pET System (manufactured by Novagen), pME18SFL3 (manufactured by Toyobo Co., Ltd.), and the like.

As the promoter, any promoter may be used as long as it can exhibit its function in a host cell to be used.

Examples thereof include promoters such as a trp promoter (Ptrp), a lac promoter, a PL promoter, a PR promoter, or a T7 promoter, which are derived from E. coli or phage. In addition, examples thereof also include promoters such as a tandem promoter with two tandemly arrayed Ptrps, a tac promoter, a lacT7 promoter, or a let I promoter, which are artificially designed and altered.

Examples of the host cell include E. coli XL1-Blue, E. coli XL2-Blue, E. coli DH1, E. coli MC1000, E. coli KY3276, E. coli W1485, E. coli JM109, E. coli HB101, E. coli No.49, E. coli W3110, E. coli NY49, E. coli DH5$\alpha$, and the like.

As a method for introducing a recombinant vector in a host cell, any method can be used as long as it is a method by which DNA is introduced into a host cell to be used. Examples thereof include a method using calcium ions [Proc. Natl. Acad. Sci. USA, 69, 2110 (1972), Gene, 17, 107 (1982), Molecular & General Genetics, 168, 111 (1979)].

In a case of using an animal cell as a host, as the expression vector, any vector can be used as long as it can exhibit its function in the animal cell. Examples thereof include pcDNAI (manufactured by Invitrogen), pcDM8 (manufactured by Funakoshi Co., Ltd.), pAGE107 [JP-A-H3-22979; Cytotechnology, 3,133 (1990)], pAS3-3 (JP-A-H2-227075), pCDM8 [Nature, 329, 840 (1987)], pcDNAI/Amp (manufactured by Invitrogen), pcDNA3.1 (manufactured by Invitrogen), pREP4 (manufactured by Invitrogen), pAGE103 [J. Biochemistry, 101, 1307 (1987)], pAGE210, pME18SFL3, pKANTEX93(International Publication No. 97/10354), and the like.

As the promoter, any promoter can be used as long as it can exhibit its function in the animal cell. Examples thereof include a promoter of cytomegalovirus (CMV) immediate early (IE) gene, an early promoter of SV40, a retroviral promoter, a metallothionein promoter, a heat-shock promoter, a SR$\alpha$ promoter, a promoter of Moloney murine leukemia virus, or an enhancer. In addition, a promoter may be used together with an enhancer of human CMV IE gene.

Examples of the host cell include a human Burkitt's lymphoma cell Namalwa, an African Green Monkey kidney cell COS, a Chinese hamster ovary cell CHO, a human leukemia cell HBT5637 (JP-A-S63-000299), and the like.

As a method for introducing a recombinant vector in a host cell, any method can be used as long as it is a method by which DNA is introduced into an animal cell. Examples thereof include the electroporation [Cytotechnology, 3, 133 (1990)], the calcium phosphate transfection method (JP-A-H2-227075), or the lipofection [Proc. Natl. Acad. Sci. USA, 84, 7413 (1987)], and the like.

It is possible to produce at least one of the TRAILR2 and the PSMA by culturing a microorganism having a recombinant vector into which DNA that encodes at least one of TRAILR2 and PSMA is introduced, or a transfectant derived from an animal cell obtained as above or the like, in a medium, generating and accumulating at least one of the TRAILR2 and the PSMA in the culture, and then collecting it from the culture. A method of culturing the transfectant in a medium can be performed according to a usual method used for a host culture.

In a case of being expressed in the cells derived from a eukaryote, it is possible to obtain at least one of TRAILR2 and PSMA added with sugars or sugar chains.

When culturing a microorganism that is transformed by a recombinant vector comprising an inducible promoter, an inducer may be added to a medium if necessary. For example, isopropyl-$\beta$-D-thiogalactopyranoside or the like may be added to a medium for a case of culturing a microorganism that is transformed by a recombinant vector comprising a lac promoter, and indoleacrylic acid or the like may be added to a medium for a case of culturing a microorganism that is transformed by a recombinant vector comprising a trp promoter.

Examples of the medium in which the transfectant derived from an animal cell as a host is cultured include RPMI 1640 Medium [The Journal of the American Medical Association, 199, 519 (1967)], Eagle's MEM Medium [Science, 122, 501 (1952)], Dulbecco's Modified MEM Medium [Virology, 8, 396 (1959)], Medium 199 [Proc. Soc. Exp. Biol. Med., 73, 1 (1950)], Iscove's Modified Dulbecco's Medium (IMDM), which are generally used, or a medium in which fetal bovine serum (FBS) or the like is added to these media. Culture is usually performed under the conditions of pH 6 to 8 and 30°C to 40°C, and in the presence of 5% $CO_2$ for 1 to 7 days. In addition, during the culture, antibiotics such as kanamycin and penicillin may be added to a medium, as necessary.

As a method for expressing a gene that encodes at least one of TRAILR2 and PSMA, it is possible to use a method of a secretory production or fused protein expression [Molecular Cloning, A Laboratory Manual, Second Edition, Cold Spring Harbor Laboratory Press (1989)], in addition to direct expression. Examples of a method for producing at least one of TRAILR2 and PSMA include a method of producing in a host cell, a method of secretion out of the host cell, and a method of producing on an outer membrane of the host cell. It is possible to select an appropriate method by changing a host cell to be used, and the structure of at least one of TRAILR2 and PSMA to be produced.

For example, it is possible to produce an antigen-fusion protein by producing DNA by linking DNA that encodes the Fc region of an antibody, DNA that encodes glutathione S-transferase (GST), or DNA that encodes a FLAG tag or DNA that encodes a Histidine tag, and the like, to DNA that encodes amino acid sequences of an extracellular region, and then expressing and purifying it. Specific examples include an Fc-fusion protein in which an extracellular region of at least one of TRAILR2 and PSMA is bound to the Fc region of human IgG, a protein in which an extracellular region of at least one of TRAILR2 and PSMA is fused with glutathione S-transferase (GST).

In a case where at least one of TRAILR2 and PSMA is produced in a host cell or on an outer membrane of the host cell, it is possible to actively secrete at least one of TRAILR2 and PSMA outside the host cell by using methods described in a method by Paulson et al. [J. Biol. Chem., 264, 17619 (1989)], a method by Lowe et al. [Proc. Natl. Acad. Sci., USA, 86, 8227 (1989), Genes Develop., 4, 1288 (1990)], JP-A-H05-336963, International Publication No. 94/23021, and the like. In addition, it is possible to enhance an amount of production of at least one of TRAILR2 and PSMA by using the gene amplification using dihydrofolate reductase gene or the like (JP-A-2-227075).

It is possible to isolate and purify at least one of the produced TRAILR2 and PSMA according to the below for example.

In a case where at least one of TRAILR2 and PSMA is expressed in the cells in a dissolved state, the cells are collected by centrifugation after completing culture, suspended in an aquatic buffer solution, followed by crushing of the cells using ultrasonic crusher, French press, Manton Gaulin homogenizer, Dyno mill, or the like, and therefore cell-free extract is obtained. It is possible to obtain purified protein from a supernatant obtained by the centrifugation of the cell-free extract by using methods such as a general method for isolation and purification of proteins, that is, a solvent extraction method, a salting-out method using ammonium sulfate or the like, a desalting method, a precipitation method using an organic solvent, anion-exchange chromatography using a resin such as Diethylaminoethyl (DEAE)-Sepharose or DIAION HPA-75 (manufactured by Mitsubishi Chemical Corporation), cation-exchange chromatography using a resin such as S-Sepharose FF (manufactured by Pharmacia), hydrophobic interaction chromatography method using a resin such as Butyl Sepharose or Phenyl Sepharose, a gel filtration method using molecular decoration, affinity chromatography, a chromatofocusing method, electrophoresis such isoelectric focusing electrophoresis, and the like alone or in combination.

In a case where at least one of TRAILR2 and PSMA forms an insoluble complex to be expressed in the cells, the cells are collected and then crushed in the same manner as above, followed by the centrifugation, and then an insoluble complex of at least one of the TRAILR2 and the PSMA is collected as a precipitated fraction. The collected insoluble complex of at least one of the TRAILR2 and the PSMA is solubilized with a protein denaturant. It is possible to obtain a polypeptide-purified protein by using the same method for isolation and purification as above, after returning at least one of the TRAILR2 and the PSMA back to a normal three-dimensional structure through dilution or dialysis of the solubilized solution.

In a case where at least one of TRAILR2 and PSMA, or a derivative thereof such as a sugar-modified complex are extracellularly secreted, it is possible to collect at least one of the TRAILR2 and the PSMA, or the derivative thereof such as a sugar-modified complex in a culture supernatant. The culture supernatant is subjected to procedures using a method such as the centrifugation as in the same manner as above, thereby obtaining a soluble fraction, and then by using the same method for isolation and purification as above, it is possible to obtain a purified protein from the soluble fraction.

In addition, at least one of TRAILR2 and PSMA used in the present invention can be produced by using a chemical synthesis method such the Fmoc method or the tBoc method. Specifically, it is possible to perform chemical synthesis using a peptide synthesizer manufactured by Advanced Chemtech, PerkinElmer, Inc., Pharmacia, Protein Technology Instrument, Inc., Shinseserubega Co., Perceptive, Shimadzu Corporation, and the like.

(2) Step of Preparing Antibody-Producing Cell

By immunizing 3 to 20 week old animals such as mice, rats, hamsters, or the like with the antigen obtained in (1), an antibody-producing cell is collected from spleen, lymph nodes, or peripheral blood of the animal. In addition, examples of the animals include a conditional knockout mouse of TRAILR2 or PSMA for enhancing immunogenicity, and a transgenic mouse that produces a human derived antibody described in the document of Tomizuka. et al. [Tomizuka. et al., Proc Natl Acad Sci USA., 97, 722 (2000)], and the like as an immune animal.

Immunization is performed by administering an antigen together with an appropriate adjuvant such as Freund's complete adjuvant, aluminum hydroxide gel, Bordetella pertussis vaccine, and the like. As a method for administration of an immunogen when immunizing a mouse, any one of a subcutaneous injection, an intraperitoneal injection, an intravenous injection, an intradermal injection, an intramuscular injection, a footpad injection, and the like may be used, but the intraperitoneal injection, the footpad injection, and the intravenous injection are preferable. In a case where an antigen is partially a peptide, producing a conjugate of the antigen to a carrier protein such as BSA (bovine serum albumin) and KLH (Keyhole Limpet hemocyanin), and this is used as an immunogen.

The administration of an antigen is performed 5 to 10 times every 1 to 2 weeks after the first administration. On the 3$^{rd}$ to 7$^{th}$ day after each administration, the collection of blood from a venous plexus of the fundus of the eye is performed, and the antibody valency of the serum is measured by using an enzyme immunoassay method [Antibodies - A Laboratory Manual, Cold Spring Harbor Laboratory (1988)], and the like. If animals of which the serum exhibited sufficient antibody valency with respect the antigen used for the immunization, are used as a supply source for the antibody-producing cell for fusion, it is possible to enhance the effect of the subsequent procedures.

On the 3$^{rd}$ to 7$^{th}$ day after a final administration of the antigen, tissues including the antibody-producing cell such as the spleen, are extracted from the immunized animals, and the antibody-producing cells are collected. The antibody-producing cell is a lymphocyte that is a plasma cell and a progenitor cell thereof. This may be obtained from any site of the individuals and can be generally obtained from the spleen, the lymph node, bone marrow, tonsils, peripheral blood, the appropriate combination thereof, and the like, but a spleen cell is most generally used. In a case of using the spleen cell, the spleen is shredded and loosened, followed by the centrifugation, and then erythrocytes are removed, and therefore the antibody-producing cell for fusion is obtained.

(3) Step of Preparing Myeloma

As myeloma, it is possible to use a cell that is derived from a mammal such as a mouse, a rat, a guinea pig, a hamster, a rabbit, or a human, and that has no ability of autoantibody production. Generally, an established cell obtained from a mouse such as a 8-azaguanine resistant mouse (BALB/c derived) myeloma cell line P3-X63Ag8-U1 (P3-Ul) [Current Topics in Microbiology and Immunology, 18, 1 (1978)], P3-NS1/1-Ag41 (NS-1) [European J. Immunology, 6, 511 (1976)], SP2/0-Ag14(SP-2) [Nature, 276, 269 (1978)], P3-X63-Ag8653 (653) [J. Immunology, 123, 1548 (1979)], P3-X63-Ag8(X63) [Nature, 256, 495(1975)], and the like are used. Each cell line is subjected to subculturing with a suitable medium such as an 8-azaguanine medium [RPM1-1640 medium supplemented with glutamine, 2-mercaptoethanol, gentamicin, FCS, and 8-azaguanine], Iscove's Modified Dulbecco's Medium (hereinafter will be referred to as "IMDM"), and Dulbecco's Modified Eagle Medium (hereinafter will be referred to as "DMEM"). The above cell lines are subjected to subculturing with a normal medium (for example, DMEM containing 10% FCS) 3 to 4 days before the cell fusion, and 2 x $10^7$ or more cells are acquired on the day of the fusion.

(4) Cell Fusion

The antibody-producing cells for fusion obtained in (2) and the myeloma cells obtained in (3) are thoroughly washed with the Minimum Essential Medium (MEM) or PBS (disodium phosphate 1.83g, monopotassium phosphate 0.21 g, salt 7.65g, distilled water 1 liter, pH 7.2), mix the antibody-producing cells for fusion are mixed with the myeloma cells to become 5:1 to 10:1, followed by the centrifugation, and then the supernatant is removed. After the precipitated cell clusters are loosened thoroughly, a mixture of polyethylene glycol 1000 (PEG-1000), MEM, and dimethylsulfoxide are added thereto while stirring at 37°C. Furthermore, 1 to 2 mL of MEM is added thereto every 1 to 2 minutes for several times, and then MEM is added so that the total amount becomes 50 mL. After the centrifugation, the supernatant is removed, the precipitated cell clusters are loosened gently, and then the cells are suspended gently in the HAT medium [normal medium supplemented with hypoxanthine, thymidine and aminopterin]. This suspension is cultured in a 5% $CO_2$ incubator at 37°C for 7 to 14 days.

In addition, it is possible to perform the cell fusion by the following method. The spleen cells and the myeloma cells are washed thoroughly with a serum-free medium (for example DMEM), or phosphate buffered saline (hereinafter will be referred to as "phosphate buffer solution"), and then the spleen cells are mixed with the myeloma cells so that the ratio of the number of cells becomes approximately 5:1 to 10:1, followed by the centrifugation. The supernatant is removed, and after the precipitated cell clusters are loosened thoroughly, the serum-free medium containing 1 mL of 50% (w/v) polyethylene glycol (molecular weight 1000 to 4000) is dropped thereto while stirring. Thereafter, 10 mL of the serum-free medium is slowly added thereto, followed by the centrifugation. The supernatant is removed again, the precipitated cells are suspended in a normal medium containing an appropriate amount of hypoxanthine · aminopterin · thymidine (HAT) solution and human interleukin 2 (IL-2) (hereinafter will be referred to as "HAT medium"), and the cells are dispensed in each well of a culture plate (hereinafter will be referred to as "plate"), and then the cell is cultured in the presence of 5% carbon dioxide gas at 37°C for approximately 2 weeks. During the culture, the HAT medium is supplemented as appropriate.

(5) Selection of Hybridoma Groups

In a case where the myeloma cells used for the fusion are 8-azaguanine resistance strains, that is hypoxanthine-guanine-phosphoribosyltransferase (HGPRT)-defective strains, the unfused myeloma cells and the fused cells between the myeloma cells cannot survive in the HAT medium. On the other hand, the fused cells between the antibody-producing cells, and the hybridomas of the antibody-producing cells and the myeloma cells can survive in the HAT medium, but a life span of the fused cells between the antibody-producing cells is reached shortly. Therefore, by continuing the culture in the HAT medium, only the hybridomas of the antibody-producing cells and the myeloma cells survive, and it is possible to obtain the hybridomas as a result.

For hybridomas grown with colonies, the replacement of the medium from the HAT medium to a medium from which aminopterin has been removed (hereinafter referred to as HT medium) is performed. Thereafter, some of culture supernatant is collected, and it is possible to select hybridomas that generate antibodies using a method for measuring an antibody valency described below. Examples of the method for measuring an antibody valency include various known techniques such as the radioisotopic immunoassay method (RIA), the solid-phase enzyme immunoassay method (ELISA), a fluorescent antibody method, and a passive hemagglutination reaction method, but RIA and ELISA are preferable in terms of detection sensitivity, rapidity, accuracy, a possibility of automated operation, and the like.

The hybridomas identified to produce a desired antibody by measuring the antibody titer are transferred to another plate, and perform cloning. Examples of the cloning method include a limiting dilution method in which culture is performed by dilution to such an extent that each well of a plate contains one cell, a soft agar method in which culture is performed in a soft agar medium to collect colonies, a method of isolating one cell using a micromanipulator, a method of isolating one cell using a cell Sorter, and the like.

For well in which the antibody valency is recognized, cloning are 2 to 4 times by using, for example, the limiting dilution method, and the one in which the antibody valency is stably recognized, is select as a hybridoma strain that produces a monoclonal antibody against human TRAILR2 or PSMA.

(6) Preparation of Monoclonal Antibody

The monoclonal antibody-producing hybridomas obtained in (5) are intraperitoneally injected into 8 to 10 week mice or nude mice which are treated by pristane treatment [intraperitoneally administered 2,6,10,14-tetramethylpentadecane (Pristane) 0.5 mL and bred for 2 weeks]. In 10 to 21 days, the hybridomas become an ascites tumor. The ascites are collected from this mouse, followed by centrifugating, removing solid, and then salting out with 40% to 50% ammonium sulfate, performing the purification by caprylic acid precipitation method, DEAE-Sepharose columns, protein A columns, or gel filtration columns, and then IgG or IgM fractions are collected to have a purified monoclonal antibody. In addition, by growing the hybridomas in the peritoneal cavity of mice from the same strain (for example, BALB/c) or Nu/Nu mice, rats, guinea pigs, hamsters, rabbits, or the like, it is possible to obtain the ascites containing large amounts of monoclonal antibodies that bind to TRAILR2 or PSMA.

After culturing the monoclonal antibody-producing hybridomas obtained in (5) in RPMI1640 medium supplemented with 10%FBS addition, the supernatant is removed by the centrifugation, and it is suspended in GIT medium, Hybridoma SFM medium supplemented with 5% Daigo's GF21, or the like, and then is cultured for 3 to 7 days by flask culture, spinner culture, back culture, or the like. The obtained cell suspension is subjected to the centrifugation, the purification of the obtained supernatant is performed using protein A columns or protein G columns, and then IgG fractions are collected to have a purified monoclonal antibody. As a simple method for the purification, it is possible to use a commercially available monoclonal antibody purification kit (for example, MabTrap GII kit manufactured by Amersham Pharmacia Biotech), and the like.

Determination of subclasses of the antibody is performed by the enzyme immunoassay method using a subclass typing kit. Determination of protein content can be performed by the Lowry method or a method of calculating from the absorbance at 280 run [1.4 ($OD_{280}$) = immunoglobulin 1 mg/mL].

(7) Binding Assay of Monoclonal Antibody to TRAILR2 or PSMA

The binding activity of the monoclonal antibody to TRAILR2 or PSMA can be measured by a binding assay system such as the Ouchterlony method, the ELISA method, the RIA method, a flow cytometry method (FCM), or a surface plasmon resonance method (SPR).

The Ouchterlony method is a simple method, but concentration is needed to be managed at low antibody concentration. On the other hand, in a case of using the ELISA method or the RIA method, by causing a culture supernatant to directly react with the antigen-adsorbing solid phase and by further using antibodies corresponding to various immunoglobulin isotypes and subclasses as secondary antibodies, it is possible to identify the isotype and subclass of the antibody and to measure the binding activity of the antibody.

As specific examples of the procedure, at least one of the purified or partially purified recombinant TRAILR2 and PSMA are adsorbed on a solid phase surface of such as a 96-well plate for ELISA, and then the solid phase surface on which the antigen is not adsorbed is blocked with a protein unrelated to the antigen such as bovine serum albumin (BSA). After washing a ELISA plate with phosphate buffered saline (PBS), PBS (Tween-PBS) containing 0.05% Tween 20, and the like, followed by the reaction with a serially diluted first antibody (for example, mouse serum, culture supernatant, and the like), and then the antibody is bound to the antigen immobilized on the plate. Next, as a second antibody, an anti-immunoglobulin antibody labeled with biotin, enzyme (horse radish peroxidase (HRP), alkaline phosphatase (ALP), and the like), a chemiluminescent substance or a radioactive compound, and the like, is dispensed to cause the second antibody to react with the first antibody bound on the plate. After washing with Tween-PBS, a reaction according to the labeling substance of the second antibody is performed, and then a monoclonal antibody that specifically reacts with the target antigen is selected.

In the FCM method, it is possible to measure the binding activity of an antibody to an antigen-expressing cell [Cancer Immunol. Immunother., 36,373 (1993)]. Binding of an antibody to a membrane protein antigen expressed on cell membrane means that the antibody recognizes and binds to the three-dimensional structure of an antigen present in nature.

Examples of the SPR method include kinetics analysis by Biacore. By using Biacore T100, kinetics in binding of the antigen and a test substance are measured, and the result is analyzed with analysis software attached to the instrument. As specific examples of the procedure, after immobilizing an anti-mouse IgG antibody on a sensor chip CM5 by an amine coupling method, the test substance such as a hybridoma culture supernatant or a purified monoclonal antibody is allowed to flow to be bound by an appropriate amount, further antigens having multiple concentrations of known concentration are allowed to flow, and then binding and dissociation are measured. Next, kinetics analysis is performed with respect to the obtained data using a 1:1 binding model with the software attached to the instrument to acquire various parameters. In addition, after fixing at least one of TRAILR2 and PSMA on the sensor chip by, for example, the amine coupling method, purified monoclonal antibodies having multiple concentrations of known concentration are allowed to flow, and then binding and dissociation are measured. Kinetics analysis is performed with respect to the obtained data using a bivalent binding model with the software attached to the instrument to acquire various parameters.

In addition, in the present invention, it is possible to select an antibody that bind to TRAILR2 or PSMA by competing with the antibody against TRAILR2 or PSMA by allowing test antibodies to coexist in the above binding assay system to react with each other. That is, by screening of an antibody in which binding to an antigen is inhibited when the test antibodies are added, it is possible to obtain an antibody that competes with the antibody obtained above, for binding to TRAILR2 or PSMA.

(8) Identification of Epitope of Monoclonal Antibody against TRAILR2 or PSMA

In the present invention, the identification of the epitope that the antibody recognizes and binds thereto can be carried out as follows.

For example, a partial defect, a mutant in which amino acid residues different between species are altered, or a mutant in which a specific domain is modified of the antigen are produced, and if the reactivity of the antibody against the defect or the mutant is lowered, it becomes clear that the defective site or the amino acid-altered site is an epitope of the antibody. Such a partial defect and mutant of the antigen may be obtained using a suitable host cell such as Escherichia coli, yeast, a plant cell, or a mammalian cell, as a secretory protein, and may be prepared as an antigen-expressing cell by expressing it on the cell membrane of a host cell. In the case of a membrane antigen, it is preferable to express it on the membrane of the host cell in order to express it while maintaining the three-dimensional structure of the antigen. In addition, it is possible to confirm the reactivity of the antibody by producing a synthetic peptide mimicking the primary structure or the three-dimensional structure of the antigen. Regarding the synthetic peptide, there is a method of producing various partial peptides of the molecule using a known peptide synthesis technique.

For example, with respect to the extracellular region of human and mouse TRAILR2 or PSMA, it is possible to identify the epitope of the antibody by producing a chimeric protein appropriately combined with the domains constituting each region, and then confirming the reactivity of the antibody against the protein. Thereafter, it is possible to further identify the epitope in detail by variously synthesizing the oligopeptide of the corresponding portion, the mutant of the peptide, or the like using an oligopeptide synthesis technique well known to those skilled in the art, and then confirming the reactivity of the antibody against the peptide. As a simple method for obtaining various types of oligopeptides, it is also possible to use a commercially available kit [for example, SPOTs Kit (manufactured by Genosys Biotechnologies, Inc.), a series of multipin-peptide synthesis kit (manufactured by Chiron Corporation) using a multipin synthesis method, and the like].

It is possible to obtain antibodies that bind to the same epitope to which the antibody capable of binding to TRAILR2 or PSMA binds by identifying the epitope of the antibody obtained in the binding assay system described above, producing a partial synthetic peptide of the epitope, a synthetic peptide mimicking the three-dimensional structure of the epitope, a recombinant of the epitope, or the like, and then immunizing them.

For example, if the epitope is a membrane protein, the epitope-specific antibody can be produced more efficiently by producing a recombinant fusion protein in which the entire extracellular region or a part of the extracellular domain is linked to an appropriate tag, for example, a FLAG tag, a Histidine tag, a GST protein or an antibody Fc region, and the like, and by immunizing with the recombinant protein.

2. Production of Genetically Recombinant Antibody

Examples of production of genetically recombinant antibodies are reviewed in P.J. Delves., ANTIBODY PRODUCTION ESSENTIAL TECHNIQUES., 1997 WILEY, P. Shepherd and C. Dean. Monoclonal Antibodies., 2000 OXFORD UNIVERSITY PRESS, and J.W. Goding., Monoclonal Antibodies: principles and practice., 1993 ACADEMIC PRESS, and the like, but a method for producing a chimeric antibody, a humanized antibody and a human antibody will be described below. In addition, it is also possible to produce a genetically recombinant antibody of mouse, rat, hamster and rabbit using the same method.

(1) Obtaining cDNA that Encodes V Region of Monoclonal Antibody from Hybridoma

The cDNA that encodes VH and VL of the monoclonal antibody can be obtained, for example, as follows.

First, mRNA is extracted from a hybridoma producing a monoclonal antibody, and cDNA is synthesized. Next, the synthesized cDNA is cloned into a vector such as phage or plasmid to produce a cDNA library. Recombinant phage or recombinant plasmid comprising cDNA that encodes VH or VL isolated from the library by using DNA that encodes C region or V region of the antibody as a probe. The entire nucleotide sequence of VH or VL in the isolated recombinant phage or the recombinant plasmid is isolated, and then the entire amino acid sequence of VH or VL is deduced from the nucleotide sequence.

As a non-human animal used for producing a hybridoma, mouse, rat, hamster, rabbit, or the like is used, but any animal can be used as long as a hybridoma can be produced.

For the preparation of total RNA from hybridomas, the guanidine thiocyanate-cesium trifluoroacetate method [Methods in Enzymol., 154, 3 (1987)], or a kit such as RNA easy Kit (manufactured by Qiagen), and the like are used.

To prepare mRNA from total RNA, oligo (dT) immobilized cellulose column chromatography [Molecular Cloning, A Laboratory Manual, Second Edition, Cold Spring Harbor Laboratory Press (1989)], or a kit such as Oligo-dT30 <Super> mRNA Purification Kit (Manufactured by Takara Bio Inc.), and the like is used. Furthermore, it is possible to prepare mRNA using a kit such as Fast Track mRNA Isolation Kit (manufactured by Invitrogen), or QuickPrep mRNA Purification Kit (manufactured by Pharmacia).

For the synthesis of cDNA and the production of a cDNA library, a known method [Molecular Cloning, A Laboratory Manual, Second Edition, Cold Spring Harbor Laboratory Press (1989), Current Protocols in Molecular Biology, Supplement 1, John Wiley & Sons (1987-1997)], or a kit such as SuperScript Plasmid System for cDNA Synthesis and Plasmid Cloning (manufactured by Invitrogen) or ZAP-cDNA Synthesis Kit (manufactured by Stratagene), and the like are used.

When producing the cDNA library, any vector capable of being incorporated with the cDNA can be used as a vector into which the cDNA synthesized using mRNA extracted from the hybridoma as a template is incorporated.

For example, ZAP Express [Strategies, 5, 58 (1992)], pBluescript IISK (+) [Nucleic Acids Research, 17, 9494 (1989)], λZAPII (Stratagene), λgt 10 and λgt 11 [DNA Cloning: A Practical Approach, I, 49 (1985)], Lambda Blue Mid (Clontech Laboratories, Inc.), λExCell, pT7T3-18U (Pharmacia), pcD2 [Mol. Cell. Biol., 3, 280 (1983)], pUC18 [Gene, 33, 103 (1985)], and the like are used.

Any Escherichia coli into which a cDNA library constructed by a phage or a plasmid vector is introduced can be used as long as it is capable of being introduced with the cDNA library and of expressing and maintaining. For example, XL1-Blue MRF' [Strategies, 5, 81(1992)], C600 [Genetics, 39,440 (1954)], Y1088, Y1090 [Science, 222, 778 (1983)], NM522 [J. Mol. Biol., 166, 1 (1983)], K802 [J. Mol. Biol., 16, 118 (1966)], or JM105 [Gene, 38,275 (1985)], and the like are used.

For the selection of cDNA clones that code for VH or VL of the non-human antibody from the cDNA library, a colony hybridization method using isotope or fluorescently labeled probe, or the plaque hybridization method [Molecular Cloning, A Laboratory Manual, Second Edition, Cold Spring Harbor Laboratory Press (1989)], and the like are used.

In addition, it is possible to produce cDNA that encodes VH or VL by producing a primer and performing the polymerase chain reaction method [hereinafter referred to as PCR method, Molecular Cloning, A Laboratory Manual, Second Edition, Cold Spring Harbor Laboratory Press (1989), and Current Protocols in Molecular Biology, Supplement 1, John Wiley & Sons (1987-1997)] using cDNA synthesized from mRNA or the cDNA library as a template.

The selected cDNA is cleaved with an appropriate restriction enzyme or the like, and then cloned into a plasmid such as pBluescript SK (-) (manufactured by Stratagene), and the nucleotide sequence of the cDNA is determined by a commonly used nucleotide sequence analysis method or the like. For example, after performing a reaction such as the dideoxy method [Proc. Natl. Acad. Sci. USA, 74, 5463 (1977)], an analysis is performed using an automatic nucleotide sequence analyzer such as A.L.F. DNA sequencer (Pharmacia).

By deducing the total amino acid sequence of each of VH and VL from the determined entire nucleotide sequence and comparing it with the total amino acid sequence of VH and VL of a known antibody [Sequences of Proteins of Immunological Interest, US Dept. Health and Human Services (1991)], it is confirmed that whether the obtained cDNA encodes the complete amino acid sequence of each of the VH and VL of the antibody comprising a secretion signal sequence.

Regarding the complete amino acid sequence of each of the VH and VL of the antibody comprising the secretion signal sequence, by comparing with the entire amino acid sequence of VH and VL of a known antibody [Sequences of Proteins of Immunological Interest, US Dept. Health and Human Services (1991)], it is possible to deduce the length of the secretion signal sequence and the N-terminus amino acid sequence and to identify a subgroup to which they belong.

In addition, it is possible to deduce the amino acid sequence of each CDR of VH and VL by comparing with the amino acid sequence of VH and VL of a known antibody [Sequences of Proteins of Immunological Interest, US Dept. Health and Human Services (1991)].

Furthermore, with respect to the obtained complete amino acid sequence of VH and VL, it is possible to confirm whether the complete amino acid sequence of VH and VL is new by carrying out homology search by the BLAST method [J. Mol. Biol., 215, 403 (1990)] and the like using any database such as SWISS-PROT or PIR-Protein.

(2) Construction of Expression Vector for Genetically Recombinant Antibody

An expression vector for a genetically recombinant antibody can be constructed by cloning DNA that encodes at least one of CH and CL of a human antibody into an expression vector for animal cells.

As the C region of a human antibody, it is possible to use CH and CL of any human antibody, and for example, CH of $\gamma 1$ subclass and CL of $\kappa$ class of the human antibody, and the like can be used. As DNA that encodes CH and CL of the human antibody, cDNA is used, but it is also possible to use chromosomal DNA consisting of exons and introns.

As the expression vector for animal cells, any vector can be used as long as it is capable of being incorporated with a gene that encodes the C region of the human antibody so as to express. For example, it is possible to use pAGE107 [Cytotechnol., 3, 133 (1990)], pAGE103 [J. Biochem., 101, 1307 (1987)], pHSG274 [Gene, 27,223 (1984)], pKCR [Proc. Natl. Acad. Sci. USA, 78,1527 (1981)], pSG1bd2-4 [Cytotechnol., 4, 173 (1990)], or pSE1UK1Sed1-3 [Cytotechnol., 13, 79 (1993)], INPEP4 (manufactured by Biogen-IDEC), N5KG1val (United States Patent No. 6,001,358), transposon vector (International Publication No. 2010/143698), and the like.

As a promoter and an enhancer of the expression vector for animal cells, it is possible to use the early promoter of SV40 [J. Biochem., 101, 1307 (1987)], the Moloney murine leukemia virus LTR [Biochem. Biophys. Res. Commun., 149,960 (1987), the CMV promoter (United States Patent No. 5,168,062), or the promoter of immunoglobulin H chain [Cell, 41, 479 (1985)], and the enhancer [Cell, 33, 717 (1983)], and the like.

For the expression of the genetically recombinant antibody, a vector carrying both genes of the antibody H chain and L chain (tandem type vector) [J. Immunol. Methods, 167, 271 (1994)] is used from the viewpoints of ease of vector construction, ease of introduction into animal cells, balance of expression levels between the antibody H chain and L chain in cells, and the like, but multiple vectors carrying separately each gene of the antibody H chain and L chain (separation type vector) can be used in combination.

As the tandem type expression vector for a genetically recombinant antibody, pKANTEX93 (International Publication No. 97/10354), pEE18 [Hybridoma, 17, 559 (1998)], N5KG1val (United States Patent No. 6,001,358), Tol2 transposon vector (International Publication No. 2010/143698), and the like are used.

(3) Construction of Chimeric Antibody Expression Vector

By cloning the cDNA that encodes VH or VL of a non-human antibody obtained in (1) upstream of each gene that encodes CH or CL of a human antibody in the expression vector for a genetically recombinant antibody obtained in (2), a chimeric antibody expression vector can be constructed.

First, in order to link the 3' terminus side of the cDNA that encodes VH or VL of the non-human antibody with the 5' terminus side of CH or CL of the human antibody, cDNA of VH and VL in which the base sequence of the linking part is designed to encode an appropriate amino acid, and to become an appropriate restriction enzyme recognition sequence is produced. Next, the produced cDNAs of VH and VL are each cloned upstream of each gene that encodes CH or CL of a human antibody in the expression vector for a genetically recombinant antibody obtained in (2) so that they are expressed in an appropriate form, and therefore a chimeric antibody expression vector is constructed.

In addition, each of cDNAs that encodes VH or VL of the non-human antibody are amplified by the PCR method using synthetic DNA comprising an appropriate restriction enzyme recognition sequence at both ends, and are cloned into the expression vector for a genetically recombinant antibody obtained in (2), and therefore it is also possible to construct a chimeric antibody expression vector.

(4) Production of cDNA Encoding V Region of Humanized Antibody

A cDNA that codes for VH or VL of a humanized antibody can be produced as follows. First, each amino acid sequence in framework regions (hereinafter referred to as FR) of VH or VL of a human antibody, to which the amino acid sequence of the CDR of VH or VL of a non-human antibody obtained in (1) is to be transplanted is selected.

Any amino acid sequence derived from a human antibody can be used as selected amino acid sequence of the FR. For example, the amino acid sequence of the FR of a human antibody registered in a database such as Protein Data Bank, or the common amino acid sequence in each subgroup of the FR of a human antibody [Sequences of Proteins of Immunological Interest, US Dept. Health and Human Services (1991)], and the like are used. In order to suppress the decrease in binding activity of the antibody, the amino acid sequence of the human FR having as high homology (60% or more) as possible to the amino acid sequence of the FR of VH or VL of the original non-human antibody is selected.

Next, each of the amino acid sequences of the CDRs of the original non-human antibody are transplanted to the selected amino acid sequences of the FR of VH or VL of a human antibody, and each of the amino acid sequence of VH or VL of a humanized antibody is designed. By converting the designed amino acid sequence into a DNA sequence in consideration of the use frequency of codons found in the nucleotide sequence of the antibody gene [Sequences of Proteins of Immunological Interest, US Dept. Health and Human Services (1991)], each of cDNA sequence of VH or VL of a humanized antibody is designed.

Based on the designed cDNA sequence, several synthetic DNAs having lengths of 100 to 150 bases are synthesized and the PCR reaction is carried out using them. In this case, from the viewpoint of the reaction efficiency in the PCR reaction and the synthesizable length of DNA, preferably 4 to 6 synthetic DNAs are designed for each of the H chain and the L chain. In addition, it is possible to synthesize and use synthetic DNA having the full length of the variable region.

Furthermore, by introducing an appropriate restriction enzyme recognition sequence at the 5' terminus of the synthetic DNA located at both ends, it is possible to easily clone cDNA that encodes VH or VL of a humanized antibody into the expression vector for a genetically recombinant antibody obtained in (2). After the PCR reaction, each of the amplified products are cloned into a plasmid such as pBluescript SK (-) (manufactured by Stratagene), the nucleotide sequence is determined by the same method described in (1), and therefore a plasmid comprising a DNA sequence that encodes the amino acid sequence of VH or VL of a desired humanized antibody is obtained.

(5) Alteration of Amino Acid Sequence of V Region of Humanized Antibody

If the FRs of VH and VL of a human antibody are merely being transplanted with the CDRs of VH and VL of the non-human antibody, the antigen binding activity of the humanized antibody is lower than that of the original non-human antibody [BIO/TECHNOLOGY, 9,266 (1991)]. For this reason, by identifying amino acid residues directly related to antigen binding, amino acid residues interacting with amino acid residues of CDR, and amino acid residues that maintain the three-dimensional structure of the antibody and is indirectly related to antigen binding, among amino acid sequences of FRs of VH and VL of a human antibody, and by substituting these amino acid residues with the amino acid residues of the original non-human antibody, it is possible to increase the antigen binding activity of the humanized antibody which has been lowered.

In order to identify amino acid residues of FR related to the antigen binding activity, it is possible to construct and analyze the three-dimensional structure of the antibody by using X-ray crystallography [J. Mol. Biol., 112, 535 (1977)], or computer modeling [Protein Engineering, 7, 1501 (1994)], and the like. Furthermore, it is possible to obtain a modified humanized antibody having necessary antigen binding activity by producing various types of variants for each antibody, repeatedly examining their correlation with each antigen binding activity, and through trial and error.

The amino acid residues of FR of VH and VL of a human antibody can be modified by carrying out the PCR reaction described in (4) using synthetic DNA for the modification. For the amplified product after the PCR reaction, the nucleotide sequence is determined and whether the intended modification has been carried out is confirmed by using the method described in (1).

(6) Construction of Expression Vector for Humanized Antibody

By cloning each of the cDNAs that encodes VH or VL of the constructed humanized antibody upstream of each gene that encodes CH or CL of a human antibody in the expression vector for a genetically recombinant antibody obtained in (2), an expression vector for a humanized antibody can be constructed.

For example, the cloning is carried out upstream of each gene that encodes CH or CL of a human antibody in the expression vector for a genetically recombinant antibody obtained in (2) by introducing an appropriate restriction enzyme recognition sequence at the 5' terminus of the synthetic DNA located at both ends among the synthetic DNAs used for constructing the VH or VL of the humanized antibody obtained in (4) and (5), so that they are expressed in an appropriate form.

(7) Construction of Expression Vector for Human Antibody

In a case where a hybridoma that produces a monoclonal antibody is established using an animal that produces a human antibody as an immunized animal, it is possible to obtain the amino acid sequence and cDNA sequence of VH and VL of a human antibody in (1). By cloning each gene that encodes VH or VL of a human antibody obtained in (1) upstream of each gene that encodes CH or CL of a human antibody in the expression vector for a genetically recombinant antibody obtained in (2), an expression vector for a human antibody can be constructed.

(8) Transient Expression of Genetically Recombinant Antibody

By transgenically expressing a genetically recombinant antibody using the expression vector of a genetically recombinant antibody obtained in (3), (6) and (7), or a modified expression vector therefrom, it is possible to efficiently evaluate the antigen binding activity of the various types of genetically recombinant antibodies obtained.
As a host cell into which the expression vector is introduced, any cell can be used as long as it is a host cell capable of expressing the genetically recombinant antibody. For example, COS-7 cells [American Type Culture Collection (ATCC) number: CRL1651] are used. For introduction of the expression vector into the COS-7 cells, the DEAE-dextran method [Methods in Nucleic Acids Res., CRC press (1991)], or the lipofection method [Proc. Natl. Acad. Sci. USA, 84,7413 (1987)], and the like are used.
After introduction of the expression vector, the expression level and the antigen binding activity of the genetically recombinant antibody in a culture supernatant are measured by using the enzyme immunoassay method [Monoclonal Antibodies-Principles and practice, Third Edition, Academic Press (1996), Antibodies-A Laboratory Manual, Cold Spring Harbor Laboratory (1988), A manual for monoclonal antibody experiments, Kodansha scientific books (1987)], and the like.

(9) Acquisition of Stable Expression Strain of Genetically Recombinant Antibody and Preparation of Genetically Recombinant Antibody

By introducing the expression vector for a genetically recombinant antibody obtained in (3), (6) and (7) into an appropriate host cell, a transformant stably expressing the genetically recombinant antibody can be obtained.
For the introduction of an expression vector into a host cell, there are, for example, the electroporation method [JP-A-H2-257891, Cytotechnology, 3, 133 (1990)], a calcium ion method, an electroporation method, a spheroplast method, a lithium acetate method, a calcium phosphate method, a lipofection method, and the like. As a method of introducing a gene into an animal described below, there are, for example, a microinjection method, a method of introducing a gene into an ES cell by an electroporation or a lipofection method, a nuclear transplantation method, and the like.
As a host cell into which the expression vector for a genetically recombinant antibody is introduced, any cell can be used as long as it is a host cell capable of expressing the genetically recombinant antibody. For example, mouse SP2/0-Ag14 cells (ATCC CRL 1581), mouse P3X63-Ag8.653 cells (ATCC CRL 1580), Chinese hamster CHO-K1 cells (ATCC CCL-61), DUKXB11 (ATCC CCL-9096), Pro-5 cells (ATCC CCL-1781), CHO-S cells (Life Technologies, Cat No. 11619), CHO cells in which the dihydrofolate reductase gene (dhfr) is deficient (CHO/DG44 cell) [Proc. Natl. Acad. Sci. USA, 77,4216 (1980)], Lec 13 cells that acquired lectin tolerance [Somatic Cell and Molecular Genetics, 12, 55 (1986)], CHO cells in which the $\alpha$1,6-fucosyltransferase gene is deficient (International Publication No. 2005/035586, International Publication No. 02/31140), Rat YB2/3HL. P2. G11. 16Ag. 20 cells (ATCC No.: CRL 1662), and the like are used.
In addition, it is possible to use a host cell in which the activity of proteins such as enzymes related to intracellular synthesis of sugar nucleotide GDP-fucose, proteins such as enzymes related to glycosylation modification in which the 1-position of fucose is $\alpha$-bonded to the 6-position of N-acetylglucosamine at the reducing terminus of a N-glycoside-linked complex type sugar chain, proteins related to intracellular transport of sugar nucleotide GDP-fucose to the Golgi body, and the like, is reduce or lost, for example, CHO cells in which the $\alpha$1,6-fucosyltransferase gene is deficient (International Publication No. 2005/035586, International Publication No. 02/31140), and the like.
After introduction of the expression vector, a transformant stably expressing a genetically recombinant antibody is selected by culturing the transformant in a medium for animal cell culture containing a drug such as G418 sulfate (hereinafter referred to as G418) (JP-A-H2-257891).
As the medium for animal cell culture, RPMI 1640 medium (manufactured by Invitrogen), GIT medium (manufactured by Nippon Pharmaceutical Co., Ltd.), EX-CELL 301 medium (manufactured by Jay Earl H., Inc.), EX-CELL 302 medium (manufactured by Jay Earl H., Inc.), EX-CELL 325 medium (manufactured by Jay Earl H., Inc.), IMDM medium (manufactured by Invitrogen) or Hybridoma-SFM medium (manufactured by Invitrogen), or a medium in which various additives such as FBS to are added to these media, and the like are used. A genetically recombinant antibody is expressed and accumulated in a culture supernatant by culturing the obtained transformant in the medium. It is possible to measure the expression level and the antigen binding activity of the genetically recombinant antibody in the culture supernatant by the ELISA method and the like. In addition, the expression level of the genetically recombinant antibody produced by the transformant can be increased by using the DHFR amplification system (JP-A-H2-257891) and the like.
It is possible to purify the genetically recombinant antibody using a protein A column from the culture supernatant of the transformant [Monoclonal Antibodies - Principles and Practice, Third Edition, Academic Press (1996), Antibodies - A Laboratory Manual, Cold Spring Harbor Laboratory (1988)]. In addition, it is also possible to purify by combining methods used for purifying proteins, such as gel filtration, ion exchange chromatography and ultrafiltration.
It is possible to measure molecular weights of H chain, L chain, or whole antibody molecules of the purified genetically recombinant antibody by using polyacrylamide gel electrophoresis [Nature, 227, 680 (1970)], or Western blotting method [Monoclonal Antibodies - Principles and Practice, Third Edition, Academic Press (1996), Antibodies - A Laboratory Manual, Cold Spring Harbor Laboratory (1988)] and the like.

3. Production of Bispecific Antibody

The bispecific antibody of the present invention can be produced by, for example, first, obtaining a plurality of monoclonal antibodies that bind to different epitopes using the method described in 1. above, followed by determining the cDNA sequences of VH and VL of each antibody using method described in 2. above, and then designing an antibody to which the antigen binding site of each antibody is linked.

Specifically, it is possible to produce the antibody by synthesizing DNA in which the antigen binding site of each antibody is appropriately combined with a linker and incorporating the DNA into the genetically recombinant antibody expression vector described in 2. (2) above, and then allowing the bispecific antibody to be expressed. More specifically, it is possible to produce the antibody by synthesizing DNA that encodes a polypeptide in which VH of each antibody is linked via a linker, synthesizing DNA that encodes VL of each antibody, and incorporating these DNAs into the expression vector for a genetically recombinant antibody described in 2. (2) above, and then allowing the bispecific antibody to be expressed.

The antigen binding site can be isolated and obtained by techniques such as a phage display method and a yeast display method in addition to the method using hybridomas described in 1. above [Emmanuelle Laffy et. Al., Human Antibodies 14,33-55, (2005)].

The linker is a peptide which connects between one antigen binding domain and another antigen binding domain and is usually formed of a polypeptide of 10 or more amino acid residues. In the present invention, as an example of the linker, fragments of all or a part of the amino acid sequence formed of CH1, hinge, CH2 and CH3 of the antibody can be appropriately combined to be used. In addition, it is possible to use the amino acid sequences by partially deleting them or changing the order.

The animal species of the antibody used for the linker is not particularly limited, but human is preferable. In addition, a subclass of the antibody used for the linker is not particularly limited, but it is preferably IgM or IgG4, and more preferably IgG4.

In the present invention, specific examples of the linker include a linker formed of 14 amino acid residues from the N-terminus to the 14$^{th}$ position of CH1 of IgG4 represented by SEQ ID NO: 11, a linker formed of amino acid residues from the N-terminus to the 14$^{th}$ position of CH1 of IgM represented by SEQ ID NO: 12, a linker formed of CH1 of IgG4 represented by SEQ ID NO: 93, and the like, and the linker formed of CH1 of IgG4 represented by SEQ ID NO: 93 is preferable.

In addition, in the case of producing a bispecific antibody formed of a plurality of VHs and a single VL, a screening using a phage display method or the like is carried out and each VH most suitable for a single VL is selected so that each antigen binding site contained in the bispecific antibody reacts with each specific antigen.

Specifically, first, an animal is immunized with a first antigen using the method described in 1. above to produce a hybridoma from its spleen, and a DNA sequence that encodes a binding site of the first antigen is cloned. Next, an animal is immunized with a second antigen to prepare cDNA library from its spleen, and DNA that encodes the amino acid sequence of VH is obtained from the library by the PCR.

Subsequently, a phage library expressing scFv in which VH obtained by immunization of the second antigen and VL of the binding site of the first antigen are linked is produced, and phages displaying scFv that specifically bind to the second antigen by panning using the phage library are selected. From the selected phages, a DNA sequence that codes for the amino acid sequence of VH of the binding site of the second antigen is cloned.

Furthermore, a DNA sequence that encodes the amino acid sequence of the polypeptide in which the VH of the binding site of the first antigen and the VH of the binding site of the second antigen are linked via the above described linker is designed, and the DNA sequence and a DNA sequence that codes for the amino acid sequence of a single VL are inserted into, for example, the expression vector for a genetically recombinant antibody described in 2. (2) above, and therefore it is possible to construct the expression vector for the bispecific antibody of the present invention.

4. Evaluation on Activity of Bispecific Antibody or Antibody Fragment Thereof of Present Invention

The activity of the purified antibody or an antibody fragment thereof can be evaluated as follows.

It is possible to measure the binding activity of the bispecific antibody of the present invention to a cell line expressing at least one of TRAILR2 and PSMA using the binding assay system described in 1. (7) above.

It is possible to measure the CDC activity or the ADCC activity on cells expressing at least one of TRAILR2 and PSMA by a known measuring method [Cancer Immunol. Immunother., 36, 373 (1993)].

It is possible to measure the cell death-inducing activity of the bispecific antibody of the present invention by the following method. For example, cells are seeded in a 96-well plate, and after adding antibodies and culturing for a certain period of time, a reaction is caused with WST-8 reagent (manufactured by Dojindo Molecular Technologies, Inc.), and then the absorbance at 450 nm is measured with a plate reader to measure the cell survival rate.

5. Therapeutic Method for Disease Using Bispecific Antibody or Antibody Fragment Thereof of Present Invention

The bispecific antibody or an antibody fragment thereof of the present invention can be used for the treatment of diseases involving TRAILR2 and PSMA-expressing cells. Examples of the diseases involving TRAILR2 and PSMA include malignant tumors, cancer, and the like.

Examples of the malignant tumor and the cancer include, large intestine cancer, colorectal cancer, lung cancer, breast cancer, glioma, malignant melanoma (melanoma), thyroid cancer, renal cell carcinoma, leukemia, lymphoma, T cell lymphoma, stomach cancer, pancreatic cancer, cervical cancer, endometrial cancer, ovarian cancer, esophageal cancer, liver cancer, head and neck squamous cell carcinoma, skin cancer, urinary tract cancer, bladder cancer, prostate cancer, chorionic carcinoma, pharyngeal cancer, laryngeal cancer, mesothelioma, pleural tumor, arrhenoblastoma, endometrial hyperplasia, endometriosis, embryoma, fibrosarcoma, kaposi's sarcoma, angioma, cavernous hemangioma, angioblastoma, retinoblastoma, astrocytoma, neurofibroma, oligodendroglioma, medulloblastoma, neuroblastoma, glioma, rhabdomyosarcoma, glioblastoma, osteogenic sarcoma, leiomyosarcoma, thyroid sarcoma, Wilm's tumor, and the like.

The therapeutic agent containing the bispecific antibody or an antibody fragment thereof of the present invention, or a derivative thereof may contain only the antibody or the antibody fragment, or a derivative thereof as an active ingredient, but is generally mixed with one or more pharmacologically acceptable carriers to be provided as medicinal formulation that is produced by methods known in the technical field of pharmaceutical science.

Examples of the route of administration include oral administration or parenteral administration such as intraoral, airway, intrarectal, subcutaneous, intramuscular or intravenous administration. Examples of the form of administration include a spray, a capsule, a tablet, a powder, a granule, a syrup, an emulsion, a suppository, an injection, an ointment, or a tape, and the like. Various formulations can be produced by a common method using commonly used an excipient, a filler, a binder, a wetting agent, a disintegrating agent, a surfactant, a lubricant, a dispersant, a buffer, a preservative, a solubilizing agent, an antiseptic, a coloring agent, a flavoring agent, a stabilizer and the like.

Examples of the excipients include lactose, fructose, glucose, corn starch, sorbit, crystalline cellulose, sterilized water, ethanol, glycerol, a saline solution, a buffer solution, and the like. Examples of the disintegrating agents include starch, sodium alginate, gelatin, calcium carbonate, calcium citrate, dextrin, magnesium carbonate, synthetic magnesium silicate, and the like.

Examples of the binders include methyl cellulose or a salt thereof, ethyl cellulose, gum arabic, gelatin, hydroxypropyl cellulose, polyvinyl pyrrolidone, and the like. Examples of the lubricants include talc, magnesium stearate, polyethylene glycol, hydrogenated vegetable oil, and the like.

Examples of the stabilizers include amino acids such as arginine, histidine, lysine and methionine, human serum albumin, gelatin, dextran 40, methyl cellulose, sodium sulfite, sodium metasulfite, and the like.

Examples of other additives include syrup, vaseline, glycerin, ethanol, propylene glycol, citric acid, sodium chloride, sodium nitrite, sodium phosphate and the like.

Examples of the preparations suitable for oral administration include emulsions, syrups, capsules, tablets, powders, granules, and the like.

Liquid preparations such as emulsions or syrups are produced by using water, sugars such as sucrose, sorbitol or fructose, glycols such as polyethylene glycol or propylene glycol, oils such as sesame oil, olive oil or soybean oil, preservatives such as p-hydroxybenzoic acid esters, or flavors such as strawberry flavor or peppermint, and the like, as additives.

The capsules, tablets, powders, granules, and the like are produced by using excipients such as lactose, glucose, sucrose or mannitol, disintegrating agents such as starch or sodium alginate, lubricants such as magnesium stearate or talc, binders such as polyvinyl alcohol, hydroxypropyl cellulose or gelatin, surfactants such as a fatty acid ester, or plasticizers such as glycerin, as an additive.

Formulations suitable for parenteral administration include, for example, injections, suppositories, sprays, and the like. The injections are produced by using a salt solution, a glucose solution, carriers formed of a mixture of these solutions, and the like.

The Suppositories are produced using carriers such as cocoa butter, hydrogenated fats or carboxylic acids. The sprays are produced by using a carrier which do not stimulate the oral and respiratory mucosa of a recipient and in which the monoclonal antibody or an antibody fragment thereof of the present invention is dispersed as fine particles, and which makes it easy to be absorbed, and the like. Examples of the carriers include lactose or glycerin and the like. In addition, it can also be produced as an aerosol or a dry powder. Furthermore, also for the above parenteral preparations, it is possible to add the components exemplified as the additives for the formulations suitable for oral administration.

An effective amount administered as a combination of an effective amount of the bispecific antibody of the present invention and a suitable diluent and a pharmacologically acceptable carrier are 0.0001 mg to 100 mg per kg body weight per one time, and are administered at interval of 2 days to 8 weeks.

6. Diagnostic Method for Disease Using Bispecific Antibody or Antibody Fragment Thereof of Present Invention

By detecting or measuring cell in which at least one of TRAILR2 and PSMA is expressed using the bispecific antibody or the antibody fragment of the present invention, it is possible to diagnose diseases related to TRAILR2 and PSMA-expressing cell.

Diagnosis of a malignant tumor or a cancer which is the disease related to TRAILR2 and PSMA-expressing cells can be performed by, for example, detecting or measuring at least one of TRAILR2 and PSMA as follows.

First, detecting or measuring at least one of TRAILR2 and PSMA is performed with respect to a biological sample collected from a plurality of healthy subjects by the following immunological method using the bispecific antibody or the antibody fragment of the present invention, or a derivative thereof, and then the abundance of at least one of TRAILR2 and PSMA in the biological sample of the healthy subjects is examined.

Next, similarly, also the abundance of at least one of TRAILR2 and PSMA in a biological sample of a subject is examined, and then its abundance is compared with the abundance of the healthy subjects. In a case where the abundance of at least one of TRAILR2 and PSMA of the subject increases compared with that of the healthy subjects, the subject is diagnosed as having cancer. Other diseases related to TRAILR2 and PSMA expressing-cells can also be diagnosed by the same method.

The immunological method is a method of detecting or measuring the amount of antibody or the amount of antigen using a labeled antigen or antibody. Examples thereof include a radioactive material labeled immune antibody method, the enzyme immunoassay method, the fluorescence immunoassay method, the luminescent immunoassay method, the western blotting method, or the physicochemical method, and the like.

Examples of the radioactive material labeled immune antibody method include a method in which the bispecific antibody or the antibody fragment of the present invention is reacted with antigen or cells expressing an antigen, or the like, and then reacted with an anti-immunoglobulin antibody or a binding fragment subjected to radiolabeling, followed by measurement with a scintillation counter or the like.

Examples of the enzyme immunoassay method include a method in which the bispecific antibody or the antibody fragment of the present invention is reacted with antigen, cells expressing an antigen or the like, and then reacted with an anti-immunoglobulin antibody or a binding fragment subjected to labeling, followed by measurement of the coloring dye with an absorptiometer. Examples thereof include a sandwich ELISA method and the like.

As a labeling substance used in the enzyme immunoassay method, a known enzyme label [enzyme immunoassay method, Igaku-Shoin Ltd. (1987)] can be used. For example, alkaline phosphatase label, peroxidase label, luciferase label, or biotin label, and the like are used.

The sandwich ELISA method is a method in which after binding an antibody to a solid phase, a target antigen to be detected or to be measured is trapped, and then a second antibody is reacted with the trapped antigen. In the ELISA method, two types of antibodies having different antigen binding sites, which are antibodies or antibody fragments which bind to the antigen to be detected or measured are prepared, and among these, a first antibody or an antibody fragment is adsorbed on a plate in advance (for example, a 96-well plate), followed by labeling a second antibody or an antibody fragment with a fluorescent substance such as FITC, an enzyme such as peroxidase, or biotin, and the like. A plate on which the antibody is adsorbed is allowed to react with the cells separated from the living body or a lysate thereof, tissues or a lysate thereof, a cell culture supernatant, serum, pleural effusion, ascites, or intraocular fluid, and the like, and then to react with the labeled antibody or antibody fragments, followed by the detection reaction according to the labeled material. From a calibration curve prepared by serially diluting a known concentration of antigen, the antigen concentration in the test sample is calculated.

As the antibody used in the sandwich ELISA method, either a polyclonal antibody or a monoclonal antibody may be used, and antibody fragments such as Fab, Fab', $F(ab)_2$, and the like may be used. The combination of the two kinds antibodies used in the sandwich ELISA method may be a combination of monoclonal antibodies or antibody fragments thereof which bind to different epitopes, or may be a combination of a polyclonal antibody and a monoclonal antibody or an antibody fragment thereof.

As the fluorescence immunoassay method, measurement is carried out by, for example, a method described in the document [Monoclonal Antibodies-Principles and Practice, Third Edition, Academic Press (1996), A manual for monoclonal antibody experiments, Kodansha scientific books (1987)], and the like. As a labeling substance used in the fluorescence immunoassay method, a known fluorescent label [fluorescent antibody method, Soft Science (1983)] can be used. For example, FITC or RITC or the like is used.

As the luminescence immunoassay method, measurement is carried out by, for example, a method described in the document [Bioluminescence and Chemiluminescence Clinical Test 42, Hirokawa-Shoten Ltd. (1998)], and the like. As a labeling substance used in the luminescence immunoassay method, there is a known luminescent label, and for example, acridinium esters or lophines, and the like are used.

As the western blotting method, measurement is carried out by after fractionating antigens or cells expressing an antigen or the like by SDS (sodium dodecyl sulfate) - PAGE [Antibodies - A Laboratory Manual Cold Spring Harbor Laboratory (1988)], blotting the gel on polyvinylidene fluoride (PVDF) membranes or nitrocellulose membranes, reacting the antibody or antibody fragment that binds to the antigen with the membrane, and then further reacting it with an anti-IgG antibody or an antibody fragment thereof subjected to a labeling with fluorescent substance such as FITC, labeling with an enzyme label such as peroxidase, or biotin labeling or the like, and then visualizing the label. An example is shown below.

First, cells and tissues expressing a polypeptide having a desired amino acid sequence are lysed, and 0.1 to 30 μg as a protein amount per lane is subjected to electrophoresis by the SDS-PAGE method under reducing conditions. Next, the electrophoresed protein is transferred to a PVDF membrane and is reacted with PBS containing 1 to 10% BSA (hereinafter referred to as BSA-PBS) for 30 minutes at room temperature to perform blocking operation. The bispecific antibody of the present invention is reacted therewith, and is washed with PBS containing 0.05% to 0.1% Tween-20 (Tween-PBS), and then a goat anti-mouse IgG labeled with peroxidase is reacted therewith for 2 hours at room temperature. By washing with Tween-PBS, and detecting a band to which the antibody is bound using ECL Western Blotting Detection Reagents (manufactured by Amersham) or the like, an antigen is detected. As the antibodies used for detection by western blotting, an antibody capable of binding to polypeptides that do not retain the natural three-dimensional structure is used.

As the physicochemical method, for example, by binding at least one of TRAILR2 and PSMA which are the antigens with the bispecific antibody or the antibody fragment thereof of the present invention, an aggregate is formed, and therefore the aggregate is detected. As another physicochemical method, it is possible to use a capillary tube method, a one-dimensional immunodiffusion method, an immunoturbidimetric method, or a latex immunoturbidimetric method [Outline of Clinical Examination Method, KANEHARA & Co., LTD. (1998)], and the like.

In the latex immunoturbidimetric method, when a carrier such as a polystyrene latex having a particle size of approximately 0.1 to 1 μm sensitized with an antibody or an antigen is used to cause the antigen-antibody reaction with a corresponding antigen or antibody, scattered light is increased in a reaction solution and the transmitted light is decreased. The antigen concentration and the like in the test sample are measured by detecting this change as absorbance or integrating sphere turbidity.

On the other hand, for detection or measurement of cells expressing at least one of TRAILR2 and PSMA, a known immunological detection method can be used, but it is preferable to use the immunoprecipitation method, the immunocytochemical staining method, the immunohistochemical staining method, or the fluorescent antibody staining method, and the like.

As the immunoprecipitation method, a cell expressing at least one of TRAILR2 and PSMA or the like is reacted with the bispecific antibody or the antibody fragment thereof of the present invention, and then a carrier having specific binding ability to an immunoglobulin such as Protein G Sepharose is added thereto, and therefore an antigen-antibody complex is precipitated.

Alternatively, the method can also be carried out by the following method. First, the bispecific antibody or the antibody fragment thereof of the present invention is immobilized on a 96-well plate for ELISA, and then blocked with BSA-PBS. Next, discard BSA-PBS, thoroughly wash with PBS, and then lysates of cells and tissues expressing at least one of TRAILR2 and PSMA are reacted therewith. After washed thoroughly, immunoprecipitates are extracted from the plate with a sample buffer for SDS-PAGE, and then detected by the above western blotting.

The immunocytostaining method or the immunohistochemical staining method is a method in which cells or tissues or the like expressing an antigen are treated with a surfactant or methanol or the like in order to improve the permeability of the antibody in some cases, and then reacted with the bispecific antibody of the present invention, further reacted with an anti-immunoglobulin antibody or a binding fragment thereof subjected to labeling with fluorescent such as FITC, labeling with an enzyme label such as peroxidase or biotin labeling, the label is visualized and then observed with a microscope. In addition, detection can be carried out by the fluorescent antibody staining method in which a fluorescent-labeled antibody is reacted with cells and analyzed with a flow cytometer [Monoclonal Antibodies - Principles and Practice, Third edition, Academic Press (1996), A manual for monoclonal antibody experiments, Kodansha scientific books (1987)]. Particularly, with respect to the bispecific antibody or the antibody fragment thereof of the present invention, it is possible to detect at least one of TRAILR2- and PSMA-expressing on the cell membrane by the fluorescent antibody staining method.

In addition, when using the FMAT 8100 HTS system (manufactured by Applied Biosystems) or the like among the fluorescent antibody staining methods, it is possible to measure the amount of antigen or the amount of antibody without separating the formed antibody-antigen complex from a free antibody or antigen not involved in formation of the antibody-antigen complex.

Hereinafter, the present invention will be described in more detail, but the present invention is not limited to the following Examples.

EXAMPLES

[Example 1] Construction of Expression Plasmid Vector for Anti-hTRAILR2 Agonistic Monoclonal Antibody KMDA2 Antibody

An anti-hTRAILR2 agonistic monoclonal antibody KMDA2 antibody is produced as a positive control antibody of an anti-human TRAILR2 (hTRAILR2) antibody based on the amino acid sequences of a heavy chain variable region (VH) and a light chain variable region (VL) of the clone 0304 described in International Publication No. 2002/094880.

The entire nucleotide sequence of VH of the KMDA2 antibody is represented by SEQ ID NO: 1, the entire amino acid sequence of VH which comprises a signal sequence deduced from the sequence is represented by SEQ ID NO: 7, and the amino acid sequence in which the signal sequence is excluded from the amino acid sequence represented by SEQ ID NO: 7 is represented by SEQ ID NO: 114. In addition, the entire nucleotide sequence of VL of the KMDA2 antibody is represented by SEQ ID NO: 2, the entire amino acid sequence of VL which comprises a signal sequence deduced from the sequence is represented by SEQ ID NO: 8, and the amino acid sequence in which the signal sequence is excluded from the amino acid sequence represented by SEQ ID NO: 8 is represented by SEQ ID NO: 115. An expression plasmid vector for the KMDA2 antibody was constructed by the method described below.

Using a L chain cDNA of the KMDA2 antibody as a template, the variable region and the leader sequence of the L chain were amplified by the PCR by using primers that are represented by SEQ ID NO: 3 and SEQ ID NO: 4 and designed to add a restriction enzyme recognition site to the terminus of VL. The PCR fragment was collected by ethanol precipitation, and then digested with restriction enzymes BglII and BsiWI, and therefore a DNA fragment of approximately 400 bp was obtained.

On the other hand, a plasmid vector N5KG4PE (described in International Publication No. 2002/088186) was digested with the restriction enzymes BglII and BsiWI, and then dephosphorylated with alkaline phosphatase (E. coli C75) (Takara Shuzo Co., Ltd.), and therefore a DNA fragment of slightly less than about 9 kb was obtained. These two DNA fragments were linked by T4 DNA ligase and a plasmid N5KG4PE-KMDA2L was obtained.

Next, N5KG4PE-KMDA2L was digested with restriction enzymes NheI and SalI and dephosphorylated, and therefore a DNA fragment of approximately 9.3 kb was obtained. On the other hand, using the H chain cDNA of the KMDA 2 antibody as a template, the variable region and the leader sequence of the H chain of KMDA 2 were amplified by the PCR by using primers represented by SEQ ID NO: 5 and SEQ ID NO: 6. The PCR fragment was digested with the restriction enzymes NheI and SalI and a DNA fragment of approximately 450 bp was obtained. These two DNA fragments were linked and the expression plasmid vector N5KG4PE_KMDA2 of the anti-hTRAILR2 agonistic monoclonal antibody KMDA2 antibody was produced.

The H chain PCR fragment amplified by the above method is formed of the 5' untranslated region in the H chain, the signal sequence, the variable region (VH) and a portion of the constant region. Similarly, the PCR-amplified fragment of the L chain is formed of the 5' untranslated region in the L chain, the leader sequence, the variable region (VL) and a portion of the constant region. The leader sequence is a secretory signal sequence of an antibody and is an amino acid sequence separated from a mature antibody protein.

[Example 2] Construction of Expression Plasmid Vector for Anti-hTRAILR2 Monoclonal Antibody E11 Antibody

An anti-hTRAILR2 monoclonal antibody E11 antibody was produced for use in the production of a bispecific antibody that binds to hTRAILR2 and hPSMA described below. E11 antibody was produced based on the amino acid sequences of VH and VL of the clone E-11-13 described in International Publication No. 2002/094880. Sequence information of E11 antibody is shown in Table 1.

In Table 1, the entire nucleotide sequence that codes for the amino acid sequence of VH or VL of the antibody is described as a nucleotide sequence that codes for VH or VL (including a signal sequence), an amino acid sequence deduced from the nucleotide sequence is described as an amino acid sequence of VH or VL (including a signal sequence), and an amino acid sequence in which a signal sequence is excluded from the amino acid sequence is described as an amino acid sequence of VH or VL (excluding signal sequence).

In addition, the amino acid sequences of CDRs 1 to 3 of the heavy chain or the light chain of the antibody are described as amino acid sequences of HCDRs 1 to 3 or LCDRs 1 to 3, respectively. A nucleotide sequence that encodes the amino acid sequences of VH and VL of E11 antibody was inserted into the plasmid vector NSKG1 (described in United States Patent No. 6,001,358) or N5KG4PE [R409K] (described in International Publication No. 2002/088186). Therefore, an expression plasmid vector N5KG1_E11 of an E11 antibody which is an anti-hTRAILR2 monoclonal antibody of the human IgG1 type, and an expression plasmid vector N5KG4PE [R409K]_E11 of an E11 antibody which is an anti-hTRAILR2 monoclonal antibody of the mutated human IgG4 type (human IgG4 containing amino acid modifications of S228P, L235E and R409K in the constant region) were obtained.

[Table 1]

| Sequence information of anti-hTRAILR2 monoclonal antibody E11 antibody | |
|---|---|
| Nucleotide sequence that encodes VH (including signal sequence) | SEQ ID NO: 9 |
| Amino acid sequence of VH (including signal sequence) | SEQ ID NO: 69 |
| Amino acid sequence of VH (excluding signal sequence) | SEQ ID NO: 118 |
| Amino acid sequence of HCDR 1 | SEQ ID NO: 70 |
| Amino acid sequence of HCDR 2 | SEQ ID NO: 71 |
| Amino acid sequence of HCDR 3 | SEQ ID NO: 72 |
| Nucleotide sequence that encodes VL (including signal sequence) | SEQ ID NO: 10 |
| Amino acid sequence of VL (including signal sequence) | SEQ ID NO: 73 |
| Amino acid sequence of VL (excluding signal sequence) | SEQ ID NO: 119 |
| Amino acid sequence of LCDR 1 | SEQ ID NO: 74 |
| Amino acid sequence of LCDR 2 | SEQ ID NO: 75 |
| Amino acid sequence of LCDR 3 | SEQ ID NO: 76 |

[Example 3] Acquisition of Anti-hPSMA Monoclonal Antibody

(1) Acquisition of anti-hPSMA monoclonal antibody comprising the same amino acid sequences of VL as anti-hTRAILR2 monoclonal antibody E11 antibody, and production of expression plasmid vector for this antibody

An anti-hPSMA monoclonal antibody comprising the same amino acid sequences of VL as the anti-hTRAILR2 monoclonal antibody E11 antibody was obtained by the method described below.

Human antibody-producing mice [Ishida & Lonberg, IBC's 11th Antibody Engineering, Abstract 2000; Ishida, 1. et al., Cloning & Stem Cells 4, 85-96 (2002) and Ishida Isao (2002) Experimental Medicine 20, 6, 846-851] were intraperitoneally administered and immunized with a mixture of FLAG-PSMA of Example 5 described below and Sigma Adjuvant System (manufactured by Sigma-Aldrich Co. LLC.) as an immunogen.

The spleen was surgically removed from the immunized mouse, and placed on a cell strainer (manufactured by Falcon). While slightly crushing the spleen with a silicon rod, the cells were transferred to a tube, followed by the centrifugation to precipitate the cells, and then the cells were reacted with a reagent for removing red blood cells in ice for 3 minutes, followed by the centrifugation again.

RNA was extracted from the obtained spleen cells using ISOGEN (Nippon Gene Co., Ltd.), cDNA was amplified with SMARTer RACE cDNA amplification kit (manufactured by Clontech Laboratories, Inc.), and a VH gene fragment was further amplified by the PCR. Each of the VH gene fragment and the VL gene of E11 described in Table 1 was inserted into the phagemid pCANTAB 5E (Amersham Pharmacia Biotech Inc.). Escherichia coli TGI (manufactured by Lucigen) was transformed and a plasmid was obtained. By infecting the obtained plasmid with M13K07 Helper Phage (manufactured by Invitrogen), a human antibody M13 phage library in which the VH gene was libraryized was obtained.

Using this human antibody M13 phage library and the phage display method described below, an anti-hPSMA monoclonal antibody having the same amino acid sequence of VL as the anti-hTRAILR2 monoclonal antibody E11 antibody was obtained. MAXISORP STARTUBE (manufactured by NUNC) in which the FLAG-PSMA of Example 5 described below was immobilized thereon and a site not bound by FLAG-PSMA was blocked using SuperBlock Blockig Buffer (manufactured by Thermo Fisher Scientific Inc.) was reacted with a human antibody M 13 phage library at room temperature for 1 hour, washed 5 times with PBS, and then the phage was eluted with 0.1 M Gly-HCl (pH 2.2).

TGl competent cells were infected with the eluted phage and the phage was amplified. Thereafter, FLAG-PSMA immobilized on MAXISORP STARTUBE was reacted again, and washing and elution were carried out. This procedure was repeated two or three times to concentrate the phage representing the scFv specifically binding to FLAG-PSMA.

The concentrated phage was monocloned and clones having the affinity to FLAG-PSMA were selected by ELISA. In ELISA, MAXISORP STARTUBE (manufactured by NUNC) in which the FLAG-PSMA of Example 5 described below was immobilized thereon, and a site not bound by FLAG-PSMA was blocked by using SuperBlock Blockig Buffer (manufactured by Thermo Fisher Scientific Inc.) was used.

Each phage clone was added to each well and allowed to react at room temperature for 30 minutes, and then each well was washed three times with PBS containing 0.1% Tween 20 (hereinafter referred to as PBS-T). Subsequently, a solution in which horseradish peroxidase-labeled anti-M13 antibody (manufactured by GE Healthcare) was diluted 5000 times with PBS-T containing 10% Block Ace (manufactured by Dainippon Pharma Co., Ltd.) was added to each well by 50 μL and incubated at room temperature for 30 minutes.

After washing the microplate 3 times with PBS-T, 50 μL of TMB chromogenic substrate solution (manufactured by DAKO) was added to each well and incubated at room temperature for 10 minutes. Finally, 0.5 M sulfuric acid (50 μL/well) was added to each well to stop the chromogenic reaction, and the absorbance at a wavelength of 450 nm (reference wavelength 570 nm) was measured with a microplate reader (1420 ARVO multilabel counter: manufactured by WALLAC).

Sequence analysis was performed on the clones bound to FLAG-PSMA. Anti-hPSMA monoclonal antibody PI134 antibody, PI101 antibody, PN7 antibody, PI105 antibody, PI108 antibody, PI115 antibody, PI118 antibody, PI127 antibody, PI143 antibody, PL223 antibody and PN170 antibody, which have the same amino acid sequence of VL as the anti-hTRAILR2 monoclonal antibody E11 antibody shown in Table 1, were obtained. Sequence information of the heavy chain of each hPSMA monoclonal antibody is shown in Table 2 by the same style as Table 1.

[Table 2]

| Sequence information on heavy chain of anti-hPSMA monoclonal antibody | | | | | | | | | | | |
|---|---|---|---|---|---|---|---|---|---|---|---|
| Clone name | PN7 | PI134 | PI143 | PI105 | PI127 | PI108 | PI115 | PL223 | PI101 | PI118 | PN170 |
| Nucleotide sequence that codes for VH (including signal sequence) | SEQ ID NO: 15 | SEQ ID NO: 13 | SEQ ID NO: 21 | SEQ ID NO: 16 | SEQ ID NO: 20 | SEQ ID NO: 17 | SEQ ID NO: 18 | SEQ NO: 22 | SEQ ID NO: 14 | SEQ ID NO: 19 | SEQ ID NO: 94 |
| Amino acid sequence of VH (including signal sequence) | SEQ ID NO: 83 | SEQ ID NO: 77 | SEQ ID NO: 90 | SEQ ID NO: 85 | SEQ ID NO: 89 | SEQ ID NO: 86 | SEQ ID NO: 87 | SEQ ID NO: 91 | SEQ ID NO: 81 | SEQ ID NO: 88 | SEQ ID NO: 95 |
| Amino acid sequence of VH (excluding signal sequence) | SEQ ID NO: 122 | SEQ ID NO: 120 | SEQ ID NO: 128 | SEQ ID NO: 123 | SEQ ID NO: 127 | SEQ ID NO: 124 | SEQ ID NO: 125 | SEQ ID NO: 129 | SEQ ID NO: 121 | SEQ ID NO: 126 | SEQ ID NO: 130 |
| Ammo acid sequence of HCDR 1 | SEQ ID NO: 78 | SEQ ID NO: 78 | SEQ ID NO: 78 | SEQ NO: 78 | SEQ ID NO: 78 | SEQ ID NO: 78 | SEQ ID NO: 78 | SEQ ID NO: 78 | SEQ ID NO: 78 | SEQ ID NO: 78 | SEQ ID NO: 96 |
| Amino acid sequence of HCDR 2 | SEQ ID NO: 82 | SEQ ID NO: 79 | SEQ ID NO: 79 | SEQ ID NO: 79 | SEQ ID NO: 79 | SEQ ID NO: 79 | SEQ ID NO: 79 | SEQ ID NO: 79 | SEQ ID NO: 82 | SEQ ID NO: 82 | SEQ ID NO: 97 |
| Amino acid sequence of HCDR 3 | SEQ ID NO: 84 | SEQ ID NO: 80 | SEQ ID NO: 80 | SEQ ID NO: 80 | SEQ ID NO: 80 | SEQ ID NO: 80 | SEQ ID NO: 80 | SEQ ID NO: 80 | SEQ ID NO: 80 | SEQ ID NO: 80 | SEQ ID NO: 98 |

Nucleotide sequences that encodes each of the amino acid sequences of VH and VL of PI134 antibody, PI101 antibody, PI105 antibody, PI108 antibody, PI115 antibody, PI118 antibody, PI127 antibody, PI143 antibody, PL223 antibody and PN170 antibody, were synthesized. Expression plasmid vectors inserted into plasmid vector N5KG1 by the same method as in Example 1 were produced, and each one was named N5KG1_E11VL_PI134, N5KG1_E11VL_PI101, N5KG1_E11VL_PI105, N5KG1_E11VL_PI108, N5KG1_E11VL_PI115, N5KG1_E11VL-PI118, N5KG1_E11VL_PI127, N5KG1_E11VL_PI143, N5KG1_E11VL_PL223, and N5KG1_E11VL_PN170.

In addition, a nucleotide sequence that encodes the amino acid sequences of VH and VL of PN7 antibody was synthesized, inserted into plasmid vector N5KG4PE [R409K] (described in International Publication No. 2002/088186) by the same method as in Example 1 to produce an expression plasmid vector, and the vector was named N5KG4PE [R409K]_E11VL_PN7.

(2) Production of Expression Plasmid Vector for Anti-hPSMA Monoclonal Antibody 2A10 Antibody

The anti-hPSMA monoclonal antibody 2A10 antibody described in International Publication No. 2006/089230 was produced as a positive control antibody for the anti-hPSMA monoclonal antibody. The entire nucleotide sequence of VH of 2A10 antibody is represented by SEQ ID NO: 23, the entire amino acid sequence of VH which comprises a signal sequence deduced from the sequence is represented by SEQ ID NO: 24, and the amino acid sequence in which the signal sequence is excluded from the amino acid sequence represented by SEQ ID NO: 24, is represented by SEQ ID NO: 116.

In addition, the entire nucleotide sequence of VL of 2A10 antibody is represented by SEQ ID NO: 25, the entire amino acid sequence of the VL which comprises a signal sequence deduced from the sequence is represented by SEQ ID NO: 26, and the amino acid sequence in which the signal sequence is excluded from the amino acid sequence represented by SEQ ID NO: 26 is represented by SEQ ID NO: 117. An expression plasmid vector for the 2A10 antibody was produced by the method described below.

A gene that encodes the amino acid sequence of the VL of 2A10 antibody was synthesized and subcloned into the BglII-BsiWI site of the plasmid vector N5KG1, and therefore N5KG1_2A10VL was obtained. Next, a gene that encodes the VH of 2A10 was synthesized as a template, and a gene fragment of the VH region was amplified by the PCR using the primers represented by SEQ ID NO: 27 and SEQ ID NO: 28, and KOD plus DNA Polymerase (manufactured by Toyobo Co., Ltd.). The PCR was carried out for 25 cycles of reactions at 94°C for 30 seconds, 58°C for 30 seconds, and 68°C for 45 seconds.

Using the obtained gene fragment of the VH region as a template, a gene fragment in which the signal sequence is linked to the VH region was amplified by the PCR using the primers represented by SEQ ID NO: 29 and SEQ ID NO: 28 and KOD polymerase. The PCR was carried out for 25 cycles of reactions at 94°C for 30 seconds, 58°C for 30 seconds, and 68°C for 90 seconds. The obtained gene fragment was inserted into the SalI-NheI site of the N5KG1_2A10VL vector, and therefore an expression plasmid vector N5KG1_2A10 of anti-hPSMA monoclonal antibody 2A10 antibody was obtained.

[Example 4] Construction of Expression Vector for Bispecific Antibody Binding to hTRAILR2 and hPSMA

A bispecific antibody that binds to hTRAILR2 and hPSMA (hereinafter referred to as hTRAILR2-hPSMA bispecific antibody or simply bispecific antibody) and that has the structure illustrated in Fig. 1, was produced by the following method. The heavy chain of the bispecific antibody includes the amino acid sequences of VH of the anti-hTRAILR2 monoclonal antibody E11 antibody, the linker, and VH of the anti-hPSMA monoclonal antibody in order from the N-terminus side in Fig. 1, and all four light chains of the antibody include the amino acid sequence of VL of E11 antibody. Like this bispecific antibody, an antibody in which VLs of two different antigen binding sites are in common is referred to as a bispecific antibody having the common light chain.

The names of the hTRAILR2-hPSMA bispecific antibody, the clone name of the anti-TRAILR2 monoclonal antibody used for producing the antibody, the structure of the linker, the clone name of the anti-hPSMA monoclonal antibody, and the name of the expression plasmid vector for the bispecific antibody are shown in Table 3. Furthermore, with respect to this bispecific antibody, the amino acid sequences of the polypeptide formed of VH of the E11 antibody, the linker and VH of the anti-hPSMA monoclonal antibody (hereinafter also simply referred to as VH-linker-VH) and the nucleotide sequences that encode the amino acid sequences are shown in Table 4.

[Table 3]

| Name of bispecific antibody | Anti-TRAILR2 antibody | Linker structure | Anti-PSMA antibody | Name of expression vector for antibody |
|---|---|---|---|---|
| E11-CH1-PI134VH | E11 | IgG4 CH1 | PI134 | N5KG1_E11VL_E11-CH1-PI134VH |
| E11-CH1-PI101VH | | | PI101 | N5KG1_E11VL_E11-CH1-PI101VH |
| E11-CH1-PI105VH | | | PI105 | N5KG1_E11VL_E11-CH1-PI105VH |
| E11-CH1-PI108VH | | | PI108 | N5KG1_E11VL_E11-CH1-PI108VH |
| E11-CH1-PI115VH | | | PI115 | N5KG1_E11VL_E11-CH1-PI115VH |
| E11-CH1-PI118VH | | | PI118 | N5KG1_E11VL_E11-CH1-PI118VH |
| E11-CH1-PI127VH | | | PI127 | N5KG1_E11VL_E11-CH1-PI127VH |
| E11-CH1-PI143VH | | | PI143 | N5KG1_E11VL_E11-CH1-PI143VH |
| E11-CH1-PL223VH | | | PL223 | N5KG1_1VL_E11-CH1-PL223VH |
| E11-CH1-PN7VH | | | PN7 | N5K_G4PE[R409K]_E11VL_E11-CH1-PN7VH |
| E11-CH1-PN170VH | | | PN170 | N5KG1_E11VL_E11-CH1-PN170VH |
| E11-GL-PI134VH | | 14 Amino acid residue from N-terminus of IgG4 CH1 | PI134 | N5KG1_E11VL_E11-GL-PI134VH |
| E11-ML-PI101VH | | 14 Amino acid residue from N-terminus of IgM CH1 | PI101 | N5KG1_E11VL_E11-ML-PI101VH |

[Table 4]

| Name of bispecific antibody | Nucleotide sequence that encodes VH-linker-VH (including signal sequence) | Amino acid sequence of VH-linker-VH (including signal sequence) | Amino acid sequence of VH-linker-VH (excluding signal sequence) |
|---|---|---|---|
| E11-CH1-PI134VH | SEQ ID NO: 34 | SEQ ID NO: 99 | SEQ ID NO: 131 |
| E11-CH1-PI101VH | SEQ ID NO: 36 | SEQ ID NO: 100 | SEQ ID NO: 132 |
| E11-CH1-PI105VH | SEQ ID NO: 39 | SEQ ID NO: 101 | SEQ ID NO: 133 |
| E11-CH1-PI108VH | SEQ ID NO: 40 | SEQ ID NO: 102 | SEQ ID NO:134 |
| E11-CH1-PI115VH | SEQ ID NO: 41 | SEQ ID NO: 103 | SEQ ID NO: 135 |
| E11-CH1-PI118VH | SEQ ID NO: 43 | SEQ ID NO: 104 | SEQ ID NO: 136 |

(continued)

| Name of bispecific antibody | Nucleotide sequence that encodes VH-linker-VH (including signal sequence) | Amino acid sequence of VH-linker-VH (including signal sequence) | Amino acid sequence of VH-linker-VH (excluding signal sequence) |
|---|---|---|---|
| E11-CH1-PI127VH | SEQ ID NO: 45 | SEQ ID NO: 105 | SEQ ID NO: 137 |
| E11-CH1-PI143VH | SEQ ID NO: 48 | SEQ ID NO: 106 | SEQ ID NO: 138 |
| E11-CH1-PL223VH | SEQ ID NO: 51 | SEQ ID NO: 107 | SEQ ID NO: 139 |
| E11-CH1-PN7VH | SEQ ID NO: 53 | SEQ ID NO: 108 | SEQ ID NO: 140 |
| E11-CH1-PN170VH | SEQ ID NO: 56 | SEQ ID NO: 109 | SEQ ID NO: 141 |
| E11-GL-PI134VH | SEQ ID NO: 58 | SEQ ID NO: 110 | SEQ ID NO: 142 |
| E11-ML-PI101VH | SEQ ID NO: 63 | SEQ ID NO: 111 | SEQ ID NO: 143 |

(1) Production of Expression Plasmid Vector for hTRAILR2-hPSMA Bispecific Antibody comprising CH1 of Human IgG4 as Linker

An expression plasmid vector for the bispecific antibody comprising CH1 of human IgG4 as a linker among the bispecific antibodies described in Tables 3 and 4, was produced by the method described below. The amino acid sequence of the linker is represented by SEQ ID NO: 93.

Using the nucleotide sequence of the constant region of human IgG4 as a template, the gene fragment of the linker portion was amplified by the PCR using the primers represented by SEQ ID NO: 30 and SEQ ID NO: 31 and KOD plus DNA Polymerase (manufactured by Toyobo Co., Ltd.). On the other hand, the gene fragment of the VH region of each anti-hPSMA monoclonal antibody was amplified by the PCR using a vector as VH amplification template, a primer for VH amplification and KOD polymerase shown in Table 5. For every amplification, the PCR was carried out for 25 cycles of reactions at 94°C for 30 seconds, 58°C. for 30 seconds, and 68°C for 45 seconds.

Next, using each gene fragment of the above linker portion and the VH region of each anti-hPSMA monoclonal antibody as a template, and the gene fragment in which the linker portion and the VH region of each anti-hPSMA monoclonal antibody are linked was amplified by the PCR using the primer for VH-linker binding shown in Table 5 and KOD polymerase.

The PCR was carried out for 25 cycles of reactions at 94°C for 30 seconds, 58°C for 30 seconds, and 68°C for 90 seconds. An expression plasmid vector for each hTRAILR2-hPSMA bispecific antibody shown in Table 5 was obtained by linking the amplified gene fragment to N5KG1_E11 or N5KG4PE [R409K]_E11 cleaved by NheI.

[Table 5]

| Expression plasmid vector for antibody | Vector as VH amplification template | Primer for VH amplification | Primer for VH-linker binding |
|---|---|---|---|
| N5KG1_E11VL_E11-CH1-PI134VH | N5KG1_E11VL_PI134 | SEQ ID NOs: 32, 33 | SEQ ID NOs: 50, 33 |
| N5KG1_E11VL_Ell-CH1-PI101VH | N5KG1_E11VL_PI101 | SEQ ID NOs: 35, 33 | SEQ ID NOs: 30, 33 |
| N5KG1_E11VL_E11-CH1-PI105VH | NSKG1_E11VL_PI105 | SEQ ID NOs: 37, 38 | SEQ ID NOs: 30, 38 |
| N5KG1_E11VL_E11-CH1-PI108VH | N5KG1_E11VL_PI108 | SEQ ID NOs: 32, 38 | SEQ ID NOs: 30, 38 |

(continued)

| Expression plasmid vector for antibody | Vector as VH amplification template | Primer for VH amplification | Primer for VH-linker binding |
|---|---|---|---|
| N5KG1_E11VL_E11-CH1-PI115VH | N5KG1_E11VL_PI115 | SEQ ID NOs: 35, 33 | SEQ ID NOs: 30, 33 |
| N5KG1_E11VL_E11-CH1-PI118VH | N5KG1_E11VL_PI118 | SEQ ID NOs: 42, 33 | SEQ ID NOs: 30, 33 |
| NSKG1_E11VL_E11-CH1-PI127VH | N5KG1_E11VL_PI127 | SEQ ID NOs: 44, 38 | SEQ ID NOs: 30, 38 |
| N5KG1_E11VL_E11-CH1-PI143VH | N5KG1_E11VL_PI143 | SEQ ID NOs: 46, 47 | SEQ ID NOs: 30, 47 |
| N5KG1_E11VL_E11-CH1-PL223VH | N5KG1_E11VL_PI223 | SEQ ID NOs: 49, 50 | SEQ ID NOs: 30, 50 |
| NSK G4PE [R409K]_E11VL_E11-CH1-PN7VH | N5KG4PE [R409K]_E11VL_PN7 | SEQ ID NOs: 52, 33 | SEQ ID NOs: 30, 33 |
| N5KG1_E11VL_E11-CH1-PN170VH | N5KGI_E11VL_PN170 | SEQ ID NOs: 54, 55 | SEQ ID NOs: 30, 55 |

(2) Production of expression plasmid vector for hTRAILR2-hPSMA bispecific antibody comprising the amino acid sequence from the N-terminus to the 14th position of CH1 of human IgG4 as linker (GL linker)

An expression plasmid vector for hTRAILR2-hPSMA bispecific antibody E11-GL-PI1134VH antibody shown in Table 3 and Table 4 was produced by the method described below. The linker of the bispecific antibody is the amino acid sequence from the N-terminus to the 14th position of CH1 of human IgG4 represented by SEQ ID NO: 11. Using the plasmid vector N5KG1_E11VL_PI134 as a template, the gene fragment of the VH region of anti-hPSMA monoclonal antibody PI134 antibody was amplified by the PCR using the primers represented by SEQ ID NO: 57 and SEQ ID NO: 33 and KOD polymerase.

The PCR was carried out for 25 cycles of reactions at 94°C for 30 seconds, 58°C for 30 seconds, and 68°C for 45 seconds. An expression plasmid vector N5KG1_E11VL_E11-GL-PI134VH for the hTRAILR2-hPSMA bispecific antibody E11-GL-PI1134VH antibody was produced by linking the gene fragment to N5KG1_E11 cleaved by the restriction enzyme NheI.

(3) Production of expression plasmid vector for hTRAILR2-hPSMA bispecific antibody comprising amino acid sequence from N-terminus to 14th position of CH1 of human IgM as linker (ML linker)

An expression plasmid vector for the hTRAILR2-hPSMA bispecific antibody E11-ML-PI101VH antibody shown in Table 3 and Table 4 was produced by the method described below. The linker of the bispecific antibody is the amino acid sequence from the N-terminus to the 14th position of CH1 of human IgM represented by SEQ ID NO: 12. Using N5KG1_E11 as a template, the gene fragment of the VH region of the anti-hTRAILR2 monoclonal antibody E11 antibody was amplified by the PCR using the primers represented by SEQ ID NO: 59 and SEQ ID NO: 60 and KOD polymerase.

The PCR was carried out for 25 cycles of reactions at 94°C for 30 seconds, 58°C for 30 seconds, and 68°C for 45 seconds. On the other hand, using N5KG1_E11VL_PI101 as a template, the gene fragment of the VH region of the anti-hPSMA monoclonal antibody PI101 antibody was amplified by the PCR using the primers represented by SEQ ID NO: 61 and SEQ ID NO: 62 and KOD polymerase. The PCR was carried out for 25 cycles of reactions at 94°C for 30 seconds, 58°C for 30 seconds, and 68°C for 45 seconds.

Next, the above gene fragments of the VH region of E11 antibody and the VH region of PI101 antibody were inserted into N5KG1 cleaved by the restriction enzymes SalI and NheI, and therefore an expression plasmid vector N5KG1_E11VL_E11-ML-PI101VH of the hTRAILR2-hPSMA bispecific antibody E11-ML-PI101VH antibody was produced.

[Example 5] Preparation of Soluble hPSMAAntigen

As a soluble antigen of human prostate specific membrane antigen (hPSMA), an extracellular domain protein of hPSMA in which a FLAG-tag was added to the N-terminus was produced by the method described below. The nucleotide sequence that encodes the amino acid sequence of hPSMA is represented by SEQ ID NO: 64, and the amino acid sequence deduced from this nucleotide sequence is represented by SEQ ID NO: 112.

The gene sequence of the extracellular domain of hPSMA was synthesized and inserted into the SpeI-BamHI site of the N5 (manufactured by IDEC) vector into which the FLAG-tag was inserted, and therefore a plasmid vector N5/FLAG-hPSMA for expressing the extracellular domain of hPSMA in which the FLAG-tag was added to the N-terminus side, was produced. The nucleotide sequence of FLAG-hPSMA is represented by SEQ ID NO: 92, and the amino acid sequence deduced from this nucleotide sequence is represented by SEQ ID NO: 113.

N5/FLAG-hPSMA was introduced into suspension cell 293 using the FreeStyle (trade mark) 293 Expression System (manufactured by Invitrogen) and cultured to express the protein in a transient expression system. The culture supernatant was collected 7 days after introduction of the vector, and filtered through a membrane filter (manufactured by Millipore Corporation.) having a pore size of 0.22 μm.

The culture supernatant was charged to ANTI-FLAG M2 Affinity Gel (column volume 2 mL) (manufactured by Sigma-Aldrich Co. LLC.) for purification of FLAG-fusion protein, washed with PB S (-), and eluted with an eluate [20 mM citric acid, 50 mM NaCl (pH 3.4)], and collected in a tube containing 1M sodium phosphate buffer solution (pH 7.0).

Subsequently, the solvent of the eluate was replaced with PBS by ultrafiltration using VIVASPIN (manufactured by Sartrius stealin), and then the solution was sterilized by filtration with a membrane filter having a pore size of 0.22 μm (Millex-GV, Merck Millipore Corporation.), and therefore a FLAG-fusion protein solution was obtained. The concentration of the purified FLAG-hPSMA-fusion protein was measured by the absorbance at 280 nm.

[Example 6] Production of Expression Plasmid Vector for Membrane hPSMA

An expression plasmid vector for the membrane hPSMA in which a signal sequence and a membrane binding site were added to the N-terminus side of hPSMA, was produced by the method described below using extension PCR.

Using the synthetic DNA of hPSMA gene (SEQ ID NO: 64) as a template, the PCR amplification was carried out for 25 cycles of reactions at 94°C for 30 seconds, 58°C for 30 seconds, and 68°C for 3 minutes using the primers represented by SEQ ID NO: 65 and SEQ ID NO: 66, and KOD polymerase.

Using the PCR product as a template, the PCR amplification was carried out for 25 cycles of reactions at 94°C for 30 seconds, 58°C for 30 seconds, and 68°C for 3 minutes using the primers represented by SEQ ID NO: 67 and SEQ ID NO: 66, and KOD polymerase.

Furthermore, using the PCR product as a template, the PCR amplification was carried out for 25 cycles of reactions at 94°C for 30 seconds, 58°C for 30 seconds, and 68°C for 3 minutes using the primers represented by SEQ ID NO: 68 and SEQ ID NO: 66, and KOD polymerase.

TOPO cloning was performed on the amplification product using pEF6/V5-His TOPO TA Expression Kit (manufactured by Invitrogen), and an expression plasmid vector pEF6-hPSMA full for the membrane hPSMA was obtained by selecting clones to which the hPSMA gene was properly inserted.

[Example 7] Preparation of Anti-hTRAILR2 Monoclonal Antibody, Anti-hPSMA Monoclonal Antibody and hTRAILR2-hPSMA Bispecific Antibody

The expression plasmid vectors for various antibodies produced in Examples 1 to 4 were gene-transferred into suspension cell 293 by FreeStyle (trade mark) 293 Expression System (manufactured by Invitrogen) to express the antibodies in the transient expression system. The culture supernatant was collected 7 days after the introduction of the vector, and filtered through a membrane filter (manufactured by Millipore Corporation.) having a pore size of 0.22 μm, and then the antibody was affinity-purified using Protein A resin (MabSelect, manufactured by GE Healthcare BioSciences). A phosphate buffer solution was used as a wash solution.

The antibody adsorbed on Protein A was eluted with an eluate [20 mM sodium citrate, 50 mM NaCl buffer solution (pH 3.4)], and collected in a tube containing 1M sodium phosphate buffer solution (pH 7.0). Next, the solvent of the eluate was replaced with PBS by the ultrafiltration using VIVASPIN (manufactured by Sartrius stealin).

Further, a monomer fraction was fractionated from the antibody solution by AKTA FPLC (manufactured by GE Healthcare) using Superdex High-performance Columns (manufactured by GE Healthcare), and was sterilized by the filtration with the membrane filter having a pore size of 0.22 μm (Millex-GV, manufactured by Millipore Corporation.). By the filtration sterilization with the membrane filter having a pore size of 0.22 μm (Millex-GV, manufactured by Millipore Corporation.) of AKTA system, a targeted purified antibody solution was obtained. The absorbance of the antibody solution at 280 nm was measured, and the concentration of the purified antibody was calculated by converting the concentration of 1 mg/mL to 1.40 Optimal density.

[Example 8] Preparation of L929 and PC3 expressing hPSMA

The expression plasmid vector for the membrane PSMA obtained in Example 6 was introduced into cells of mouse connective tissue-derived fibroblast L929 [American Type Culture Collection (ATCC) number: CCL-1], and human prostate cancer cell PC3 [ATCC number: CRL-1435] using the HilyMax (manufactured by Dojindo Laboratories).

The gene-transferred cells were selected using the antibiotic substance, Blasticidin (manufactured by Invitrogen), and then cloned by the limiting dilution method, and therefore L929 cells (hereinafter abbreviated as hPSMA/L929) expressing hPSMA on the cell surface and PC3 cells (hereinafter abbreviated as hPSMA/PC3) expressing hPSMA on the cell surface were obtained.

[Example 9] Evaluation on Affinity of hTRAILR2-hPSMA Bispecific Antibody to hTRAILR2 and hPSMA by Flow Cytometer

The affinity of various hTRAILR2-hPSMA bispecific antibodies obtained in Example 7 to hTRAILR2 and hPSMA was evaluated by the flow cytometry according to the following procedure.

The affinity to hTRAILR2/L929 cells (described in International Publication No. 2002/094880) and hPSMA/L929 cells obtained in Example 8 was analyzed by FACS using the various hTRAILR2-hPSMA bispecific antibodies obtained in Example 7, anti-hPSMA monoclonal antibody, or anti-hTRAILR2 monoclonal antibody.

The cells were suspended in a staining buffer [PBS containing 0.1% $NaN_3$ and 1% FBS] at a concentration of $1 \times 10^6$ cells/mL, and dispensed in a round-bottom 96-well plate (manufactured by Becton Dickinson) by 100 μL/well. After the centrifugation (2000 rpm, 4°C, 2 minutes), each antibody obtained in Example 7 was added to the pellet obtained by removing the supernatant, and suspended, and then left stand at ice temperature for 30 minutes.

After the further centrifugation (2000 rpm, 4°C, 2 minutes), the pellet obtained by removing the supernatant was washed twice with 200 μL/well of the staining buffer. Thereafter, 1 μg/mL of RPE fluorescently labeled mouse anti-human antibody (hinge region) clone 4E3 antibody (manufactured by Southern Bioblot) was added thereto by 50 μL/well and incubated at ice temperature for 30 minutes. After washing twice with the staining buffer, the cells were suspended in 200 μL/well of the staining buffer, and the fluorescence intensity of each cell was measured with the flow cytometer FACSCANTO II (manufactured by Becton Dickinson). The obtained results are illustrated in Fig. 2 and Fig. 3.

As the cells, hTRAILR2/L929 cells (described in International Publication No. 2002/094880) and hPSMA/L929 cells obtained in Example 8 were used. hTRAILR2/L929 cells were stained with anti-hPSMA monoclonal antibody 2A10 antibody or PI134 antibody, or hTRAILR2-hPSMA bispecific antibody E11-GL-PI134VH antibody, E11-CH1-PI134VH antibody, E11-CH1-PI101 VH antibody, E11-CH1-PN7VH antibody or E11-CH1-PN170VH antibody. The obtained results are illustrated in Fig. 3.

hPSMA/L929 cells were stained with anti-TRAILR2 monoclonal antibody E11 antibody, anti-hPSMA monoclonal antibody PI134 antibody, or hTRAILR2-hPSMA bispecific antibody E11-CH1-PI101VH antibody, E11-CH1-PN7VH antibody or E11-CH1-PN170VH antibody. The obtained results are illustrated in Fig. 2.

As illustrated in Fig. 3, anti-hTRAILR2 monoclonal antibody E11 antibody, and hTRIALR2-hPSMA bispecific antibody E11-CH1-PI101VH antibody, E11-CH1-PN7VH antibody and E11-CH1-PN170VH antibody were bound to hTRAILR2/L929 cells, but anti-hPSMA monoclonal antibody PI134 antibody was not bound thereto.

According to this, it became clear that hTRAILR2/L929 cells express hTRAILR2 and do not express hPSMA. In addition, this indicates that E11-CH1-PI101VH antibody, E11-CH1-PN7VH antibody and E11-CH1-PN170VH antibody which are hTRIALR2-hPSMA bispecific antibodies, have the affinity to hTRAILR2.

As illustrated in Fig. 2, 2A10 antibody and PI134 antibody, which are anti-hPSMA monoclonal antibodies, and E11-CH1-PI134VH antibody, E11-GL-PI134VH antibody, E11-CH1-PI101VH antibody, E11-CH1-PN7VH antibody and E11-CH1-PN170VH antibody, which are hTRAILR2-hPSMA bispecific antibodies were all bound to hPSMA/L929 cells.

This indicates that hPSMA/L929 cells express hPSMA, and E11-CH1-PI134VH antibody, E11-GL-PI134VH antibody, E11-CH1-PI101VH antibody, E11-CH1-PN7VH antibody and E11-CH1-PN170VH antibody which are hTRAILR2-hPSMA bispecific antibodies, have the affinity to hPSMA.

It was confirmed that the other hTRAILR2-hPSMA bispecific antibodies shown in Table 3 bind to both PC3 cells (expressing TRAILR2) and hPSMA/PC3 cells produced in Example 8 (expressing both hPSMA and TRAILR2) by the same method as above (no data disclosed). hPSMA/PC3 cells exhibited higher expression level of PSMA than TRAILR2, and the fluorescence intensity exhibited by hPSMA/PC3 cells with respect to every antibodies was higher than the fluorescence intensity exhibited by PC3 cells.

This indicates that the hTRAILR2-hPSMA bispecific antibodies of the present invention bind to any one of hTRAILR2 and hPSMA.

[Example 10] Analysis of Antigen Expression of PC3 Cell, hPSMA/PC3 Cell and LNCaP Clone FGC Cell by Flow Cytometer

Whether hTRAILR2 or hPSMA is expressed in PC3 cells, hPSMA/PC3 cells produced in Example 8 and human prostate cancer cell line LNCaP clone FGC (CRL-1740) was evaluated by the FACS method according to the following procedure.

PC3 cells, hPSMA/PC3 cells or LNCaP clone FGC cells were suspended in the staining buffer (PBS containing 0.1% $NaN_3$ and 1% FBS) at a concentration of $1 \times 10^6$ cells/mL, and dispensed in a round-bottom 96-well plate (manufactured by Becton Dickinson) by 100 μL/well. After the centrifugation (2000 rpm, 4°C, 2 minutes), each antibody obtained in Example 7 was added to the pellet obtained by removing the supernatant, and suspended, and then left stand at ice temperature for 30 minutes.

After the further centrifugation (2000 rpm, 4°C, 2 minutes), the pellet obtained by removing the supernatant was washed twice with 200 μL/well of the staining buffer. Thereafter, 1 μg/mL of RPE fluorescently labeled mouse anti-human antibody (hinge region) clone 4E3 antibody (manufactured by Southern Bioblot) was added thereto by 50 μL/well and incubated at ice temperature for 30 minutes. After washing twice with the staining buffer, the cells were suspended in 200 μL/well of the staining buffer, and the fluorescence intensity of each cell was measured with the flow cytometer FACSCANTO II (manufactured by Becton Dickinson).

As the antibody, anti-hTRAILR2 monoclonal antibody E11 antibody, anti-hPSMA monoclonal antibody 2A10 antibody or PI134 antibody, hTRAILR2-hPSMA bispecific antibody E11-CH1-PI101VH antibody or a known anti-DNP monoclonal antibody as a negative control was used. The obtained results are illustrated in Fig. 4(A), Fig. 4(B), and Fig. 5.

As illustrated in Fig. 4(A), anti-hTRAILR2 monoclonal antibody E11 antibody and bispecific antibody E11-CH1-PI101VH antibody were bound to PC3 cells, and anti-hPSMA monoclonal antibody PI134 antibody was not bound thereto.

As illustrated in Fig. 4(B), E11 antibody, E11-CH1-PI101VH antibody and PI134 antibody were bound to hPSMA/PC3 cells. Furthermore, it is illustrated that PI134 antibody and E11-CH1-PI101VH antibody have higher affinity than E11 antibody.

This result indicates that PC3 cells do not express hPSMA but express only hTRAILR2, and hPSMA/PC3 cells express both hTRAILR2 and hPSMA antigens.

As illustrated in Fig. 5, anti-hTRAILR2 monoclonal antibody E11 antibody and anti-hPSMA monoclonal antibody 2A10 antibody were bound to LNCaP clone FGC. This result indicates that LNCaP clone FGC expresses both hTRAILR2 and hPSMA antigens.

[Example 11] Proliferation Test on PC3 Cell and hPSMA/PC3 Cell Using hTRAILR2-hPSMA Bispecific Antibody

The proliferations of PC3 cells and the hPSMA/PC3 cells obtained in Example 8 were evaluated as follows using the various antibodies obtained in Example 7. According to this, it is possible to confirm that whether the antibody obtained in Example 7 inhibits the cell proliferation and/or induces the cell death of the above cells.

It was confirmed in Example 10 that the PC3 cells are cells expressing hTRAILR2 and not expressing hPSMA, and the hPSMA/PC3 cells are cells expressing hTRAILR2 and hPSMA.

As the antibody, anti-hTRAILR2 monoclonal antibody E11 antibody, anti-hTRAILR2 agonistic monoclonal antibody KMDA2 antibody, hTRAILR2-hPSMA bispecific antibody E11-CH1-PI134VH antibody, E11-CH1-PI101VH antibody, E11-CH1-PN7VH antibody, E11-CH1-PN170VH antibody, E11-GL-PI134VH, E11-CH1-PI105VH antibody, E11-CH1-PI108VH antibody, E11-CH1-PI115VH antibody, E11-CH1-PI118VH antibody, E11-CH1-PI127VH antibody, E11-CH1-PI143VH antibody, or E11-CH1-PL223VH antibody, or the known anti-DNP monoclonal antibody as a negative control antibody was used.

The cells of 2 to $10 \times 10^4$ cells/mL were seeded in a flat-bottom 96-well plate by 50 μL/well and cultured at 37°C under 5.0% carbon dioxide for 16 hours. The above antibodies adjusted to various concentrations were added thereto so that a total volume became 100 μl/well and cultured at 37°C under 5.0% carbon dioxide for 72 hours, and then 10 μL of Cell Counting Kit-8 (WST-8) (manufactured by Dojindo Laboratories) was added to each well. The evaluation was performed using 4 independent wells for each condition.

After further culture at 37°C under 5.0% carbon dioxide for 4 hours, the reaction was stopped by adding 0.1 M HCl, and then the absorbance at a wavelength of 450 nm (reference wavelength 630 nm) was measured with the microplate reader (1420 ARVO multilabel counter: manufactured by WALLAC).

The cell survival rate in each well was calculated by the following equation.

$$\text{Survival rate (\%)} = 100 \times (a-b) / (c-b)$$

(In the equation, a represents the absorbance of the test well, b represents the absorbance of the cell-free well, and c represents the absorbance of the well with no antibody added).

The obtained results are illustrated in Figs. 6 to 10. The results for PC3 cells are illustrated in Figs. 6 and 7, and the results for hPSMA/PC3 cells are illustrated in Figs. 8 to 10. As illustrated in Figs. 7 and 9, the anti-hTRAILR2 monoclonal antibody E11 antibody did not lower the survival rate of every cells, as same as the anti-DNP monoclonal antibody. On the other hand, as illustrated in Figs. 6 to 10, the anti-TRAILR2 agonistic monoclonal antibody KMDA 2 antibody lowered the survival rate of every cells than the anti-DNP monoclonal antibody.

As illustrated in Figs. 6 and 7, E11-CH1-PI134VH antibody, E11-CH1-PI101VH antibody, E11-CH1-PN7VH antibody and E11-CH1-PN170VH antibody which are hTRAILR2-hPSMA bispecific antibodies, did not lower the survival rate of PC3 cells, as same as the anti-DNP monoclonal antibody.

On the other hand, as illustrated in Figs. 8 to 10, E11-GL-PI134VH antibody, E11-CH1-PI134VH antibody, E11-CH1-PI101VH antibody, E11-CH1-PN7VH antibody, E11-CH1-PN170VH antibody, E11-CH1-PI105VH antibody, E11-CH1-PI108VH antibody, E11-CH1-PI115VH antibody, E11-CH1-PI118VH antibody, E11-CH1-PI127VH antibody, E11-CH1-PI143VH antibody, and E11-CH1-PL223VH antibody which are hTRAILR2-hPSMA bispecific antibodies, all lowered the survival rate of hPSMA/PC3 cells than the anti-DNP monoclonal antibody.

In addition, each anti-hPSMA monoclonal antibody 2A10 antibody, PI134 antibody, PN7 antibody did not lower the survival rate of the cells, as same as the anti-DNP monoclonal antibody (no date disclosed).

That is, the hTRAILR2-hPSMA bispecific antibodies of the present invention do not lower the survival rate of the cells expressing hTRAILR2 and not expressing hPSMA, and lower the survival rate of the cells expressing both hTRAILR2 and hPSMA.

Therefore, it is indicated that the hTRAILR2-hPSMA bispecific antibodies of the present invention do not inhibit the cell proliferation and/or induce the cell death when bound to only hTRAILR2, but induce the above reactions when bound to HTRAILR22 and hPSMA.

In addition, it is indicated that the anti-hTRAILR2 monoclonal antibody E11 antibody, which is the parent antibody of the hTRAILR2-hPSMA bispecific antibody of the present invention, does not lower the survival rate of both of the cells expressing hTRAILR2 and not expressing hPSMA, and the cells expressing both hTRAILR2 and hPSMA.

Therefore, it revealed that the anti-TRAILR2 monoclonal antibody used in the present invention inhibits the cell proliferation and/or induce the cell death only after allowing the monoclonal antibody to become a bispecific antibody that binds to the binding domain to PSMA.

[Example 12] Proliferation Test on Cancer Cell Using hTRAILR2-hPSMA Bispecific Antibody

The proliferation of LNCaP clone FGC cells (ATCC, CRL_1740) was evaluated as follows using each antibody obtained in Example 7. As the antibody, the anti-hTRAILR2 agonistic monoclonal antibody KMDA2 antibody, the anti-hTRAILR2 monoclonal antibody E11 antibody, the hTRAILR2-hPSMA bispecific antibody E11-CH1-PI101VH antibody, the E11-CH1-PN7VH antibody, or the E11-CH1-PN170VH antibody, or the known anti-DNP monoclonal antibody as a negative control antibody was used. It was confirmed in Example 10 that the LNCaP clone FGC cells express both hTRAILR2 and hPSMA.

Seeded were $4 \times 10^4$ cells/mL of the LNCaP clone FGC cells in a flat-bottom 96-well plate by 50 μL/well and cultured at 37°C under 5.0% carbon dioxide for 16 hours. The above antibodies adjusted to various concentrations were added thereto so that a total volume became 100 μl/well and cultured at 37°C under 5.0% carbon dioxide for 72 hours, and then 10 μL of Cell Counting Kit-8 (WST-8) (manufactured by Dojindo Laboratories) was added to each well.

The evaluation was performed using 4 independent wells for each condition. After further culture at 37°C under 5.0% carbon dioxide for 4 hours, the reaction was stopped by adding 0.1 M HCl, and then the absorbance at a wavelength of 450 nm (reference wavelength 630 nm) was measured with the microplate reader (1420 ARVO multilabel counter: manufactured by WALLAC).

The cell survival rate in each well was calculated by the following equation.

$$\text{Survival rate (\%)} = 100 \times (a-b) / (c-b)$$

(In the equation, a represents the absorbance of the test well, b represents the absorbance of the cell-free well, and c represents the absorbance of the well with no antibody added).

The obtained results are illustrated in Fig. 11. As illustrated in Fig. 11, the anti-hTRAILR2 monoclonal antibody E11 antibody did not lower the survival rate of the LNCaP clone FGC cells, as same as the anti-DNP monoclonal antibody. On the other hand, the anti-hTRAILR2 agonistic monoclonal antibody KMDA2 antibody, and E11-CH1-PI101VH antibody, E11-CH1-PN7VH antibody and E11-CH1-PN170VH antibody which are the hTRAILR2-hPSMA bispecific antibodies lowered the survival rate of the LNCaP clone FGC cells than the anti-DNP monoclonal antibody.

The results indicate that as long as the cells are cells expressing hTRAILR2 and hPSMA, the hTRAILR2-hPSMA bispecific antibody of the present invention inhibits the cell proliferation and/or induces the cell death of even a normal cell line that is not a forcibly expressed cell line.

[Example 13] Detection of Caspase by Flow Cytometer

Whether the hTRAILR2-hPSMA bispecific antibody E11-CH1-PI101VH antibody obtained in Example 7 induces the apoptosis in the PC3 cells and the hPSMA/PC3 cells was confirmed by detecting activated caspase using the flow cytometer and FAM-FLICA Caspase Detection Kit (manufactured by ImmunoChemistry) as described below. In Example 10, it was confirmed that the PC3 cells are cells expressing hTRAILR2 and not expressing hPSMA, and the hPSMA/PC3 cells are cells not expressing hTRAILR2 and hPSMA.

The PC3 cells or the hPSMA/PC3 cells were dispensed in the round-bottom 96-well plate (manufactured by Becton Dickinson) at $5 \times 10^4$ cells/well. After the centrifugation (2000 rpm, 4°C, 2 minutes), the negative control antibody (anti-DNP monoclonal antibody), E11-CH1-PI101VH antibody or TRAILR2 ligand TRAIL/Apo2 (manufactured by R&D Systems, Inc.) were diluted to make a final concentration of 1 μg/mL with a medium, and added to the pellet obtained by removing the supernatant, and then incubated at 37°C for 6 hours. After removing the supernatant by the centrifugation (2000 rpm, 4°C, 2 minutes), 50 μL/mL of FLICA attached to the kit diluted with the medium was added thereto, followed by the incubation at 37°C for 30 minutes.

Thereafter, the cells were washed 4 times with the apoptosis wash buffer attached to the kit, and suspended in 100 μL/well of the apoptosis wash buffer, and the fluorescence intensity of each cells was measured with the flow cytometer FACSCANTO II (manufactured by Becton Dickinson). The obtained results are illustrated in Figs. 12(A) and 12(B).

As illustrated in Fig. 12(A), in the PC3 cells, activated caspase was not detected when E11-CH1-PI101VH antibody was added, but was detected only when TRAIL was added. On the other hand, as illustrated in Fig. 12(B), in the hPSMA/PC3 cells, activated caspase was detected both when E11-CH1-PI101VH antibody was added and when TRAIL was added.

From this result, it was reveled that the E11-CH1-PI101VH antibody does not induce apoptosis in the cells expressing hTRAILR2 and not expressing hPSMA, and specifically induces apoptosis in the cells expressing both hTRAILR2 and hPSMA.

In addition, when the E11-CH1-PI101VH antibody was added to the hPSMA/PC3 cells, activated caspase was detected as same as when TRAILR2 ligand TRAIL was added.

Therefore, it was indicated that the hTRAILR2-hPSMA bispecific antibodies of the present invention do not induce apoptosis when bound to only hTRAILR2, but induce apoptosis when bound to hTRAILR2 and hPSMA. It was also indicated that the apoptosis occurs via TRAILR2.

[Example 14] Safety Evaluation on hTRAILR2-hPSMA Bispecific Antibody by Proliferation Test on Normal Hepatocyte

The proliferation of normal hepatocytes (Celsis IVT, IVT-M00995-P) was evaluated as follows using each antibody obtained in Example 7. According to this, it is possible to confirm that whether each antibody obtained in Example 7 induce at least one of the inhibition of the cell proliferation and the cell death of the normal hepatocytes.

As the antibody, the anti-hTRAILR2 agonistic monoclonal antibody KMDA2 antibody, the anti-hTRAILR2 monoclonal antibody E11 antibody, the hTRAILR2-hPSMA bispecific antibody E11-CH1-PI101VH antibody or the E11-CH1-PN7VH antibody, or the known anti-DNP monoclonal antibody as a negative control antibody was used. In addition, as a positive control, the TRAILR2 ligand TRAIL/Apo2 was also used instead of the antibody.

Seeded were $2 \times 10^4$ cells/mL of the normal hepatocytes cells in the flat-bottom 96-well plate by 50 μL/well and cultured at 37°C under 5.0% carbon dioxide for 16 hours. The above antibodies adjusted to various concentrations were added thereto so that a total volume became 100 μl/well and cultured at 37°C under 5.0% carbon dioxide for 72 hours, and then 10 μL of Cell Counting Kit-8 (WST-8) (manufactured by Dojindo Laboratories) was added to each well. The evaluation was performed using 4 independent wells for each condition.

After further culture at 37°C under 5.0% carbon dioxide for 4 hours, the reaction was stopped by adding 0.1 M HCl, and then the absorbance at a wavelength of 450 nm (reference wavelength 630 nm) was measured with the microplate reader (1420 ARVO multilabel counter: manufactured by WALLAC).

The cell survival rate in each well was calculated by the following equation.

$$\text{Survival rate (\%)} = 100 \times (a-b) / (c-b)$$

(In the equation, a represents the absorbance of the test well, b represents the absorbance of the cell-free well, and c represents the absorbance of the well with no antibody added).

The obtained results are illustrated in Figs. 13 and 14. As illustrated in Figs. 13 and 14, the anti-hTRAILR2 agonistic monoclonal antibody KMDA2 antibody and the TRILR2 ligand TRAIL/Apo2 lowered the survival rate of the normal hepatocytes than the anti-DNP monoclonal antibody, whereas the anti-hTRAILR2 monoclonal antibody E11 antibody, and E11-CH1-PI101VH antibody and E11-CH1-PN7VH antibody which are hTRAILR2-hPSMA bispecific antibodies did not lower the survival rate of the normal hepatocytes as same as the anti-DNP monoclonal antibody.

The results indicate that the hTRAILR2-hPSMA bispecific antibodies of the present invention do not inhibit the cell proliferation and/or induce the cell death of the normal hepatocytes.

Therefore, it was indicated that the hTRAILR2-hPSMA bispecific antibodies of the present invention significantly decrease toxicity to normal hepatocytes induced by the anti-TRAILR2 agonistic monoclonal antibody or TRAIL/Apo2.

[Example 15] Construction of Expression Plasmid Vector for hTRAILR2-hPSMA Bispecific Antibody or Antibody Fragment Thereof

In order to investigate the relationship between at least one of the cell proliferation inhibitory activity and the cell death-inducing activity of the hTRAILR2-hPSMA bispecific antibody measured in Example 11 and the structure of the bispecific antibody, E11-CH1-PN7VH F(ab')$_2$ and E11-CH1-PN7VH Fab which are hTRAILR2-hPSMA bispecific antibody fragment, and E11_PN7 Hetero antibody which is hTRAILR2-hPSMA heterobispecific antibody, each illustrated in Figs. 15(B) to 15(D), were produced, and at least one of the cell proliferation inhibitory activity and the cell death-inducing activity thereof was evaluated.

(1) Construction of Expression Plasmid Vector for hTRAILR2-hPSMA Bispecific Antibody Fragment E11-CH1-PN7VH F(ab')$_2$

E11-CH1-PN7VH F(ab')$_2$ is a bispecific antibody fragment in which CH2 and CH3 of the hTRAILR2-hPSMA bispecific antibody E11-CH1-PN7VH produced in Examples 4 and 7 are deleted [Fig. 15(B)].

An expression plasmid vector for hTRAILR2-hPSMA bispecific antibody fragment E11-CH1-PN7VH F(ab')$_2$ was produced by the following method. The heavy chain of the bispecific antibody fragment includes the amino acid sequences of the VH of the anti-hTRAILR2 monoclonal antibody E11 antibody, the linker (CH1 region of human IgG4), and the VH of the anti-hPSMA monoclonal antibody PN7 antibody in order from the N-terminus side, and includes FLAG-tag and His-tag as purification tags in the C-terminus. Furthermore, all of the light chains of the bispecific antibody fragment include the amino acid sequences of the VL of E11 antibody. E11-CH1-PN7VH F(ab')$_2$ forms a dimer through cysteine in the hinge region and divalently binds to each hTRAILR2 and hPSMA [Fig. 15(B)].

Using the nucleotide sequence of the constant region of human IgG4 as a template, the gene fragment of the linker portion was amplified by the PCR using the primer 33 (SEQ ID NO: 144), the primer 34 (SEQ ID NO: 145), and KOD plus DNA Polymerase (manufactured by Toyobo Co., Ltd.). In addition, using N5KG4PE [R409K]_E11VL_PN7 vector described in Example 3 as a template, the gene fragment of the VH region of PN7 antibody was amplified by the PCR using the primer 35 (SEQ ID NO: 146), the primer 36 (SEQ ID NO: 147), and KOD plus DNA Polymerase (manufactured by Toyobo Co., Ltd.). Furthermore, using the nucleotide sequence of the constant region of human IgG4 as a template, the gene fragment of the CH1 and the hinge region parts was amplified by the PCR using the primer 37 (SEQ ID NO: 148), the primer 38 (SEQ ID NO: 149), and KOD Polymerase.

The gene fragment amplified by the PCR reaction was linked to the N5KG1_E11 vector described in Example 2 which was cleaved by the restriction enzymes NheI and BamHI, and therefore an expression plasmid vector N5KG_E11VL_E11-CH1-PN7VH F(ab')$_2$ of the hTRAILR2-hPSMA bispecific antibody fragment E11-CH1-PN7VH F(ab')$_2$ was produced.

The nucleotide sequence excluding the signal sequence of the heavy chain of E11-CH1-PN7VH F(ab')$_2$ is represented by SEQ ID NO: 150, and the amino acid sequence deduced from this nucleotide sequence is represented by SEQ ID NO: 151.

(2) Construction of Expression Plasmid Vector for hTRAILR2-hPSMA Bispecific Antibody Fragment E11-CH1-PN7VH Fab

An expression plasmid vector for the hTRAILR2-hPSMA bispecific antibody fragment E11-CH1-PN7VH Fab was produced by the following method. E11-CH1-PN7VH Fab has a structure in which the hinge region is deleted from E11-CH1-PN7VH F(ab')$_2$ described in (1), and monovalently binds to each hTRAILR2 and hPSMA [Fig. 15(C)].

Using the nucleotide sequence of the constant region of human IgG4 as a template, the gene fragment of the linker portion was amplified by the PCR using the primer 33 (SEQ ID NO: 144), the primer 34 (SEQ ID NO: 145), and KOD plus DNA Polymerase (manufactured by Toyobo Co., Ltd.). In addition, using N5KG4PE [R409K]_E11VL_PN7 vector described in Example 3 as a template, the gene fragment of the VH region of the anti-hPSMA monoclonal antibody PN7 antibody was amplified by the PCR using the primer 35 (SEQ ID NO: 146), the primer 36 (SEQ ID NO: 147), and KOD plus DNA Polymerase (manufactured by Toyobo Co., Ltd.). Furthermore, using the nucleotide sequence of the constant region of human IgG4 as a template, the gene fragment of the CH1 part was amplified by the PCR using the primer 37 (SEQ ID NO: 148), the primer 39 (SEQ ID NO: 152), and KOD Polymerase.

The gene fragment amplified by the PCR reaction was linked to N5KG1_E11 vector which was cleaved by the restriction enzymes NheI and BamHI, and therefore an expression plasmid vector N5KG_E11VL_E11-CH1-PN7VH Fab of the hTRAILR2-hPSMA bispecific antibody fragment E11-CH1-PN7VH Fab was produced.

The nucleotide sequence excluding the signal sequence of the heavy chain of E11-CH1-PN7VH Fab is represented by SEQ ID NO: 153, and the amino acid sequence deduced from this nucleotide sequence is represented by SEQ ID NO: 154.

(3) Construction of Expression Plasmid Vector for hTRAILR2-hPSMA Heterobispecific Antibody E11_PN7 Hetero Antibody

An expression plasmid vector for hTRAILR2-hPSMA heterobispecific antibody E11_PN7 Hetero antibody was produced by the following method. This bispecific antibody is an antibody in which the heavy chain comprising the VH of the anti-hTRAILR2 monoclonal antibody E11 antibody and the heavy chain comprising the VH of the antibody hPSMA monoclonal antibody PN7 antibody form a heterodimer, and which monovalently binds to each hTRAILR2 and hPSMA.

One heavy chain of the bispecific antibody is a heavy chain formed of the amino acid sequence of the VH of E11 antibody and the amino acid sequence of the constant region of human IgG1 into which the amino acid mutation of K409R is introduced (hereinafter referred to as E11 K409R). The other heavy chain is a heavy chain formed of the amino acid sequence of the VH of PN7 antibody and the amino acid sequence of the constant region of human IgG1 into which the amino acid mutation of F405L is introduced (hereinafter referred to as PN7 F405L).

By the E11 K409R and PN7 F405L comprising the amino acid mutations of K409R and F405L, respectively, the formation of the heterodimer between these heavy chains is promoted [Nature Protocols, 9,2450-2463 (2014)] [Fig. 15(D)]. In addition, all VL of the antibody [E11 VL and PN7 VL of Fig. 15(D)] are the same and comprise the amino acid sequence represented by SEQ ID NO: 119.

The method for producing an E11_PN7 Hetero antibody will be described in Example 16. First, an anti-hTRAILR2 monoclonal antibody in which the heavy chain is E11 K409R and the light chain comprises the amino acid sequence of the VL of E11 antibody, and an anti-hPSMA monoclonal antibody in which the heavy chain is PN7 F405L and the light chain comprises the amino acid sequence of the VL of E11 antibody are produced. Next, by mixing these two types of antibodies to perform a reduction reaction, it is possible to produce E11_PN7 Hetero antibody in which E11 K409R and PN7 F405L each of which is the heavy chain of each antibody form heterodimer.

An expression plasmid vector N5KG1_E11VL_E11VH K409R for the anti-hTRAILR2 antibody in which the heavy chain is E11 K409R and the light chain comprises the amino acid sequence of the VL of E11 was produced by linking the gene fragment of the constant region of the heavy chain of the human IgG1 into which the amino acid mutation of K409R is introduced, with the expression plasmid vector N5KG1_E11 of the human IgG1-type anti-hTRAILR2 monoclonal antibody E11 antibody which was cleaved by the restriction enzymes NheI and BamHI.

An expression plasmid vector N5KG1_E11VL_PN7VH F405L for the anti-hPSMA monoclonal antibody in which the heavy chain is PN7 F405L and the light chain comprises the amino acid sequence of the VL of E11 antibody was produced by linking the gene fragment of the constant region of the heavy chain of the human IgG1 into which the amino acid mutation of F405L is introduced, with the expression plasmid vector N5KG4PE [R409K]_E11VL_PN7 of the mutated human IgG4-type anti-hPSMA monoclonal antibody PN7 antibody which was cleaved by the restriction enzymes NheI and BamHI.

The nucleotide sequence of E11 K409R excluding the signal sequence is represented by SEQ ID NO: 155, and the amino acid sequence deduced from this nucleotide sequence is represented by SEQ ID NO: 156. The nucleotide sequence excluding the signal sequence of PN7 F405L is represented by SEQ ID NO: 157, and the amino acid sequence deduced from this nucleotide sequence is represented by SEQ ID NO: 158.

[Example 16] Preparation of hTRAILR2-hPSMA Bispecific Antibody or Antibody Fragment Thereof

(1) Preparation of hTRAILR2-hPSMA Bispecific Antibody Fragment E11-CH1-PN7VH F(ab')$_2$ and E11-CH1-PN7VH Fab

N5KG_E11VL_E11-CH1-PN7VH F(ab')$_2$ or N5KG_E11VL_E11-CH1-PN7VH Fab, which are the expression plasmid vectors of the hTRAILR2-hPSMA bispecific antibody fragment produced in Example 15, were gene-transferred into suspension cell Expi293F (trade mark) using Expi293 (trade mark) Expression System (manufactured by Thermo Fisher Scientific Inc.), and therefore E11-CH1-PN7VH F(ab')$_2$ or E11-CH1-PN7VH Fab were transiently expressed.

The culture supernatant was collected 4 days after the gene transfer, and filtered through a membrane filter having a pore size of 0.22 μm (manufactured by Thermo Fisher Scientific Inc.). The culture supernatant was charged to ANTI-FLAG M2 Affinity Gel (column volume 2 mL) (manufactured by Sigma-Aldrich Co. LLC.) for purification of FLAG-fusion protein, washed with PBS (-), and eluted with an elution buffer [20 mM citric acid, 50 mM NaCl (pH 3.4)], and collected in a tube containing 200 mM sodium phosphate buffer solution (pH 7.0).

Subsequently, the eluate was concentrated using VIVASPIN (manufactured by Sartrius stealin), and the solvent was replaced with PBS using NAP (registered trademark) column (manufactured by GE Healthcare BioSciences). Furthermore, fractions corresponding to the molecular weight of E11-CH1-PN7VH F(ab')$_2$ or E11-CH1-PN7VH Fab were collected from the eluate using Superdex High-performance Columns (manufactured by GE Healthcare BioSciences) by AKTA Explore (manufactured by GE Healthcare), and sterilized by filtration with a membrane filter having a pore size of 0.22 μm (Millex-GV, Merck Millipore Corporation.), and therefore an antibody solution containing the hTRAILR2-hPSMA bispecific antibody fragment E11-CH1-PN7VH F(ab')$_2$ or E11-CH1-PN7VH Fab was obtained.

It was confirmed by the SDS-PAGE method that both the E11-CH1-PN7VH F(ab')$_2$ or E11-CH1-PN7VH Fab in the obtained antibody solution have 95% or more of purity. It was confirmed by the flow cytometry that both bispecific antibody fragments have the affinity to hTRAILR2 and hPSMA.

(2) Preparation of hTRAILR2-hPSMA Heterobispecific Antibody E11_PN7 Hetero Antibody

The expression plasmid vector N5KG1_E11VL_E11VH K409R produced in Example 15 was gene-transferred into suspension cell Expi293F (trade mark) using Expi293 (trade mark) Expression System (manufactured by Thermo Fisher Scientific Inc.), and therefore the anti-hTRAILR2 monoclonal antibody in which the heavy chain is E11 K409R and the light chain comprises the amino acid sequence of the VL of E11 antibody, was transiently expressed.

The expression plasmid vector N5KG1_E11VL_PN7VH F405L produced in Example 15 was gene-transferred into suspension cell Expi293F (trade mark) using the same method, and therefore the anti-hPSMA monoclonal antibody in which the heavy chain is PN7 F405L and the light chain comprises the amino acid sequence of the VL of E11 antibody, was transiently expressed.

For each gene-transferred cells, the culture supernatant was collected 4 days after the gene transfer, and filtered through a membrane filter having a pore size of 0.22 μm (manufactured by Thermo Fisher Scientific Inc.), and then the antibody was affinity-purified using Protein A resin (MabSelect, manufactured by GE Healthcare BioSciences). A phosphate buffer solution was used as a wash solution.

The antibody adsorbed on Protein A was eluted with an elution buffer [20 mM sodium citrate, 50 mM NaCl buffer solution (pH 3.4)], and collected in a tube containing 200M sodium phosphate buffer solution (pH 7.0). Next, the eluate was concentrated using VIVASPIN (manufactured by Sartrius stealin), and the solvent was replaced with PBS using NAP (trademark) column (manufactured by GE Healthcare BioSciences).

Therefore, an antibody solution containing the anti-hTRAILR2 monoclonal antibody in which the heavy chain is E11 K409R and the light chain comprises the amino acid sequence of the VL of E11 antibody, or the anti-hPSMA monoclonal antibody in which the heavy chain is PN7 F405L and the light chain comprises the amino acid sequence of the VL of E11 antibody, was obtained.

Next, the hTRAILR2-hPSMA heterobispecific antibody hE11_PN7 Hetero antibody was produced by performing the following reactions according to Nature Protocols, 9, 2450-2463 (2014) using the above two types of the antibody solutions.

The above anti-hTRAILR2 monoclonal antibody solution and the anti-hPSMA monoclonal antibody solution were mixed and the reduction reaction was performed for 90 minutes using 75 mM 2-MEA, PBS by mixing. The obtained reaction solution was replaced with PBS using NAP (trademark) column (manufactured by GE Healthcare BioSciences), followed by the incubation at 4°C for 16 hours. Therefore, E11 K409R and PN7 F405L each of which is the heavy chain of each antibody form a heterodimer.

Furthermore, fractions corresponding to E11_PN7 Hetero were collected from the reaction solution using Superdex High-performance Columns (manufactured by GE Healthcare BioSciences) and AKTA Explore (manufactured by GE Healthcare), and sterilized by filtration with the membrane filter having a pore size of 0.22 μm (Millex-GV, Merck Millipore Corporation.), and therefore an antibody solution containing the hTRAILR2-hPSMA heterobispecific antibody E11_PN7 Hetero antibody was obtained.

It was confirmed by the SDS-PAGE method that the E11_PN7 Hetero antibody in the obtained antibody solution has 95% or more of purity. In addition, it was confirmed by the flow cytometry that the E11_PN7 Hetero antibody has the affinity to hTRAILR2 and hPSMA.

[Example 17] Proliferation Test on PC3 Cell and hPSMA/PC3 Cell Using hTRAILR2-hPSMA Bispecific Antibody

The proliferation of the PC3 cells and the hPSMA/PC3 cells obtained in Example 8 was evaluated as follows using the hTRAILR2-hPSMA bispecific antibody E11-CH1-PN7VH antibody produced in Example 7, the hTRAILR2-hPSMA bispecific antibody fragment E11-CH1-PN7VH F(ab')$_2$ and E11-CH1-PN7VH Fab produced in Example 16, and the hTRAILR2-hPSMA heterobispecific antibody E11_PN7 Hetero antibody.

According to this, it is possible to confirm that whether the antibodies inhibit the cell proliferation and/or induce the cell death of the PC3 cells and the hPSMA/PC3 cells. In addition, the known anti-DNP monoclonal antibody was used as a negative control.

It was confirmed in Example 10 that PC3 cells express hTRAILR2 and do not express hPSMA, and the hPSMA/PC3 cells express hTRAILR2 and hPSMA.

2 to 10 $\times$ $10^4$ cells/mL of the cells were seeded in a flat-bottom 96-well plate by 50 $\mu$L/well and cultured at 37°C under 5.0% carbon dioxide for 16 hours. The above antibodies adjusted to various concentrations were added thereto so that a total volume became 100 $\mu$l/well and cultured at 37°C under 5.0% carbon dioxide for 72 hours, and then 10 $\mu$L of Cell Counting Kit-8 (WST-8) (manufactured by Dojindo Laboratories) was added to each well. The evaluation was performed using 4 independent wells for each condition.

After further culture at 37°C under 5.0% carbon dioxide for 4 hours, the reaction was stopped by adding 0.1 M HCl, and then the absorbance at a wavelength of 450 nm (reference wavelength 630 nm) was measured with the microplate reader (1420 ARVO multilabel counter: manufactured by WALLAC). The obtained results are illustrated in Figs. 16 and 17. The antibody concentration is given in the molar concentration. The obtained absorbance values reflect the number of cells in each well.

The results for the PC3 cells are illustrated in Fig. 16, and the results for the hPSMA/PC3 cells are illustrated in Fig. 17. As illustrated in Figs. 16 and 17, every cells was slightly decreased in the wells to which 10 nM of the anti-hTRAILR2 agonistic monoclonal antibody KMDA2 antibody was added compared to anti-DNP monoclonal antibody. The number of the PC3 cells was equivalent in all the wells to which other antibodies or antibody fragments and the anti-DNP monoclonal antibody were added.

The number of the hPSMA/PC3 cells was decreased in the wells to which E11-CH1-PN7VH antibody or E11-CH1-PN7VH F(ab')$_2$ was added than in the wells to which the KMDA2 antibody was added. On the other hand, the number of cells in the wells to which E11-CH1-PN7VH Fab and E11_PN7 Hetero antibody were added, was the same as in the wells to which the anti-DNP monoclonal antibody was added.

Therefore, it is revealed that E11-CH1-PN7VH antibody and E11-CH1-PN7VH F(ab')$_2$ do not inhibit the cell proliferation and/or induce the cell death of the cells expressing hTRAILR2 and not expressing hPSMA, and inhibit the cell proliferation and/or induce the cell death of the cells expressing hTRAILR2 and hPSMA. On the other hand, it is revealed that E11-CH1-PN7VH Fab and E11_PN7 Hetero antibody do not induce the above reaction even of the cells expressing hTRAILR2 and hPSMA.

E11-CH1-PN7VH antibody, E11-CH1-PN7VH F(ab')$_2$, E11-CH1-PN7VH Fab and E11 PN7 Hetero antibody were all produced using the variable region of the same anti-hTRAILR2 monoclonal antibody E11 antibody and anti-hPSMA monoclonal antibody PN7 antibody, but in terms of the structure of each antibody or the antibody fragment, E11-CH1-PN7VH antibody and E11-CH1-PN7VH F(ab')$_2$ divalently bind to each hTRAILR2 and hPSMA, and E11-CH1-PN7VH Fab and E11_PN7 Hetero antibody monovalently bind to each hTRAILR2 and hPSMA.

From the above, it was indicated that the hTRAILR2-hPSMA bispecific antibodies or the antibody fragments thereof of the present invention inhibit the cell proliferation and/or induce the cell death when divalently bound to each hTRAILR2 and hPSMA, and do not generate the above reaction when monovalently bound to each antigen.

Industrial Applicability

According to the present invention, it is possible to provide a bispecific antibody or an antibody fragment thereof comprising an antigen binding domain that binds to TRAILR2, and an antigen binding domain that binds to PSMA; a nucleic acid comprising a nucleotide sequence that encodes the antibody or the antibody fragment; a recombinant vector including the nucleic acid; a transformant comprising the recombinant vector; a method for producing the bispecific antibody or the antibody fragment by using the transformed cells; and a reagent for detection or measurement, a diagnostic agent, and a therapeutic agent, each of which includes the bispecific antibody or the antibody fragment.

The invention has been described in detail using the specific aspects, but it is obvious for those skilled in the art that various changes and modifications can be made without departing from the spirit and scope of the invention. The present application is based on U.S. provisional application (62/101042) filed on January 8, 2015, which is incorporated by reference in its entirety.

Sequence Listing Free Text

Definition of SEQ ID NO: 1-artificial sequence: Nucleotide sequence of VH of KMDA2
Definition of SEQ ID NO: 2-artificial sequence: Nucleotide sequence of VL of KMDA2
Definition of SEQ ID NO: 3-artificial sequence: Nucleotide sequence of primer 1

Definition of SEQ ID NO: 4-artificial sequence: Nucleotide sequence of Primer 2
Definition of SEQ ID NO: 5-artificial sequence: Nucleotide sequence of primer 3
Definition of SEQ ID NO: 6-artificial sequence: Nucleotide sequence of primer 4
Definition of SEQ ID NO: 7-artificial sequence: Amino acid sequence of VH of KMDA2
Definition of SEQ ID NO: 8-artificial sequence: Amino acid sequence of VL of KMDA2
Definition of SEQ ID NO: 9-artificial sequence: Nucleotide sequence of VH of E11
Definition of SEQ ID NO: 10-artificial sequence: Nucleotide sequence of VL of E11
Definition of SEQ ID NO: 11-artificial sequence: Amino acid sequence of GL linker
Definition of SEQ ID NO: 12-artificial sequence: Amino acid sequence of ML linker
Definition of SEQ ID NO: 13-artificial sequence: Nucleotide sequence of VH of PI134
Definition of SEQ ID NO: 14-artificial sequence: Nucleotide sequence of VH of PI101
Definition of SEQ ID NO: 15-artificial sequence: Nucleotide sequence of VH of PN7
Definition of SEQ ID NO: 16-artificial sequence: Nucleotide sequence of VH of PI105
Definition of SEQ ID NO: 17-artificial sequence: Nucleotide sequence of VH of PI108
Definition of SEQ ID NO: 18-artificial sequence: Nucleotide sequence of VH of PI115
Definition of SEQ ID NO: 19-artificial sequence: Nucleotide sequence of VH of PI118
Definition of SEQ ID NO: 20-artificial sequence: Nucleotide sequence of VH of PI127
Definition of SEQ ID NO: 21-artificial sequence: Nucleotide sequence of VH of PI143
Definition of SEQ ID NO: 22-artificial sequence: Nucleotide sequence of VH of PL223
Definition of SEQ ID NO: 23-artificial sequence: Nucleotide sequence of VH of 2A10
Definition of SEQ ID NO: 24-artificial sequence: Amino acid sequence of VH of 2A10
Definition of SEQ ID NO: 25-artificial sequence: Nucleotide sequence of VL of 2A10
Definition of SEQ ID NO: 26-artificial sequence: Amino acid sequence of VL of 2A10
Definition of SEQ ID NO: 27-artificial sequence: Nucleotide sequence of primer 5
Definition of SEQ ID NO: 28-artificial sequence: Nucleotide sequence of primer 6
Definition of SEQ ID NO: 29-artificial sequence: Nucleotide sequence of primer 7
Definition of SEQ ID NO: 30-artificial sequence: Nucleotide sequence of primer 8
Definition of SEQ ID NO: 31-artificial sequence: Nucleotide sequence of primer 9
Definition of SEQ ID NO: 32-artificial sequence: Nucleotide sequence of primer 10
Definition of SEQ ID NO: 33-artificial sequence: Nucleotide sequence of primer 11
Definition of SEQ ID NO: 34-artificial sequence: Nucleotide sequence of E11_VH-CH1-PI134_VH
Definition of SEQ ID NO: 35-artificial sequence: Nucleotide sequence of primer 12
Definition of SEQ ID NO: 36-artificial sequence: Nucleotide sequence of E11_VH-CH1-PI101_VH
Definition of SEQ ID NO: 37-artificial sequence: Nucleotide sequence of primer 13
Definition of SEQ ID NO: 38-artificial sequence: Nucleotide sequence of primer 14
Definition of SEQ ID NO: 39-artificial sequence: Nucleotide sequence of E11_VH-CH1-PI105_VH
Definition of SEQ ID NO: 40-artificial sequence: Nucleotide sequence of E11_VH-CH1-PI108_VH
Definition of SEQ ID NO: 41-artificial sequence: Nucleotide sequence of E11_VH-CH1-PI115_VH
Definition of SEQ ID NO: 42-artificial sequence: Nucleotide sequence of primer 15
Definition of SEQ ID NO: 43-artificial sequence: Nucleotide sequence of E11_VH-CH1-PI118_VH
Definition of SEQ ID NO: 44-artificial sequence: Nucleotide sequence of primer 16
Definition of SEQ ID NO: 45-artificial sequence: Nucleotide sequence of E11_VH-CH1-PI127_VH
Definition of SEQ ID NO: 46-artificial sequence: Nucleotide sequence of primer 17
Definition of SEQ ID NO: 47-artificial sequence: Nucleotide sequence of primer 18
Definition of SEQ ID NO: 48-artificial sequence: Nucleotide sequence of E11_VH-CH1-PI143_VH
Definition of SEQ ID NO: 49-artificial sequence: Nucleotide sequence of primer 19
Definition of SEQ ID NO: 50-artificial sequence: Nucleotide sequence of primer 20
Definition of SEQ ID NO: 51-artificial sequence: Nucleotide sequence of E11_VH-CH1-PL223_VH
Definition of SEQ ID NO: 52-artificial sequence: Nucleotide sequence of primer 21
Definition of SEQ ID NO: 53-artificial sequence: Nucleotide sequence of E11_VH-CH1-PN7_VH
Definition of SEQ ID NO: 54-artificial sequence: Nucleotide sequence of primer 22
Definition of SEQ ID NO: 55-artificial sequence: Nucleotide sequence of primer 23
Definition of SEQ ID NO: 56-artificial sequence: Nucleotide sequence of E11_VH-CH1-PN170_VH
Definition of SEQ ID NO: 57-artificial sequence: Nucleotide sequence of primer 24
Definition of SEQ ID NO: 58-artificial sequence: Nucleotide sequence of E11_VH-GL-PI134 VH
Definition of SEQ ID NO: 59-artificial sequence: Nucleotide sequence of primer 25
Definition of SEQ ID NO: 60-artificial sequence: Nucleotide sequence of primer 26
Definition of SEQ ID NO: 61-artificial sequence: Nucleotide sequence of primer 27

Definition of SEQ ID NO: 62-artificial sequence: Nucleotide sequence of primer 28
Definition of SEQ ID NO: 63-artificial sequence: Nucleotide sequence of E11_VH-ML-PI101_VH
Definition of SEQ ID NO: 65-artificial sequence: Nucleotide sequence of primer 29
Definition of SEQ ID NO: 66-artificial sequence: Nucleotide sequence of primer 30
Definition of SEQ ID NO: 67-artificial sequence: Nucleotide sequence of primer 31
Definition of SEQ ID NO: 68-artificial sequence: Nucleotide sequence of primer 32
Definition of SEQ ID NO: 69-artificial sequence: Amino acid sequence of VH of E11
Definition of SEQ ID NO: 70-artificial sequence: Amino acid sequence of HCDR1 of E11
Definition of SEQ ID NO: 71-artificial sequence: Amino acid sequence of HCDR2 of E11
Definition of SEQ ID NO: 72-artificial sequence: Amino acid sequence of HCDR3 of E11
Definition of SEQ ID NO: 73-artificial sequence: Amino acid sequence of VL of E11
Definition of SEQ ID NO: 74-artificial sequence: Amino acid sequence of LCDR1 of E11
Definition of SEQ ID NO: 75-artificial sequence: Amino acid sequence of LCDR2 of E11
Definition of SEQ ID NO: 76-artificial sequence: Amino acid sequence of LCDR3 of E11
Definition of SEQ ID NO: 77-artificial sequence: Amino acid sequence of VH of PI134
Definition of SEQ ID NO: 78-artificial sequence: Amino acid sequence of HCDR1 of PI134, PI143, PI105, PI127, PI108, PI115, PL223, PN7, PI101, and PI118
Definition of SEQ ID NO: 79-artificial sequence: Amino acid sequence of HCDR2 of PI134, PI143, PI105, PI127, PI108, PI115, and PL223
Definition of SEQ ID NO: 80-artificial sequence: Amino acid sequence of HCDR3 of PI134, PI143, PI105, PI127, PI108, PI115, PL223, PI101, and PI118
Definition of SEQ ID NO: 81-artificial sequence: Amino acid sequence of VH of PI101
Definition of SEQ ID NO: 82-artificial sequence: Amino acid sequence of HCDR2 of PI101, PN7, and PI118
Definition of SEQ ID NO: 83-artificial sequence: Amino acid sequence of VH of PN7
Definition of SEQ ID NO: 84-artificial sequence: Amino acid sequence of HCDR3 of PN7
Definition of SEQ ID NO: 85-artificial sequence: Amino acid sequence of VH of PI105
Definition of SEQ ID NO: 86-artificial sequence: Amino acid sequence of VH of PI108
Definition of SEQ ID NO: 87-artificial sequence: Amino acid sequence of VH of PI115
Definition of SEQ ID NO: 88-artificial sequence: Amino acid sequence of VH of PI118
Definition of SEQ ID NO: 89-artificial sequence: Amino acid sequence of VH of PI127
Definition of SEQ ID NO: 90-artificial sequence: Amino acid sequence of VH of PI143
Definition of SEQ ID NO: 91-artificial sequence: Amino acid sequence of VH of PL223
Definition of SEQ ID NO: 92-artificial sequence: Nucleotide sequence of N-terminus FLAG -fused PSMA extracellular domain
Definition of SEQ ID NO: 93-artificial sequence: Amino acid sequence of IgG4 CH1 linker
Definition of SEQ ID NO: 94-artificial sequence: Nucleotide sequence of VH of PN170
Definition of SEQ ID NO: 95-artificial sequence: Amino acid sequence of VH of PN170
Definition of SEQ ID NO: 96-artificial sequence: Amino acid sequence of HCDR1 of PN170
Definition of SEQ ID NO: 97-artificial sequence: Amino acid sequence of HCDR2 of PN170
Definition of SEQ ID NO: 98-artificial sequence: Amino acid sequence of HCDR3 of PN170
Definition of SEQ ID NO: 99-artificial sequence: Amino acid sequence of E11_VH-CH1-PI134_VH
Definition of SEQ ID NO: 100-artificial sequence: Amino acid sequence of E11_VH-CH1-PI101_VH
Definition of SEQ ID NO: 101-artificial sequence: Amino acid sequence of E11_VH-CH1-PI105_VH
Definition of SEQ ID NO: 102-artificial sequence: Amino acid sequence of E11_VH-CH1-PI108_VH
Definition of SEQ ID NO: 103-artificial sequence: Amino acid sequence of E11_VH-CH1-PI115_VH
Definition of SEQ ID NO: 104-artificial sequence: Amino acid sequence of E11_VH-CH1-PI118_VH
Definition of SEQ ID NO: 105-artificial sequence: Amino acid sequence of E11_VH-CH1-PI127_VH
Definition of SEQ ID NO: 106-artificial sequence: Amino acid sequence of E11_VH-CH1-PI143_VH
Definition of SEQ ID NO: 107-artificial sequence: Amino acid sequence of E11_VH-CH1-PL223_VH
Definition of SEQ ID NO: 108-artificial sequence: Amino acid sequence of E11_VH-CH1-PN7_VH
Definition of SEQ ID NO: 109-artificial sequence: Amino acid sequence of E11_VH-CH1-PN170_VH
Definition of SEQ ID NO: 110-artificial sequence: Amino acid sequence of E11_VH-GL-PI134_VH
Definition of SEQ ID NO: 111-artificial sequence: Amino acid sequence of E11_VH-ML-PI101_VH
Definition of SEQ ID NO: 113-artificial sequence: Amino acid sequence of N-terminus FLAG fused PSMA extracellular domain
Definition of SEQ ID NO: 114-artificial sequence: Amino acid sequence of VH of KMDA2 excluding signal sequence
Definition of SEQ ID NO: 115-artificial sequence: Amino acid sequence of VL of KMDA2 excluding signal sequence
Definition of SEQ ID NO: 116-artificial sequence: Amino acid sequence of VH of 2A10 excluding signal sequence

Definition of SEQ ID NO: 117-artificial sequence: Amino acid sequence of VL of 2A10 excluding signal sequence
Definition of SEQ ID NO: 118-artificial sequence: Amino acid sequence of VH of E11 excluding signal sequence
Definition of SEQ ID NO: 119-artificial sequence: Amino acid sequence of VL of E11 excluding signal sequence
Definition of SEQ ID NO: 120-artificial sequence: Amino acid sequence of VH of PI134 excluding signal sequence
Definition of SEQ ID NO: 121-artificial sequence: Amino acid sequence of VH of PI101 excluding signal sequence
Definition of SEQ ID NO: 122-artificial sequence: Amino acid sequence of VH of PN7 excluding signal sequence
Definition of SEQ ID NO: 123-artificial sequence: Amino acid sequence of VH of PI105 excluding signal sequence
Definition of SEQ ID NO: 124-artificial sequence: Amino acid sequence of VH of PI108 excluding signal sequence
Definition of SEQ ID NO: 125-artificial sequence: Amino acid sequence of VH of PI115 excluding signal sequence
Definition of SEQ ID NO: 126-artificial sequence: Amino acid sequence of VH of PI118 excluding signal sequence
Definition of SEQ ID NO: 127-artificial sequence: Amino acid sequence of VH of PI127 excluding signal sequence
Definition of SEQ ID NO: 128-artificial sequence: Amino acid sequence of VH of PI143 excluding signal sequence
Definition of SEQ ID NO: 129-artificial sequence: Amino acid sequence of VH of PL223 excluding signal sequence
Definition of SEQ ID NO: 130-artificial sequence: Amino acid sequence of VH of PN170 excluding signal sequence
Definition of SEQ ID NO: 131-artificial sequence: Amino acid sequence of E11_VH-CH1-PI134_VH excluding signal sequence
Definition of SEQ ID NO: 132-artificial sequence: Amino acid sequence of E11_VH-CH1-PI101_VH excluding signal sequence
Definition of SEQ ID NO: 133-artificial sequence: Amino acid sequence of E11_VH-CH1-PI105_VH excluding signal sequence
Definition of SEQ ID NO: 134-artificial sequence: Amino acid sequence of E11_VH-CH1-PI108_VH excluding signal sequence
Definition of SEQ ID NO: 135-artificial sequence: Amino acid sequence of E11_VH-CH1-PI115_VH excluding signal sequence
Definition of SEQ ID NO: 136-artificial sequence: Amino acid sequence of E11_VH-CH1-PI118_VH excluding signal sequence
Definition of SEQ ID NO: 137-artificial sequence: Amino acid sequence of E11_VH-CH1-PI127_VH excluding signal sequence
Definition of SEQ ID NO: 138-artificial sequence: Amino acid sequence of E11_VH-CH1-PI143_VH excluding signal sequence
Definition of SEQ ID NO: 139-artificial sequence: Amino acid sequence of E11_VH-CH1-PL223_VH excluding signal sequence
Definition of SEQ ID NO: 140-artificial sequence: Amino acid sequence of E11_VH-CH1-PN7_VH excluding signal sequence
Definition of SEQ ID NO: 141-artificial sequence: Amino acid sequence of E11_VH-CH1-PN170_VH excluding signal sequence
Definition of SEQ ID NO: 142-artificial sequence: Amino acid sequence of E11_VH-GL-PI134_VH excluding signal sequence
Definition of SEQ ID NO: 143-artificial sequence: Amino acid sequence of E11_VH-ML-PI101_VH excluding signal sequence
Definition of SEQ ID NO: 144-artificial sequence: primer 33
Definition of SEQ ID NO: 145-artificial sequence: Nucleotide sequence of primer 34
Definition of SEQ ID NO: 146-artificial sequence: Nucleotide sequence of primer 35
Definition of SEQ ID NO: 147-artificial sequence: Nucleotide sequence of primer 36
Definition of SEQ ID NO: 148-artificial sequence: Nucleotide sequence of primer 37
Definition of SEQ ID NO: 149-artificial sequence: Nucleotide sequence of primer 38
Definition of SEQ ID NO: 150-artificial sequence: Nucleotide sequence of E11-CH1-PN7VH F(ab')2
Definition of SEQ ID NO: 151-artificial sequence: Amino acid sequence of synthetic construct
Definition of SEQ ID NO: 152-artificial sequence: Nucleotide sequence of primer 39
Definition of SEQ ID NO: 153-artificial sequence: Nucleotide sequence of E11-CH1-PN7VH Fab
Definition of SEQ ID NO: 154-artificial sequence: Amino acid sequence of synthetic construct
Definition of SEQ ID NO: 155-artificial sequence: Nucleotide sequence of E11 K409R
Definition of SEQ ID NO: 156-artificial sequence: Amino acid sequence of synthetic construct
Definition of SEQ ID NO: 157-artificial sequence: Nucleotide sequence of PN7 F405L
Definition of SEQ ID NO: 158-artificial sequence: Amino acid sequence of synthetic construct

## Claims

1. A bispecific antibody or an antibody fragment thereof, comprising:

   an antigen binding domain that binds to a tumor necrosis factor (TNF)-related apoptosis inducing ligand receptor 2 (TRAILR2); and
   an antigen binding domain that binds to a prostate-specific membrane antigen (PSMA).

2. The bispecific antibody or the antibody fragment thereof according to Claim 1, which activates TRAILR2, only when binding to TRAILR2 and PSMA.

3. The bispecific antibody or the antibody fragment thereof according to Claim 1 or 2, which divalently binds to each TRAILR2 and PSMA.

4. The bispecific antibody or the antibody fragment thereof according to any one of Claims 1 to 3, wherein the antigen binding domain that binds to TRAILR2 is located closer to the N-terminus side than the antigen binding domain that binds to PSMA.

5. The bispecific antibody or the antibody fragment thereof according to any one of Claims 1 to 4, wherein the antigen binding domain that binds to TRAILR2 is a variable region (V region) of an antibody that binds to TRAILR2, and the antigen binding domain that binds to PSMA is a V region of an antibody that binds to PSMA.

6. The bispecific antibody or the antibody fragment thereof according to Claim 5, wherein amino acid sequences of complementarity determining regions (CDRs) 1 to 3 of a heavy chain variable region (hereinafter will be described as VH) of the antibody that binds to TRAILR2 comprise the amino acid sequences represented by SEQ ID NOs: 70 to 72, respectively, and amino acid sequences of CDRs 1 to 3 of a light chain variable region (hereinafter will be described as VL) of the antibody that binds to TRAILR2 comprise the amino acid sequences represented by SEQ ID NOs: 74 to 76, respectively.

7. The bispecific antibody or the antibody fragment thereof according to Claim 5 or 6, wherein an amino acid sequence of the VH of the antibody that binds to TRAILR2 comprise the amino acid sequence represented by SEQ ID NO: 118, and an amino acid sequence of the VL of the antibody that binds to TRAILR2 comprise the amino acid sequence represented by SEQ ID NO: 119.

8. The bispecific antibody or the antibody fragment thereof according to any one of Claims 5 to 7, wherein amino acid sequences of CDRs 1 to 3 of the VL of the antibody that binds to PSMA comprise the amino acid sequences represented by SEQ ID NOs: 74 to 76, respectively, and amino acid sequences of CDRs 1 to 3 of the VH of the antibody that binds to PSMA comprise any one of amino acid sequences selected from the group consisting of (a) to (d) below;

   (a) the amino acid sequences represented by SEQ ID NOs: 78, 82, and 84, respectively,
   (b) the amino acid sequences represented by SEQ ID NOs: 78, 79, and 80, respectively,
   (c) the amino acid sequences represented by SEQ ID NOs: 78, 82, and 80, respectively, and
   (d) the amino acid sequences represented by SEQ ID NOs: 96 to 98, respectively.

9. The bispecific antibody or the antibody fragment thereof according to any one of Claims 5 to 8, wherein an amino acid sequence of the VL of the antibody that binds to PSMA comprise the amino acid sequence represented by SEQ ID NO: 119, and an amino acid sequence of the VH of the antibody that binds to PSMA comprise any one of amino acid sequences selected from the groups consisting of (e) to (o) below;

   (e) the amino acid sequence represented by SEQ ID NO: 122,
   (f) the amino acid sequence represented by SEQ ID NO: 120,
   (g) the amino acid sequence represented by SEQ ID NO: 128,
   (h) the amino acid sequence represented by SEQ ID NO: 123,
   (i) the amino acid sequence represented by SEQ ID NO: 127,
   (j) the amino acid sequence represented by SEQ ID NO: 124,
   (k) the amino acid sequence represented by SEQ ID NO: 125,
   (1) the amino acid sequence represented by SEQ ID NO: 129,

(m) the amino acid sequence represented by SEQ ID NO: 121,
(n) the amino acid sequence represented by SEQ ID NO: 126, and
(o) the amino acid sequence represented by SEQ ID NO: 130.

**10.** The bispecific antibody or the antibody fragment thereof according to any one of Claims 5 to 9, which comprises a heavy chain comprising a polypeptide wherein the VH of the antibody that binds to TRAILR2 and the VH of the antibody that binds to PSMA are linked via a linker comprising an immunoglobulin domain or a domain fragment thereof.

**11.** The bispecific antibody or the antibody fragment thereof according to Claim 10, wherein the linker is a linker derived from the subclass of IgG4 or IgM.

**12.** The bispecific antibody or the antibody fragment thereof according to Claim 10 or 11, wherein an amino acid sequence of a polypeptide consisting of the VH of the antibody that binds to TRAILR2, the linker and the VH of the antibody that binds to PSMA is any one of amino acid sequences selected from the group consisting of (A) to (M) below;

(A) the amino acid sequence represented by SEQ ID NO: 131,
(B) the amino acid sequence represented by SEQ ID NO: 132,
(C) the amino acid sequence represented by SEQ ID NO: 133,
(D) the amino acid sequence represented by SEQ ID NO: 134,
(E) the amino acid sequence represented by SEQ ID NO: 135,
(F) the amino acid sequence represented by SEQ ID NO: 136,
(G) the amino acid sequence represented by SEQ ID NO: 137,
(H) the amino acid sequence represented by SEQ ID NO: 138,
(I) the amino acid sequence represented by SEQ ID NO: 139,
(J) the amino acid sequence represented by SEQ ID NO: 140,
(K) the amino acid sequence represented by SEQ ID NO: 141,
(L) the amino acid sequence represented by SEQ ID NO: 142, and
(M) the amino acid sequence represented by SEQ ID NO: 143.

**13.** A nucleic acid, comprising:

a nucleotide sequence that encodes the bispecific antibody or the antibody fragment thereof according to any one of Claims 1 to 12.

**14.** A recombinant vector, comprising:

the nucleic acid according to Claim 13.

**15.** A transformant, comprising:

the recombinant vector according to Claim 14.

**16.** A method for producing the bispecific antibody or the antibody fragment thereof according to any one of Claims 1 to 12, comprising:

culturing the transformant according to Claim 15 in a medium and collecting the bispecific antibody or the antibody fragment thereof from a culture supernatant.

**17.** A reagent for detecting or measuring at least one of TRAILR2 and PSMA, comprising:

the bispecific antibody or the antibody fragment thereof according to any one of Claims 1 to 12.

**18.** A diagnostic agent for a disease related to a TRAILR2- and PSMA-expressing cell, comprising:

the bispecific antibody or the antibody fragment thereof according to any one of Claims 1 to 12.

**19.** The diagnostic agent according to Claim 18,
wherein the disease related to a TRAILR2- and PSMA-expressing cell is a malignant tumor and a cancer.

**20.** A therapeutic agent for a disease related to a TRAILR2- and PSMA-expressing cell, comprising:

the bispecific antibody or the antibody fragment thereof according to any one of Claims 1 to 12 as an active ingredient.

**21.** The therapeutic agent according to Claim 20,
wherein the disease related to a TRAILR2- and PSMA-expressing cell is a malignant tumor and a cancer.

**22.** A method for detecting or measuring at least one of TRAILR2 and PSMA,
by using the bispecific antibody or the antibody fragment thereof according to any one of Claims 1 to 12.

**23.** A diagnostic method for a disease related to a TRAILR2- and PSMA-expressing cell, comprising:

detecting or measuring at least one of TRAILR2 and PSMA by using the bispecific antibody or the antibody fragment thereof according to any one of Claims 1 to 12.

**24.** The diagnostic method according to Claim 23,
wherein the disease related to a TRAILR2- and PSMA-expressing cell is a malignant tumor and a cancer.

**25.** A therapeutic method for a disease related to a TRAILR2- and PSMA-expressing cell,
by using the bispecific antibody or the antibody fragment thereof according to any one of Claims 1 to 12.

**26.** The therapeutic method according to Claim 25,
wherein the disease related to TRAILR2- and PSMA-expressing cell is a malignant tumor and a cancer.

**27.** Use of the bispecific antibody or the antibody fragment thereof according to any one of Claims 1 to 12, for the manufacture of a diagnostic agent for a disease related to a TRAILR2- and PSMA-expressing cell.

**28.** The use according to Claim 27,
wherein the disease related to a TRAILR2- and PSMA-expressing cell is a malignant tumor and a cancer.

**29.** Use of the bispecific antibody or the antibody fragment thereof according to any one of Claims 1 to 12, for the manufacture of a therapeutic agent for a disease related to a TRAILR2- and PSMA-expressing cell.

**30.** The use according to Claim 29,
wherein the disease related to a TRAILR2- and PSMA-expressing cell is a malignant tumor and a cancer.

[Fig. 1]

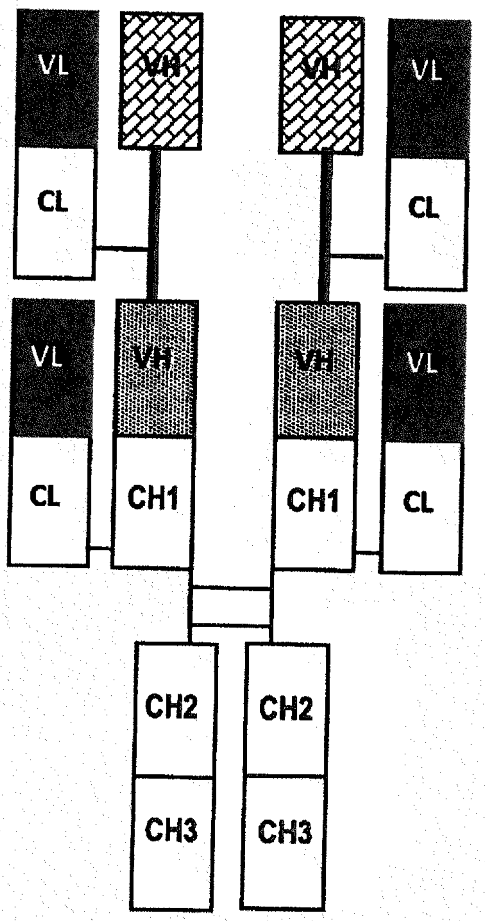

[Fig. 2]

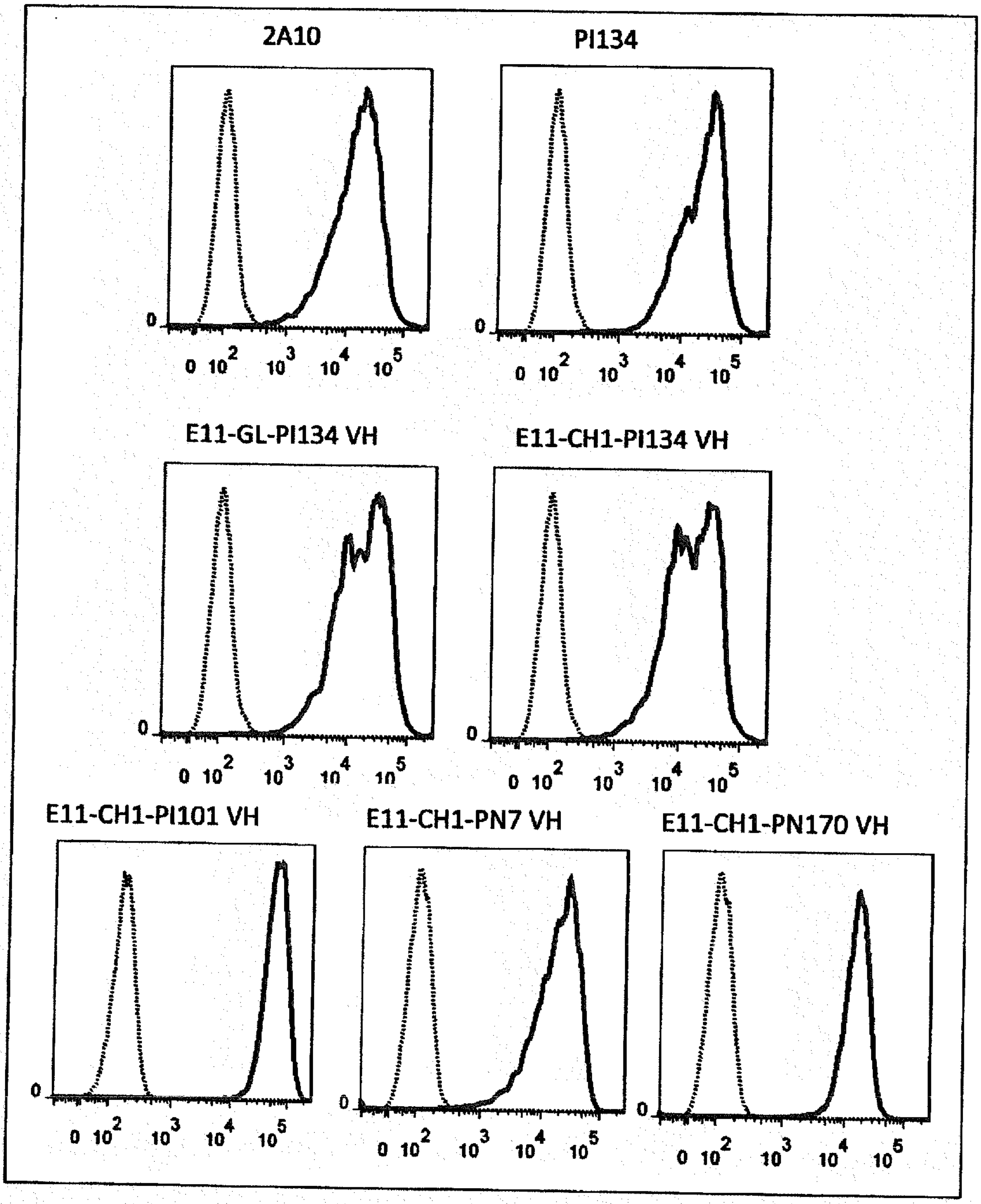
2A10
PI134
E11-GL-PI134 VH
E11-CH1-PI134 VH
E11-CH1-PI101 VH
E11-CH1-PN7 VH
E11-CH1-PN170 VH
0
0 10^2 10^3 10^4 10^5

[Fig. 3]

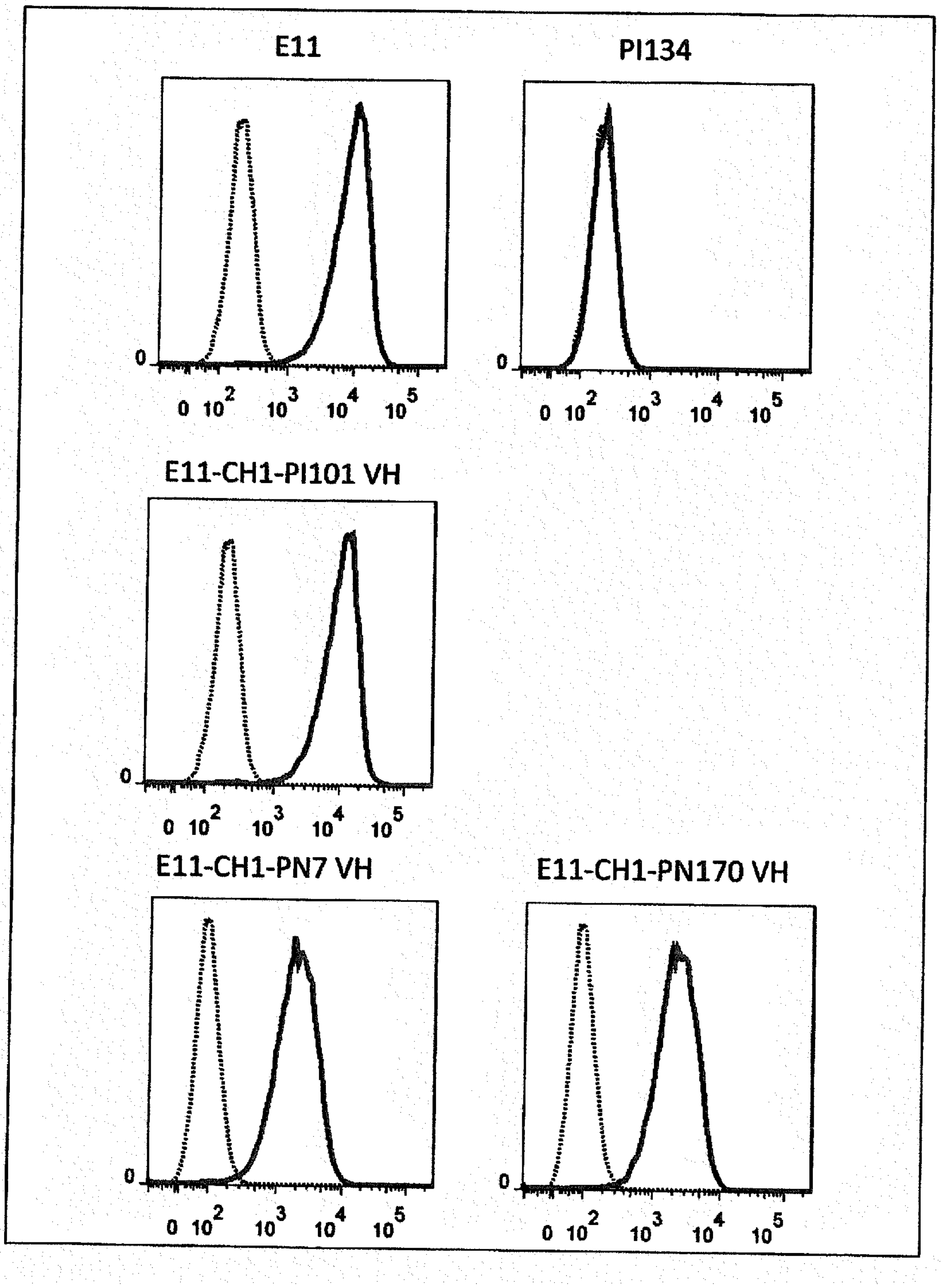
E11
PI134
E11-CH1-PI101 VH
E11-CH1-PN7 VH
E11-CH1-PN170 VH
0
0 10^2 10^3 10^4 10^5

[Fig. 4]

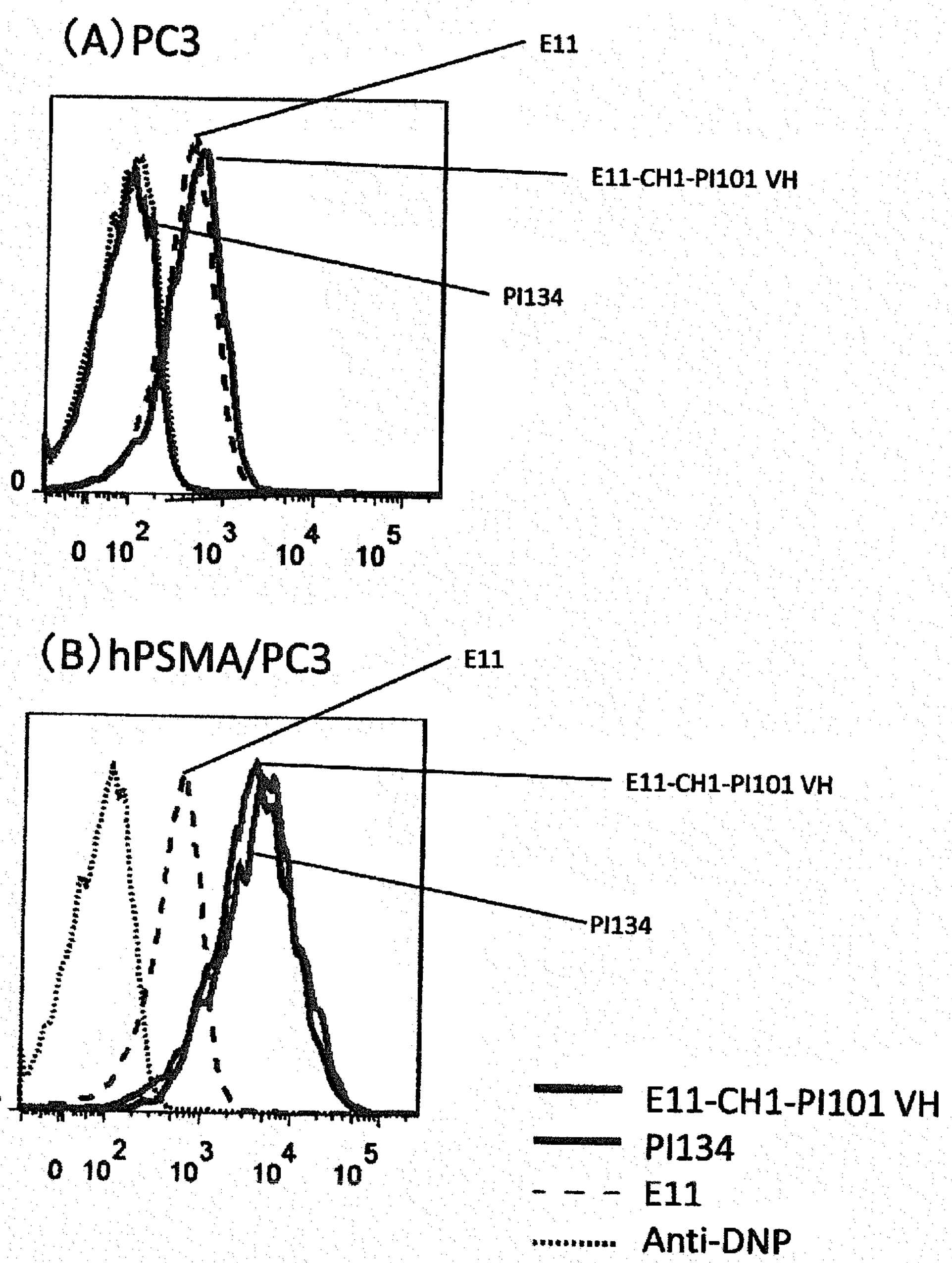
(A) PC3
E11
E11-CH1-PI101 VH
PI134
0
0 10^2 10^3 10^4 10^5
(B) hPSMA/PC3
E11
E11-CH1-PI101 VH
PI134
0
0 10^2 10^3 10^4 10^5
E11-CH1-PI101 VH
PI134
E11
Anti-DNP

[Fig. 5]

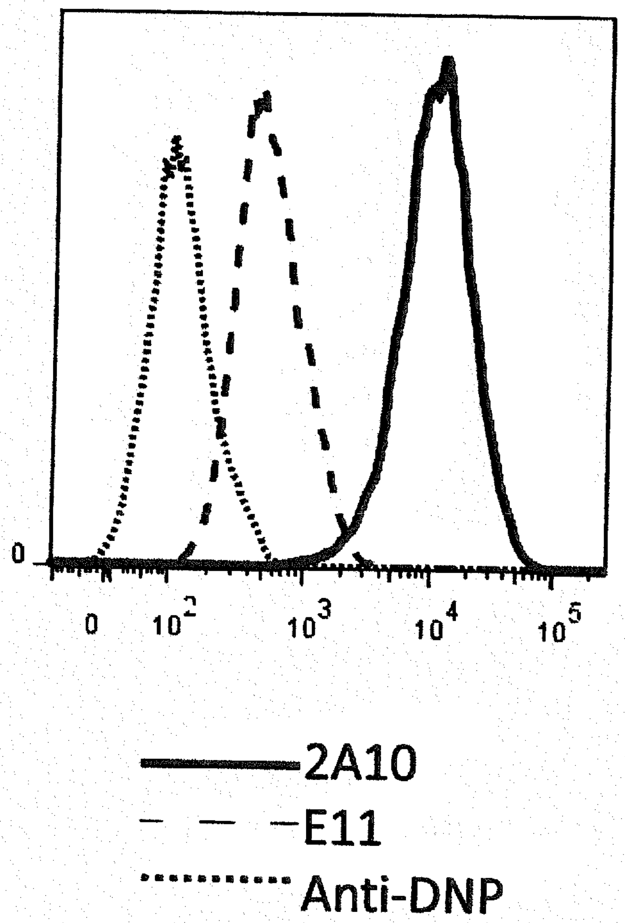

[Fig. 6]

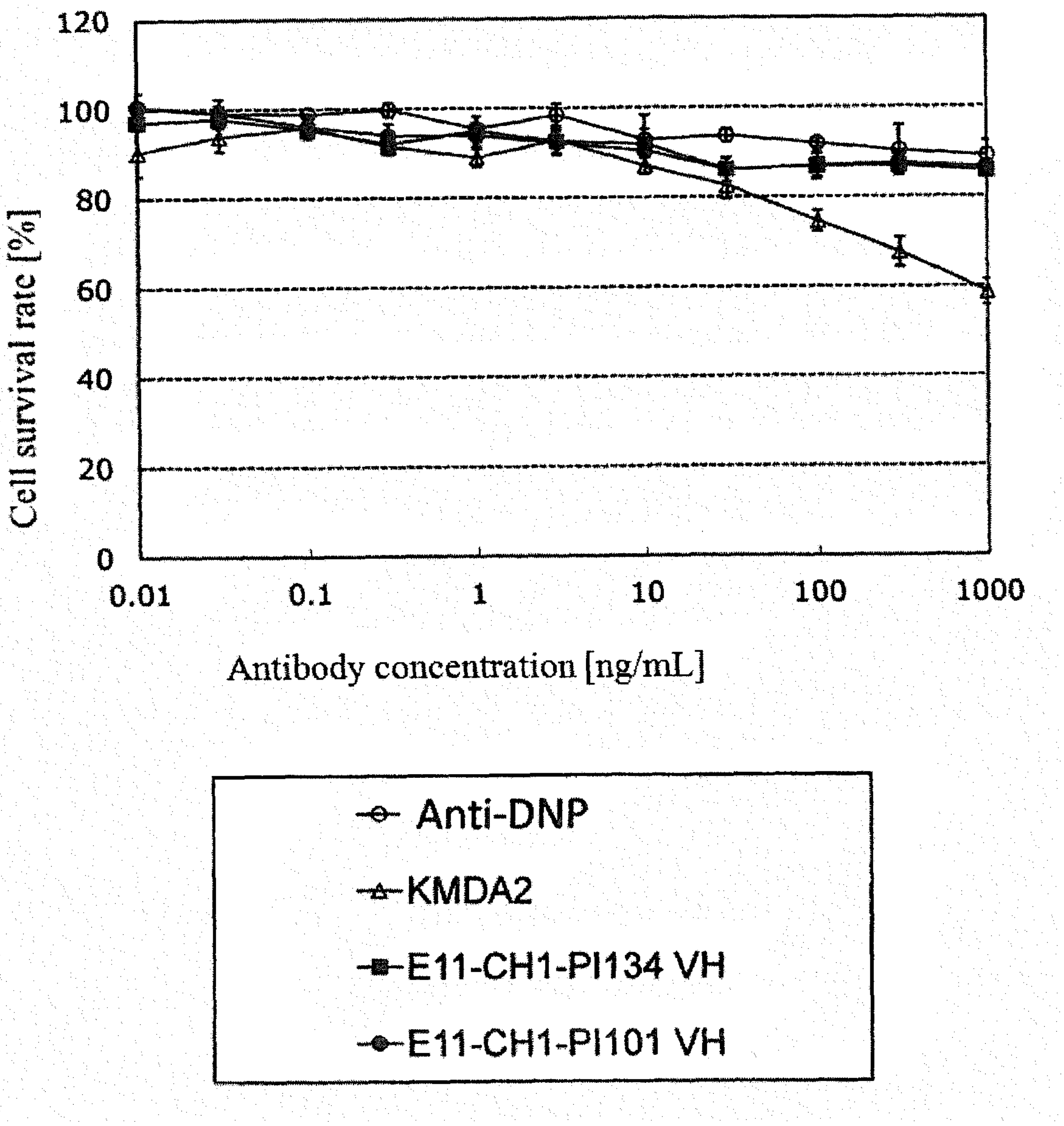
120
100
80
60
40
20
0
Cell survival rate [%]
0.01
0.1
1
10
100
1000
Antibody concentration [ng/mL]
Anti-DNP
KMDA2
E11-CH1-PI134 VH
E11-CH1-PI101 VH

[Fig. 7]

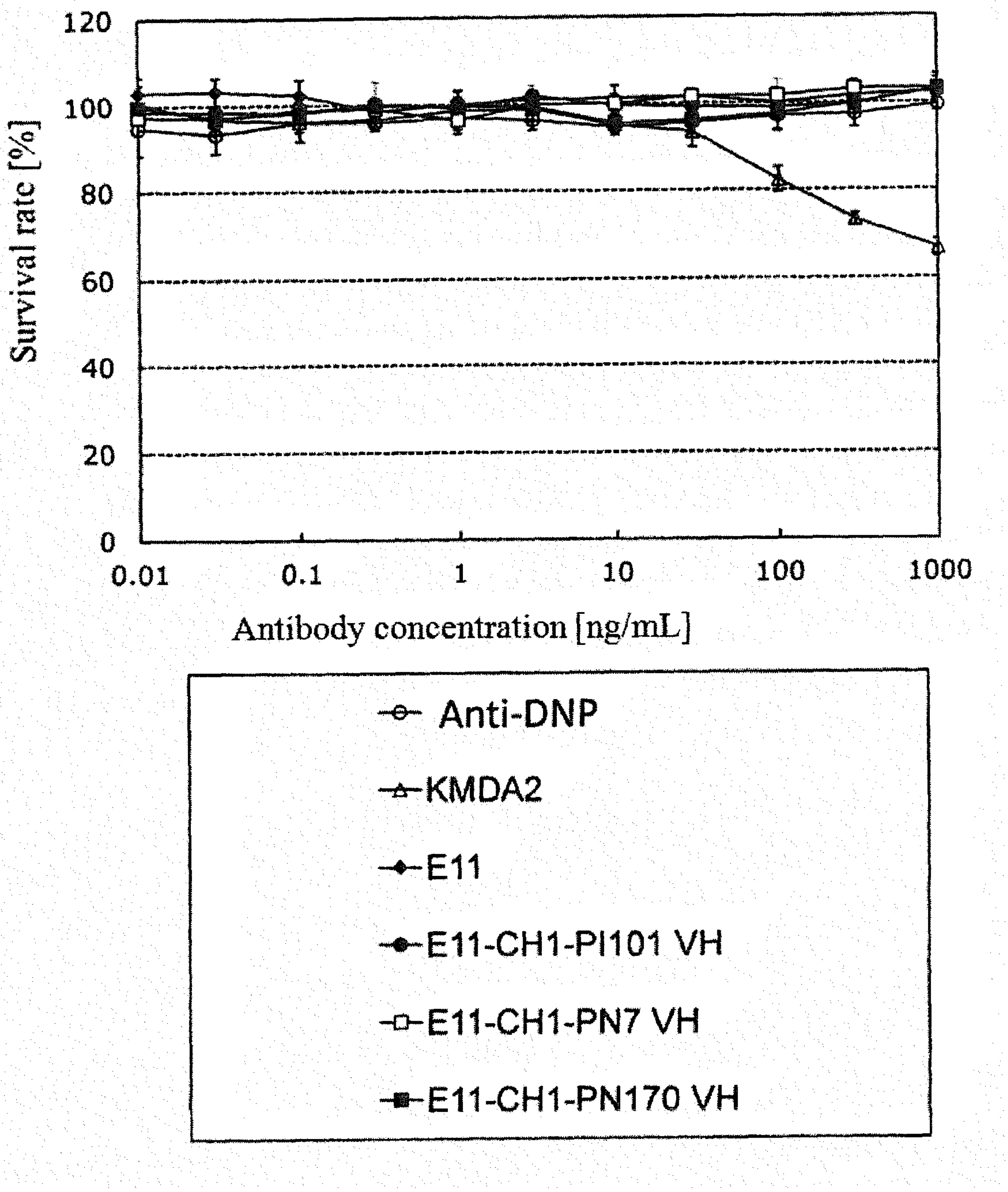
120
100
80
60
40
20
0
Survival rate [%]
0.01
0.1
1
10
100
1000
Antibody concentration [ng/mL]
Anti-DNP
KMDA2
E11
E11-CH1-PI101 VH
E11-CH1-PN7 VH
E11-CH1-PN170 VH

[Fig. 8]

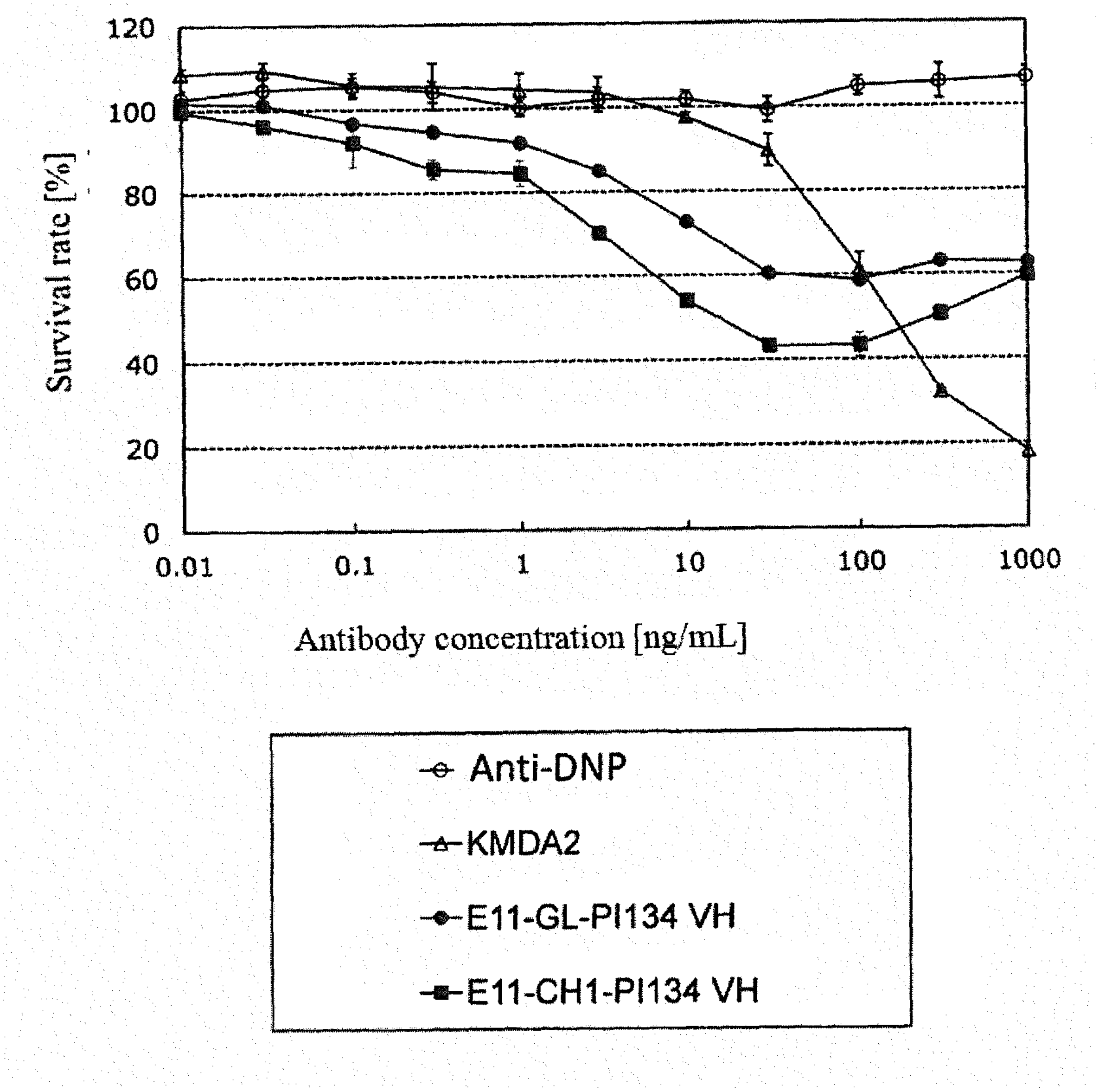
120
100
80
60
40
20
0
Survival rate [%]
0.01
0.1
1
10
100
1000
Antibody concentration [ng/mL]
Anti-DNP
KMDA2
E11-GL-PI134 VH
E11-CH1-PI134 VH

[Fig. 9]

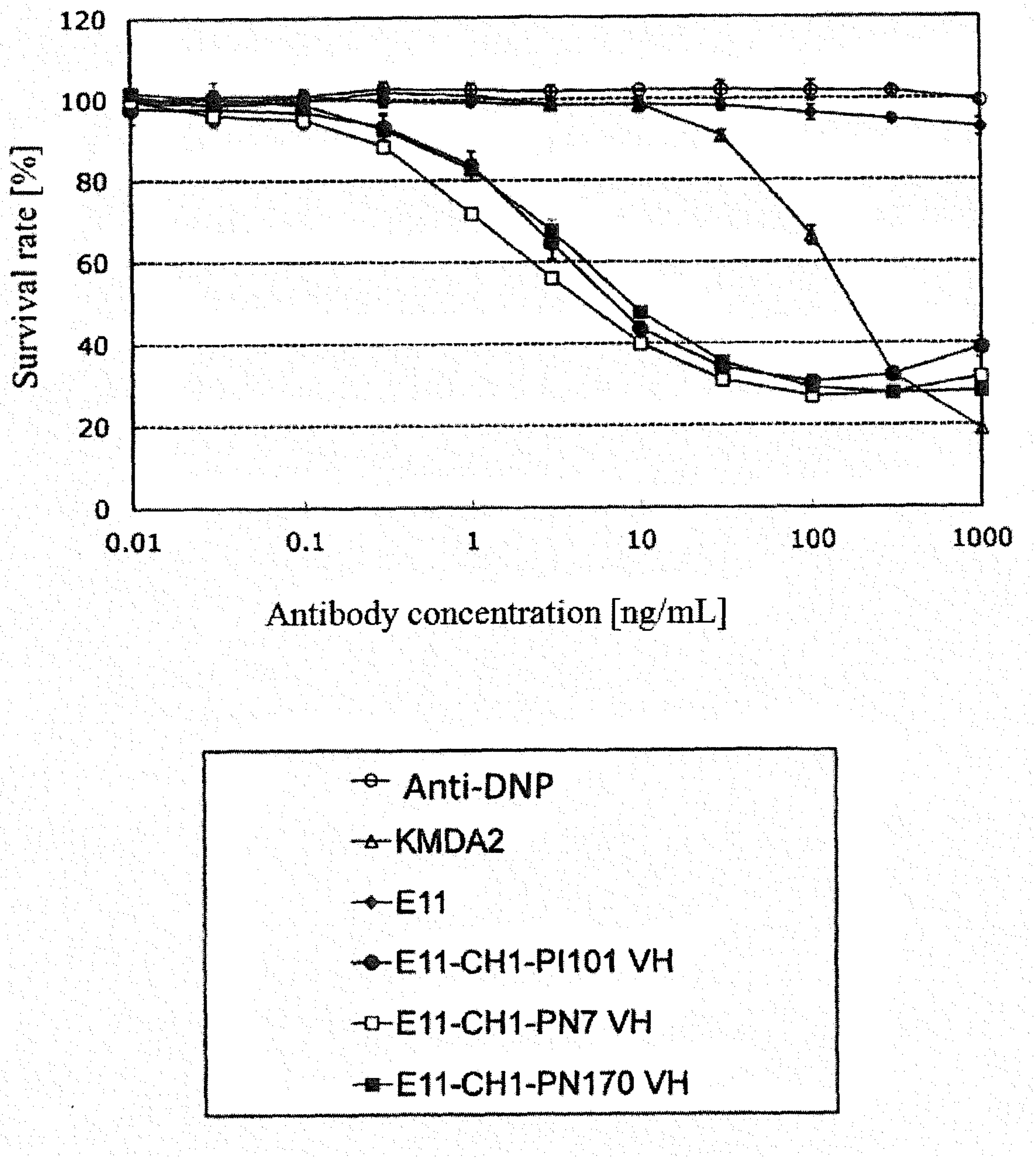
120
100
80
60
40
20
0
0.01
0.1
1
10
100
1000
Survival rate [%]
Antibody concentration [ng/mL]
Anti-DNP
KMDA2
E11
E11-CH1-PI101 VH
E11-CH1-PN7 VH
E11-CH1-PN170 VH

[Fig. 10]

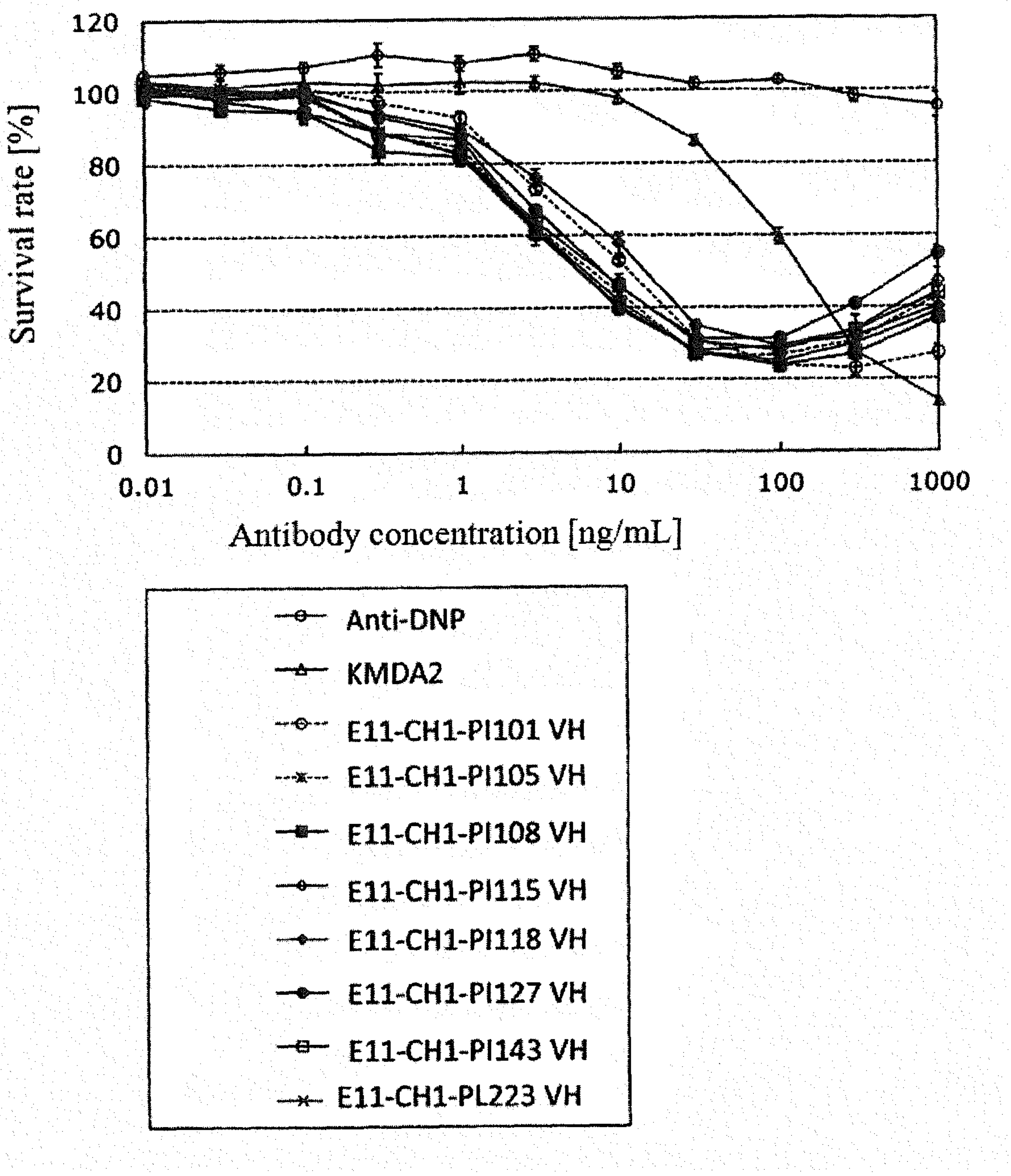
120
100
80
60
40
20
0
Survival rate [%]
0.01
0.1
1
10
100
1000
Antibody concentration [ng/mL]
Anti-DNP
KMDA2
E11-CH1-PI101 VH
E11-CH1-PI105 VH
E11-CH1-PI108 VH
E11-CH1-PI115 VH
E11-CH1-PI118 VH
E11-CH1-PI127 VH
E11-CH1-PI143 VH
E11-CH1-PL223 VH

[Fig. 11]

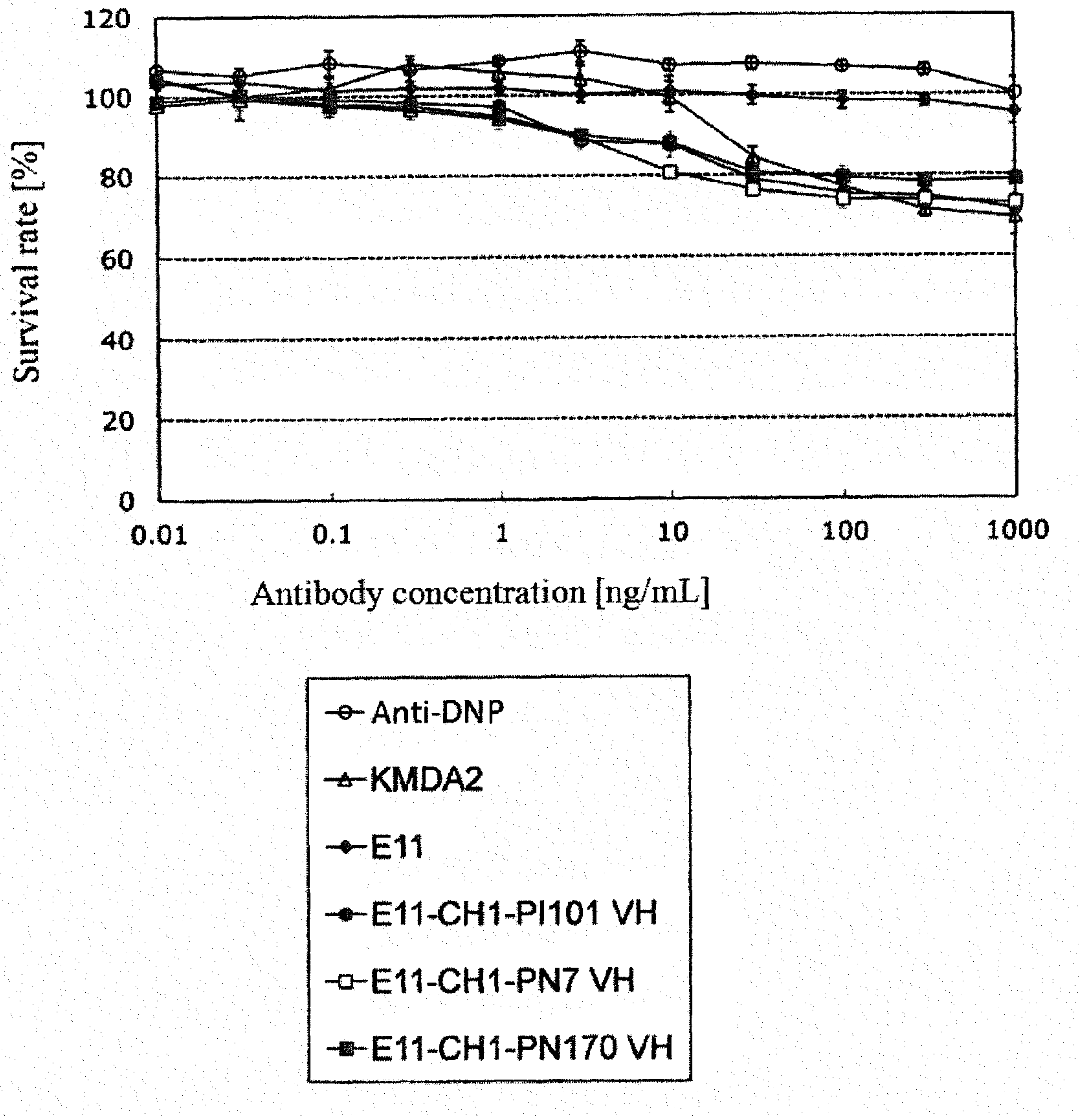
120
100
80
60
40
20
0
Survival rate [%]
0.01
0.1
1
10
100
1000
Antibody concentration [ng/mL]
Anti-DNP
KMDA2
E11
E11-CH1-PI101 VH
E11-CH1-PN7 VH
E11-CH1-PN170 VH

[Fig. 12]

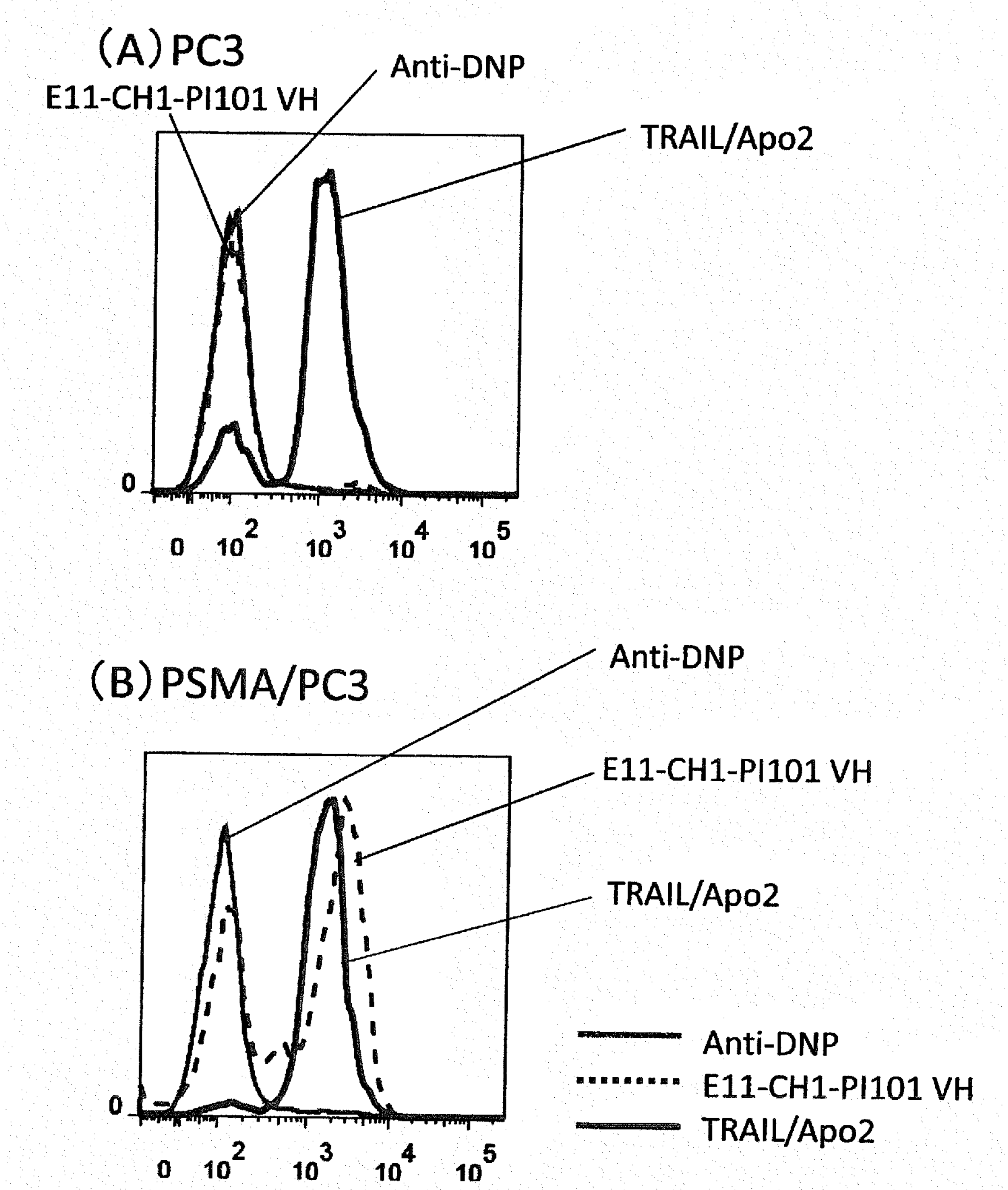
(A) PC3
E11-CH1-PI101 VH
Anti-DNP
TRAIL/Apo2
0
0
10^2
10^3
10^4
10^5
(B) PSMA/PC3
Anti-DNP
E11-CH1-PI101 VH
TRAIL/Apo2
Anti-DNP
E11-CH1-PI101 VH
TRAIL/Apo2
0
0
10^2
10^3
10^4
10^5

[Fig. 13]

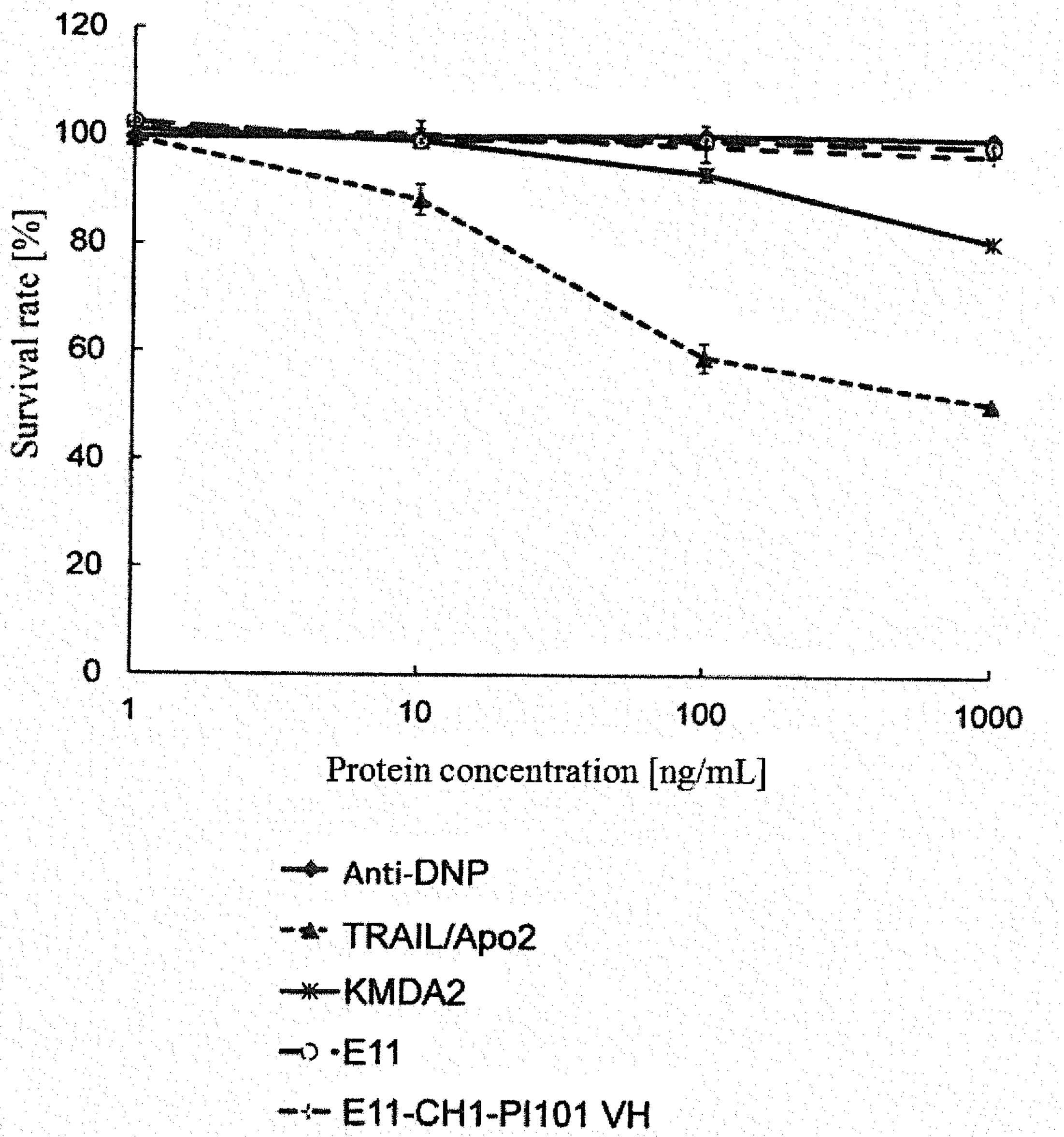
120
100
80
60
40
20
0
Survival rate [%]
1
10
100
1000
Protein concentration [ng/mL]
Anti-DNP
TRAIL/Apo2
KMDA2
E11
E11-CH1-PI101 VH

[Fig. 14]

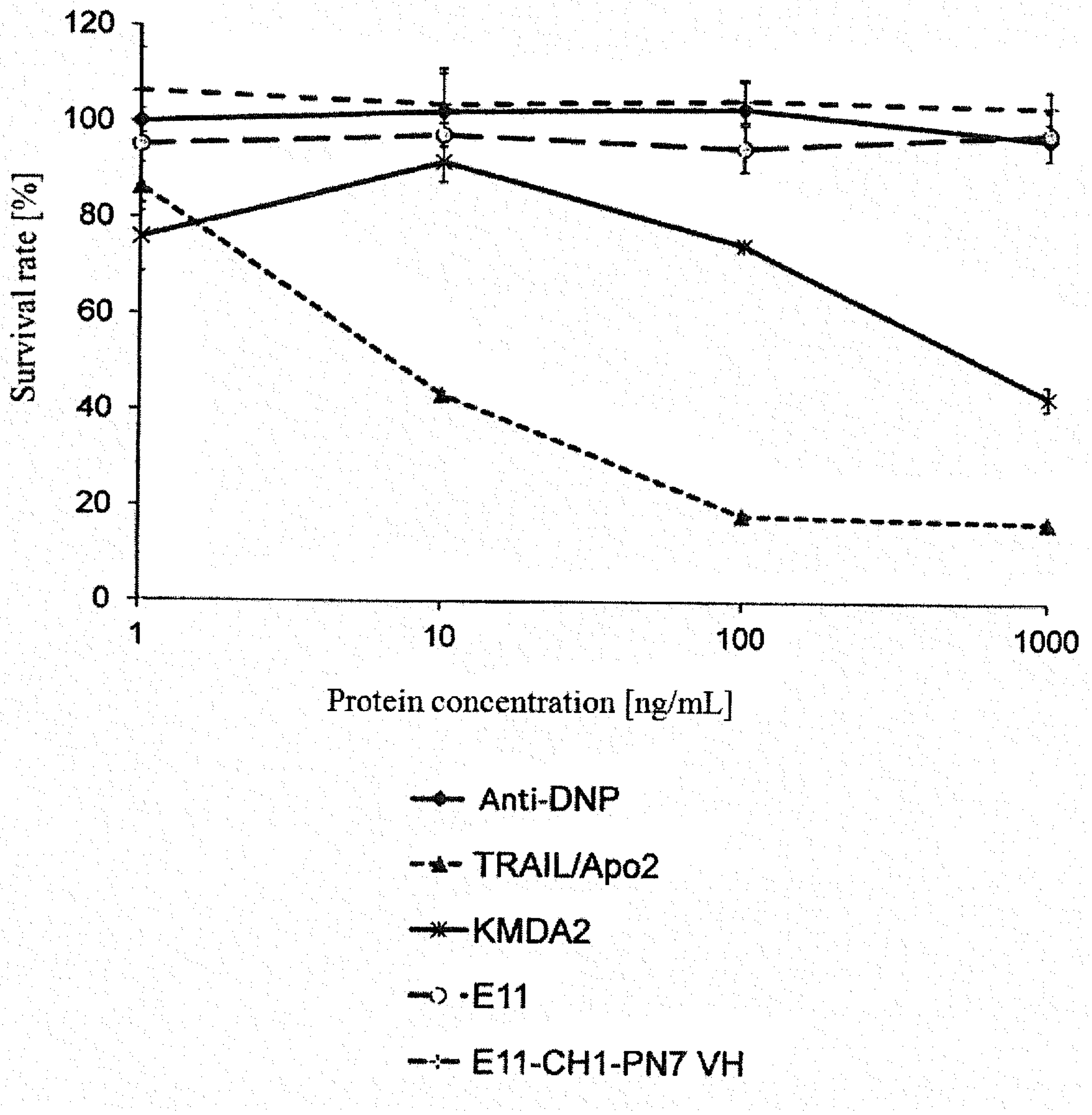
120
100
80
60
40
20
0
Survival rate [%]
1
10
100
1000
Protein concentration [ng/mL]
Anti-DNP
TRAIL/Apo2
KMDA2
E11
E11-CH1-PN7 VH

[Fig. 15]

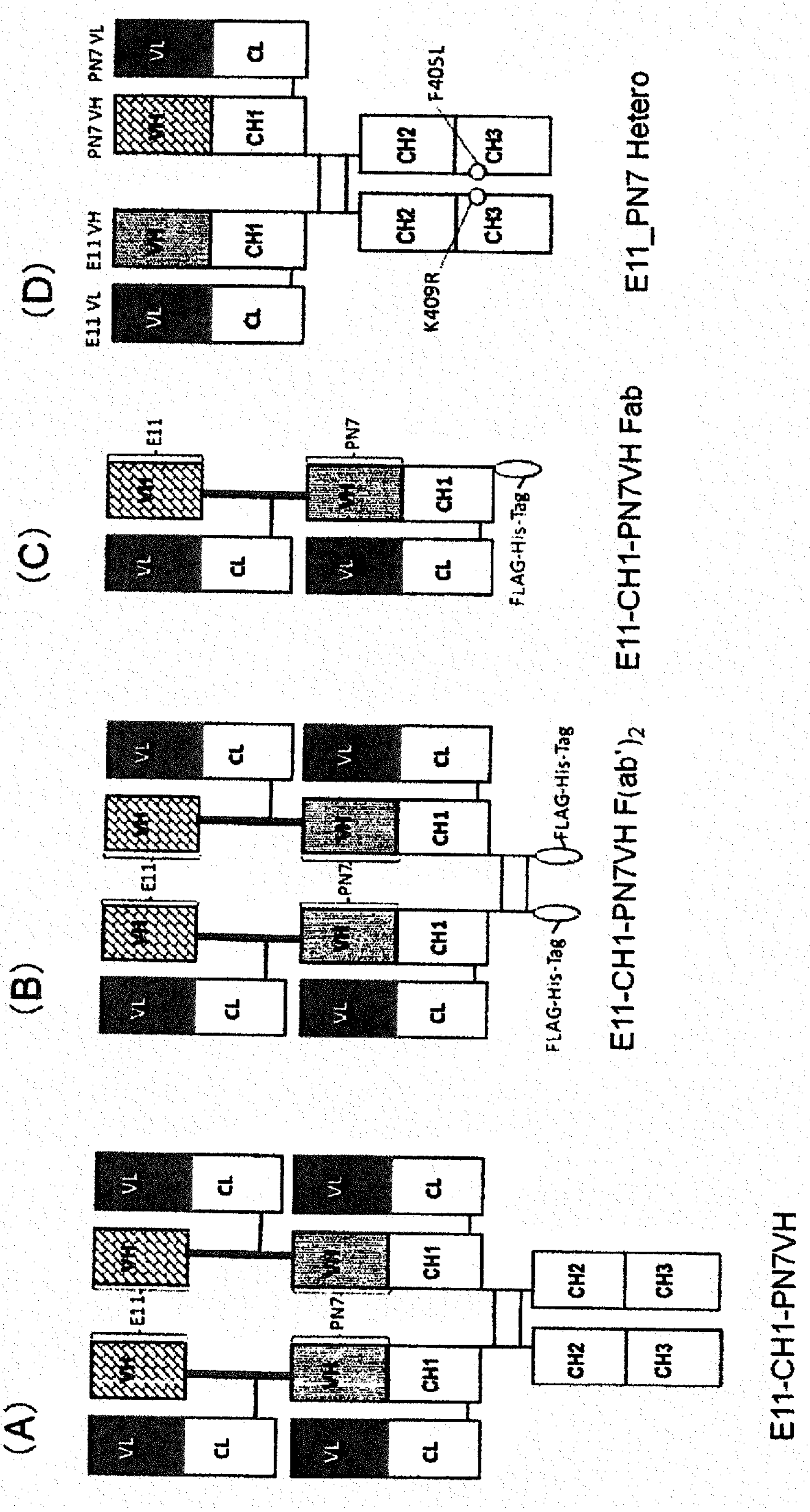
(A)
E11-CH1-PN7VH
(B)
E11-CH1-PN7VH F(ab')2
(C)
E11-CH1-PN7VH Fab
(D)
E11_PN7 Hetero
E11 VL
E11 VH
PN7 VH
PN7 VL
K409R
F405L
FLAG-His-Tag
E11
PN7

[Fig. 16]

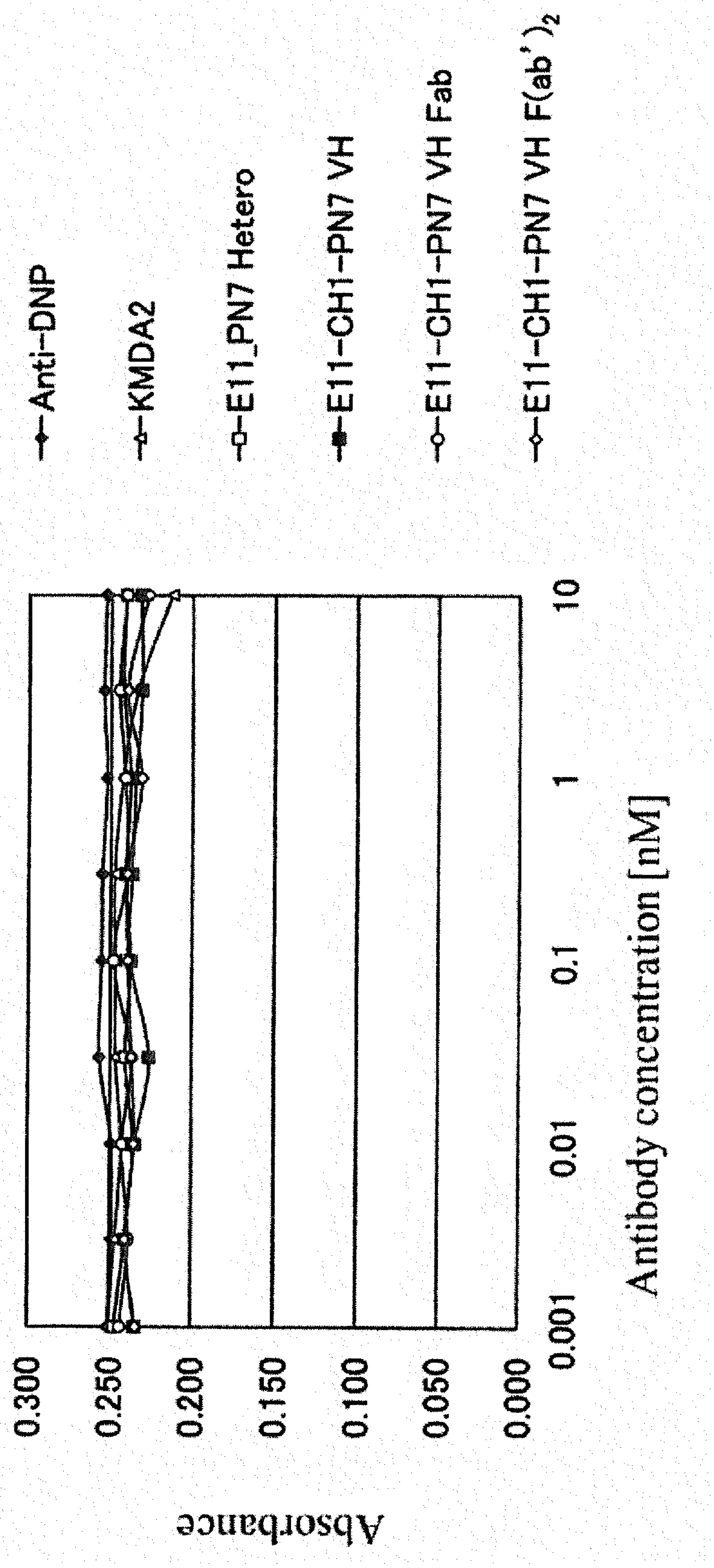
PC3
Absorbance
0.300
0.250
0.200
0.150
0.100
0.050
0.000
0.001
0.01
0.1
1
10
Antibody concentration [nM]
Anti-DNP
KMDA2
E11_PN7 Hetero
E11-CH1-PN7 VH
E11-CH1-PN7 VH Fab
E11-CH1-PN7 VH F(ab')2

[Fig. 17]

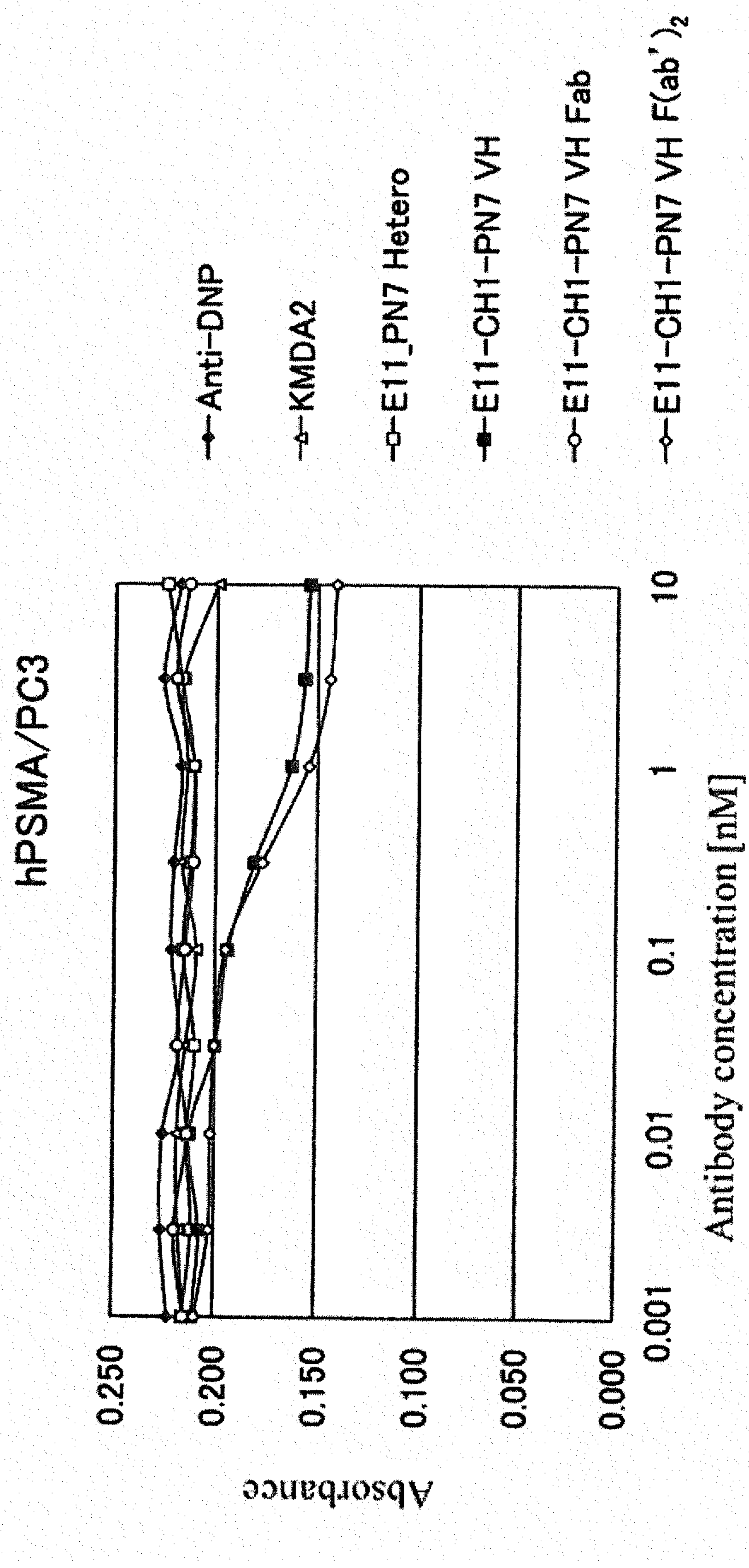
hPSMA/PC3
Absorbance
0.250
0.200
0.150
0.100
0.050
0.000
0.001
0.01
0.1
1
10
Antibody concentration [nM]
Anti-DNP
KMDA2
E11_PN7 Hetero
E11-CH1-PN7 VH
E11-CH1-PN7 VH Fab
E11-CH1-PN7 VH F(ab')2

**INTERNATIONAL SEARCH REPORT**

International application No.
PCT/JP2016/050434

A. CLASSIFICATION OF SUBJECT MATTER
*C07K16/28*(2006.01)i, *A61K39/395*(2006.01)i, *A61P35/00*(2006.01)i, *C12N5/10*(2006.01)i, *C12N15/09*(2006.01)i, *C12P21/08*(2006.01)i

According to International Patent Classification (IPC) or to both national classification and IPC

B. FIELDS SEARCHED

Minimum documentation searched (classification system followed by classification symbols)
C07K16/28, A61K39/395, A61P35/00, C12N5/10, C12N15/09, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
WPI, JSTPlus/JMEDPlus/JST7580(JDreamIII), GeneCards

C. DOCUMENTS CONSIDERED TO BE RELEVANT

| Category* | Citation of document, with indication, where appropriate, of the relevant passages | Relevant to claim No. |
|---|---|---|
| X | WO 2005/014618 A2 (IMMUNOMEDICS, INC.), 17 February 2005 (17.02.2005), claims 7 to 9 & JP 2007-516213 A & US 2005/0079184 A1 & EP 1651663 A2 | 1-30 |
| Y | WO 2012/145714 A2 (EMERGENT PRODUCT DEVELOPMENT SEATTLE, LLC), 26 October 2012 (26.10.2012), claim 39; paragraph [0216] & JP 2014-518615 A & US 2014/0161800 A1 & EP 2699596 A2 & CN 103687872 A & RU 2013142600 A | 1-30 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 March 2016 (17.03.16) | 29 March 2016 (29.03.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2016/050434

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2006/083971 A2 (GENENTECH, INC.), 10 August 2006 (10.08.2006), claims 1 to 76<br>& JP 2008-532487 A & US 2006/0269554 A1<br>& EP 1844077 A2 & KR 10-2007-0102585 A<br>& CN 101247825 A | 1-30 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070071675 A
- US 2001077342 A
- US 2009131239 A
- US 2002094880 A
- US 5773292 A
- WO 1998050431 A
- WO 20017734 A
- WO 2002002773 A
- WO 2009131239 A
- US 2005035586 A
- US 200231140 B
- US 0061739 A
- US 0042072 A
- US 6737056 B
- US 7297775 B
- US 7317091 B
- JP S61178926 A
- JP S5623587 A
- JP H2117920 A
- JP S58110600 A
- JP S60221091 A
- US 4686191 A
- US 4939094 A
- US 5160735 A
- JP H322979 A
- JP H2227075 A
- JP 9010354 A
- JP S63000299 A
- JP H05336963 A
- JP 6023021 A
- JP 2227075 A
- US 6001358 A
- US 2010143698 A
- US 5168062 A
- US 9710354 B
- JP H2257891 A
- JP 2005035586 A
- JP 2031140 A
- WO 2002094880 A
- WO 2002088186 A
- US 2002088186 A
- US 62101042 A


### Non-patent literature cited in the description


- **CHARLES A. J.** Immunobiology. Current Biology Ltd/Garland Publishing Inc, 1997
- **EMMANUELLE LAFFY.** *Human Antibodies,* 2005, vol. 14, 33-55
- **SURESH.** *Methods Enzymol.,* 1986, vol. 121, 210-228
- **KRANZ.** *J. Hematother Immunol.,* 1995, vol. 5, 403-408
- **WU.** *Nat. Biothech.,* 2007, vol. 25, 1290-1297
- **KERR et al.** *Br. J. Cancer,* 1972, vol. 26, 239
- **ITOH, S. et al.** *Cell,* 1991, vol. 66, 233-243
- **SCHMID et al.** *Proc.Natl.Acad.Sci.,* 1986, vol. 83, 1881
- **DEALTRY et al.** *Eur.J.Immunol.,* 1987, vol. 17, 689
- **KRAMMER et al.** *Curr.Op.Immunol.,* 1994, vol. 6, 279-289
- **NAGATA et al.** *Science,* 1995, vol. 267, 1449-1456
- **NAGATA et al.** *Cell,* 1997, vol. 88, 355-365
- **CHUNTHARAPAI A.** *J. Immunol.,* 2001, vol. 166 (8), 4891
- **ASHKENAZI A.** *Nat Rev Cancer,* 2002, vol. 2, 420
- **WILEY et al.** *Immunity,* 1995, vol. 3, 673-682
- **PITT, R. M. et al.** *J.Biol.Chem.,* 1996, vol. 271, 12687-12690
- **MARSTERS, S. A. et al.** *Curr. Biol.,* 1996, vol. 6, 750-752
- **PAN, GEL et al.** *Science,* 1997, vol. 276, 111-113
- **PAN, G et al.** *Science,* 1997, vol. 277, 815-818
- **WALCZAK, H. et al.** *EMBO J.,* 1997, vol. 16, 5386-5397
- **SCREATON, G R. et al.** *Curr Biol.,* 1997, vol. 7, 693-696
- **ARAI, T. et al.** *Cancer Letters,* 1998, vol. 133, 197-204
- **HYMOWITZ S. G. et al.** *Mol Cell.,* 1999, vol. 4, 563-71
- **GRIFFITH, T. S. et al.** *Curr.Opin.Immunol.,* 1998, vol. 10, 559-563
- **WALCZAK, H. et al.** *Nature Medicine,* 1999, vol. 5 (2), 157-163
- **ASHKENAZI, A. et al.** *J.Clin.Invest.,* 1999, vol. 104, 155-162
- **MORI, E. et al.** *Cell Death and Differentiation,* 2004, vol. 11, 203-207

- **JO, M. et al.** *Nature Medicine,* 2000, vol. 6, 564-567
- **ELSASSER-BEILE U. et al.** *Curr. Drug Targets,* 2009, vol. 10, 118-125
- **GREGORAKIS, A.K. et al.** *Semin. Urol. Oncol.,* 1998, vol. 16, 2-12
- **SILVER, D.A.** *Clin. Cancer Reseach,* 1997, vol. 3, 81-85
- **CHANG, S.S.** *Curr. Opin. Invest. Drugs,* 2004, vol. 5, 611-615
- Molecular Cloning, A Laboratory Manual. Cold Spring Harbor Laboratory Press, 1989
- Current Protocols in Molecular Biology. John Wiley & Sons, 1987
- *Nucleic Acids Research,* 1982, vol. 10, 6487
- *Proc. Natl. Acad. Sci. USA,* 1982, vol. 79, 6409
- *Gene,* 1985, vol. 34, 315
- *Nucleic Acids Research,* 1985, vol. 13, 4431
- *Proceeding of the National Academy of Sciences in USA,* 1985, vol. 82, 488
- DNA Cloning 1: Core Techniques, A Practical Approach. Oxford University, 1995
- *J. Mol. Biol.,* 1990, vol. 215, 403
- *Nucleic Acids Research,* 1997, vol. 25, 3389
- *Genome Research,* 1997, vol. 7, 649, http://www.ncbi.nlm.nih.gov/Education/BLASTinfo/information3.htm
- Monoclonal Antibodies - Principles and Practice. Academic Press, 1996
- Antibodies - A Laboratory Manual. Cold Spring Harbor Laboratory, 1988
- A manual for monoclonal antibody experiments. Kodansha scientific books, 1987
- **KABAT et al.** Sequences of proteins of immunological interest. 1991
- *Nature Protocols,* 2014, vol. 9, 2450-2463
- Molecular Cloning. Cold Spring Harbor Laboratory Press, 1989
- Current Protocols in Molecular Biology. John Willy & Sons, 1987
- *Proc. Natl. Acad. Sci., USA,* 1982, vol. 79, 6409
- *Proc. Natl. Acad. Sci USA,* 1985, vol. 82, 488
- **BECK et al.** *Analytical Chemistry,* 2013, vol. 85, 715-736
- *Cancer Immunol. Immunother.,* 1993, vol. 36, 373
- *Annu. Rev. Immunol.,* 1991, vol. 9, 457-492
- Introduction to Antibody Engineering. Chijinshokan Co., Ltd, 1994
- *Clinical oncology, Cancer and chemotherapy,* 1996
- Inflammation and anti-inflammatory therapy. Ishiyaku Pub, Inc, 1982
- Bioconjugate pharmaceutical product. Hirokawa-Shoten Ltd, 1993
- Current Protocols In Molecular Biology. John Wiley & Sons, 1987
- *Agricultural Biological Chemistry,* 1984, vol. 48, 669
- *Agric. Biol. Chem.,* 1989, vol. 53, 277
- *Proc. Natl. Acad. Sci. USA,* 1985, vol. 82, 4306
- *J. Bacteriol.,* 1990, vol. 172, 2392
- *Proc. Natl. Acad. Sci. USA,* 1972, vol. 69, 2110
- *Gene,* 1982, vol. 17, 107
- *Molecular & General Genetics,* 1979, vol. 168, 111
- *Cytotechnology,* 1990, vol. 3, 133
- *Nature,* 1987, vol. 329, 840
- *J. Biochemistry,* 1987, vol. 101, 1307
- *Proc. Natl. Acad. Sci. USA,* 1987, vol. 84, 7413
- *The Journal of the American Medical Association,* 1967, vol. 199, 519
- *Science,* 1952, vol. 122, 501
- *Virology,* 1959, vol. 8, 396
- *Proc. Soc. Exp. Biol. Med.,* 1950, vol. 73, 1
- **PAULSON et al.** *J. Biol. Chem.,* 1989, vol. 264, 17619
- **LOWE et al.** *Proc. Natl. Acad. Sci., USA,* 1989, vol. 86, 8227
- *Genes Develop.,* 1990, vol. 4, 1288
- **TOMIZUKA et al.** *Proc Natl Acad Sci USA.,* 2000, vol. 97, 722
- *Current Topics in Microbiology and Immunology,* 1978, vol. 18, 1
- *European J. Immunology,* 1976, vol. 6, 511
- *Nature,* 1978, vol. 276, 269
- *J. Immunology,* 1979, vol. 123, 1548
- *Nature,* 1975, vol. 256, 495
- **P.J. DELVES.** ANTIBODY PRODUCTION ESSENTIAL TECHNIQUES. WILEY, 1997
- **P. SHEPHERD ; C. DEAN.** Monoclonal Antibodies. OXFORD UNIVERSITY PRESS, 2000
- **J.W. GODING.** Monoclonal Antibodies: principles and practice. ACADEMIC PRESS, 1993
- *Methods in Enzymol.,* 1987, vol. 154, 3
- Current Protocols in Molecular Biology. John Wiley & Sons, 1987, vol. 1
- *Strategies,* 1992, vol. 5, 58
- *Nucleic Acids Research,* 1989, vol. 17, 9494
- *DNA Cloning: A Practical Approach,* 1985, vol. I, 49
- *Mol. Cell. Biol.,* 1983, vol. 3, 280
- *Gene,* 1985, vol. 33, 103
- *Strategies,* 1992, vol. 5, 81
- *Genetics,* 1954, vol. 39, 440
- *Science,* 1983, vol. 222, 778
- *J. Mol. Biol.,* 1983, vol. 166, 1

- *J. Mol. Biol.,* 1966, vol. 16, 118
- *Gene,* 1985, vol. 38, 275
- *Proc. Natl. Acad. Sci. USA,* 1977, vol. 74, 5463
- Sequences of Proteins of Immunological Interest. US Dept. Health and Human Services, 1991
- J. Mol. Biol. 1990, vol. 215, 403
- *Cytotechnol.,* 1990, vol. 3, 133
- *J. Biochem.,* 1987, vol. 101, 1307
- *Gene,* 1984, vol. 27, 223
- *Proc. Natl. Acad. Sci. USA,* 1981, vol. 78, 1527
- *Cytotechnol.,* 1990, vol. 4, 173
- *Cytotechnol.,* 1993, vol. 13, 79
- *Biochem. Biophys. Res. Commun.,* 1987, vol. 149, 960
- *Cell,* 1985, vol. 41, 479
- *Cell,* 1983, vol. 33, 717
- *J. Immunol. Methods,* 1994, vol. 167, 271
- *Hybridoma,* 1998, vol. 17, 559
- *BIO/TECHNOLOGY,* 1991, vol. 9, 266
- *J. Mol. Biol.,* 1977, vol. 112, 535
- *Protein Engineering,* 1994, vol. 7, 1501
- Methods in Nucleic Acids Res. CRC press, 1991
- Monoclonal Antibodies-Principles and practice. Academic Press, 1996
- Antibodies-A Laboratory Manual. Cold Spring Harbor Laboratory, 1988
- *Proc. Natl. Acad. Sci. USA,* 1980, vol. 77, 4216
- *Somatic Cell and Molecular Genetics,* 1986, vol. 12, 55
- *Nature,* 1970, vol. 227, 680
- enzyme immunoassay method. Igaku-Shoin Ltd, 1987
- Monoclonal Antibodies-Principles and Practice. Academic Press, 1996
- *fluorescent antibody method, Soft Science,* 1983
- Bioluminescence and Chemiluminescence Clinical Test. Hirokawa-Shoten Ltd, 1998, vol. 42
- Outline of Clinical Examination Method. KANEHARA & Co., LTD, 1998
- **ISHIDA ; LONBERG.** *IBC's 11th Antibody Engineering,* 2000
- **ISHIDA, 1. et al.** *Cloning & Stem Cells,* 2002, vol. 4, 85-96
- **ISHIDA ISAO.** *Experimental Medicine,* 2002, vol. 20 (6), 846-851